(19) Europäisches Patentamt
European Patent Office
Office européen des brevets

(11) Veröffentlichungsnummer: **0 296 109 A2**

(12) **EUROPÄISCHE PATENTANMELDUNG**

(21) Anmeldenummer: **88810376.9**

(22) Anmeldetag: **08.06.88**

(51) Int. Cl.[4]: **C 07 D 401/04**
C 07 D 409/14,
C 07 D 401/14, A 01 N 43/50,
C 07 D 213/80,
C 07 D 405/04,
C 07 D 487/14,
C 07 D 491/04,
C 07 D 213/82, C 07 D 471/04
//(C07D487/00,239:00,221:00,
209:00),(C07D491/00,307:00,
221:00),(C07D471/00,221:00,
209:00)

(30) Priorität: **18.06.87 CH 2301/87**

(43) Veröffentlichungstag der Anmeldung:
**21.12.88 Patentblatt 88/51**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Szczepanski, Henry, Dr.**
**Bodenmatt**
**CH-4323 Wallbach (CH)**

**Dürr, Dieter, Dr.**
**Brändelistalweg 16**
**CH-4103 Bottmingen (CH)**

(54) **2-(Imidazolin-2-yl)nicotinsäurederivate.**

(57) Die Erfindung betrifft Verfahren zur Herstellung von in der 6-Stellung substituierte 2-(Imidazolin-2-yl)-nicotinsäurederivate, welche sich durch hervorragende herbizide und den Pflanzenwuchs regulierende Eigenschaften auszeichnen, sowie die Zwischenprodukte und Verfahren zur Herstellung der Zwischenprodukte. Die Derivate entsprechen der Formel I

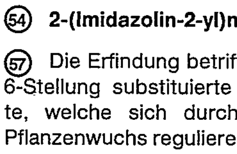

(I)

worin

$R_1$ Wasserstoff, ein Salz-, Ester- oder Amidrest,
$R_2$ und $R_3$ unabhängig je $C_1$-$C_4$-Alkyl oder zusammen auch einen $C_3$-$C_5$-Alkylenrest,
X Wasserstoff oder Methyl,
Y Wasserstoff, Halogen, Alkyl, Haloalkyl, Alkoxy, Alkylthio, Phenoxy, Nitro, Cyan, Alkylamino, Phenyl, Alkenyloxy oder Alkinyloxy und
Z einen Rest $-CQ_1Q_2Q_3$ oder $-CQ_1Q_4Q_5$ bedeutet worin
$Q_1$ und $Q_2$ je Wasserstoff oder $C_1$-$C_4$-Alkyl,
$Q_3$ $C_1$-$C_6$-Alkoxy oder Phenoxy welche unsubstituiert oder substituiert sind oder $C_3$-$C_6$-Alkenyloxy oder $C_3$-$C_6$-Alkinyloxy und
$Q_4$ und $Q_5$ zusammen einen Cycloalkyl-, Furyl- oder Pyran-, Dioxan-oder Dioxolan-Rest bedeuten oder Z bedeutet einen 5-6-gliedrigen heterocyclischen Rest.

2-(Imidazolin-2-yl)-nicotinsäurederivate sind bekannt, in der 6-Stellung substituierte Derivate sind z.T. bekannt. Es ist jedoch sehr schwierig, sie herzustellen und die Substitution erstreckte sich auf einen Niederalkyl- oder annellierten Phenylrest. Die Verbindungen mit den definierten Substituenten Z werden hergestellt, indem man zuerst gemäss einem in der EP-A 169 051 beschriebenen Verfahren den Substituenten Z in einen Pyridin-2,3-diocarbonsäurediester einführt und daraus gemäss dem z.B. aus der EP-A 41 623 bekannten Verfahren die 2-(Imidazolin-2-yl)-nicotinsäurederivate herstellt.

EP 0 296 109 A2

**Beschreibung**

**2-(Imidazolin-2-yl)nicotinsäurederivate**

Die vorliegende Erfindung betrifft Verfahren zur Herstellung von in der 6-Stellung substituierten 2-(Imidazolin-2-yl)nicotinsäurederivaten mit herbizider und den Pflanzenwuchs regulierender Wirkung, sowie die Zwischenprodukte und Verfahren zur Herstellung der Zwischenprodukte.

Bei den herzustellenden Derivaten handelt es sich um in der 6-Stellung substituierte 2-(Imidazolin-2-yl)nicotinsäurederivate der Formel I

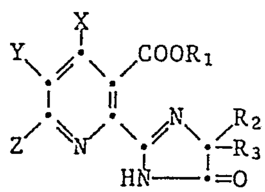

(I)

worin

$R_1$ Wasserstoff, das Kationäquivalent eines Alkalimetall-, Erdalkalimetall-, Magnesium-, Kupfer-, Eisen-, Zink-, Kobalt-, Blei-, Silber-, Nickel- oder quaternären Ammonium- oder Alkylammonium-Salzes, $C_1$-$C_6$-Alkyl, unsubstituiert oder substituiert durch Halogen, Hydroxyl, $C_1$-$C_3$-Alkoxy, $C_3$-$C_6$-Cycloalkyl, Benzyloxy, Furyl, Phenyl, Halophenyl, Niederalkylphenyl, Niederalkoxyphenyl, Nitrophenyl, Carboxyl, $C_1$-$C_4$-Alkoxycarbonyl oder Cyan; $C_3$-$C_6$-Cycloalkyl unsubstituiert oder 1-2fach substituiert durch $C_1$-$C_3$-Alkyl; $C_3$-$C_6$-Alkenyl, unsubstituiert oder substituiert durch Halogen, $C_1$-$C_3$-Alkoxy, Phenyl oder $C_1$-$C_4$-Alkoxycarbonyl; $C_3$-$C_6$-Alkinyl, unsubstituiert oder 1-2fach substituiert durch $C_1$-$C_3$-Alkyl;

$R_2$ $C_1$-$C_4$-Alkyl,

$R_3$ $C_1$-$C_4$-Alkyl oder $C_3$-$C_6$-Cycloalkyl oder

$R_2$ und $R_3$ zusammen mit dem Kohlenstoffatom, an das sie gebunden sind, auch einen $C_3$-$C_6$-Cycloalkylrest, der durch Methylreste substituiert sein kann,

X Wasserstoff oder Methyl,

Y Wasserstoff, Halogen, $C_1$-$C_6$-Alkyl, $C_1$-$C_6$-Halogenalkyl, $C_1$-$C_6$-Hydroxyalkyl, $C_1$-$C_6$-Alkoxy, $C_1$-$C_6$-Alkylthio, Phenoxy, Nitro, Cyan, $C_1$-$C_4$-Alkylamino, Di-$C_1$-$C_4$-alkylamino, $C_1$-$C_4$-Alkylsulfonyl, phenyl, Halophenyl, Niederalkylphenyl, Niederalkoxyphenyl, $C_3$-$C_8$-Alkenyloxy, $C_3$-$C_8$-Haloalkenyloxy, $C_3$-$C_8$-Alkinyloxy, $C_3$-$C_8$-Haloalkinyloxy und

Z einen Rest $-CQ_1Q_2Q_3$ oder $-CQ_1Q_4Q_5$ bedeuten worin

$Q_1$ Wasserstoff oder $C_1$-$C_4$-Alkyl,

$Q_2$ Wasserstoff oder $C_1$-$C_4$-Alkyl,

$Q_3$ $C_1$-$C_6$-Alkoxy welches unsubstituiert oder durch $C_1$-$C_4$-Alkyl, $C_1$-$C_4$-Alkoxy, $C_4$-$C_9$-Alkoxyalkoxy, Cyan oder Carbamoyl substituiert ist, Phenoxy welches unsubstituiert oder durch Halogen, $C_1$-$C_4$-Alkyl, $C_1$-$C_4$-Alkoxy, $C_1$-$C_4$-Haloalkyl oder Nitro substituiert ist; $C_3$-$C_6$-Alkenyloxy oder $C_3$-$C_6$-Alkinyloxy,

$Q_4$ und $Q_5$ zusammen mit dem Kohlenstoffatom an das sie gebunden sind $C_3$-$C_6$-Cycloalkyl oder einen durch ein oder zwei sich nicht in vicinaler Stellung befindliche Sauerstoffatome enthaltenden 5-6 gliedrigen Ring welche durch $C_1$-$C_4$-Alkyl substituiert sein können, bedeuten oder

Z bedeutet einen über Kohlenstoff gebundenen 5-6 gliedrigen gesättigten oder ungesättigten heterocyclischen Rest, der ungesättigt oder durch Niederalkyl substituiert ist.

In diesen Definitionen können die Alkyl- wie auch die Alkenyl- und Alkinylreste sowohl geradkettig wie verzweigt sein.

Die Alkylreste können die Bedeutung von Methyl, Aethyl, n-Propyl, Isopropyl, n-Butyl, sec.Butyl, tert.Butyl, Isobutyl, n-Pentyl, Iospentyl, Neopentyl, tert.Pentyl, n-Hexyl oder Isohexyl haben. Niederalkyl oder Niederalkoxy bedeutet, dass die Alkylreste 1-4 Kohlenstoffe enthalten.

Unter "Niederalkyl" wird ein Alkylrest mit 1-4 Kohlenstoffatomen bezeichnet.

Die Alkenyl- und Alkinylreste haben vorzugsweise zwischen 3 und 6 Kohlenstoffatome und können durch folgende Reste verkörpert sein: Allyl, Methallyl, Propinyl, 2-Butenyl. 1,3-Butadienyl, 1-Methylvinyl, Pentenyl, Butinyl, Pentinyl.

Unter Halogen wird Fluor, Chlor, Brom und Jod verstanden. Die 5-6 gliedrigen heterocyclischen Reste umfassen z.B. Furan, Tetrahydrofuran, Thiophen, Pyrrol, Pyrrolidin, Oxazol, Oxazolidin, Triazol, Imidazol, Oxadiazol, Thiadiazol, Triazol, Pyran, Pyridin, Piperidin, Dioxan, Pyrimidin, Morpholin, Thiomorpholin oder Triazin.

Verbindungen dieser Art, d.h. 2-Imidazolyl -nicotinsäuren sowie 2-Imidazolyl-3-chinoncarbonsäuren und ihre Derivate sind bekannt, siehe z.B. die EP-A 41 623, die US-P 4 518 780, die EP-A 61 423 oder die US-P 4 647 301.

Es ist jedoch bisher nicht oder nur mit Schwierigkeit gelungen, Substituenten in die 6-Stellung der

2-Imidazolylnicotinsäure zu bringen resp. solche Säuren und Ester herzustellen.

Gute Wirkung zeigten diejenigen Verbindungen der Formel I,

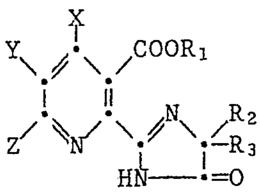

(I)

in denen $R_1$ Wasserstoff oder $C_1$-$C_6$-Alkyl, $R_2$, $R_3$ und X die unter der Formel I gegebene Bedeutung haben, Y Wasserstoff, $C_1$-$C_6$-Alkyl oder Halogen, Z einen heterocyclischen Rest oder in der Bedeutung $-CQ_1Q_2Q_3$ einen $C_1$-$C_6$-Alkoxy-, $C_1$-$C_4$-alkyl-, Phenoxy-$C_1$-$C_4$-alkyl oder Carbamoyl-$C_1$-$C_4$-alkoxy-alkylrest oder in der Bedeutung von $-CQ_1$-$Q_4Q_5$ einen $C_3$-$C_6$-Cycloalkylrest, der durch $C_1$-$C_4$-Alkyl substituiert sein kann, bedeuten.

Ganz besonders aktiv waren diejenigen Verbindungen der Formel I

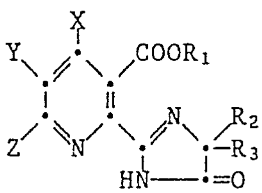

(I)

in denen $R_1$ Wasserstoff oder $C_1$-$C_6$-Alkyl, $R_2$ Methyl, $R_3$ Isopropyl,X Wasserstoff oder Methyl, Y $C_1$-$C_6$-Alkyl, Chlor oder Brom und Z einen $C_1$-$C_6$-Alkoxyalkyl-, Phenoxy-$C_1$-$C_4$-alkyl-carbamoyl-$C_1$-$C_4$-alkoxy-$C_1$-$C_4$-alkyl, einen unsubstituierten oder durch $C_1$-$C_4$-Alkyl substituierten $C_3$-$C_6$-Cycloalkyl-, einen Thiophenyl-, Pyridinyl-, Methylpyrimidin-Furanyl- oder Dioxanyl-Rest bedeuten.

Selbst wenn solche Verbindungen beschrieben wurden, ist in der 6-Stellung kaum je ein anderer als ein Niederalkyl- oder ein benzannellierter Phenylrest postuliert worden.

Es ist daher ein Ziel der vorliegenden Erfindung, ausgehend von gut zugänglichen und leicht handhabbaren Ausgangsmaterialien ein Verfahren zur Herstellung der 2-(2-Imidazolin-2-yl)-nicotinsäuren der Formel I bereitzustellen, das die Herstellung dieser Verbindungen auf einfache Weise und in guter Ausbeute ermöglicht.

Gemäss einem ersten Verfahren wird vorgeschlagen, die in der 6-Stellung substituierten 2-Imidazolin-2-yl-nicotinsäurederivate der Formel I herzustellen indem man einen in der 6-Stellung unsubstituierten Pyridin-2,3-dicarbonsäure-diester der Formel V

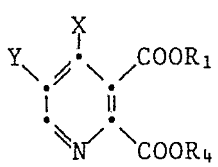

(V)

worin $R_1$, X und Y die unter der Formel I gegebene Bedeutung haben, und $R_4$ einen $C_1$-$C_8$-Alkylphenyl- oder Phenyl-$C_1$-$C_4$-alkylrest bedeutet, in wässriger Lösung, in Gegenwart einer katalytischen Menge von Silber II-ionen und einem Peroxysulfatsalz, mit einer Carbonsäure der Formel XVII

Z−COOH (XVII)

worin Z die unter der Formel I gegebene Bedeutung hat, umsetzt zum in der 6-Stellung substituierten Pyridin-2,3-dicarbonsäure-diester der Formel (IV)

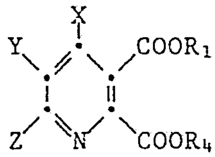

(IV)

worin $R_1$, $R_4$, X, Y und Z die unter der Formel I gegebene Bedeutung haben und diesen Ester in einem inerten organischen Lösungsmittel, in Gegenwart einer starken Base mit den 2-Aminoalkancarbonsäureamid der Formel XV

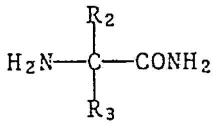

(XV)

in welchem $R_2$ und $R_3$ die unter der Formel I gegebene Bedeutung haben, umsetzt, das gebildete Salz der 2-(Imidazol-2-yl)nicotinsäure der Formel III in wässriger saurer Lösung aufnimmt, die freie 2-(Imidazolin-2-yl)nicotinsäure der Formel III

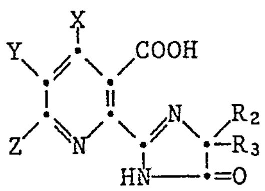

(III)

worin $R_2$, $R_3$, X, Y und Z die unter der Formel I gegebene Bedeutung haben, isoliert und dann mit einem wasserentziehenden Mittel oder Reagens zu einer tricyclischen 2H-Imidazo[1',2':1,2]pyrrolo[3,4-b]pyridin-3,5-dion Verbindung der Formel II umlagert,

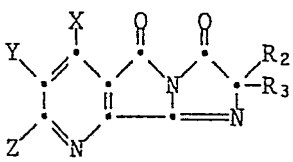

(II)

worin $R_2$, $R_3$, X, Y und Z die unter der Formel I gegebene Bedeutung haben, und diese Verbindung dann bei erhöhter Temperatur mit einem Hydroxid oder Alkohol der Formel XVI

$HOR_1$ (XVI)

worin $R_1$ die unter der Formel I gegebene Bedeutung hat, zum 2-(Imidazolin-2-yl)nicotinsäurederivat der Formel I aufspaltet.

Die 2-(Imidazolin-2-yl)nicotinsäure der Formel III und ihre Salze fallen unter die allgemeine Formel I. Sie haben ebenfalls aussergewöhnliche herbizide und den Pflanzenwuchs regulierende Wirkung. Sie und ihre Herstellung, ausgehend von den Pyridin-2,3-dicarbonsäurediestern der Formel V gemäss dem oben beschriebenen Verfahren, sind ebenfalls Gegenstand dieser Erfindung.

Die in der 6-Stellung substituierten Pyridin-2,3-dicarbonsäurediester der Formel IV sind neue Zwischenprodukte. Sie und ihre Herstellung aus den Pyridin-2,3-dicarbonsäurediestern der Formel V durch Anlagerung einer Carbonsäure der Formel XVII an einen in der 6-Stellung unsubstituierten Pyridin-2,3-dicarbonsäurediester der Formel V in Gegenwart von Silberionen und einem Peroxysulfat-Salz ist ebenfalls Gegenstand dieser Erfindung.

Die als Ausgangsmaterialien benötigten Pyridin-2,3-dicarbonsäureester der Formel V können in einfacher Weise durch Veresterung von Pyridin-2,3-dicarbonsäure mit entsprechenden Alkoholen hergestellt werden. Die Pyridin-2,3-dicarbonsäure kann ihrerseits durch Oxidation von Chinolin (vgl. DE-PS 1 010 524 und US-PS 2 512 482) hergestellt werden. Ferner können die Pyridin-2,3-dicarbonsäureester der Formel II nach der in den

publizierten Europäischen Patent anmeldungen 0 161 221 und 0 172 140 beschriebenen Methoden ausgehend von einem Hydrazon einer α,β-ungesättigten Carbonylverbindung und einem Maleinsäurederivat nach dem Schema einer Diels-Alder-Reaktion hergestellt werden. In der 6-Stellung nicht substituierte Chinolin-2,3-dicarbonsäureester können z.B. nach der in J. Org. Chem. 49, 4999-5000, 1984 beschriebenen Methode erhalten werden. Die 2-Aminoalkancarbon-säureamide der Formel II sind grösstenteils bekannte Verbindungen, die in bekannter Weise durch Umsetzung entsprechender Ketone mit Ammoniak und Blausäure hergestellt werden können.

Die Einführung des Substituenten Z in die 6-Position des Pyridinringes erfolgt mittels einer Carbonsäure Z-COOH, worin Z dem gewünschten Substitutionsrest entspricht. Die Anlagerung wird gemäss einem in der EP-A 169 051 für Pyridazinverbindungen beschriebenen Verfahren in Gegenwart eines Peroxysulfat-Ion-Aequivalentes und einer katalytischen Menge von Silberionen in einer wässrigen Mineralsäure-Lösung bei Temperaturen von 40-80°C vollzogen. Es ist darauf zu achten, dass die Mineralsäure die Silberionen nicht ausfällt. Es kommen z.B. Schwefelsäure, Perchlorsäure oder Trifluoressigsäure in Betracht. Die Silberionen können von einem wasserlöslichen Silbersalz, wie Silbernitrat, Silberfluorid, Silbertrifluoracetat oder Silberperchlorat, vorzugsweise von Silbernitrat, herkommen.

Bei der Reaktion wird die Carbonsäure Z-COOH oxidativ decarboxyliert, wobei sich die vom Silbersalz stammenden Silber I-ionen in Gegenwart des Peroxydisulfat-Salzes zu Silber II-ionen oxidieren. Die Reaktion verläuft selektiv, so dass nur stoechiometrische Mengen an Carbonsäure und Peroxysulfatsalz eingesetzt werden müssen. Die Reaktionstemperatur liegt zwischen 40° und 80°C, vorzugsweise zwischen 70° und 80°C. Als Peroxydisulfatsalze kommen vorzugsweise das Ammonium, Natrium, Kaliumsalz in Betracht.

Wenn bei dieser Umsetzung X durch Wasserstoff besetzt ist, so entstehen bei der radikalen Alkylierung auch 4- und 4,6-substituierte Pyridin-2,3-dicarbonsäureester entsprechend der Gleichung

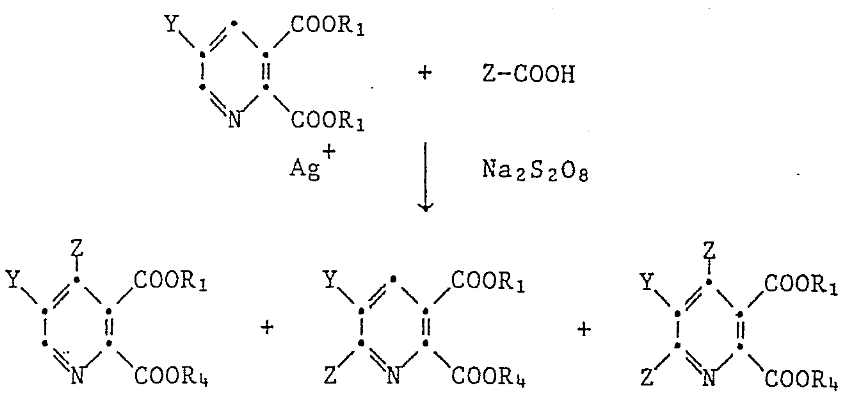


XIII + IV + XIV

Solche Estergemische können durch Chromatographie oder fraktionierte Destillation in ihre Komponenten aufgetrennt werden.

Als Carbonsäuren der Formel XVIII geeignet sind z.B. Pivaloylsäure (Trimethylessigsäure), Buttersäure, Isobuttersäure, Propionsäure, Essigsäure, Cyclobutancarbonsäure, Cyclopropancarbonsäure, Cyclopentencarbonsäure, Phenoxyessigsäure, Glykolsäure.

Die Umsetzung des in 6-Stellung substituierten Pyridin-2,3-dicarbonsäureesters der Formel IV mit einem 2-Aminoalkancarbonsäureamid der Formel $H_2N-C(R_2)R_3-CONH_2$ findet in einem inerten organischen Lösungsmittel in Gegenwart einer sterilen Base bei einer Temperatur zwischen Raumtemperatur und der Rückflusstemperatur des Reaktionsgemisches statt.

Als inerte Lösungsmittel kommen beispielsweise flüssige aromatische Kohlenwasserstoffe und Halogenkohlenwasserstoffe, wie Benzol, Toluol, Chlorbenzol und Xylole, $C_1$-$C_{10}$-Alkanole, insbesondere $C_1$-$C_4$-Alkanole, wie Methanol, Ethanol, Propanol, Isopropanol, n-Butanol, sec.Butanol, tert.Butanol und Isobutanol, etherartige Flüssigkeiten, die Tetrahydrofuran und Dioxan, sowie stark polare Lösungsmittel, wie Acetonitril, N,N-Dimethylformamid und Dimethylsulfoxid in Betracht. Vorteilhaft werden mit Wasser nicht mischbare Lösungsmittel verwendet, wie Benzol, Toluol, Chlorbenzol oder Xylole.

Als starke Basen, in deren Gegenwart die Umsetzung des Pyridin-2,3-dicarbonsäureesters der Formel IV mit einem 2-Aminoalkancarbonsäureamid der Formel XV durchgeführt wird, sind Alkalimetallhydroxide und Alkalimetallalkoholate geeignet. Bevorzugte Basen sind Alkalimetallalkoholate, insbesondere solche, die sich von $C_1$-$C_4$-Alkanolen ableiten, wie Natriummethylat, Natriumethylat, Natriumisopropylat und Kalium-tert.butylat. Alkalimetallhydroxide wie Natriumhydroxid und Kaliumhydroxid sind insbesondere bei Verwendung von Alkanolen als inerte Lösungsmittel geeignet. Die Umsetzung kann ferner in Gegenwart von Pottasche in Ethanol durchgeführt werden. Die starke Base wird in der Regel in mindestens equimolarer Menge bezogen auf eingesetzten Pyridin- 2,3-dicarbonsäureester der Formel IV eingesetzt. Bei der Durchführung des erfindungsgemässen Verfahrens hat sich die Verwendung von 1-3 Mol Base pro Mol Pyridin-2,3-dicarbonsäureester der Formel IV als geeignet erwiesen. Vorzugsweise werden 1,5-2,5 Mol Base pro Mol Pyridin-2,3-dicarbonsäureester der Formel IV verwendet.

Innerhalb des angegebenen Temperaturbereichs von Raumtemperatur bis Rückflusstemperatur des Reaktionsgemisches sind für die Durchführung des erfindungsgemässen Verfahrens Temperaturen von 50-90° C bevorzugt.

Die Kondensation der 2-(Imidazolin-2-yl)nicotinsäure der Formel III zum tricyclischen 2H-Imidazo[1′,2′:1,2]pyrrolo[3,4-b]pyridin-3,5-dion der Formel II erfolgt mittels eines wasserentziehenden Mittels oder Reagens.

Als solche kommen beispielsweise das Kochen am Wasserabscheider mit einem mit Wasser nicht mischbaren organischen Lösungsmittel, wie Benzol oder Toluol, in Frage, ferner das Kochen in einem Säureanhydrid oder mit konzentrierter Schwefelsäure resp. einem organischen Lösungsmittel, das ein Anhydrid, z.B. Essigsäureanhydrid oder konzentrierte Schwefelsäure, enthält. Als wasserentziehende Mittel kommen ferner Säurehalogenide, wie Phosgen, Thionylchlorid, aber auch andere Reagentien, wie Dicyclohexyldiimid oder auch Phosphorpentoxid, in Frage. Sie werden vorzugsweise in organischen Lösungsmitteln gelöst angewandt und die Reaktion findet unter Rühren bei Temperaturen zwischen Raumtemperatur und dem Siedepunkt des Reaktionsgemisches statt.

Die Nomenklatur der Verbindung II 2H-Imidazo[1′,2′:1,2]pyrrolo[3,4-b]pyridin-3,5-dion ist aus Chemical Abstract übernommen worden. Der Grundkörper dieses Dreiringes wird wie folgt bezeichnet und numeriert:

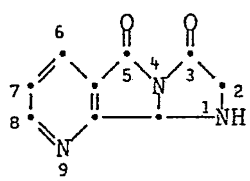

Gemäss einem zweiten Verfahren werden die in der 6-Stellung substituierten 2-(Imidazolin-2-yl)nicotinsäurederivate der Formel I hergestellt, indem man einen in der 6-Stellung substituierten Pyridin-2,3-dicarbonsäurediester der Formel IV

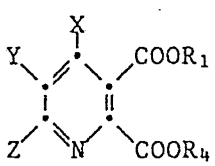

(IV)

worin $R_1$, $R_4$, X, Y und Z die unter der Formel I gegebene Bedeutung haben, mit einer starken Base in wässrigem Milieu verseift und die entstandene in 6-Stellung substituierte Pyridin-2,3-dicarbonsäure der Formel VI

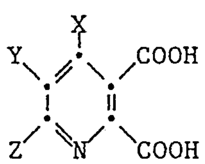

(VI)

worin X, Y und Z die unter der Formel I gegebene Bedeutung haben, in einem wasserentziehenden Milieu zum in der 6-Stellung substituierten Pyridin-2,3-dicarbonsäureanhydrid der Formel VII

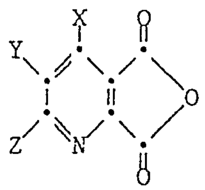

(VII)

worin X, Y und Z die unter der Formel I gegebene Bedeutung haben, und dieses Anhydrid dann mit einem Glycylamid der Formel XVIII

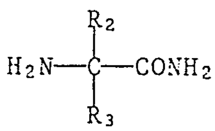

(XVIII)

worin $R_2$ und $R_3$ die unter der Formel I gegebene Bedeutung haben, kondensiert, die daraus entstandene, in der 6-Stellung substituierte 2-(N-Carbamoylmethylcarbamoyl)-nicotinsäuren der Formel VIII

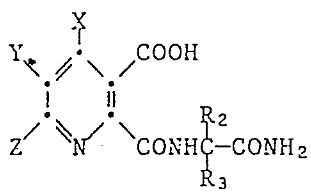

(VIII)

worin $R_2$, $R_3$, X, Y und Z die unter der Formel I gegebene Bedeutung haben, in einem inerten trockenen Lösungsmittel zum Ringschluss bringt, wobei die in der 6-Stellung substituierte 2-(Imidazolin-2-yl)nicotinsäure der Formel III entsteht

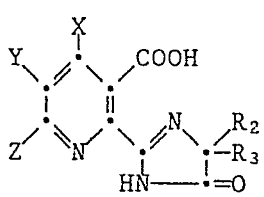

(III)

worin $R_2$, $R_3$, X, Y und Z die unter der Formel I gegebene Bedeutung haben isoliert und dann mit einem wasserentziehenden Mittel oder Reagens zu einer tricyclischen 2H-Imidazo[1,2:1′,2′]pyrrolo[3,4-b]pyridin-3,5-dion Verbindung der Formel II

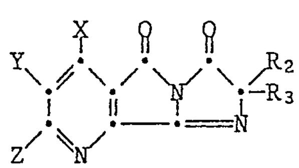

(II)

worin $R_2$, $R_3$, X, Y und Z die unter der Formel I gegebene Bedeutung haben, und diese Verbindung dann bei erhöhter Temperatur mit einem Hydroxid oder Alkohol der Formel XVI

$HOR_1$ (XVI)

worin $R_1$ die unter der Formel I gegebene Bedeutung hat, zum 2-(Imidazolin-2-yl)nicotinsäurederivat der Formel I aufspaltet.

Die Verseifung des Pyridin-2,3-dicarbonsäurediesters der Formel IV zur entsprechenden Säure ist eine

konventionelle Verseifung, die mit einer sterilen Base in wässrigem Milieu bei Siedetemperatur des Reaktionsmilieus vorgenommen wird.

Als Basen eignen sich die Alkali- oder Erdalkalimetallhydroxyde oder -carbonate als Lösungsmittel, Wasser allein oder in Mischung mit einem Alkohol, Keton oder mit Dimethylformamid.

Die Umsetzung der Pyridin-2,3-dicarbonsäure der Formel VI zum entsprechenden Anhydrid erfolgt durch Kochen in einem wasserentziehenden Milieu. Als solches kommt z.B. ein Säureanhydrid, wie z.B. Acetanhydrid, auch konzentrierte Schwefelsäure, in Betracht.

Die Anlagerung des Glycylamides der Formel XVIII and das Säureanhydrid der Formel VII erfolgt in einem inerten organischen Lösungsmittel bei einer Temperatur zwischen Raumtemperatur und dem Siedepunkt des Lösungsmittels. Die Kondensation der 2-(N-Carbamoylmethylcarbamoyl)nicotinsäure der Formel VIII zur 2-(Imidazolin-2-yl)nicotinsäure der Formel III erfolgt durch Kochen in einem inerten organischen Lösungsmittel in Gegenwart einer starken Base unter anhydriden Bedingungen. Dabei werden vorzugsweise Alkalimetallalkoholate, z.B. das Kalium-tert.Butyloxyd, in einem Alkanol als Lösungsmittel verwendet.

Die ganze Umsetzung, angefangen vom Pyridindicarbonsäurediester der Formel V bis zur 2-(Imidazolin-2-yl)nicotinsäure der Formel III, wird vorzugsweise in einem Eintopf-Verfahren durchgeführt, indem man den Ester, das Glycylamid und das Alkoholat in einem inerten organischen Lösungsmittel, wie z.B. Benzol, Toluol, Xylol, Cyclohexan, kocht, das Lösungsmittel dann verdampft und den Rückstand ansäuert. Man erhält die Nicotinsäuren der Formel III in reiner Form und frei von Isomeren.

Die Verfahrensstufen, welche von der 2-(Imidazolin-2-yl)nicotinsäure der Formel III über das 2H-Imidazo[1′,2′:1,2]pyrrolo[3,4-b]pyridin-3,5-dion der Formel II zum 2-(Imidazolin-2-yl)nicotinsäurederivat der Formel I führen, sind mit den im ersten Verfahren beschriebenen Stufen identisch.

Gemäss einem dritten Verfahren erhält man die in der 6-Stellung substituierten 2-(Imidazolin-2-yl)nicotinsäurederivate der Formel I dadurch, dass man ein in der 6-Stellung substituiertes Pyridin-2,3-dicarbonsäureanhydrid der Formel VII

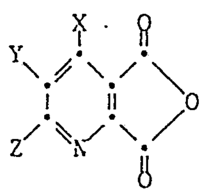

(VII)

worin X, Y und Z die unter der Formel I gegebene Bedeutung haben, in einem inerten organischen Lösungsmittel mit einem Glycylnitril der Formel XIX

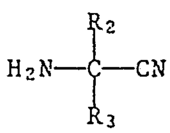

(XIX)

worin $R_2$ und $R_3$ die unter der Formel I gegebene Bedeutung haben, umsetzt, die dabei erhaltene in der 6-Stellung substituierte 2-(Cyanomethylcarbamoyl)-nicotinsäure der Formel IX

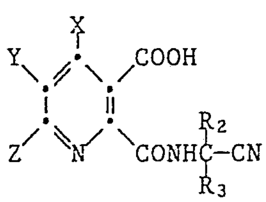

(IX)

worin $R_2$, $R_3$, X, Y und Z die unter der Formel I gegebene Bedeutung haben, dann in wasserentziehendem Milieu oder einem wasserentziehenden Mittel behandelt, so dass ein N-(Cyanomethylcarbamoyl)-pyridin-2,3-dicarbonsäureimid der Formel X entsteht,

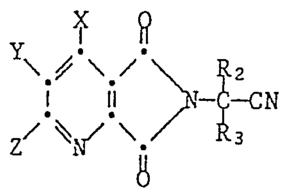

(X)

worin $R_2$, $R_3$, X, Y und Z die unter der Formel I gegebene Bedeutung haben, diese Imid in wässrig-saurem Medium, bei erhöhter Temperatur zum N-(Carbamoylmethyl)-pyridin-2,3-dicarbonsäureimid der Formel XI hydrolisiert,

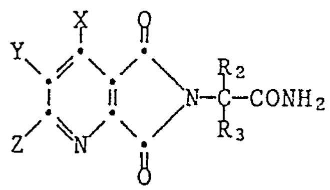

(XI)

worin $R_2$, $R_3$, X, Y und Z die unter der Formel I gegebene Bedeutung haben, welche Verbindung dann durch Erwärmen in basischem Milieu zur tricyclischen 3H-Imidazo[1,2:1′,2′]pyrrolo[3,4-b]pyridin-2,5-dion Verbindung der Formel XII umgewandelt wird,

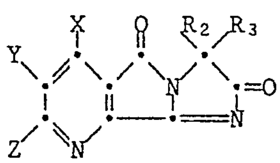

(XII)

worin $R_2$, $R_3$, X, Y und Z die unter der Formel I gegebene Bedeutung haben, und dieses Produkt dann bei erhöhter Temperatur mit einem Hydroxid oder Alkohol der Formel XVI

$HOR_1$ (XVI)

worin $R_1$ die unter der Formel I gegebene Bedeutung hat, zum 2-(Imidazolin-2-yl)nicotinsäurederivat der Formel I aufspaltet.

Die Kondensation des Glycylnitrils der Formel XIX mit dem Pyridin-2,3-dicarbonsäureanhydrid der Formel VII erfolgt durch Rühren und Erwärmen in einem inerten organischen Lösungsmittel.

Die nachfolgende Kondensation zum Imid der Formel X kann durch Kochen am Wasserabscheider oder auch nur durch Eindampfen des Reaktionsmilieus bewerkstelligt werden.

Wenn man das N-(Glycylnitril)-2,3-pyridindicarbonsäureimid der Formel X zuerst mit konzentrierter Schwefelsäure behandelt, erhält man unter Hydrolyse der Nitrilgruppe das N-(Carbamoylmethylcarbamoyl)-2,3-pyridincarbonsäureimid der Formel XI welches unter basischen Bedingungen zum 3H-Imidazo[1′,2′:1.2]pyrrolo[3,4-b]-pyridin-2,5-dion der Formel XII kondensiert. Dieses kann man dann in Gegenwart eines Hydroxyds oder Alkohols zum 2-Imidazolin-2-yl)nicotinsäurederivat der Formel I umwandeln. Diese Reaktionsschritte sind z.B. auch in der Europäischen Offenlegungsschrift EP-A 41 623 beschrieben.

Die Nomenklatur der Verbindung XII 3H-Imidazo[1′,2′:1,2]pyrrolo[3,4-b]pyridin-2,5-dion ist aus Chemical Abstract übernommen worden. Der Grundkörper dieses Dreiringes wird wie folgt bezeichnet und numeriert:

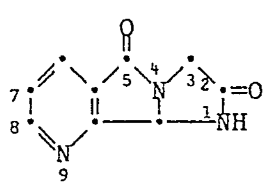

Die in der 8-Stellung substituierten 2H-Imidazol[1′,2′:1,2]pyrrolo[3,4-b]pyridin-3,5-dion-Verbindungen der Formel II sowie die in der 8 Stellung substituierten 3H-Imidazo[1′,2′:1,2]pyrrolo[3,4-b] pyridin-2,5-dion-Verbindungen der Formel XII sind neu. Sie zeichnen sich durch ausgezeichnete herbizide und den Pflanzenwuchs regulierende Wirkung aus. Diese Verbindungen und ihre Herstellung sind ebenfalls Gegenstand dieser Erfindung.

Gute Wirkung zeigten diejenigen Verbindungen der Formel

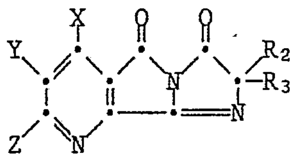 (II) oder 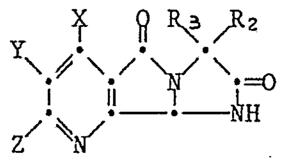 (XII)

in denen $R_1$ Wasserstoff oder $C_1$-$C_6$-Alkyl, $R_2$, $R_3$ und X die unter der Formel I gegebene Bedeutung haben, Y Wasserstoff, $C_1$-$C_6$-Alkyl oder Halogen, Z einen heterocyclischen Rest oder in der Bedeutung $-CQ_1Q_2Q_3$ einen $C_1$-$C_6$-Alkoxy-, $C_1$-$C_4$-alkyl-, Phenoxy-$C_1$-$C_4$-alkyl oder Carbamoyl-$C_1$-$C_4$-alkoxy-alkylrest oder in der Bedeutung von $-CQ_1Q_4Q_5$ einen $C_3$-$C_6$-Cycloalkylrest, der durch $C_1$-$C_4$-Alkyl substituiert sein kann bedeuten.

Ganz besonders aktiv waren diejenigen Verbindungen der Formeln

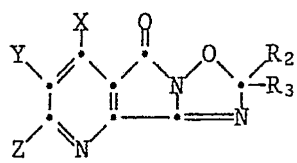 (II) oder 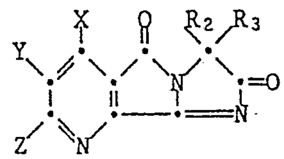 (XII)

in denen $R_1$ Wasserstoff oder $C_1$-$C_6$-Alkyl, $R_2$ Methyl, $R_3$ Isopropyl, Wasserstoff oder Methyl, Y $C_1$-$C_6$-Alkyl, Chlor oder Brom und Z einen $C_1$-$C_6$-Alkoxyalkyl-, Phenoxy-$C_1$-$C_4$-alkyl-carbamoyl-$C_1$-$C_4$-alkoxy-$C_1$-$C_4$-alkyl, einen unsubstituierten oder durch $C_1$-$C_4$-Alkyl substituierten $C_3$-$C_6$-Cycloalkyl-, einen Thiophenyl-, Pyridinyl-, Methylpyrimidin-Furanyl- oder Dioxanyl-Rest bedeuten.

Die in der 6-Stellung substituierten 2-(Cyanomethylcarbamoyl)nicotinsäuren der Formel IX, die N-(Cyanomethylcarbamoyl)pyridin-2,3-dicarbonsäureamide der Formel X und die N-(Carbamoylmethylcarbamoyl)pyridin-dicarbonsäureamide der Formel XI sind neue Zwischenprodukte. Sie und ihre Herstellung sind ebenfalls Gegenstand dieser Erfindung.

Gemäss einem weiteren Verfahen werden die in der 6-Stellung substituierten 2-(Imidazolin-2-yl)nicotinsäurederivate der Formel I hergestellt, indem man ein Methylketon der Formel XXIV

$Z-COCH_3$ (XXIV)

worin Z die unter der Formel I gegebene Bedeutung hat mit einem sekundären Amin der Formel XX

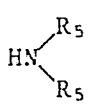 (XX)

umsetzt, worin $R_5$ $C_1$-$C_4$-Alkyl, Phenyl-$C_1$-$C_4$-alkyl oder beide $R_5$ zusammen mit dem Stickstoffatom an das sie gebunden sind, einen 5-6 gliedrigen, gesättigten oder ungesättigten Ring bilden, der durch Sauerstoff unterbrochen sein kann, das entstandene Methylenamin der Formel XXI

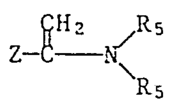 (XXI)

worin $R_5$ die oben gegebene Bedeutung hat, dann mit einem Alkoxymethylenoxalessigester der Formel XXII

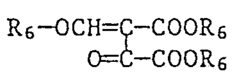 (XXII)

worin die $R_6$ unabhängig voneinander $C_1$-$C_6$-Alkyl- oder Phenyl-$C_1$-$C_6$-alkylreste bedeuten, kondensiert wobei ein 6-Amino-3-alkoxycarbonyl-hexan-3,5-dier·carbonsäureester der Formel XXIII entsteht,

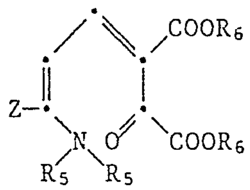

(XXIII)

worin $R_5$ und $R_6$ die obige und Z die unter der Formel I gegebene Bedeutung haben, diesem Ester dann in organischer Lösung mit Ammoniak ringschliesst zum in der 6-Stellung substituierten Pyridin-2,3-dicarbonsäurediester der Formel IVa

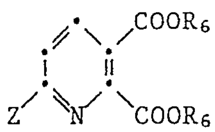

(IVa)

worin Z die unter der Formel I gegebene Bedeutung hat und $R_6$ einem $C_1$-$C_6$-Alkyl- oder Phenyl-$C_1$-$C_6$-alkylrest bedeutet steht, welcher dann gemäss dem Verfahren des Anspruchs 2 in Gegenwart einer starken Base mit dem 2-Glycylamid der Formel XVIII

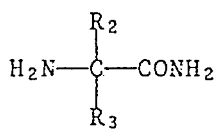

(XVIII)

in welchem $R_2$ und $R_3$ die unter der Formel I gegebene Bedeutung haben, umsetzt, das gebildete Salz der 2-(Imidazol-2-yl)nicotinsäure der Formel III wässriger saurer Lösung aufnimmt, die freie 2-(Imidazolin-2-yl)nicotinsäure der Formel III

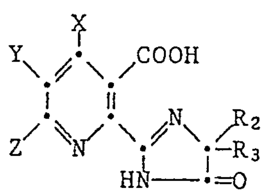

(III)

worin $R_2$, $R_3$, X, Y und Z die unter der Formel I gegebene Bedeutung haben, isoliert und dann mit einem wasserentziehenden Mittel oder Reagens zu einer tricyclischen 2H-Imidazo[1,2:1',2']pyrrolo[3,4-b]pyridin-3,5-dion Verbindung der Formel II umlagert,

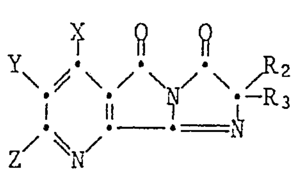

(II)

worin $R_2$, $R_3$, X, Y und Z die unter der Formel I gegebene Bedeutung haben, und diese Verbindung dann bei erhöhterter Temperatur mit einem Hydroxid oder Alkohol der Formel XVI

$HOR_1$ (XVI)

worin $R_1$ die unter der Formel I gegebene Bedeutung hat, zum 2-(Imidazolin-2-yl)nicotinsäurederivat der Formel I aufspaltet.

Die Reaktionen werden, soweit sie sich nicht schon bei Raumtemperatur durchführen lassen, bei

Temperaturen zwischen 0°C und 200°C durchgeführt, d.h. man erhitzt - falls nötig - bis zum Siedepunkt des Reaktionsgemisches und kühlt wenn nötig mit Eis/Wasser oder Eis/Solebad ab.

Als Basen kommen für diese Kondensationen resp. Hydrolysierungen vor allem anorganische Basen wie Natriumhydroxid, Natriumcarbonat, Calciumhydroxid, Calciumcarbonat, Kaliumhydroxid, Kaliumcarbonat, Ammoniak sowie tertiäre organische Basen wie Triäthylamin in Frage.

Geeignete Lösungsmittel sind z.B. polare, aprotische Lösungsmittel, die alleine oder in Mischungen aus mindestens zwei Lösungsmitteln verwendet werden können. Beispiele sind: Ether wie Dibutylether, Tetrahydrofuran, Dioxan, Methylenglykol, Dimethylethylenglykol, Diethyldiethylenglykol, Dimethyltriethylenglykol, halogenierte Kohlenwasserstoffe wie Methylenchlorid, Chloroform, 1,2-Dichlorethan, 1,1,1-Trichlorethan, 1,1,2,2-Tetrachlorethan, Carbonsäureester und Lactone wie Essigsäureethylester, Propionsäuremethylester, Benzoesäureethylester, 2-Methoxyethylacetat, γ-Butyrolacton, o-Valerolacton und Pivalolacton, Carbonsäureamide und Lactame wie Formamid, Acetamid, N-Methylformamid, N,N-Dimethylformamid, N,N-Di ethylformamid, N-N-Dimethylacetamid, N,N-Diethylacetamid, γ-Butyrolactam, ε-Caprolactam, N-Methylpyrrolidon, N-Acetylpyrrolidon, N-Methylcaprolactam, Tetramethylharnstoff, Hexamethylphosphorsäuretriamid, Sulfoxide wie Dimethylsulfoxid, Sulfone wie Dimethylsulfon, Diethylsulfon, Trimethylensulfon, Tetramethylen-sulfon, Trimethylamin, Triethylamin, N-Methylpyrrolidin, N-Methylpiperidin, N-Methylmorpholin, substituierte Benzole wie Chlorbenzol, Nitrobenzol, Nitrile wie z.B. Acetonitril.

Bevorzugt als Herbizide oder zur Regulierung des Pflanzenwuchses sind diejenigen Derivate der 2-(Imidazolin-2-yl)nicotinsäuren und Derivate der Formeln III und I sowie der 2H-Imidazo[1′,2′:1,2]pyrrolo[3,4-b]pyridin-2,5-dion-Verbindungen der Formel II und XII, worin Z einen $C_3$-$C_6$-Cycloalkyl-, $C_2$-$C_8$-Alkoxyalkyl-, Phenoxyalkyl-, Furanyl-, Dioxanyl oder Carbamoyloxymethoxymethylrest bedeutet.

Die Erfindung betrifft auch alle diastereomeren und enantiomeren Isomeren der Verbindungen der Formel I.

Die Wirkstoffe der Formel I werden in der Regel mit Aufwandmengen von 0,05 bis 4 kg/ha insbesondere 0,1 bis 1 kg/ha erfolgreich eingesetzt.

Bei geringeren Aufwandmengen zeichnen sich die Verbindungen der Formel I durch gute selektiv-wuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei - im Vergleich zu anderen Herbiziden und Wuchsregulatoren - sehr geringen Aufwandmengen.

Die Verbindungen der Formel I haben ausserdem starke pflanzenwuchshemmende, Eigenschaften. Es werden sowohl Monokotyledonen als auch Dikotyledonen in ihrem Wachstum beeinträchtigt.

So können z.B. die in der Landwirtschaft in tropischen Gegenden häufig als "cover crops" (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten-und Fruchtbildung zugute kommen, während das vegetative Wachstum eingeschränkt wird.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie

Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation-und/oder anionaktive Tenside mit guten Emulgier-, Dispergier-und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfate liegen in der Regel als Alkali, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nicht ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 10 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffdatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte, Tributylphenoxypolyethanol Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl-oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben: "Mc Cutcheon's Detergents and Emulsifiers Annual" MC Publishing Corp., Ridgewood, New Jersey, 1979. Dr. Helmut Stache "Tensid Taschenbuch" Carl Hanser Verlag, München/Wien 1981.

Die pestiziden Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent)

Emulgierbare Konzentrate:

Wirkstoff der Formel I: 1 bis 20 % bevorzugt 5 bis 10 %
oberflächenaktives Mittel: 5 bis 30 %,vorzugsweise 10 bis 20 %
flüssiges Trägermittel: 50 bis 94%,vorzugsweise 70 bis 85 %.

Stäube:
Wirkstoff der Formel I: 0,1 bis 10 %, vorzugsweise 0,1 bis 1 %
festes Trägermittel: 99,9 bis 90 %, vorzugsweise 99,9 bis 99 %.

Suspensions-Konzentrate:
Wirkstoff der Formel I: 5 bis 75 %, vorzugsweise 10 bis 50 %
Wasser: 94 bis 25 %, vorzugsweise 90 bis 30 %
oberflächenaktives Mittel: 1 bis 40 %, vorzugsweise 2 bis 30 %.

Benetzbare Pulver:
Wirkstoff der Formel I: 0,5 bis 90 %, vorzugsweise 1 bis 80 %
oberflächenaktives Mittel: 0,5 bis 20 %, vorzugsweise 1 bis 15 %
festes Trägermittel: 5 bis 95 %, vorzugsweise 15 bis 90 %.

Granulate:
Wirkstoff der Formel I: 0,5 bis 30 %, vorzugsweise 3 bis 15 %
festes Trägermittel: 99,5 bis 70 %, vorzugsweise 97 bis 85 %.

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,005 bis 5 kg As/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1 Herstellung von 6-Cyclopropyl-2,3-pyridindicarbonsäure-diäthylester

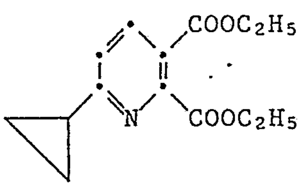

40 g 2,3-Pyridindicarbonsäure-diäthylester werden in einem Gemisch von 400 g Wasser und 36g konzentrierter Schwefelsäure und 6.1 g Silbernitrat ($AgNO_3$) gelöst. Anschliessend gibt man 32 g Cyclopropancarbonsäure zu und erwärmt das Reaktionsgemisch auf 70°. Unter starkem Rühren tropft man dann eine Lösung von 82,1 g Ammonium-peroxy-disulfat $(NH_4)_2S_2O_8$ in 300 ml Wasser dazu. Nach Beendigung der $CO_2$-Entwicklung wird noch 20 Minuten gerührt. Dann wird das Reaktionsgemisch auf 15° abgekühlt und zweimal mit je 200 ml Methylenchlorid extrahiert. Die organische Phase wird gesammelt, über Magnesiumsulfat getrocknet, filtriert und eingedampft. Der Rückstand wird bei 13 millibar destilliert und das Destillat noch über eine Kieselgelsäule mit Hexan/Methylenchlorid/Aether chromatographiert. Nach Verdampfen der Lösungsmittel verbleiben 11,2 g des obigen Diesters als farbloses Oel.

Beispiel 2 Herstellung von 6-Methoxymethyl-2,3-pyridindicarbonsäure-diäthylester

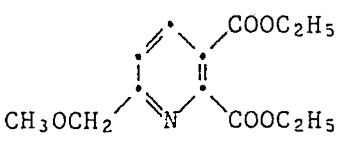

Zu einer Lösung von 400 g Wasser, 36 g konzentrierter Schwefelsäure und 6,1 g Silbernitrat gibt man zuerst 40 g 2,3-Pyridindicarbonsäure-diäthylester und dann 33,3 g Methoxyessigsäure. Das Reaktionsgemisch wird dann auf 70° erwärmt und unter starkem Rühren gibt man tropfenweise eine Lösung von 60,7 g Ammonium-peroxy-disulfat $(NH_4)_2S_2O_8$ in 225 ml Wasser zu. Nach Beendigung der Kohlensäureentwicklung wird noch 20 Minuten nachgerührt. Dann kühlt man das Reaktionsgemisch auf 15° ab und extrahiert zweimal mit je 200 ml Methylenchlorid. Die organischen Phasen werden gesammelt, über Magnesiumsulfat getrocknet,

filtriert und eingedamft. Das zurückbleibende Oel wird über eine Kieselgelsäule mit Aether/Hexan 1:1 chromatographiert, wobei man aus verschiedenen Fraktionen 9,1 g 6-Methoxymethyl-2,3-pyridindicarbonsäure als Kristalle mit Smp. 52-54°; 12,9 g 4,6-Bis-methoxymethyl-2,3-pyridindicarbonsäurediäthylester als Oel und 10,2 g 4-Methoxymethyl-2,3-pyridindicarbonsäure-diäthylester, ebenfalls als Oel eluiert.

Beispiel 3 Herstellung von 5-Aethyl-6-methoxymethyl-2,3-pyridindicarbonsäure-diäthylester

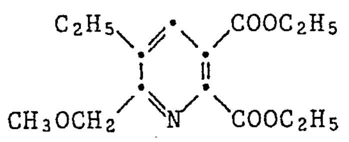

Zu einer Lösung von 20 g konzentrierter Schwefelsäure und 3,4 g Silbernitrat ($AgNO_3$) in 200 ml Wasser gibt man zuerst 25,1 g 5-Aethyl-2,3-pyridindicarbonsäure-diäthylester und anschliessend 13,5 g Methoxyessigsäure zu. Dann wird auf 70° erwärmt und unter starkem Rühren eine Lösung von 34,2 g Ammonium-peroxy-disulfat $(NH_4)_2S_2O_8$ zugetropft. Nachdem die Kohlensäureentwicklung aufgehört hat, wird noch 20 Minuten weitergerührt, dann auf 15° abgekühlt und das Reaktionsgemisch zweimal mit je 200 ml Methylenchlorid extrahiert. Die Methylenchlorid-Extrakte werden über Magnesiumsulfat getrocknet, filtriert und eingedampft. Das verbleibende Oel wird durch Chromatographie über eine Kieselgelsäule mittels Aether/Hexan 1:1 gereinigt. Aus dem Eluat gewinnt man in verschiedenen Fraktionen 7,2 g 5-Aethyl-6-methoxymethyl-2,3-pyridindicarbonsäure-diäthylester als hellgelbes Oel, 5,3 g 4,6-bis-Methoxymethyl-5-äthyl-2,3-pyridindicarbonsäure-diäthylester als helles Oel, sowie 1 g 5-Aethyl-4-methoxymethyl-2,3-pyridindicarbonsäure-diäthylester, ebenfalls als Oel.

Beispiel 4 Herstellung von 5-Aethyl-6-cyclopropyl-2,3-pyridindicarbonsäure-diäthylester

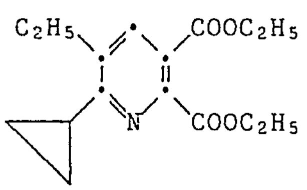

Zu einer Lösung von 32 ml konzentrierter Schwefelsäure und 6,8 g Silbernitrat ($AgNO_3$) in 400 ml Wasser gibt man zuerst 50 g 5-Aethyl-2,3-pyridindicarbonsäure-diäthylester und, wenn eine homogene Lösung erreicht ist, 25,8 g Cyclopropancarbonsäure. Die Lösung wird dann auf 70° erwärmt und man tropft unter Starkem Rühren eine Lösung von 68,2 g Ammonium-peroxy-disulfonat $(NH_4)_2S_2O_2$ zu. Nachdem die Karbonsäureentwicklung aufgehört hat, wird noch 20 Minuten weitergerührt, dann auf 15° abgekühlt und das Reaktionsgemisch zweimal mit je 200 ml Methylenchlorid extrahiert. Das Methylenchlorid-Extrakt wird über Magnesiumsulfat getrocknet, filtriert und eingeengt. Der ölige Rückstand wird durch Chromatographie über eine Silikagelsäule mittels Aether/Hexan 1:1 gereinigt. Nach Verdampfen der Lösungsmittel erhält man 20 g 5-Aethyl-6-cyclopropyl-2,3-pyridindicarbonsäure als gelbes Oel.

Zu den Beispielen 1-4 werden in analoger Weise die folgenden Diester der Formel IV hergestellt:

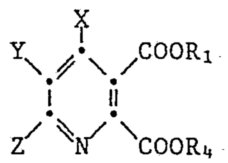

(IV)

Tabelle 1.00

| No. | X | Y | $R_1$ | $R_4$ | Z | phys. Daten |
|---|---|---|---|---|---|---|
| 1.0001 | H | H | $C_2H_5$ | $C_2H_5$ | $C_3H_5$(Cyclo) | Sdp.155-163/0.13 mbar |
| 1.0002 | H | $CH_3$ | $C_2H_5$ | $C_2H_5$ | $C_3H_5$(Cyclo) | Oel |
| 1.0003 | H | $C_2H_5$ | $C_2H_5$ | $C_2H_5$ | $C_3H_5$(Cyclo) | Oel |
| 1.0004 | H | $C_3H_7$ | $C_2H_5$ | $C_2H_5$ | $C_3H_5$(Cyclo) | |
| 1.0005 | H | $OCH_3$ | $C_2H_5$ | $C_2H_5$ | $C_3H_5$(Cyclo) | |
| 1.0006 | H | $OC_2H_5$ | $C_2H_5$ | $C_2H_5$ | $C_3H_5$(Cyclo) | |
| 1.0007 | H | $OCH_2CH=CH_2$ | $C_2H_5$ | $C_2H_5$ | $C_3H_5$(Cyclo) | |
| 1.0008 | H | $OCH_2CH=CHCH_3$ | $C_2H_5$ | $C_2H_5$ | $C_3H_5$(Cyclo) | |
| 1.0009 | H | $OCH_2C(Cl)=CH_2$ | $C_2H_5$ | $C_2H_5$ | $C_3H_5$(Cyclo) | |
| 1.0010 | H | $OCH_2CH=CHCl$ | $C_2H_5$ | $C_2H_5$ | $C_3H_5$(Cyclo) | |
| 1.0011 | H | $OCH_2C\equiv CH$ | $C_2H_5$ | $C_2H_5$ | $C_3H_5$(Cyclo) | |
| 1.0012 | H | $OCHF_2$ | $C_2H_5$ | $C_2H_5$ | $C_3H_5$(Cyclo) | |
| 1.0013 | H | $OCF_3$ | $C_2H_5$ | $C_2H_5$ | $C_3H_5$(Cyclo) | |
| 1.0014 | H | $OCF_2CHFCF_3$ | $C_2H_5$ | $C_2H_5$ | $C_3H_5$(Cyclo) | |
| 1.0015 | H | $OC_6H_5$ | $C_2H_5$ | $C_2H_5$ | $C_3H_5$(Cyclo) | |
| 1.0016 | H | $CF_3$ | $C_2H_5$ | $C_2H_5$ | $C_3H_5$(Cyclo) | |
| 1.0017 | H | Br | $C_2H_5$ | $C_2H_5$ | $C_3H_5$(Cyclo) | Oel |
| 1.0018 | H | Cl | $C_2H_5$ | $C_2H_5$ | $C_3H_5$(Cyclo) | |
| 1.0019 | H | F | $C_2H_5$ | $C_2H_5$ | $C_3H_5$(Cyclo) | |
| 1.0020 | H | $SCH_3$ | $C_2H_5$ | $C_2H_5$ | $C_3H_5$(Cyclo) | |
| 1.0021 | H | $SC_2H_5$ | $C_2H_5$ | $C_2H_5$ | $C_3H_5$(Cyclo) | |
| 1.0022 | H | $SC_3H_7$-n | $C_2H_5$ | $C_2H_5$ | $C_3H_5$(Cyclo) | |
| 1.0023 | H | $SO_2CH_3$ | $C_2H_5$ | $C_2H_5$ | $C_3H_5$(Cyclo) | |
| 1.0024 | H | $SO_2C_2H_5$ | $C_2H_5$ | $C_2H_5$ | $C_3H_5$(Cyclo) | |
| 1.0025 | H | $NO_2$ | $C_2H_5$ | $C_2H_5$ | $C_3H_5$(Cyclo) | |
| 1.0026 | H | CN | $C_2H_5$ | $C_2H_5$ | $C_3H_5$(Cyclo) | |
| 1.0027 | H | $CH_2OH$ | $C_2H_5$ | $C_2H_5$ | $C_3H_5$(Cyclo) | |
| 1.0028 | H | $CH_2CH_2OH$ | $C_2H_5$ | $C_2H_5$ | $C_3H_5$(Cyclo) | |
| 1.0029 | H | $CH_2CH_2CH_2OH$ | $C_2H_5$ | $C_2H_5$ | $C_3H_5$(Cyclo) | |
| 1.0030 | H | $C_6H_5$ | $C_2H_5$ | $C_2H_5$ | $C_3H_5$(Cyclo) | |
| 1.0031 | H | $CH_2Cl$ | $C_2H_5$ | $C_2H_5$ | $C_3H_5$(Cyclo) | |
| 1.0032 | H | $CH_2CH_2Cl$ | $C_2H_5$ | $C_2H_5$ | $C_3H_5$(Cyclo) | |
| 1.0033 | H | $CH_2CH_2CH_2Cl$ | $C_2H_5$ | $C_2H_5$ | $C_3H_5$(Cyclo) | |
| 1.0034 | H | H | $C_2H_5$ | $C_3H_7$-iso | $C_3H_5$(Cyclo) | |
| 1.0035 | H | H | $C_3H_7$ | $CH_2C_6H_5$ | $C_3H_5$(Cyclo) | |
| 1.0036 | H | H | $CH_3$ | $CH_3$ | $C_3H_5$(Cyclo) | |
| 1.0037 | H | H | $CH_2C_6H_5$ | $CH_2C_6H_5$ | $C_3H_5$(Cyclo) | |

Tabelle 1.01

| No. | X | Y | $R_1$ | $R_4$ | Z | phys. Daten |
|---|---|---|---|---|---|---|
| 1.0101 | H | H | $C_2H_5$ | $C_2H_5$ | $C_4H_7$(Cyclo) | |
| 1.0102 | H | $CH_3$ | $C_2H_5$ | $C_2H_5$ | $C_4H_7$(Cyclo) | |
| 1.0103 | H | $C_2H_5$ | $C_2H_5$ | $C_2H_5$ | $C_4H_7$(Cyclo) | |
| 1.0104 | H | $C_3H_7$ | $C_2H_5$ | $C_2H_5$ | $C_4H_7$(Cyclo) | |
| 1.0105 | H | $OCH_3$ | $C_2H_5$ | $C_2H_5$ | $C_4H_7$(Cyclo) | |
| 1.0106 | H | $OC_2H_5$ | $C_2H_5$ | $C_2H_5$ | $C_4H_7$(Cyclo) | |
| 1.0107 | H | $OCH_2CH{=}CH_2$ | $C_2H_5$ | $C_2H_5$ | $C_4H_7$(Cyclo) | |
| 1.0108 | H | $OCH_2CH{=}CHCH_3$ | $C_2H_5$ | $C_2H_5$ | $C_4H_7$(Cyclo) | |
| 1.0109 | H | $OCH_2C(Cl){=}CH_2$ | $C_2H_5$ | $C_2H_5$ | $C_4H_7$(Cyclo) | |
| 1.0110 | H | $OCH_2CH{=}CHCl$ | $C_2H_5$ | $C_2H_5$ | $C_4H_7$(Cyclo) | |
| 1.0111 | H | $OCH_2C{\equiv}CH$ | $C_2H_5$ | $C_2H_5$ | $C_4H_7$(Cyclo) | |
| 1.0112 | H | $OCHF_2$ | $C_2H_5$ | $C_2H_5$ | $C_4H_7$(Cyclo) | |
| 1.0113 | H | $OCF_3$ | $C_2H_5$ | $C_2H_5$ | $C_4H_7$(Cyclo) | |
| 1.0114 | H | $OCF_2CHFCF_3$ | $C_2H_5$ | $C_2H_5$ | $C_4H_7$(Cyclo) | |
| 1.0115 | H | $OC_6H_5$ | $C_2H_5$ | $C_2H_5$ | $C_4H_7$(Cyclo) | |
| 1.0116 | H | $CF_3$ | $C_2H_5$ | $C_2H_5$ | $C_4H_7$(Cyclo) | |
| 1.0117 | H | Br | $C_2H_5$ | $C_2H_5$ | $C_4H_7$(Cyclo) | |
| 1.0118 | H | Cl | $C_2H_5$ | $C_2H_5$ | $C_4H_7$(Cyclo) | |
| 1.0119 | H | F | $C_2H_5$ | $C_2H_5$ | $C_4H_7$(Cyclo) | |
| 1.0120 | H | $SCH_3$ | $C_2H_5$ | $C_2H_5$ | $C_4H_7$(Cyclo) | |
| 1.0121 | H | $SC_2H_5$ | $C_2H_5$ | $C_2H_5$ | $C_4H_7$(Cyclo) | |
| 1.0122 | H | $SC_3H_7$-n | $C_2H_5$ | $C_2H_5$ | $C_4H_7$(Cyclo) | |
| 1.0123 | H | $SO_2CH_3$ | $C_2H_5$ | $C_2H_5$ | $C_4H_7$(Cyclo) | |
| 1.0124 | H | $SO_2C_2H_5$ | $C_2H_5$ | $C_2H_5$ | $C_4H_7$(Cyclo) | |
| 1.0125 | H | $NO_2$ | $C_2H_5$ | $C_2H_5$ | $C_4H_7$(Cyclo) | |
| 1.0126 | H | CN | $C_2H_5$ | $C_2H_5$ | $C_4H_7$(Cyclo) | |
| 1.0127 | H | $CH_2OH$ | $C_2H_5$ | $C_2H_5$ | $C_4H_7$(Cyclo) | |
| 1.0128 | H | $CH_2CH_2OH$ | $C_2H_5$ | $C_2H_5$ | $C_4H_7$(Cyclo) | |
| 1.0129 | H | $CH_2CH_2CH_2OH$ | $C_2H_5$ | $C_2H_5$ | $C_4H_7$(Cyclo) | |
| 1.0130 | H | $C_6H_5$ | $C_2H_5$ | $C_2H_5$ | $C_4H_7$(Cyclo) | |
| 1.0131 | H | $CH_2Cl$ | $C_2H_5$ | $C_2H_5$ | $C_4H_7$(Cyclo) | |
| 1.0132 | H | $CH_2CH_2Cl$ | $C_2H_5$ | $C_2H_5$ | $C_4H_7$(Cyclo) | |
| 1.0133 | H | $CH_2CH_2CH_2Cl$ | $C_2H_5$ | $C_2H_5$ | $C_4H_7$(Cyclo) | |

Tabelle 1.02

| No. | X | Y | $R_1$ | $R_4$ | Z | phys. Daten |
|---|---|---|---|---|---|---|
| 1.0201 | H | H | $C_2H_5$ | $C_2H_5$ | $C_5H_9$(Cyclo) | |
| 1.0202 | H | $CH_3$ | $C_2H_5$ | $C_2H_5$ | $C_5H_9$(Cyclo) | |
| 1.0203 | H | $C_2H_5$ | $C_2H_5$ | $C_2H_5$ | $C_5H_9$(Cyclo) | |
| 1.0204 | H | $C_3H_7$ | $C_2H_5$ | $C_2H_5$ | $C_5H_9$(Cyclo) | |
| 1.0205 | H | $OCH_3$ | $C_2H_5$ | $C_2H_5$ | $C_5H_9$(Cyclo) | |
| 1.0206 | H | $OC_2H_5$ | $C_2H_5$ | $C_2H_5$ | $C_5H_9$(Cyclo) | |
| 1.0207 | H | $OCH_2CH=CH_2$ | $C_2H_5$ | $C_2H_5$ | $C_5H_9$(Cyclo) | |
| 1.0208 | H | $OCH_2CH=CHCH_3$ | $C_2H_5$ | $C_2H_5$ | $C_5H_9$(Cyclo) | |
| 1.0209 | H | $OCH_2C(Cl)=CH_2$ | $C_2H_5$ | $C_2H_5$ | $C_5H_9$(Cyclo) | |
| 1.0210 | H | $OCH_2CH=CHCl$ | $C_2H_5$ | $C_2H_5$ | $C_5H_9$(Cyclo) | |
| 1.0211 | H | $OCH_2C{\equiv}CH$ | $C_2H_5$ | $C_2H_5$ | $C_5H_9$(Cyclo) | |
| 1.0212 | H | $OCHF_2$ | $C_2H_5$ | $C_2H_5$ | $C_5H_9$(Cyclo) | |
| 1.0213 | H | $OCF_3$ | $C_2H_5$ | $C_2H_5$ | $C_5H_9$(Cyclo) | |
| 1.0214 | H | $OCF_2CHFCF_3$ | $C_2H_5$ | $C_2H_5$ | $C_5H_9$(Cyclo) | |
| 1.0215 | H | $OC_6H_5$ | $C_2H_5$ | $C_2H_5$ | $C_5H_9$(Cyclo) | |
| 1.0216 | H | $CF_3$ | $C_2H_5$ | $C_2H_5$ | $C_5H_9$(Cyclo) | |
| 1.0217 | H | Br | $C_2H_5$ | $C_2H_5$ | $C_5H_9$(Cyclo) | |
| 1.0218 | H | Cl | $C_2H_5$ | $C_2H_5$ | $C_5H_9$(Cyclo) | |
| 1.0219 | H | F | $C_2H_5$ | $C_2H_5$ | $C_5H_9$(Cyclo) | |
| 1.0220 | H | $SCH_3$ | $C_2H_5$ | $C_2H_5$ | $C_5H_9$(Cyclo) | |
| 1.0221 | H | $SC_2H_5$ | $C_2H_5$ | $C_2H_5$ | $C_5H_9$(Cyclo) | |
| 1.0222 | H | $SC_3H_7$-n | $C_2H_5$ | $C_2H_5$ | $C_5H_9$(Cyclo) | |
| 1.0223 | H | $SO_2CH_3$ | $C_2H_5$ | $C_2H_5$ | $C_5H_9$(Cyclo) | |
| 1.0224 | H | $SO_2C_2H_5$ | $C_2H_5$ | $C_2H_5$ | $C_5H_9$(Cyclo) | |
| 1.0225 | H | $NO_2$ | $C_2H_5$ | $C_2H_5$ | $C_5H_9$(Cyclo) | |
| 1.0226 | H | CN | $C_2H_5$ | $C_2H_5$ | $C_5H_9$(Cyclo) | |
| 1.0227 | H | $CH_2OH$ | $C_2H_5$ | $C_2H_5$ | $C_5H_9$(Cyclo) | |
| 1.0228 | H | $CH_2CH_2OH$ | $C_2H_5$ | $C_2H_5$ | $C_5H_9$(Cyclo) | |
| 1.0229 | H | $CH_2CH_2CH_2OH$ | $C_2H_5$ | $C_2H_5$ | $C_5H_9$(Cyclo) | |
| 1.0230 | H | $C_6H_5$ | $C_2H_5$ | $C_2H_5$ | $C_5H_9$(Cyclo) | |
| 1.0231 | H | $CH_2Cl$ | $C_2H_5$ | $C_2H_5$ | $C_5H_9$(Cyclo) | |
| 1.0232 | H | $CH_2CH_2Cl$ | $C_2H_5$ | $C_2H_5$ | $C_5H_9$(Cyclo) | |
| 1.0233 | H | $CH_2CH_2CH_2Cl$ | $C_2H_5$ | $C_2H_5$ | $C_5H_9$(Cyclo) | |

Tabelle 1.03

| No. | X | Y | $R_1$ | $R_4$ | Z | phys. Daten |
|---|---|---|---|---|---|---|
| 1.0301 | H | H | $C_2H_5$ | $C_2H_5$ | $C_6H_{11}$(Cyclo) | Oel |
| 1.0302 | H | $CH_3$ | $C_2H_5$ | $C_2H_5$ | $C_6H_{11}$(Cyclo) | |
| 1.0303 | H | $C_2H_5$ | $C_2H_5$ | $C_2H_5$ | $C_6H_{11}$(Cyclo) | |
| 1.0304 | H | $C_3H_7$ | $C_2H_5$ | $C_2H_5$ | $C_6H_{11}$(Cyclo) | |
| 1.0305 | H | $OCH_3$ | $C_2H_5$ | $C_2H_5$ | $C_6H_{11}$(Cyclo) | |
| 1.0306 | H | $OC_2H_5$ | $C_2H_5$ | $C_2H_5$ | $C_6H_{11}$(Cyclo) | |
| 1.0307 | H | $OCH_2CH{=}CH_2$ | $C_2H_5$ | $C_2H_5$ | $C_6H_{11}$(Cyclo) | |
| 1.0308 | H | $OCH_2CH{=}CHCH_3$ | $C_2H_5$ | $C_2H_5$ | $C_6H_{11}$(Cyclo) | |
| 1.0309 | H | $OCH_2C(Cl){=}CH_2$ | $C_2H_5$ | $C_2H_5$ | $C_6H_{11}$(Cyclo) | |
| 1.0310 | H | $OCH_2CH{=}CHCl$ | $C_2H_5$ | $C_2H_5$ | $C_6H_{11}$(Cyclo) | |
| 1.0311 | H | $OCH_2C{\equiv}CH$ | $C_2H_5$ | $C_2H_5$ | $C_6H_{11}$(Cyclo) | |
| 1.0312 | H | $OCHF_2$ | $C_2H_5$ | $C_2H_5$ | $C_6H_{11}$(Cyclo) | |
| 1.0313 | H | $OCF_3$ | $C_2H_5$ | $C_2H_5$ | $C_6H_{11}$(Cyclo) | |
| 1.0314 | H | $OCF_2CHFCF_3$ | $C_2H_5$ | $C_2H_5$ | $C_6H_{11}$(Cyclo) | |
| 1.0315 | H | $OC_6H_5$ | $C_2H_5$ | $C_2H_5$ | $C_6H_{11}$(Cyclo) | |
| 1.0316 | H | $CF_3$ | $C_2H_5$ | $C_2H_5$ | $C_6H_{11}$(Cyclo) | |
| 1.0317 | H | Br | $C_2H_5$ | $C_2H_5$ | $C_6H_{11}$(Cyclo) | |
| 1.0318 | H | Cl | $C_2H_5$ | $C_2H_5$ | $C_6H_{11}$(Cyclo) | |
| 1.0319 | H | F | $C_2H_5$ | $C_2H_5$ | $C_6H_{11}$(Cyclo) | |
| 1.0320 | H | $SCH_3$ | $C_2H_5$ | $C_2H_5$ | $C_6H_{11}$(Cyclo) | |
| 1.0321 | H | $SC_2H_5$ | $C_2H_5$ | $C_2H_5$ | $C_6H_{11}$(Cyclo) | |
| 1.0322 | H | $SC_3H_7$-n | $C_2H_5$ | $C_2H_5$ | $C_6H_{11}$(Cyclo) | |
| 1.0323 | H | $SO_2CH_3$ | $C_2H_5$ | $C_2H_5$ | $C_6H_{11}$(Cyclo) | |
| 1.0324 | H | $SO_2C_2H_5$ | $C_2H_5$ | $C_2H_5$ | $C_6H_{11}$(Cyclo) | |
| 1.0325 | H | $NO_2$ | $C_2H_5$ | $C_2H_5$ | $C_6H_{11}$(Cyclo) | |
| 1.0326 | H | CN | $C_2H_5$ | $C_2H_5$ | $C_6H_{11}$(Cyclo) | |
| 1.0327 | H | $CH_2OH$ | $C_2H_5$ | $C_2H_5$ | $C_6H_{11}$(Cyclo) | |
| 1.0328 | H | $CH_2CH_2OH$ | $C_2H_5$ | $C_2H_5$ | $C_6H_{11}$(Cyclo) | |
| 1.0329 | H | $CH_2CH_2CH_2OH$ | $C_2H_5$ | $C_2H_5$ | $C_6H_{11}$(Cyclo) | |
| 1.0330 | H | $C_6H_5$ | $C_2H_5$ | $C_2H_5$ | $C_6H_{11}$(Cyclo) | |
| 1.0331 | H | $CH_2Cl$ | $C_2H_5$ | $C_2H_5$ | $C_6H_{11}$(Cyclo) | |
| 1.0332 | H | $CH_2CH_2Cl$ | $C_2H_5$ | $C_2H_5$ | $C_6H_{11}$(Cyclo) | |
| 1.0333 | H | $CH_2CH_2CH_2Cl$ | $C_2H_5$ | $C_2H_5$ | $C_6H_{11}$(Cyclo) | |

Tabelle 1.04

| No. | X | Y | $R_1$ | $R_4$ | Z | phys. Daten |
|---|---|---|---|---|---|---|
| 1.0401 | H | H | $C_2H_5$ | $C_2H_5$ | $CH_2OCH_3$ | Smp. 52-54°C |
| 1.0402 | H | $CH_3$ | $C_2H_5$ | $C_2H_5$ | $CH_2OCH_3$ | Oel |
| 1.0403 | H | $C_2H_5$ | $C_2H_5$ | $C_2H_5$ | $CH_2OCH_3$ | Oel |
| 1.0404 | H | $C_3H_7$ | $C_2H_5$ | $C_2H_5$ | $CH_2OCH_3$ | |
| 1.0405 | H | $OCH_3$ | $C_2H_5$ | $C_2H_5$ | $CH_2OCH_3$ | |
| 1.0406 | H | $OC_2H_5$ | $C_2H_5$ | $C_2H_5$ | $CH_2OCH_3$ | |
| 1.0407 | H | $OCH_2CH=CH_2$ | $C_2H_5$ | $C_2H_5$ | $CH_2OCH_3$ | |
| 1.0408 | H | $OCH_2CH=CHCH_3$ | $C_2H_5$ | $C_2H_5$ | $CH_2OCH_3$ | |
| 1.0409 | H | $OCH_2C(Cl)=CH_2$ | $C_2H_5$ | $C_2H_5$ | $CH_2OCH_3$ | |
| 1.0410 | H | $OCH_2CH=CHCl$ | $C_2H_5$ | $C_2H_5$ | $CH_2OCH_3$ | |
| 1.0411 | H | $OCH_2C\equiv CH$ | $C_2H_5$ | $C_2H_5$ | $CH_2OCH_3$ | |
| 1.0412 | H | $OCHF_2$ | $C_2H_5$ | $C_2H_5$ | $CH_2OCH_3$ | |
| 1.0413 | H | $OCF_3$ | $C_2H_5$ | $C_2H_5$ | $CH_2OCH_3$ | |
| 1.0414 | H | $OCF_2CHFCF_3$ | $C_2H_5$ | $C_2H_5$ | $CH_2OCH_3$ | |
| 1.0415 | H | $OC_6H_5$ | $C_2H_5$ | $C_2H_5$ | $CH_2OCH_3$ | |
| 1.0416 | H | $CF_3$ | $C_2H_5$ | $C_2H_5$ | $CH_2OCH_3$ | |
| 1.0417 | H | Br | $C_2H_5$ | $C_2H_5$ | $CH_2OCH_3$ | |
| 1.0418 | H | Cl | $C_2H_5$ | $C_2H_5$ | $CH_2OCH_3$ | |
| 1.0419 | H | F | $C_2H_5$ | $C_2H_5$ | $CH_2OCH_3$ | |
| 1.0420 | H | $SCH_3$ | $C_2H_5$ | $C_2H_5$ | $CH_2OCH_3$ | |
| 1.0421 | H | $SC_2H_5$ | $C_2H_5$ | $C_2H_5$ | $CH_2OCH_3$ | |
| 1.0422 | H | $SC_3H_7$-n | $C_2H_5$ | $C_2H_5$ | $CH_2OCH_3$ | |
| 1.0423 | H | $SO_2CH_3$ | $C_2H_5$ | $C_2H_5$ | $CH_2OCH_3$ | |
| 1.0424 | H | $SO_2C_2H_5$ | $C_2H_5$ | $C_2H_5$ | $CH_2OCH_3$ | |
| 1.0425 | H | $NO_2$ | $C_2H_5$ | $C_2H_5$ | $CH_2OCH_3$ | |
| 1.0426 | H | CN | $C_2H_5$ | $C_2H_5$ | $CH_2OCH_3$ | |
| 1.0427 | H | $CH_2OH$ | $C_2H_5$ | $C_2H_5$ | $CH_2OCH_3$ | |
| 1.0428 | H | $CH_2CH_2OH$ | $C_2H_5$ | $C_2H_5$ | $CH_2OCH_3$ | |
| 1.0429 | H | $CH_2CH_2CH_2OH$ | $C_2H_5$ | $C_2H_5$ | $CH_2OCH_3$ | |
| 1.0430 | H | $C_6H_5$ | $C_2H_5$ | $C_2H_5$ | $CH_2OCH_3$ | |
| 1.0431 | H | $CH_2Cl$ | $C_2H_5$ | $C_2H_5$ | $CH_2OCH_3$ | |
| 1.0432 | H | $CH_2CH_2Cl$ | $C_2H_5$ | $C_2H_5$ | $CH_2OCH_3$ | |
| 1.0433 | H | $CH_2CH_2CH_2Cl$ | $C_2H_5$ | $C_2H_5$ | $CH_2OCH_3$ | |

Tabelle 1.05

| No. | X | Y | $R_1$ | $R_4$ | Z | phys. Daten |
|---|---|---|---|---|---|---|
| 1.0501 | H | H | $C_2H_5$ | $C_2H_5$ | $CH(CH_3)OCH_3$ | |
| 1.0502 | H | $CH_3$ | $C_2H_5$ | $C_2H_5$ | $CH(CH_3)OCH_3$ | |
| 1.0503 | H | $C_2H_5$ | $C_2H_5$ | $C_2H_5$ | $CH(CH_3)OCH_3$ | |
| 1.0504 | H | $C_3H_7$ | $C_2H_5$ | $C_2H_5$ | $CH(CH_3)OCH_3$ | |
| 1.0505 | H | $OCH_3$ | $C_2H_5$ | $C_2H_5$ | $CH(CH_3)OCH_3$ | |
| 1.0506 | H | $OC_2H_5$ | $C_2H_5$ | $C_2H_5$ | $CH(CH_3)OCH_3$ | |
| 1.0507 | H | $OCH_2CH=CH_2$ | $C_2H_5$ | $C_2H_5$ | $CH(CH_3)OCH_3$ | |
| 1.0508 | H | $OCH_2CH=CHCH_3$ | $C_2H_5$ | $C_2H_5$ | $CH(CH_3)OCH_3$ | |
| 1.0509 | H | $OCH_2C(Cl)=CH_2$ | $C_2H_5$ | $C_2H_5$ | $CH(CH_3)OCH_3$ | |
| 1.0510 | H | $OCH_2CH=CHCl$ | $C_2H_5$ | $C_2H_5$ | $CH(CH_3)OCH_3$ | |
| 1.0511 | H | $OCH_2C{\equiv}CH$ | $C_2H_5$ | $C_2H_5$ | $CH(CH_3)OCH_3$ | |
| 1.0512 | H | $OCHF_2$ | $C_2H_5$ | $C_2H_5$ | $CH(CH_3)OCH_3$ | |
| 1.0513 | H | $OCF_3$ | $C_2H_5$ | $C_2H_5$ | $CH(CH_3)OCH_3$ | |
| 1.0514 | H | $OCF_2CHFCF_3$ | $C_2H_5$ | $C_2H_5$ | $CH(CH_3)OCH_3$ | |
| 1.0515 | H | $OC_6H_5$ | $C_2H_5$ | $C_2H_5$ | $CH(CH_3)OCH_3$ | |
| 1.0516 | H | $CF_3$ | $C_2H_5$ | $C_2H_5$ | $CH(CH_3)OCH_3$ | |
| 1.0517 | H | Br | $C_2H_5$ | $C_2H_5$ | $CH(CH_3)OCH_3$ | |
| 1.0518 | H | Cl | $C_2H_5$ | $C_2H_5$ | $CH(CH_3)OCH_3$ | |
| 1.0519 | H | F | $C_2H_5$ | $C_2H_5$ | $CH(CH_3)OCH_3$ | |
| 1.0520 | H | $SCH_3$ | $C_2H_5$ | $C_2H_5$ | $CH(CH_3)OCH_3$ | |
| 1.0521 | H | $SC_2H_5$ | $C_2H_5$ | $C_2H_5$ | $CH(CH_3)OCH_3$ | |
| 1.0522 | H | $SC_3H_7$-n | $C_2H_5$ | $C_2H_5$ | $CH(CH_3)OCH_3$ | |
| 1.0523 | H | $SO_2CH_3$ | $C_2H_5$ | $C_2H_5$ | $CH(CH_3)OCH_3$ | |
| 1.0524 | H | $SO_2C_2H_5$ | $C_2H_5$ | $C_2H_5$ | $CH(CH_3)OCH_3$ | |
| 1.0525 | H | $NO_2$ | $C_2H_5$ | $C_2H_5$ | $CH(CH_3)OCH_3$ | |
| 1.0526 | H | CN | $C_2H_5$ | $C_2H_5$ | $CH(CH_3)OCH_3$ | |
| 1.0527 | H | $CH_2OH$ | $C_2H_5$ | $C_2H_5$ | $CH(CH_3)OCH_3$ | |
| 1.0528 | H | $CH_2CH_2OH$ | $C_2H_5$ | $C_2H_5$ | $CH(CH_3)OCH_3$ | |
| 1.0529 | H | $CH_2CH_2CH_2OH$ | $C_2H_5$ | $C_2H_5$ | $CH(CH_3)OCH_3$ | |
| 1.0530 | H | $C_6H_5$ | $C_2H_5$ | $C_2H_5$ | $CH(CH_3)OCH_3$ | |
| 1.0531 | H | $CH_2Cl$ | $C_2H_5$ | $C_2H_5$ | $CH(CH_3)OCH_3$ | |
| 1.0532 | H | $CH_2CH_2Cl$ | $C_2H_5$ | $C_2H_5$ | $CH(CH_3)OCH_3$ | |
| 1.0533 | H | $CH_2CH_2CH_2Cl$ | $C_2H_5$ | $C_2H_5$ | $CH(CH_3)OCH_3$ | |

Tabelle 1.06

| No. | X | Y | $R_1$ | $R_4$ | Z | phys. Daten |
|---|---|---|---|---|---|---|
| 1.0601 | H | H | $C_2H_5$ | $C_2H_5$ | $CH_2OC_2H_5$ | Sdp.140-145°/0.13 mbar |
| 1.0602 | H | $CH_3$ | $C_2H_5$ | $C_2H_5$ | $CH_2OC_2H_5$ | Oel |
| 1.0603 | H | $C_2H_5$ | $C_2H_5$ | $C_2H_5$ | $CH_2OC_2H_5$ | Oel |
| 1.0604 | H | $C_3H_7$ | $C_2H_5$ | $C_2H_5$ | $CH_2OC_2H_5$ | |
| 1.0605 | H | $OCH_3$ | $C_2H_5$ | $C_2H_5$ | $CH_2OC_2H_5$ | |
| 1.0606 | H | $OC_2H_5$ | $C_2H_5$ | $C_2H_5$ | $CH_2OC_2H_5$ | |
| 1.0607 | H | $OCH_2CH=CH_2$ | $C_2H_5$ | $C_2H_5$ | $CH_2OC_2H_5$ | |
| 1.0608 | H | $OCH_2CH=CHCH_3$ | $C_2H_5$ | $C_2H_5$ | $CH_2OC_2H_5$ | |
| 1.0609 | H | $OCH_2C(Cl)=CH_2$ | $C_2H_5$ | $C_2H_5$ | $CH_2OC_2H_5$ | |
| 1.0610 | H | $OCH_2CH=CHCl$ | $C_2H_5$ | $C_2H_5$ | $CH_2OC_2H_5$ | |
| 1.0611 | H | $OCH_2C{\equiv}CH$ | $C_2H_5$ | $C_2H_5$ | $CH_2OC_2H_5$ | |
| 1.0612 | H | $OCHF_2$ | $C_2H_5$ | $C_2H_5$ | $CH_2OC_2H_5$ | |
| 1.0613 | H | $OCF_3$ | $C_2H_5$ | $C_2H_5$ | $CH_2OC_2H_5$ | |
| 1.0614 | H | $OCF_2CHFCF_3$ | $C_2H_5$ | $C_2H_5$ | $CH_2OC_2H_5$ | |
| 1.0615 | H | $OC_6H_5$ | $C_2H_5$ | $C_2H_5$ | $CH_2OC_2H_5$ | |
| 1.0616 | H | $CF_3$ | $C_2H_5$ | $C_2H_5$ | $CH_2OC_2H_5$ | |
| 1.0617 | H | Br | $C_2H_5$ | $C_2H_5$ | $CH_2OC_2H_5$ | |
| 1.0618 | H | Cl | $C_2H_5$ | $C_2H_5$ | $CH_2OC_2H_5$ | |
| 1.0619 | H | F | $C_2H_5$ | $C_2H_5$ | $CH_2OC_2H_5$ | |
| 1.0620 | H | $SCH_3$ | $C_2H_5$ | $C_2H_5$ | $CH_2OC_2H_5$ | |
| 1.0621 | H | $SC_2H_5$ | $C_2H_5$ | $C_2H_5$ | $CH_2OC_2H_5$ | |
| 1.0622 | H | $SC_3H_7$-n | $C_2H_5$ | $C_2H_5$ | $CH_2OC_2H_5$ | |
| 1.0623 | H | $SO_2CH_3$ | $C_2H_5$ | $C_2H_5$ | $CH_2OC_2H_5$ | |
| 1.0624 | H | $SO_2C_2H_5$ | $C_2H_5$ | $C_2H_5$ | $CH_2OC_2H_5$ | |
| 1.0625 | H | $NO_2$ | $C_2H_5$ | $C_2H_5$ | $CH_2OC_2H_5$ | |
| 1.0626 | H | CN | $C_2H_5$ | $C_2H_5$ | $CH_2OC_2H_5$ | |
| 1.0627 | H | $CH_2OH$ | $C_2H_5$ | $C_2H_5$ | $CH_2OC_2H_5$ | |
| 1.0628 | H | $CH_2CH_2OH$ | $C_2H_5$ | $C_2H_5$ | $CH_2OC_2H_5$ | |
| 1.0629 | H | $CH_2CH_2CH_2OH$ | $C_2H_5$ | $C_2H_5$ | $CH_2OC_2H_5$ | |
| 1.0630 | H | $C_6H_5$ | $C_2H_5$ | $C_2H_5$ | $CH_2OC_2H_5$ | |
| 1.0631 | H | $CH_2Cl$ | $C_2H_5$ | $C_2H_5$ | $CH_2OC_2H_5$ | |
| 1.0632 | H | $CH_2CH_2Cl$ | $C_2H_5$ | $C_2H_5$ | $CH_2OC_2H_5$ | |
| 1.0633 | H | $CH_2CH_2CH_2Cl$ | $C_2H_5$ | $C_2H_5$ | $CH_2OC_2H_5$ | |

Tabelle 1.07

| No. | X | Y | $R_1$ | $R_4$ | Z | phys. Daten |
|---|---|---|---|---|---|---|
| 1.0701 | H | H | $C_2H_5$ | $C_2H_5$ | $CH(CH_3)OC_2H_5$ | |
| 1.0702 | H | $CH_3$ | $C_2H_5$ | $C_2H_5$ | $CH(CH_3)OC_2H_5$ | |
| 1.0703 | H | $C_2H_5$ | $C_2H_5$ | $C_2H_5$ | $CH(CH_3)OC_2H_5$ | |
| 1.0704 | H | $C_3H_7$ | $C_2H_5$ | $C_2H_5$ | $CH(CH_3)OC_2H_5$ | |
| 1.0705 | H | $OCH_3$ | $C_2H_5$ | $C_2H_5$ | $CH(CH_3)OC_2H_5$ | |
| 1.0706 | H | $OC_2H_5$ | $C_2H_5$ | $C_2H_5$ | $CH(CH_3)OC_2H_5$ | |
| 1.0707 | H | $OCH_2CH=CH_2$ | $C_2H_5$ | $C_2H_5$ | $CH(CH_3)OC_2H_5$ | |
| 1.0708 | H | $OCH_2CH=CHCH_3$ | $C_2H_5$ | $C_2H_5$ | $CH(CH_3)OC_2H_5$ | |
| 1.0709 | H | $OCH_2C(Cl)=CH_2$ | $C_2H_5$ | $C_2H_5$ | $CH(CH_3)OC_2H_5$ | |
| 1.0710 | H | $OCH_2CH=CHCl$ | $C_2H_5$ | $C_2H_5$ | $CH(CH_3)OC_2H_5$ | |
| 1.0711 | H | $OCH_2C\equiv CH$ | $C_2H_5$ | $C_2H_5$ | $CH(CH_3)OC_2H_5$ | |
| 1.0712 | H | $OCHF_2$ | $C_2H_5$ | $C_2H_5$ | $CH(CH_3)OC_2H_5$ | |
| 1.0713 | H | $OCF_3$ | $C_2H_5$ | $C_2H_5$ | $CH(CH_3)OC_2H_5$ | |
| 1.0714 | H | $OCF_2CHFCF_3$ | $C_2H_5$ | $C_2H_5$ | $CH(CH_3)OC_2H_5$ | |
| 1.0715 | H | $OC_6H_5$ | $C_2H_5$ | $C_2H_5$ | $CH(CH_3)OC_2H_5$ | |
| 1.0716 | H | $CF_3$ | $C_2H_5$ | $C_2H_5$ | $CH(CH_3)OC_2H_5$ | |
| 1.0717 | H | Br | $C_2H_5$ | $C_2H_5$ | $CH(CH_3)OC_2H_5$ | |
| 1.0718 | H | Cl | $C_2H_5$ | $C_2H_5$ | $CH(CH_3)OC_2H_5$ | |
| 1.0719 | H | F | $C_2H_5$ | $C_2H_5$ | $CH(CH_3)OC_2H_5$ | |
| 1.0720 | H | $SCH_3$ | $C_2H_5$ | $C_2H_5$ | $CH(CH_3)OC_2H_5$ | |
| 1.0721 | H | $SC_2H_5$ | $C_2H_5$ | $C_2H_5$ | $CH(CH_3)OC_2H_5$ | |
| 1.0722 | H | $SC_3H_7$-n | $C_2H_5$ | $C_2H_5$ | $CH(CH_3)OC_2H_5$ | |
| 1.0723 | H | $SO_2CH_3$ | $C_2H_5$ | $C_2H_5$ | $CH(CH_3)OC_2H_5$ | |
| 1.0724 | H | $SO_2C_2H_5$ | $C_2H_5$ | $C_2H_5$ | $CH(CH_3)OC_2H_5$ | |
| 1.0725 | H | $NO_2$ | $C_2H_5$ | $C_2H_5$ | $CH(CH_3)OC_2H_5$ | |
| 1.0726 | H | CN | $C_2H_5$ | $C_2H_5$ | $CH(CH_3)OC_2H_5$ | |
| 1.0727 | H | $CH_2OH$ | $C_2H_5$ | $C_2H_5$ | $CH(CH_3)OC_2H_5$ | |
| 1.0728 | H | $CH_2CH_2OH$ | $C_2H_5$ | $C_2H_5$ | $CH(CH_3)OC_2H_5$ | |
| 1.0729 | H | $CH_2CH_2CH_2OH$ | $C_2H_5$ | $C_2H_5$ | $CH(CH_3)OC_2H_5$ | |
| 1.0730 | H | $C_6H_5$ | $C_2H_5$ | $C_2H_5$ | $CH(CH_3)OC_2H_5$ | |
| 1.0731 | H | $CH_2Cl$ | $C_2H_5$ | $C_2H_5$ | $CH(CH_3)OC_2H_5$ | |
| 1.0732 | H | $CH_2CH_2Cl$ | $C_2H_5$ | $C_2H_5$ | $CH(CH_3)OC_2H_5$ | |
| 1.0733 | H | $CH_2CH_2CH_2Cl$ | $C_2H_5$ | $C_2H_5$ | $CH(CH_3)OC_2H_5$ | |

Tabelle 1.08

| No. | X | Y | $R_1$ | $R_4$ | Z | phys. Daten |
|---|---|---|---|---|---|---|
| 1.0801 | H | H | $C_2H_5$ | $C_2H_5$ | $C(CH_3)_2OCH_3$ | Oel |
| 1.0802 | H | $CH_3$ | $C_2H_5$ | $C_2H_5$ | $C(CH_3)_2OCH_3$ | |
| 1.0803 | H | $C_2H_5$ | $C_2H_5$ | $C_2H_5$ | $C(CH_3)_2OCH_3$ | |
| 1.0804 | H | $C_3H_7$ | $C_2H_5$ | $C_2H_5$ | $C(CH_3)_2OCH_3$ | |
| 1.0805 | H | $OCH_3$ | $C_2H_5$ | $C_2H_5$ | $C(CH_3)_2OCH_3$ | |
| 1.0806 | H | $OC_2H_5$ | $C_2H_5$ | $C_2H_5$ | $C(CH_3)_2OCH_3$ | |
| 1.0807 | H | $OCH_2CH=CH_2$ | $C_2H_5$ | $C_2H_5$ | $C(CH_3)_2OCH_3$ | |
| 1.0808 | H | $OCH_2CH=CHCH_3$ | $C_2H_5$ | $C_2H_5$ | $C(CH_3)_2OCH_3$ | |
| 1.0809 | H | $OCH_2C(Cl)=CH_2$ | $C_2H_5$ | $C_2H_5$ | $C(CH_3)_2OCH_3$ | |
| 1.0810 | H | $OCH_2CH=CHCl$ | $C_2H_5$ | $C_2H_5$ | $C(CH_3)_2OCH_3$ | |
| 1.0811 | H | $OCH_2C{\equiv}CH$ | $C_2H_5$ | $C_2H_5$ | $C(CH_3)_2OCH_3$ | |
| 1.0812 | H | $OCHF_2$ | $C_2H_5$ | $C_2H_5$ | $C(CH_3)_2OCH_3$ | |
| 1.0813 | H | $OCF_3$ | $C_2H_5$ | $C_2H_5$ | $C(CH_3)_2OCH_3$ | |
| 1.0814 | H | $OCF_2CHFCF_3$ | $C_2H_5$ | $C_2H_5$ | $C(CH_3)_2OCH_3$ | |
| 1.0815 | H | $OC_6H_5$ | $C_2H_5$ | $C_2H_5$ | $C(CH_3)_2OCH_3$ | |
| 1.0816 | H | $CF_3$ | $C_2H_5$ | $C_2H_5$ | $C(CH_3)_2OCH_3$ | |
| 1.0817 | H | Br | $C_2H_5$ | $C_2H_5$ | $C(CH_3)_2OCH_3$ | |
| 1.0818 | H | Cl | $C_2H_5$ | $C_2H_5$ | $C(CH_3)_2OCH_3$ | |
| 1.0819 | H | F | $C_2H_5$ | $C_2H_5$ | $C(CH_3)_2OCH_3$ | |
| 1.0820 | H | $SCH_3$ | $C_2H_5$ | $C_2H_5$ | $C(CH_3)_2OCH_3$ | |
| 1.0821 | H | $SC_2H_5$ | $C_2H_5$ | $C_2H_5$ | $C(CH_3)_2OCH_3$ | |
| 1.0822 | H | $SC_3H_7$-n | $C_2H_5$ | $C_2H_5$ | $C(CH_3)_2OCH_3$ | |
| 1.0823 | H | $SO_2CH_3$ | $C_2H_5$ | $C_2H_5$ | $C(CH_3)_2OCH_3$ | |
| 1.0824 | H | $SO_2C_2H_5$ | $C_2H_5$ | $C_2H_5$ | $C(CH_3)_2OCH_3$ | |
| 1.0825 | H | $NO_2$ | $C_2H_5$ | $C_2H_5$ | $C(CH_3)_2OCH_3$ | |
| 1.0826 | H | CN | $C_2H_5$ | $C_2H_5$ | $C(CH_3)_2OCH_3$ | |
| 1.0827 | H | $CH_2OH$ | $C_2H_5$ | $C_2H_5$ | $C(CH_3)_2OCH_3$ | |
| 1.0828 | H | $CH_2CH_2OH$ | $C_2H_5$ | $C_2H_5$ | $C(CH_3)_2OCH_3$ | |
| 1.0829 | H | $CH_2CH_2CH_2OH$ | $C_2H_5$ | $C_2H_5$ | $C(CH_3)_2OCH_3$ | |
| 1.0830 | H | $C_6H_5$ | $C_2H_5$ | $C_2H_5$ | $C(CH_3)_2OCH_3$ | |
| 1.0831 | H | $CH_2Cl$ | $C_2H_5$ | $C_2H_5$ | $C(CH_3)_2OCH_3$ | |
| 1.0832 | H | $CH_2CH_2Cl$ | $C_2H_5$ | $C_2H_5$ | $C(CH_3)_2OCH_3$ | |
| 1.0833 | H | $CH_2CH_2CH_2Cl$ | $C_2H_5$ | $C_2H_5$ | $C(CH_3)_2OCH_3$ | |

Tabelle 1.09

| No. | X | Y | $R_1$ | $R_4$ | Z | phys. Daten |
|---|---|---|---|---|---|---|
| 1.0901 | H | H | $C_2H_5$ | $C_2H_5$ | $CH_2OC_3H_7$ | |
| 1.0902 | H | $CH_3$ | $C_2H_5$ | $C_2H_5$ | $CH_2OC_3H_7$ | |
| 1.0903 | H | $C_2H_5$ | $C_2H_5$ | $C_2H_5$ | $CH_2OC_3H_7$ | |
| 1.0904 | H | $C_3H_7$ | $C_2H_5$ | $C_2H_5$ | $CH_2OC_3H_7$ | |
| 1.0905 | H | $OCH_3$ | $C_2H_5$ | $C_2H_5$ | $CH_2OC_3H_7$ | |
| 1.0906 | H | $OC_2H_5$ | $C_2H_5$ | $C_2H_5$ | $CH_2OC_3H_7$ | |
| 1.0907 | H | $OCH_2CH{=}CH_2$ | $C_2H_5$ | $C_2H_5$ | $CH_2OC_3H_7$ | |
| 1.0908 | H | $OCH_2CH{=}CHCH_3$ | $C_2H_5$ | $C_2H_5$ | $CH_2OC_3H_7$ | |
| 1.0909 | H | $OCH_2C(Cl){=}CH_2$ | $C_2H_5$ | $C_2H_5$ | $CH_2OC_3H_7$ | |
| 1.0910 | H | $OCH_2CH{=}CHCl$ | $C_2H_5$ | $C_2H_5$ | $CH_2OC_3H_7$ | |
| 1.0911 | H | $OCH_2C{\equiv}CH$ | $C_2H_5$ | $C_2H_5$ | $CH_2OC_3H_7$ | |
| 1.0912 | H | $OCHF_2$ | $C_2H_5$ | $C_2H_5$ | $CH_2OC_3H_7$ | |
| 1.0913 | H | $OCF_3$ | $C_2H_5$ | $C_2H_5$ | $CH_2OC_3H_7$ | |
| 1.0914 | H | $OCF_2CHFCF_3$ | $C_2H_5$ | $C_2H_5$ | $CH_2OC_3H_7$ | |
| 1.0915 | H | $OC_6H_5$ | $C_2H_5$ | $C_2H_5$ | $CH_2OC_3H_7$ | |
| 1.0916 | H | $CF_3$ | $C_2H_5$ | $C_2H_5$ | $CH_2OC_3H_7$ | |
| 1.0917 | H | Br | $C_2H_5$ | $C_2H_5$ | $CH_2OC_3H_7$ | |
| 1.0918 | H | Cl | $C_2H_5$ | $C_2H_5$ | $CH_2OC_3H_7$ | |
| 1.0919 | H | F | $C_2H_5$ | $C_2H_5$ | $CH_2OC_3H_7$ | |
| 1.0920 | H | $SCH_3$ | $C_2H_5$ | $C_2H_5$ | $CH_2OC_3H_7$ | |
| 1.0921 | H | $SC_2H_5$ | $C_2H_5$ | $C_2H_5$ | $CH_2OC_3H_7$ | |
| 1.0922 | H | $SC_3H_7$-n | $C_2H_5$ | $C_2H_5$ | $CH_2OC_3H_7$ | |
| 1.0923 | H | $SO_2CH_3$ | $C_2H_5$ | $C_2H_5$ | $CH_2OC_3H_7$ | |
| 1.0924 | H | $SO_2C_2H_5$ | $C_2H_5$ | $C_2H_5$ | $CH_2OC_3H_7$ | |
| 1.0925 | H | $NO_2$ | $C_2H_5$ | $C_2H_5$ | $CH_2OC_3H_7$ | |
| 1.0926 | H | CN | $C_2H_5$ | $C_2H_5$ | $CH_2OC_3H_7$ | |
| 1.0927 | H | $CH_2OH$ | $C_2H_5$ | $C_2H_5$ | $CH_2OC_3H_7$ | |
| 1.0928 | H | $CH_2CH_2OH$ | $C_2H_5$ | $C_2H_5$ | $CH_2OC_3H_7$ | |
| 1.0929 | H | $CH_2CH_2CH_2OH$ | $C_2H_5$ | $C_2H_5$ | $CH_2OC_3H_7$ | |
| 1.0930 | H | $C_6H_5$ | $C_2H_5$ | $C_2H_5$ | $CH_2OC_3H_7$ | |
| 1.0931 | H | $CH_2Cl$ | $C_2H_5$ | $C_2H_5$ | $CH_2OC_3H_7$ | |
| 1.0932 | H | $CH_2CH_2Cl$ | $C_2H_5$ | $C_2H_5$ | $CH_2OC_3H_7$ | |
| 1.0933 | H | $CH_2CH_2CH_2Cl$ | $C_2H_5$ | $C_2H_5$ | $CH_2OC_3H_7$ | |

Tabelle 1.10

| No. | X | Y | $R_1$ | $R_4$ | Z | phys. Daten |
|---|---|---|---|---|---|---|
| 1.1001 | H | H | $C_2H_5$ | $C_2H_5$ | $C(CH_3)C_2H_4$(Cyclo) | Oel |
| 1.1002 | H | $CH_3$ | $C_2H_5$ | $C_2H_5$ | $C(CH_3)C_2H_4$(Cyclo) | |
| 1.1003 | H | $C_2H_5$ | $C_2H_5$ | $C_2H_5$ | $C(CH_3)C_2H_4$(Cyclo) | |
| 1.1004 | H | $C_3H_7$ | $C_2H_5$ | $C_2H_5$ | $C(CH_3)C_2H_4$(Cyclo) | |
| 1.1005 | H | $OCH_3$ | $C_2H_5$ | $C_2H_5$ | $C(CH_3)C_2H_4$(Cyclo) | |
| 1.1006 | H | $OC_2H_5$ | $C_2H_5$ | $C_2H_5$ | $C(CH_3)C_2H_4$(Cyclo) | |
| 1.1007 | H | $OCH_2CH{=}CH_2$ | $C_2H_5$ | $C_2H_5$ | $C(CH_3)C_2H_4$(Cyclo) | |
| 1.1008 | H | $OCH_2CH{=}CHCH_3$ | $C_2H_5$ | $C_2H_5$ | $C(CH_3)C_2H_4$(Cyclo) | |
| 1.1009 | H | $OCH_2C(Cl){=}CH_2$ | $C_2H_5$ | $C_2H_5$ | $C(CH_3)C_2H_4$(Cyclo) | |
| 1.1010 | H | $OCH_2CH{=}CHCl$ | $C_2H_5$ | $C_2H_5$ | $C(CH_3)C_2H_4$(Cyclo) | |
| 1.1011 | H | $OCH_2C{\equiv}CH$ | $C_2H_5$ | $C_2H_5$ | $C(CH_3)C_2H_4$(Cyclo) | |
| 1.1012 | H | $OCHF_2$ | $C_2H_5$ | $C_2H_5$ | $C(CH_3)C_2H_4$(Cyclo) | |
| 1.1013 | H | $OCF_3$ | $C_2H_5$ | $C_2H_5$ | $C(CH_3)C_2H_4$(Cyclo) | |
| 1.1014 | H | $OCF_2CHFCF_3$ | $C_2H_5$ | $C_2H_5$ | $C(CH_3)C_2H_4$(Cyclo) | |
| 1.1015 | H | $OC_6H_5$ | $C_2H_5$ | $C_2H_5$ | $C(CH_3)C_2H_4$(Cyclo) | |
| 1.1016 | H | $CF_3$ | $C_2H_5$ | $C_2H_5$ | $C(CH_3)C_2H_4$(Cyclo) | |
| 1.1017 | H | Br | $C_2H_5$ | $C_2H_5$ | $C(CH_3)C_2H_4$(Cyclo) | |
| 1.1018 | H | Cl | $C_2H_5$ | $C_2H_5$ | $C(CH_3)C_2H_4$(Cyclo) | |
| 1.1019 | H | F | $C_2H_5$ | $C_2H_5$ | $C(CH_3)C_2H_4$(Cyclo) | |
| 1.1020 | H | $SCH_3$ | $C_2H_5$ | $C_2H_5$ | $C(CH_3)C_2H_4$(Cyclo) | |
| 1.1021 | H | $SC_2H_5$ | $C_2H_5$ | $C_2H_5$ | $C(CH_3)C_2H_4$(Cyclo) | |
| 1.1022 | H | $SC_3H_7$-n | $C_2H_5$ | $C_2H_5$ | $C(CH_3)C_2H_4$(Cyclo) | |
| 1.1023 | H | $SO_2CH_3$ | $C_2H_5$ | $C_2H_5$ | $C(CH_3)C_2H_4$(Cyclo) | |
| 1.1024 | H | $SO_2C_2H_5$ | $C_2H_5$ | $C_2H_5$ | $C(CH_3)C_2H_4$(Cyclo) | |
| 1.1025 | H | $NO_2$ | $C_2H_5$ | $C_2H_5$ | $C(CH_3)C_2H_4$(Cyclo) | |
| 1.1026 | H | CN | $C_2H_5$ | $C_2H_5$ | $C(CH_3)C_2H_4$(Cyclo) | |
| 1.1027 | H | $CH_2OH$ | $C_2H_5$ | $C_2H_5$ | $C(CH_3)C_2H_4$(Cyclo) | |
| 1.1028 | H | $CH_2CH_2OH$ | $C_2H_5$ | $C_2H_5$ | $C(CH_3)C_2H_4$(Cyclo) | |
| 1.1029 | H | $CH_2CH_2CH_2OH$ | $C_2H_5$ | $C_2H_5$ | $C(CH_3)C_2H_4$(Cyclo) | |
| 1.1030 | H | $C_6H_5$ | $C_2H_5$ | $C_2H_5$ | $C(CH_3)C_2H_4$(Cyclo) | |
| 1.1031 | H | $CH_2Cl$ | $C_2H_5$ | $C_2H_5$ | $C(CH_3)C_2H_4$(Cyclo) | |
| 1.1032 | H | $CH_2CH_2Cl$ | $C_2H_5$ | $C_2H_5$ | $C(CH_3)C_2H_4$(Cyclo) | |
| 1.1033 | H | $CH_2CH_2CH_2Cl$ | $C_2H_5$ | $C_2H_5$ | $C(CH_3)C_2H_4$(Cyclo) | |

Tabelle 1.11

| No. | X | Y | $R_1$ | $R_4$ | Z | phys. Daten |
|---|---|---|---|---|---|---|
| 1.1101 | H | H | $C_2H_5$ | $C_2H_5$ | $CH_2OC_6H_5$ | Smp. 78-79°C |
| 1.1102 | H | $CH_3$ | $C_2H_5$ | $C_2H_5$ | $CH_2OC_6H_5$ | |
| 1.1103 | H | $C_2H_5$ | $C_2H_5$ | $C_2H_5$ | $CH_2OC_6H_5$ | |
| 1.1104 | H | $C_3H_7$ | $C_2H_5$ | $C_2H_5$ | $CH_2OC_6H_5$ | |
| 1.1105 | H | $OCH_3$ | $C_2H_5$ | $C_2H_5$ | $CH_2OC_6H_5$ | |
| 1.1106 | H | $OC_2H_5$ | $C_2H_5$ | $C_2H_5$ | $CH_2OC_6H_5$ | |
| 1.1107 | H | $OCH_2CH=CH_2$ | $C_2H_5$ | $C_2H_5$ | $CH_2OC_6H_5$ | |
| 1.1108 | H | $OCH_2CH=CHCH_3$ | $C_2H_5$ | $C_2H_5$ | $CH_2OC_6H_5$ | |
| 1.1109 | H | $OCH_2C(Cl)=CH_2$ | $C_2H_5$ | $C_2H_5$ | $CH_2OC_6H_5$ | |
| 1.1110 | H | $OCH_2CH=CHCl$ | $C_2H_5$ | $C_2H_5$ | $CH_2OC_6H_5$ | |
| 1.1111 | H | $OCH_2C{\equiv}CH$ | $C_2H_5$ | $C_2H_5$ | $CH_2OC_6H_5$ | |
| 1.1112 | H | $OCHF_2$ | $C_2H_5$ | $C_2H_5$ | $CH_2OC_6H_5$ | |
| 1.1113 | H | $OCF_3$ | $C_2H_5$ | $C_2H_5$ | $CH_2OC_6H_5$ | |
| 1.1114 | H | $OCF_2CHFCF_3$ | $C_2H_5$ | $C_2H_5$ | $CH_2OC_6H_5$ | |
| 1.1115 | H | $OC_6H_5$ | $C_2H_5$ | $C_2H_5$ | $CH_2OC_6H_5$ | |
| 1.1116 | H | $CF_3$ | $C_2H_5$ | $C_2H_5$ | $CH_2OC_6H_5$ | |
| 1.1117 | H | Br | $C_2H_5$ | $C_2H_5$ | $CH_2OC_6H_5$ | |
| 1.1118 | H | Cl | $C_2H_5$ | $C_2H_5$ | $CH_2OC_6H_5$ | |
| 1.1119 | H | F | $C_2H_5$ | $C_2H_5$ | $CH_2OC_6H_5$ | |
| 1.1120 | H | $SCH_3$ | $C_2H_5$ | $C_2H_5$ | $CH_2OC_6H_5$ | |
| 1.1121 | H | $SC_2H_5$ | $C_2H_5$ | $C_2H_5$ | $CH_2OC_6H_5$ | |
| 1.1122 | H | $SC_3H_7$-n | $C_2H_5$ | $C_2H_5$ | $CH_2OC_6H_5$ | |
| 1.1123 | H | $SO_2CH_3$ | $C_2H_5$ | $C_2H_5$ | $CH_2OC_6H_5$ | |
| 1.1124 | H | $SO_2C_2H_5$ | $C_2H_5$ | $C_2H_5$ | $CH_2OC_6H_5$ | |
| 1.1125 | H | $NO_2$ | $C_2H_5$ | $C_2H_5$ | $CH_2OC_6H_5$ | |
| 1.1126 | H | CN | $C_2H_5$ | $C_2H_5$ | $CH_2OC_6H_5$ | |
| 1.1127 | H | $CH_2OH$ | $C_2H_5$ | $C_2H_5$ | $CH_2OC_6H_5$ | |
| 1.1128 | H | $CH_2CH_2OH$ | $C_2H_5$ | $C_2H_5$ | $CH_2OC_6H_5$ | |
| 1.1129 | H | $CH_2CH_2CH_2OH$ | $C_2H_5$ | $C_2H_5$ | $CH_2OC_6H_5$ | |
| 1.1130 | H | $C_6H_5$ | $C_2H_5$ | $C_2H_5$ | $CH_2OC_6H_5$ | |
| 1.1131 | H | $CH_2Cl$ | $C_2H_5$ | $C_2H_5$ | $CH_2OC_6H_5$ | |
| 1.1132 | H | $CH_2CH_2Cl$ | $C_2H_5$ | $C_2H_5$ | $CH_2OC_6H_5$ | |
| 1.1133 | H | $CH_2CH_2CH_2Cl$ | $C_2H_5$ | $C_2H_5$ | $CH_2OC_6H_5$ | |

Tabelle 1.12

| No. | X | Y | $R_1$ | $R_4$ | Z | phys. Daten |
|---|---|---|---|---|---|---|
| 1.1201 | H | H | $C_2H_5$ | $C_2H_5$ | Furan-2-yl | Oel |
| 1.1202 | H | $CH_3$ | $C_2H_5$ | $C_2H_5$ | Furan-2-yl | |
| 1.1203 | H | $C_2H_5$ | $C_2H_5$ | $C_2H_5$ | Furan-2-yl | |
| 1.1204 | H | $C_3H_7$ | $C_2H_5$ | $C_2H_5$ | Furan-2-yl | |
| 1.1205 | H | $OCH_3$ | $C_2H_5$ | $C_2H_5$ | Furan-2-yl | |
| 1.1206 | H | $OC_2H_5$ | $C_2H_5$ | $C_2H_5$ | Furan-2-yl | |
| 1.1207 | H | $OCH_2CH=CH_2$ | $C_2H_5$ | $C_2H_5$ | Furan-2-yl | |
| 1.1208 | H | $OCH_2CH=CHCH_3$ | $C_2H_5$ | $C_2H_5$ | Furan-2-yl | |
| 1.1209 | H | $OCH_2C(Cl)=CH_2$ | $C_2H_5$ | $C_2H_5$ | Furan-2-yl | |
| 1.1210 | H | $OCH_2CH=CHCl$ | $C_2H_5$ | $C_2H_5$ | Furan-2-yl | |
| 1.1211 | H | $OCH_2C\equiv CH$ | $C_2H_5$ | $C_2H_5$ | Furan-2-yl | |
| 1.1212 | H | $OCHF_2$ | $C_2H_5$ | $C_2H_5$ | Furan-2-yl | |
| 1.1213 | H | $OCF_3$ | $C_2H_5$ | $C_2H_5$ | Furan-2-yl | |
| 1.1214 | H | $OCF_2CHFCF_3$ | $C_2H_5$ | $C_2H_5$ | Furan-2-yl | |
| 1.1215 | H | $OC_6H_5$ | $C_2H_5$ | $C_2H_5$ | Furan-2-yl | |
| 1.1216 | H | $CF_3$ | $C_2H_5$ | $C_2H_5$ | Furan-2-yl | |
| 1.1217 | H | Br | $C_2H_5$ | $C_2H_5$ | Furan-2-yl | |
| 1.1218 | H | Cl | $C_2H_5$ | $C_2H_5$ | Furan-2-yl | |
| 1.1219 | H | F | $C_2H_5$ | $C_2H_5$ | Furan-2-yl | |
| 1.1220 | H | $SCH_3$ | $C_2H_5$ | $C_2H_5$ | Furan-2-yl | |
| 1.1221 | H | $SC_2H_5$ | $C_2H_5$ | $C_2H_5$ | Furan-2-yl | |
| 1.1222 | H | $SC_3H_7$-n | $C_2H_5$ | $C_2H_5$ | Furan-2-yl | |
| 1.1223 | H | $SO_2CH_3$ | $C_2H_5$ | $C_2H_5$ | Furan-2-yl | |
| 1.1224 | H | $SO_2C_2H_5$ | $C_2H_5$ | $C_2H_5$ | Furan-2-yl | |
| 1.1225 | H | $NO_2$ | $C_2H_5$ | $C_2H_5$ | Furan-2-yl | |
| 1.1226 | H | CN | $C_2H_5$ | $C_2H_5$ | Furan-2-yl | |
| 1.1227 | H | $CH_2OH$ | $C_2H_5$ | $C_2H_5$ | Furan-2-yl | |
| 1.1228 | H | $CH_2CH_2OH$ | $C_2H_5$ | $C_2H_5$ | Furan-2-yl | |
| 1.1229 | H | $CH_2CH_2CH_2OH$ | $C_2H_5$ | $C_2H_5$ | Furan-2-yl | |
| 1.1230 | H | $C_6H_5$ | $C_2H_5$ | $C_2H_5$ | Furan-2-yl | |
| 1.1231 | H | $CH_2Cl$ | $C_2H_5$ | $C_2H_5$ | Furan-2-yl | |
| 1.1232 | H | $CH_2CH_2Cl$ | $C_2H_5$ | $C_2H_5$ | Furan-2-yl | |
| 1.1233 | H | $CH_2CH_2CH_2Cl$ | $C_2H_5$ | $C_2H_5$ | Furan-2-yl | |

Tabelle 1.13

| No. | X | Y | $R_1$ | $R_4$ | Z | phys. Daten |
|---|---|---|---|---|---|---|
| 1.1301 | H | H | $C_2H_5$ | $C_2H_5$ | Dioxan-2-yl | Oel |
| 1.1302 | H | $CH_3$ | $C_2H_5$ | $C_2H_5$ | Dioxan-2-yl | |
| 1.1303 | H | $C_2H_5$ | $C_2H_5$ | $C_2H_5$ | Dioxan-2-yl | |
| 1.1304 | H | $C_3H_7$ | $C_2H_5$ | $C_2H_5$ | Dioxan-2-yl | |
| 1.1305 | H | $OCH_3$ | $C_2H_5$ | $C_2H_5$ | Dioxan-2-yl | |
| 1.1306 | H | $OC_2H_5$ | $C_2H_5$ | $C_2H_5$ | Dioxan-2-yl | |
| 1.1307 | H | $OCH_2CH=CH_2$ | $C_2H_5$ | $C_2H_5$ | Dioxan-2-yl | |
| 1.1308 | H | $OCH_2CH=CHCH_3$ | $C_2H_5$ | $C_2H_5$ | Dioxan-2-yl | |
| 1.1309 | H | $OCH_2C(Cl)=CH_2$ | $C_2H_5$ | $C_2H_5$ | Dioxan-2-yl | |
| 1.1310 | H | $OCH_2CH=CHCl$ | $C_2H_5$ | $C_2H_5$ | Dioxan-2-yl | |
| 1.1311 | H | $OCH_2C\equiv CH$ | $C_2H_5$ | $C_2H_5$ | Dioxan-2-yl | |
| 1.1312 | H | $OCHF_2$ | $C_2H_5$ | $C_2H_5$ | Dioxan-2-yl | |
| 1.1313 | H | $OCF_3$ | $C_2H_5$ | $C_2H_5$ | Dioxan-2-yl | |
| 1.1314 | H | $OCF_2CHFCF_3$ | $C_2H_5$ | $C_2H_5$ | Dioxan-2-yl | |
| 1.1315 | H | $OC_6H_5$ | $C_2H_5$ | $C_2H_5$ | Dioxan-2-yl | |
| 1.1316 | H | $CF_3$ | $C_2H_5$ | $C_2H_5$ | Dioxan-2-yl | |
| 1.1317 | H | Br | $C_2H_5$ | $C_2H_5$ | Dioxan-2-yl | |
| 1.1318 | H | Cl | $C_2H_5$ | $C_2H_5$ | Dioxan-2-yl | |
| 1.1319 | H | F | $C_2H_5$ | $C_2H_5$ | Dioxan-2-yl | |
| 1.1320 | H | $SCH_3$ | $C_2H_5$ | $C_2H_5$ | Dioxan-2-yl | |
| 1.1321 | H | $SC_2H_5$ | $C_2H_5$ | $C_2H_5$ | Dioxan-2-yl | |
| 1.1322 | H | $SC_3H_7$-n | $C_2H_5$ | $C_2H_5$ | Dioxan-2-yl | |
| 1.1323 | H | $SO_2CH_3$ | $C_2H_5$ | $C_2H_5$ | Dioxan-2-yl | |
| 1.1324 | H | $SO_2C_2H_5$ | $C_2H_5$ | $C_2H_5$ | Dioxan-2-yl | |
| 1.1325 | H | $NO_2$ | $C_2H_5$ | $C_2H_5$ | Dioxan-2-yl | |
| 1.1326 | H | CN | $C_2H_5$ | $C_2H_5$ | Dioxan-2-yl | |
| 1.1327 | H | $CH_2OH$ | $C_2H_5$ | $C_2H_5$ | Dioxan-2-yl | |
| 1.1328 | H | $CH_2CH_2OH$ | $C_2H_5$ | $C_2H_5$ | Dioxan-2-yl | |
| 1.1329 | H | $CH_2CH_2CH_2OH$ | $C_2H_5$ | $C_2H_5$ | Dioxan-2-yl | |
| 1.1330 | H | $C_6H_5$ | $C_2H_5$ | $C_2H_5$ | Dioxan-2-yl | |
| 1.1331 | H | $CH_2Cl$ | $C_2H_5$ | $C_2H_5$ | Dioxan-2-yl | |
| 1.1332 | H | $CH_2CH_2Cl$ | $C_2H_5$ | $C_2H_5$ | Dioxan-2-yl | |
| 1.1333 | H | $CH_2CH_2CH_2Cl$ | $C_2H_5$ | $C_2H_5$ | Dioxan-2-yl | |

Tabelle 1.14

| No. | X | Y | $R_1$ | $R_4$ | Z | phys. Daten |
|---|---|---|---|---|---|---|
| 1.1401 | H | H | $C_2H_5$ | $C_2H_5$ | $CH_2OC_2H_4OCH_3$ | Oel |
| 1.1402 | H | $CH_3$ | $C_2H_5$ | $C_2H_5$ | $CH_2OC_2H_4OCH_3$ | |
| 1.1403 | H | $C_2H_5$ | $C_2H_5$ | $C_2H_5$ | $CH_2OC_2H_4OCH_3$ | |
| 1.1404 | H | $C_3H_7$ | $C_2H_5$ | $C_2H_5$ | $CH_2OC_2H_4OCH_3$ | |
| 1.1405 | H | $OCH_3$ | $C_2H_5$ | $C_2H_5$ | $CH_2OC_2H_4OCH_3$ | |
| 1.1406 | H | $OC_2H_5$ | $C_2H_5$ | $C_2H_5$ | $CH_2OC_2H_4OCH_3$ | |
| 1.1407 | H | $OCH_2CH{=}CH_2$ | $C_2H_5$ | $C_2H_5$ | $CH_2OC_2H_4OCH_3$ | |
| 1.1408 | H | $OCH_2CH{=}CHCH_3$ | $C_2H_5$ | $C_2H_5$ | $CH_2OC_2H_4OCH_3$ | |
| 1.1409 | H | $OCH_2C(Cl){=}CH_2$ | $C_2H_5$ | $C_2H_5$ | $CH_2OC_2H_4OCH_3$ | |
| 1.1410 | H | $OCH_2CH{=}CHCl$ | $C_2H_5$ | $C_2H_5$ | $CH_2OC_2H_4OCH_3$ | |
| 1.1411 | H | $OCH_2C{\equiv}CH$ | $C_2H_5$ | $C_2H_5$ | $CH_2OC_2H_4OCH_3$ | |
| 1.1412 | H | $OCHF_2$ | $C_2H_5$ | $C_2H_5$ | $CH_2OC_2H_4OCH_3$ | |
| 1.1413 | H | $OCF_3$ | $C_2H_5$ | $C_2H_5$ | $CH_2OC_2H_4OCH_3$ | |
| 1.1414 | H | $OCF_2CHFCF_3$ | $C_2H_5$ | $C_2H_5$ | $CH_2OC_2H_4OCH_3$ | |
| 1.1415 | H | $OC_6H_5$ | $C_2H_5$ | $C_2H_5$ | $CH_2OC_2H_4OCH_3$ | |
| 1.1416 | H | $CF_3$ | $C_2H_5$ | $C_2H_5$ | $CH_2OC_2H_4OCH_3$ | |
| 1.1417 | H | Br | $C_2H_5$ | $C_2H_5$ | $CH_2OC_2H_4OCH_3$ | |
| 1.1418 | H | Cl | $C_2H_5$ | $C_2H_5$ | $CH_2OC_2H_4OCH_3$ | |
| 1.1419 | H | F | $C_2H_5$ | $C_2H_5$ | $CH_2OC_2H_4OCH_3$ | |
| 1.1420 | H | $SCH_3$ | $C_2H_5$ | $C_2H_5$ | $CH_2OC_2H_4OCH_3$ | |
| 1.1421 | H | $SC_2H_5$ | $C_2H_5$ | $C_2H_5$ | $CH_2OC_2H_4OCH_3$ | |
| 1.1422 | H | $SC_3H_7$-n | $C_2H_5$ | $C_2H_5$ | $CH_2OC_2H_4OCH_3$ | |
| 1.1423 | H | $SO_2CH_3$ | $C_2H_5$ | $C_2H_5$ | $CH_2OC_2H_4OCH_3$ | |
| 1.1424 | H | $SO_2C_2H_5$ | $C_2H_5$ | $C_2H_5$ | $CH_2OC_2H_4OCH_3$ | |
| 1.1425 | H | $NO_2$ | $C_2H_5$ | $C_2H_5$ | $CH_2OC_2H_4OCH_3$ | |
| 1.1426 | H | CN | $C_2H_5$ | $C_2H_5$ | $CH_2OC_2H_4OCH_3$ | |
| 1.1427 | H | $CH_2OH$ | $C_2H_5$ | $C_2H_5$ | $CH_2OC_2H_4OCH_3$ | |
| 1.1428 | H | $CH_2CH_2OH$ | $C_2H_5$ | $C_2H_5$ | $CH_2OC_2H_4OCH_3$ | |
| 1.1429 | H | $CH_2CH_2CH_2OH$ | $C_2H_5$ | $C_2H_5$ | $CH_2OC_2H_4OCH_3$ | |
| 1.1430 | H | $C_6H_5$ | $C_2H_5$ | $C_2H_5$ | $CH_2OC_2H_4OCH_3$ | |
| 1.1431 | H | $CH_2Cl$ | $C_2H_5$ | $C_2H_5$ | $CH_2OC_2H_4OCH_3$ | |
| 1.1432 | H | $CH_2CH_2Cl$ | $C_2H_5$ | $C_2H_5$ | $CH_2OC_2H_4OCH_3$ | |
| 1.1433 | H | $CH_2CH_2CH_2Cl$ | $C_2H_5$ | $C_2H_5$ | $CH_2OC_2H_4OCH_3$ | |

Tabelle 1.15

| No. | X | Y | $R_1$ | $R_4$ | Z | phys. Daten |
|---|---|---|---|---|---|---|
| 1.1501 | H | H | $C_2H_5$ | $C_2H_5$ | $CH_2OC_2H_4OC_2H_4OCH_3$ | Oel |
| 1.1502 | H | $CH_3$ | $C_2H_5$ | $C_2H_5$ | $CH_2OC_2H_4OC_2H_4OCH_3$ | |
| 1.1503 | H | $C_2H_5$ | $C_2H_5$ | $C_2H_5$ | $CH_2OC_2H_4OC_2H_4OCH_3$ | |
| 1.1504 | H | $C_3H_7$ | $C_2H_5$ | $C_2H_5$ | $CH_2OC_2H_4OC_2H_4OCH_3$ | |
| 1.1505 | H | $OCH_3$ | $C_2H_5$ | $C_2H_5$ | $CH_2OC_2H_4OC_2H_4OCH_3$ | |
| 1.1506 | H | $OC_2H_5$ | $C_2H_5$ | $C_2H_5$ | $CH_2OC_2H_4OC_2H_4OCH_3$ | |
| 1.1507 | H | $OCH_2CH=CH_2$ | $C_2H_5$ | $C_2H_5$ | $CH_2OC_2H_4OC_2H_4OCH_3$ | |
| 1.1508 | H | $OCH_2CH=CHCH_3$ | $C_2H_5$ | $C_2H_5$ | $CH_2OC_2H_4OC_2H_4OCH_3$ | |
| 1.1509 | H | $OCH_2C(Cl)=CH_2$ | $C_2H_5$ | $C_2H_5$ | $CH_2OC_2H_4OC_2H_4OCH_3$ | |
| 1.1510 | H | $OCH_2CH=CHCl$ | $C_2H_5$ | $C_2H_5$ | $CH_2OC_2H_4OC_2H_4OCH_3$ | |
| 1.1511 | H | $OCH_2C\equiv CH$ | $C_2H_5$ | $C_2H_5$ | $CH_2OC_2H_4OC_2H_4OCH_3$ | |
| 1.1512 | H | $OCHF_2$ | $C_2H_5$ | $C_2H_5$ | $CH_2OC_2H_4OC_2H_4OCH_3$ | |
| 1.1513 | H | $OCF_3$ | $C_2H_5$ | $C_2H_5$ | $CH_2OC_2H_4OC_2H_4OCH_3$ | |
| 1.1514 | H | $OCF_2CHFCF_3$ | $C_2H_5$ | $C_2H_5$ | $CH_2OC_2H_4OC_2H_4OCH_3$ | |
| 1.1515 | H | $OC_6H_5$ | $C_2H_5$ | $C_2H_5$ | $CH_2OC_2H_4OC_2H_4OCH_3$ | |
| 1.1516 | H | $CF_3$ | $C_2H_5$ | $C_2H_5$ | $CH_2OC_2H_4OC_2H_4OCH_3$ | |
| 1.1517 | H | Br | $C_2H_5$ | $C_2H_5$ | $CH_2OC_2H_4OC_2H_4OCH_3$ | |
| 1.1518 | H | Cl | $C_2H_5$ | $C_2H_5$ | $CH_2OC_2H_4OC_2H_4OCH_3$ | |
| 1.1519 | H | F | $C_2H_5$ | $C_2H_5$ | $CH_2OC_2H_4OC_2H_4OCH_3$ | |
| 1.1520 | H | $SCH_3$ | $C_2H_5$ | $C_2H_5$ | $CH_2OC_2H_4OC_2H_4OCH_3$ | |
| 1.1521 | H | $SC_2H_5$ | $C_2H_5$ | $C_2H_5$ | $CH_2OC_2H_4OC_2H_4OCH_3$ | |
| 1.1522 | H | $SC_3H_7$-n | $C_2H_5$ | $C_2H_5$ | $CH_2OC_2H_4OC_2H_4OCH_3$ | |
| 1.1523 | H | $SO_2CH_3$ | $C_2H_5$ | $C_2H_5$ | $CH_2OC_2H_4OC_2H_4OCH_3$ | |
| 1.1524 | H | $SO_2C_2H_5$ | $C_2H_5$ | $C_2H_5$ | $CH_2OC_2H_4OC_2H_4OCH_3$ | |
| 1.1525 | H | $NO_2$ | $C_2H_5$ | $C_2H_5$ | $CH_2OC_2H_4OC_2H_4OCH_3$ | |
| 1.1526 | H | CN | $C_2H_5$ | $C_2H_5$ | $CH_2OC_2H_4OC_2H_4OCH_3$ | |
| 1.1527 | H | $CH_2OH$ | $C_2H_5$ | $C_2H_5$ | $CH_2OC_2H_4OC_2H_4OCH_3$ | |
| 1.1528 | H | $CH_2CH_2OH$ | $C_2H_5$ | $C_2H_5$ | $CH_2OC_2H_4OC_2H_4OCH_3$ | |
| 1.1529 | H | $CH_2CH_2CH_2OH$ | $C_2H_5$ | $C_2H_5$ | $CH_2OC_2H_4OC_2H_4OCH_3$ | |
| 1.1530 | H | $C_6H_5$ | $C_2H_5$ | $C_2H_5$ | $CH_2OC_2H_4OC_2H_4OCH_3$ | |
| 1.1531 | H | $CH_2Cl$ | $C_2H_5$ | $C_2H_5$ | $CH_2OC_2H_4OC_2H_4OCH_3$ | |
| 1.1532 | H | $CH_2CH_2Cl$ | $C_2H_5$ | $C_2H_5$ | $CH_2OC_2H_4OC_2H_4OCH_3$ | |
| 1.1533 | H | $CH_2CH_2CH_2Cl$ | $C_2H_5$ | $C_2H_5$ | $CH_2OC_2H_4OC_2H_4OCH_3$ | |

Tabelle 1.15

| No. | X | Y | $R_1$ | $R_4$ | Z | phys. Daten |
|---|---|---|---|---|---|---|
| 1.1601 | H | H | $C_2H_5$ | $C_2H_5$ | $CH_2OCH_2CONH_2$ | Oel |
| 1.1602 | H | $CH_3$ | $C_2H_5$ | $C_2H_5$ | $CH_2OCH_2CONH_2$ | |
| 1.1603 | H | $C_2H_5$ | $C_2H_5$ | $C_2H_5$ | $CH_2OCH_2CONH_2$ | |
| 1.1604 | H | $C_3H_7$ | $C_2H_5$ | $C_2H_5$ | $CH_2OCH_2CONH_2$ | |
| 1.1605 | H | $OCH_3$ | $C_2H_5$ | $C_2H_5$ | $CH_2OCH_2CONH_2$ | |
| 1.1606 | H | $OC_2H_5$ | $C_2H_5$ | $C_2H_5$ | $CH_2OCH_2CONH_2$ | |
| 1.1607 | H | $OCH_2CH{=}CH_2$ | $C_2H_5$ | $C_2H_5$ | $CH_2OCH_2CONH_2$ | |
| 1.1608 | H | $OCH_2CH{=}CHCH_3$ | $C_2H_5$ | $C_2H_5$ | $CH_2OCH_2CONH_2$ | |
| 1.1609 | H | $OCH_2C(Cl){=}CH_2$ | $C_2H_5$ | $C_2H_5$ | $CH_2OCH_2CONH_2$ | |
| 1.1610 | H | $OCH_2CH{=}CHCl$ | $C_2H_5$ | $C_2H_5$ | $CH_2OCH_2CONH_2$ | |
| 1.1611 | H | $OCH_2C{\equiv}CH$ | $C_2H_5$ | $C_2H_5$ | $CH_2OCH_2CONH_2$ | |
| 1.1612 | H | $OCHF_2$ | $C_2H_5$ | $C_2H_5$ | $CH_2OCH_2CONH_2$ | |
| 1.1613 | H | $OCF_3$ | $C_2H_5$ | $C_2H_5$ | $CH_2OCH_2CONH_2$ | |
| 1.1614 | H | $OCF_2CHFCF_3$ | $C_2H_5$ | $C_2H_5$ | $CH_2OCH_2CONH_2$ | |
| 1.1615 | H | $OC_6H_5$ | $C_2H_5$ | $C_2H_5$ | $CH_2OCH_2CONH_2$ | |
| 1.1616 | H | $CF_3$ | $C_2H_5$ | $C_2H_5$ | $CH_2OCH_2CONH_2$ | |
| 1.1617 | H | Br | $C_2H_5$ | $C_2H_5$ | $CH_2OCH_2CONH_2$ | |
| 1.1618 | H | Cl | $C_2H_5$ | $C_2H_5$ | $CH_2OCH_2CONH_2$ | |
| 1.1619 | H | F | $C_2H_5$ | $C_2H_5$ | $CH_2OCH_2CONH_2$ | |
| 1.1620 | H | $SCH_3$ | $C_2H_5$ | $C_2H_5$ | $CH_2OCH_2CONH_2$ | |
| 1.1621 | H | $SC_2H_5$ | $C_2H_5$ | $C_2H_5$ | $CH_2OCH_2CONH_2$ | |
| 1.1622 | H | $SC_3H_7$-n | $C_2H_5$ | $C_2H_5$ | $CH_2OCH_2CONH_2$ | |
| 1.1623 | H | $SO_2CH_3$ | $C_2H_5$ | $C_2H_5$ | $CH_2OCH_2CONH_2$ | |
| 1.1624 | H | $SO_2C_2H_5$ | $C_2H_5$ | $C_2H_5$ | $CH_2OCH_2CONH_2$ | |
| 1.1625 | H | $NO_2$ | $C_2H_5$ | $C_2H_5$ | $CH_2OCH_2CONH_2$ | |
| 1.1626 | H | CN | $C_2H_5$ | $C_2H_5$ | $CH_2OCH_2CONH_2$ | |
| 1.1627 | H | $CH_2OH$ | $C_2H_5$ | $C_2H_5$ | $CH_2OCH_2CONH_2$ | |
| 1.1628 | H | $CH_2CH_2OH$ | $C_2H_5$ | $C_2H_5$ | $CH_2OCH_2CONH_2$ | |
| 1.1629 | H | $CH_2CH_2CH_2OH$ | $C_2H_5$ | $C_2H_5$ | $CH_2OCH_2CONH_2$ | |
| 1.1630 | H | $C_6H_5$ | $C_2H_5$ | $C_2H_5$ | $CH_2OCH_2CONH_2$ | |
| 1.1631 | H | $CH_2Cl$ | $C_2H_5$ | $C_2H_5$ | $CH_2OCH_2CONH_2$ | |
| 1.1632 | H | $CH_2CH_2Cl$ | $C_2H_5$ | $C_2H_5$ | $CH_2OCH_2CONH_2$ | |
| 1.1633 | H | $CH_2CH_2CH_2Cl$ | $C_2H_5$ | $C_2H_5$ | $CH_2OCH_2CONH_2$ | |

Beispiel 5 Herstellung von 6-Cyclopropyl-2-(5-isopropyl-5-methyl-4-oxoimidazolin-2-yl)-pyridin-3-carbonsäure

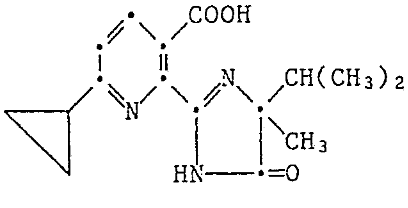

Eine Lösung von 6 g 6-Cyclopropyl-2,3-dicarbonsäure-diäthylester und 3 g 2-Amino-2,3-dimethylbutyramid in 100 ml Aethanol wird mit 10 g 30 %igem Natrium-methylat in Methanol versetzt und während 3 Stunden am Rückfluss gekocht. Anschliessend wird das Reaktionsgemisch eingeengt und der Rückstand in wenig Wasser

gelöst, und die Lösung mit Kochsalz gesättigt. Dann wird der pH mit konzentrierter Salzsäure auf pH 4 gestellt und dreimal mit je 150 ml Aethylacetat extrahiert. Die organische Phase wird über Magnesiumsulfat getrocknet, filtriert und eingeengt. Der Rückstand kristallisiert aus Aether/Hexan 1:2. Man erhält so 4 g 6-Cyclopropyl-2-(5-isopropyl-5-methyl-4-oxoimidazolin-2-yl)-pyridincarbonsäure, welche bei 118-121° schmilzt.

Beispiel 6 Herstellung von 6-Methoxy-5-methyl-2-(5-isopropyl-5-methyl-4-oxoimidazolin-2-yl)-pyridin-3-carbonsäure

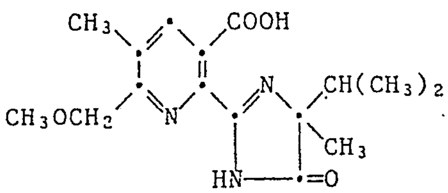

In eine Lösung von 5,7 g 6-Methoxymethyl-5-methylpyridin-2,3-dicarbonsäure-diäthylester und 2,6 g 2-Amino-2,3-dimethylbutyramid in 200 ml trockenem Toluol werden unter Rühren 5 g Kalium-tert.butylat portionsweise bei einer Temperatur von 80° C eingetragen. Die erhaltene rote Lösung wird nach beendigter Zugabe des Kalium-tert.butylats 2 Stunden bei 80° C gerührt, wobei sich das Kaliumsalz der 2-(5-Isopropyl-5-methyl-4-oxoimidazolin-2-yl)-5ethylpyridin-3-carbonsäure in Form eines dicken Kristallbreis abscheidet. Die auf Raumtemperatur abgekühlte Mischung wird abgenutscht, die Kristalle getrocknet und anschliessend in 50 ml gesättigter Kochsalzlösung gelöst. Die Lösung wird mit konzentrierter Salzsäure auf pH 4 gebracht und dann dreimal mit je 100 ml Aethylacetat extrahiert wird. Die organische Phase wird über Magnesiumsulfat getrocknet, abgenutscht und eingedampft. Der Rückstand kristallisiert aus Aether/Hexan 1:2 und man erhält so 2,8 g der obigen Säure, welche bei 151-153° C schmilzt.

Beispiel 7 Herstellung von 5-Aethyl-6-methoxymethyl-2-(5-Isopropyl-5-methyl-4-oxo-imidazol-2H-1-yl)-pyridin-3-carbonsäure

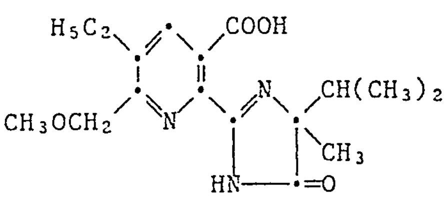

Zu einer Lösung von 18,3 g 5-Aethyl-6-methoxymethyl-2,3-pyridindicarbonsäure in 300 ml trockenem Toluol gibt man bei einer Temperatur von 80° C 17,4 g Kalium-tert.butylat. Dann wird die erhaltene Lösung während 3 Stunden bei 80° C gerührt und schliesslich auf 15° C abgekühlt. Das ausgefallene Kristallisat wird abgenutscht, getrocknet und in wenig gesättigte Kochsalzlösung aufgenommen. Die Kochsalzlösung wird mit konzentrierter Salzsäure auf pH 4 eingestellt und dann dreimal mit je 150 ml Aethylacetat extrahiert. Die organische Phase wird über Magnesiumsulfat getrocknet, abgenutscht und eingedampft. Der Rückstand kristallisiert aus Aether/Hexan 1:2. Man erhält so 11,9 g der obigen Säure mit Schmelzpunkt 134-137° C.

Beispiel 8 Herstellung von 6-(Thiophen-3-yl)-2-(5-isopropyl-5-methyl-4-oxo-imidazol-2-yl)-pyrid in-3-carbonsäure

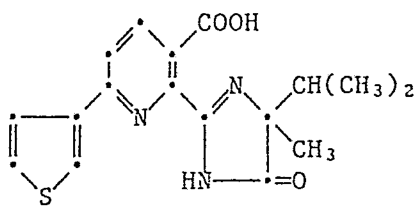

Zu einer Lösung von 14 g 6-(Thiophen-3-yl)-2,3-pyridindicarbonsäure-diäthylester und 6 g 2-Amino-2,3-dimethylbutyramid in 100 ml trockenem Toluol gibt man unter Rühren 12 g Kalium-tert.butylat und erhitzt die Lösung während 5 Stunden auf 89° C. Dann giesst man die Reaktionslösung auf Eis, trennt die Phasen und säuert die wässrige Phase mit 10 ml Eisessig an. Nach einiger Zeit kristallisiert daraus die obige Säure, welche abgenutscht und getrocknet wird. Man erhält so 13,5 g 6-(Thiophen-3-yl)-2-(5-Isopropyl-5-methyl-4-oxo-imidazol-2-yl)-pyridin-3-carbonsäure mit Schmelzpunkt 253-257° C.

Der als Ausgangspunkt benötigte 6-(Thiophen-3-yl)-2,3-pyridindicarbonsäure-diäthylester wird folgendermassen hergestellt:

a) 1-Morpholino-1-(thiophen-3-yl)-äthylen.

Zu einer gerührten Lösung von 75 g 3-Acetylthiophen und 160 g Morpholin in einem Liter Cyclohexan tropft man eine Lösung von 57 g Titantetrachlorid ($TiCl_4$) in 100 ml Cyclohexan. Nachdem alles zugegeben ist, lässt man 12 Stunden bei Raumtemperatur weiterrühren, filtert dann vom Niederschlag ab und dampft das Filtrat ein. Der Rückstand wird destilliert und hat einen Siedepunkt von 98°/0.04 millibar. Man erhält so 36.5 g 1-Morpholino-1-(thiophen-3-yl)-äthylen als farbloses Oel.

b) Eine Lösung von 25 g dieses Aethylens in 50 ml Aethanol wird mit 31 g Aethoxymethylenoxalessigester ($C_2H_5OCH=C(COOC_2H_5)COCOOC_2H_5$) verrührt. Nach 2 Stunden fügt man 27,5 g einer 9,3 %igen Lösung von Ammoniak in absolutem Alkohol dazu und rührt während einer Stunde bei Raumtemperatur weiter. Beim Stehen über Nacht scheiden aus der Lösung Kristalle aus. Diese werden abfiltriert und aus Toluol/Hexan umkristallisiert. Man erhält so 22,5 g 6-(Thiphen-3-yl)-2,3-pyridindicarbonsäure-diäthylester, welcher bei 100-101,5° C schmilzt.

In Analogie zu den Beispielen 5-8 werden die in den Tabellen 2.00-2.16 aufgeführten 2-(5-Isopropyl-2-methyl-4-oxo-imidazol-2-yl-pyridin-3-carbonsäuren (Nicotinsäuren) der Formel III hergestellt.

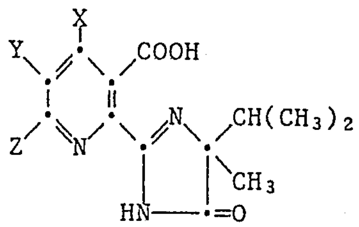

(III)

Tabelle 2.00

| No. | X | Y | Z | phys. Daten |
|---|---|---|---|---|
| 2.0001 | H | H | $C_3H_5$(Cyclo) | Smp. 118-121°C |
| 2.0002 | H | $CH_3$ | $C_3H_5$(Cyclo) | Smp. 206-208°C |
| 2.0003 | H | $C_2H_5$ | $C_3H_5$(Cyclo) | Smp. 169-172°C |
| 2.0004 | H | $C_3H_7$ | $C_3H_5$(Cyclo) | |
| 2.0005 | H | $OCH_3$ | $C_3H_5$(Cyclo) | |
| 2.0006 | H | $OC_2H_5$ | $C_3H_5$(Cyclo) | |
| 2.0007 | H | $OCH_2CH=CH_2$ | $C_3H_5$(Cyclo) | |
| 2.0008 | H | $OCH_2CH=CHCH_3$ | $C_3H_5$(Cyclo) | |
| 2.0009 | H | $OCH_2C(Cl)=CH_2$ | $C_3H_5$(Cyclo) | |
| 2.0010 | H | $OCH_2CH=CHCl$ | $C_3H_5$(Cyclo) | |
| 2.0011 | H | $OCH_2C\equiv CH$ | $C_3H_5$(Cyclo) | |
| 2.0012 | H | $OCHF_2$ | $C_3H_5$(Cyclo) | |
| 2.0013 | H | $OCF_3$ | $C_3H_5$(Cyclo) | |
| 2.0014 | H | $OCF_2CHFCF_3$ | $C_3H_5$(Cyclo) | |
| 2.0015 | H | $OC_6H_5$ | $C_3H_5$(Cyclo) | |
| 2.0016 | H | $CF_3$ | $C_3H_5$(Cyclo) | |
| 2.0017 | H | Br | $C_3H_5$(Cyclo) | Schaum |
| 2.0018 | H | Cl | $C_3H_5$(Cyclo) | |
| 2.0019 | H | F | $C_3H_5$(Cyclo) | |
| 2.0020 | H | $SCH_3$ | $C_3H_5$(Cyclo) | |
| 2.0021 | H | $SC_2H_5$ | $C_3H_5$(Cyclo) | |
| 2.0022 | H | $SC_3H_7$-n | $C_3H_5$(Cyclo) | |
| 2.0023 | H | $SO_2CH_3$ | $C_3H_5$(Cyclo) | |
| 2.0024 | H | $SO_2C_2H_5$ | $C_3H_5$(Cyclo) | |
| 2.0025 | H | $NO_2$ | $C_3H_5$(Cyclo) | |
| 2.0026 | H | CN | $C_3H_5$(Cyclo) | |
| 2.0027 | H | $CH_2OH$ | $C_3H_5$(Cyclo) | |
| 2.0028 | H | $CH_2CH_2OH$ | $C_3H_5$(Cyclo) | |
| 2.0029 | H | $CH_2CH_2CH_2OH$ | $C_3H_5$(Cyclo) | |
| 2.0030 | H | $C_6H_5$ | $C_3H_5$(Cyclo) | |
| 2.0031 | H | $CH_2Cl$ | $C_3H_5$(Cyclo) | |
| 2.0032 | H | $CH_2CH_2Cl$ | $C_3H_5$(Cyclo) | |
| 2.0033 | H | $CH_2CH_2CH_2Cl$ | $C_3H_5$(Cyclo) | |

Tabelle 2.01

| No. | X | Y | Z | phys. Daten |
|---|---|---|---|---|
| 2.0101 | H | H | $C_4H_7$(Cyclo) | |
| 2.0102 | H | $CH_3$ | $C_4H_7$(Cyclo) | |
| 2.0103 | H | $C_2H_5$ | $C_4H_7$(Cyclo) | |
| 2.0104 | H | $C_3H_7$ | $C_4H_7$(Cyclo) | |
| 2.0105 | H | $OCH_3$ | $C_4H_7$(Cyclo) | |
| 2.0106 | H | $OC_2H_5$ | $C_4H_7$(Cyclo) | |
| 2.0107 | H | $OCH_2CH=CH_2$ | $C_4H_7$(Cyclo) | |
| 2.0108 | H | $OCH_2CH=CHCH_3$ | $C_4H_7$(Cyclo) | |
| 2.0109 | H | $OCH_2C(Cl)=CH_2$ | $C_4H_7$(Cyclo) | |
| 2.0110 | H | $OCH_2CH=CHCl$ | $C_4H_7$(Cyclo) | |
| 2.0111 | H | $OCH_2C{\equiv}CH$ | $C_4H_7$(Cyclo) | |
| 2.0112 | H | $OCHF_2$ | $C_4H_7$(Cyclo) | |
| 2.0113 | H | $OCF_3$ | $C_4H_7$(Cyclo) | |
| 2.0114 | H | $OCF_2CHFCF_3$ | $C_4H_7$(Cyclo) | |
| 2.0115 | H | $OC_6H_5$ | $C_4H_7$(Cyclo) | |
| 2.0116 | H | $CF_3$ | $C_4H_7$(Cyclo) | |
| 2.0117 | H | Br | $C_4H_7$(Cyclo) | |
| 2.0118 | H | Cl | $C_4H_7$(Cyclo) | |
| 2.0119 | H | F | $C_4H_7$(Cyclo) | |
| 2.0120 | H | $SCH_3$ | $C_4H_7$(Cyclo) | |
| 2.0121 | H | $SC_2H_5$ | $C_4H_7$(Cyclo) | |
| 2.0122 | H | $SC_3H_7$-n | $C_4H_7$(Cyclo) | |
| 2.0123 | H | $SO_2CH_3$ | $C_4H_7$(Cyclo) | |
| 2.0124 | H | $SO_2C_2H_5$ | $C_4H_7$(Cyclo) | |
| 2.0125 | H | $NO_2$ | $C_4H_7$(Cyclo) | |
| 2.0126 | H | CN | $C_4H_7$(Cyclo) | |
| 2.0127 | H | $CH_2OH$ | $C_4H_7$(Cyclo) | |
| 2.0128 | H | $CH_2CH_2OH$ | $C_4H_7$(Cyclo) | |
| 2.0129 | H | $CH_2CH_2CH_2OH$ | $C_4H_7$(Cyclo) | |
| 2.0130 | H | $C_6H_5$ | $C_4H_7$(Cyclo) | |
| 2.0131 | H | $CH_2Cl$ | $C_4H_7$(Cyclo) | |
| 2.0132 | H | $CH_2CH_2Cl$ | $C_4H_7$(Cyclo) | |
| 2.0133 | H | $CH_2CH_2CH_2Cl$ | $C_4H_7$(Cyclo) | |

Tabelle 2.02

| No. | X | Y | Z | phys. Daten |
|---|---|---|---|---|
| 2.0201 | H | H | $C_5H_9$(Cyclo) | |
| 2.0202 | H | $CH_3$ | $C_5H_9$(Cyclo) | |
| 2.0203 | H | $C_2H_5$ | $C_5H_9$(Cyclo) | |
| 2.0204 | H | $C_3H_7$ | $C_5H_9$(Cyclo) | |
| 2.0205 | H | $OCH_3$ | $C_5H_9$(Cyclo) | |
| 2.0206 | H | $OC_2H_5$ | $C_5H_9$(Cyclo) | |
| 2.0207 | H | $OCH_2CH{=}CH_2$ | $C_5H_9$(Cyclo) | |
| 2.0208 | H | $OCH_2CH{=}CHCH_3$ | $C_5H_9$(Cyclo) | |
| 2.0209 | H | $OCH_2C(Cl){=}CH_2$ | $C_5H_9$(Cyclo) | |
| 2.0210 | H | $OCH_2CH{=}CHCl$ | $C_5H_9$(Cyclo) | |
| 2.0211 | H | $OCH_2C{\equiv}CH$ | $C_5H_9$(Cyclo) | |
| 2.0212 | H | $OCHF_2$ | $C_5H_9$(Cyclo) | |
| 2.0213 | H | $OCF_3$ | $C_5H_9$(Cyclo) | |
| 2.0214 | H | $OCF_2CHFCF_3$ | $C_5H_9$(Cyclo) | |
| 2.0215 | H | $OC_6H_5$ | $C_5H_9$(Cyclo) | |
| 2.0216 | H | $CF_3$ | $C_5H_9$(Cyclo) | |
| 2.0217 | H | Br | $C_5H_9$(Cyclo) | |
| 2.0218 | H | Cl | $C_5H_9$(Cyclo) | |
| 2.0219 | H | F | $C_5H_9$(Cyclo) | |
| 2.0220 | H | $SCH_3$ | $C_5H_9$(Cyclo) | |
| 2.0221 | H | $SC_2H_5$ | $C_5H_9$(Cyclo) | |
| 2.0222 | H | $SC_3H_7$-n | $C_5H_9$(Cyclo) | |
| 2.0223 | H | $SO_2CH_3$ | $C_5H_9$(Cyclo) | |
| 2.0224 | H | $SO_2C_2H_5$ | $C_5H_9$(Cyclo) | |
| 2.0225 | H | $NO_2$ | $C_5H_9$(Cyclo) | |
| 2.0226 | H | CN | $C_5H_9$(Cyclo) | |
| 2.0227 | H | $CH_2OH$ | $C_5H_9$(Cyclo) | |
| 2.0228 | H | $CH_2CH_2OH$ | $C_5H_9$(Cyclo) | |
| 2.0229 | H | $CH_2CH_2CH_2OH$ | $C_5H_9$(Cyclo) | |
| 2.0230 | H | $C_6H_5$ | $C_5H_9$(Cyclo) | |
| 2.0231 | H | $CH_2Cl$ | $C_5H_9$(Cyclo) | |
| 2.0232 | H | $CH_2CH_2Cl$ | $C_5H_9$(Cyclo) | |
| 2.0233 | H | $CH_2CH_2CH_2Cl$ | $C_5H_9$(Cyclo) | |

Tabelle 2.03

| No. | X | Y | Z | phys. Daten |
|---|---|---|---|---|
| 2.0301 | H | H | $C_6H_{11}$(Cyclo) | Smp. 188-190°C |
| 2.0302 | H | $CH_3$ | $C_6H_{11}$(Cyclo) | |
| 2.0303 | H | $C_2H_5$ | $C_6H_{11}$(Cyclo) | |
| 2.0304 | H | $C_3H_7$ | $C_6H_{11}$(Cyclo) | |
| 2.0305 | H | $OCH_3$ | $C_6H_{11}$(Cyclo) | |
| 2.0306 | H | $OC_2H_5$ | $C_6H_{11}$(Cyclo) | |
| 2.0307 | H | $OCH_2CH=CH_2$ | $C_6H_{11}$(Cyclo) | |
| 2.0308 | H | $OCH_2CH=CHCH_3$ | $C_6H_{11}$(Cyclo) | |
| 2.0309 | H | $OCH_2C(Cl)=CH_2$ | $C_6H_{11}$(Cyclo) | |
| 2.0310 | H | $OCH_2CH=CHCl$ | $C_6H_{11}$(Cyclo) | |
| 2.0311 | H | $OCH_2C\equiv CH$ | $C_6H_{11}$(Cyclo) | |
| 2.0312 | H | $OCHF_2$ | $C_6H_{11}$(Cyclo) | |
| 2.0313 | H | $OCF_3$ | $C_6H_{11}$(Cyclo) | |
| 2.0314 | H | $OCF_2CHFCF_3$ | $C_6H_{11}$(Cyclo) | |
| 2.0315 | H | $OC_6H_5$ | $C_6H_{11}$(Cyclo) | |
| 2.0316 | H | $CF_3$ | $C_6H_{11}$(Cyclo) | |
| 2.0317 | H | Br | $C_6H_{11}$(Cyclo) | |
| 2.0318 | H | Cl | $C_6H_{11}$(Cyclo) | |
| 2.0319 | H | F | $C_6H_{11}$(Cyclo) | |
| 2.0320 | H | $SCH_3$ | $C_6H_{11}$(Cyclo) | |
| 2.0321 | H | $SC_2H_5$ | $C_6H_{11}$(Cyclo) | |
| 2.0322 | H | $SC_3H_7$-n | $C_6H_{11}$(Cyclo) | |
| 2.0323 | H | $SO_2CH_3$ | $C_6H_{11}$(Cyclo) | |
| 2.0324 | H | $SO_2C_2H_5$ | $C_6H_{11}$(Cyclo) | |
| 2.0325 | H | $NO_2$ | $C_6H_{11}$(Cyclo) | |
| 2.0326 | H | CN | $C_6H_{11}$(Cyclo) | |
| 2.0327 | H | $CH_2OH$ | $C_6H_{11}$(Cyclo) | |
| 2.0328 | H | $CH_2CH_2OH$ | $C_6H_{11}$(Cyclo) | |
| 2.0329 | H | $CH_2CH_2CH_2OH$ | $C_6H_{11}$(Cyclo) | |
| 2.0330 | H | $C_6H_5$ | $C_6H_{11}$(Cyclo) | |
| 2.0331 | H | $CH_2Cl$ | $C_6H_{11}$(Cyclo) | |
| 2.0332 | H | $CH_2CH_2Cl$ | $C_6H_{11}$(Cyclo) | |
| 2.0333 | H | $CH_2CH_2CH_2Cl$ | $C_6H_{11}$(Cyclo) | |

Tabelle 2.04

| No. | X | Y | Z | phys. Daten |
|---|---|---|---|---|
| 2.0401 | H | H | $CH_2OCH_3$ | Smp. 168-169°C |
| 2.0402 | H | $CH_3$ | $CH_2OCH_3$ | Smp. 151-153°C |
| 2.0403 | H | $C_2H_5$ | $CH_2OCH_3$ | Smp. 134-137°C |
| 2.0404 | H | $C_3H_7$ | $CH_2OCH_3$ | |
| 2.0405 | H | $OCH_3$ | $CH_2OCH_3$ | |
| 2.0406 | H | $OC_2H_5$ | $CH_2OCH_3$ | |
| 2.0407 | H | $OCH_2CH=CH_2$ | $CH_2OCH_3$ | |
| 2.0408 | H | $OCH_2CH=CHCH_3$ | $CH_2OCH_3$ | |
| 2.0409 | H | $OCH_2C(Cl)=CH_2$ | $CH_2OCH_3$ | |
| 2.0410 | H | $OCH_2CH=CHCl$ | $CH_2OCH_3$ | |
| 2.0411 | H | $OCH_2C{\equiv}CH$ | $CH_2OCH_3$ | |
| 2.0412 | H | $OCHF_2$ | $CH_2OCH_3$ | |
| 2.0413 | H | $OCF_3$ | $CH_2OCH_3$ | |
| 2.0414 | H | $OCF_2CHFCF_3$ | $CH_2OCH_3$ | |
| 2.0415 | H | $OC_6H_5$ | $CH_2OCH_3$ | |
| 2.0416 | H | $CF_3$ | $CH_2OCH_3$ | |
| 2.0417 | H | Br | $CH_2OCH_3$ | |
| 2.0418 | H | Cl | $CH_2OCH_3$ | |
| 2.0419 | H | F | $CH_2OCH_3$ | |
| 2.0420 | H | $SCH_3$ | $CH_2OCH_3$ | |
| 2.0421 | H | $SC_2H_5$ | $CH_2OCH_3$ | |
| 2.0422 | H | $SC_3H_7$-n | $CH_2OCH_3$ | |
| 2.0423 | H | $SO_2CH_3$ | $CH_2OCH_3$ | |
| 2.0424 | H | $SO_2C_2H_5$ | $CH_2OCH_3$ | |
| 2.0425 | H | $NO_2$ | $CH_2OCH_3$ | |
| 2.0426 | H | CN | $CH_2OCH_3$ | |
| 2.0427 | H | $CH_2OH$ | $CH_2OCH_3$ | |
| 2.0428 | H | $CH_2CH_2OH$ | $CH_2OCH_3$ | |
| 2.0429 | H | $CH_2CH_2CH_2OH$ | $CH_2OCH_3$ | |
| 2.0430 | H | $C_6H_5$ | $CH_2OCH_3$ | |
| 2.0431 | H | $CH_2Cl$ | $CH_2OCH_3$ | |
| 2.0432 | H | $CH_2CH_2Cl$ | $CH_2OCH_3$ | |
| 2.0433 | H | $CH_2CH_2CH_2Cl$ | $CH_2OCH_3$ | |
| 2.0434 | $CH_3$ | $CH_3$ | $CH_2OCH_3$ | Oel |

Tabelle 2.05

| No. | X | Y | Z | phys. Daten |
|---|---|---|---|---|
| 2.0501 | H | H | $CH(CH_3)OCH_3$ | |
| 2.0502 | H | $CH_3$ | $CH(CH_3)OCH_3$ | |
| 2.0503 | H | $C_2H_5$ | $CH(CH_3)OCH_3$ | |
| 2.0504 | H | $C_3H_7$ | $CH(CH_3)OCH_3$ | |
| 2.0505 | H | $OCH_3$ | $CH(CH_3)OCH_3$ | |
| 2.0506 | H | $OC_2H_5$ | $CH(CH_3)OCH_3$ | |
| 2.0507 | H | $OCH_2CH=CH_2$ | $CH(CH_3)OCH_3$ | |
| 2.0508 | H | $OCH_2CH=CHCH_3$ | $CH(CH_3)OCH_3$ | |
| 2.0509 | H | $OCH_2C(Cl)=CH_2$ | $CH(CH_3)OCH_3$ | |
| 2.0510 | H | $OCH_2CH=CHCl$ | $CH(CH_3)OCH_3$ | |
| 2.0511 | H | $OCH_2C\equiv CH$ | $CH(CH_3)OCH_3$ | |
| 2.0512 | H | $OCHF_2$ | $CH(CH_3)OCH_3$ | |
| 2.0513 | H | $OCF_3$ | $CH(CH_3)OCH_3$ | |
| 2.0514 | H | $OCF_2CHFCF_3$ | $CH(CH_3)OCH_3$ | |
| 2.0515 | H | $OC_6H_5$ | $CH(CH_3)OCH_3$ | |
| 2.0516 | H | $CF_3$ | $CH(CH_3)OCH_3$ | |
| 2.0517 | H | Br | $CH(CH_3)OCH_3$ | |
| 2.0518 | H | Cl | $CH(CH_3)OCH_3$ | |
| 2.0519 | H | F | $CH(CH_3)OCH_3$ | |
| 2.0520 | H | $SCH_3$ | $CH(CH_3)OCH_3$ | |
| 2.0521 | H | $SC_2H_5$ | $CH(CH_3)OCH_3$ | |
| 2.0522 | H | $SC_3H_7$-n | $CH(CH_3)OCH_3$ | |
| 2.0523 | H | $SO_2CH_3$ | $CH(CH_3)OCH_3$ | |
| 2.0524 | H | $SO_2C_2H_5$ | $CH(CH_3)OCH_3$ | |
| 2.0525 | H | $NO_2$ | $CH(CH_3)OCH_3$ | |
| 2.0526 | H | CN | $CH(CH_3)OCH_3$ | |
| 2.0527 | H | $CH_2OH$ | $CH(CH_3)OCH_3$ | |
| 2.0528 | H | $CH_2CH_2OH$ | $CH(CH_3)OCH_3$ | |
| 2.0529 | H | $CH_2CH_2CH_2OH$ | $CH(CH_3)OCH_3$ | |
| 2.0530 | H | $C_6H_5$ | $CH(CH_3)OCH_3$ | |
| 2.0531 | H | $CH_2Cl$ | $CH(CH_3)OCH_3$ | |
| 2.0532 | H | $CH_2CH_2Cl$ | $CH(CH_3)OCH_3$ | |
| 2.0533 | H | $CH_2CH_2CH_2Cl$ | $CH(CH_3)OCH_3$ | |

Tabelle 2.06

| No. | X | Y | Z | phys. Daten |
|---|---|---|---|---|
| 2.0601 | H | H | $CH_2OC_2H_5$ | Smp. 144-151°C |
| 2.0602 | H | $CH_3$ | $CH_2OC_2H_5$ | Smp. 159-162°C |
| 2.0603 | H | $C_2H_5$ | $CH_2OC_2H_5$ | Smp. 143-145°C |
| 2.0604 | H | $C_3H_7$ | $CH_2OC_2H_5$ | |
| 2.0605 | H | $OCH_3$ | $CH_2OC_2H_5$ | |
| 2.0606 | H | $OC_2H_5$ | $CH_2OC_2H_5$ | |
| 2.0607 | H | $OCH_2CH=CH_2$ | $CH_2OC_2H_5$ | |
| 2.0608 | H | $OCH_2CH=CHCH_3$ | $CH_2OC_2H_5$ | |
| 2.0609 | H | $OCH_2C(Cl)=CH_2$ | $CH_2OC_2H_5$ | |
| 2.0610 | H | $OCH_2CH=CHCl$ | $CH_2OC_2H_5$ | |
| 2.0611 | H | $OCH_2C{\equiv}CH$ | $CH_2OC_2H_5$ | |
| 2.0612 | H | $OCHF_2$ | $CH_2OC_2H_5$ | |
| 2.0613 | H | $OCF_3$ | $CH_2OC_2H_5$ | |
| 2.0614 | H | $OCF_2CHFCF_3$ | $CH_2OC_2H_5$ | |
| 2.0615 | H | $OC_6H_5$ | $CH_2OC_2H_5$ | |
| 2.0616 | H | $CF_3$ | $CH_2OC_2H_5$ | |
| 2.0617 | H | Br | $CH_2OC_2H_5$ | |
| 2.0618 | H | Cl | $CH_2OC_2H_5$ | |
| 2.0619 | H | F | $CH_2OC_2H_5$ | |
| 2.0620 | H | $SCH_3$ | $CH_2OC_2H_5$ | |
| 2.0621 | H | $SC_2H_5$ | $CH_2OC_2H_5$ | |
| 2.0622 | H | $SC_3H_7$-n | $CH_2OC_2H_5$ | |
| 2.0623 | H | $SO_2CH_3$ | $CH_2OC_2H_5$ | |
| 2.0624 | H | $SO_2C_2H_5$ | $CH_2OC_2H_5$ | |
| 2.0625 | H | $NO_2$ | $CH_2OC_2H_5$ | |
| 2.0626 | H | CN | $CH_2OC_2H_5$ | |
| 2.0627 | H | $CH_2OH$ | $CH_2OC_2H_5$ | |
| 2.0628 | H | $CH_2CH_2OH$ | $CH_2OC_2H_5$ | |
| 2.0629 | H | $CH_2CH_2CH_2OH$ | $CH_2OC_2H_5$ | |
| 2.0630 | H | $C_6H_5$ | $CH_2OC_2H_5$ | |
| 2.0631 | H | $CH_2Cl$ | $CH_2OC_2H_5$ | |
| 2.0632 | H | $CH_2CH_2Cl$ | $CH_2OC_2H_5$ | |
| 2.0633 | H | $CH_2CH_2CH_2Cl$ | $CH_2OC_2H_5$ | |

Tabelle 2.07

| No. | X | Y | Z | phys. Daten |
|---|---|---|---|---|
| 2.0701 | H | H | $CH(CH_3)OC_2H_5$ | |
| 2.0702 | H | $CH_3$ | $CH(CH_3)OC_2H_5$ | |
| 2.0703 | H | $C_2H_5$ | $CH(CH_3)OC_2H_5$ | |
| 2.0704 | H | $C_3H_7$ | $CH(CH_3)OC_2H_5$ | |
| 2.0705 | H | $OCH_3$ | $CH(CH_3)OC_2H_5$ | |
| 2.0706 | H | $OC_2H_5$ | $CH(CH_3)OC_2H_5$ | |
| 2.0707 | H | $OCH_2CH{=}CH_2$ | $CH(CH_3)OC_2H_5$ | |
| 2.0708 | H | $OCH_2CH{=}CHCH_3$ | $CH(CH_3)OC_2H_5$ | |
| 2.0709 | H | $OCH_2C(Cl){=}CH_2$ | $CH(CH_3)OC_2H_5$ | |
| 2.0710 | H | $OCH_2CH{=}CHCl$ | $CH(CH_3)OC_2H_5$ | |
| 2.0711 | H | $OCH_2C{\equiv}CH$ | $CH(CH_3)OC_2H_5$ | |
| 2.0712 | H | $OCHF_2$ | $CH(CH_3)OC_2H_5$ | |
| 2.0713 | H | $OCF_3$ | $CH(CH_3)OC_2H_5$ | |
| 2.0714 | H | $OCF_2CHFCF_3$ | $CH(CH_3)OC_2H_5$ | |
| 2.0715 | H | $OC_6H_5$ | $CH(CH_3)OC_2H_5$ | |
| 2.0716 | H | $CF_3$ | $CH(CH_3)OC_2H_5$ | |
| 2.0717 | H | Br | $CH(CH_3)OC_2H_5$ | |
| 2.0718 | H | Cl | $CH(CH_3)OC_2H_5$ | |
| 2.0719 | H | F | $CH(CH_3)OC_2H_5$ | |
| 2.0720 | H | $SCH_3$ | $CH(CH_3)OC_2H_5$ | |
| 2.0721 | H | $SC_2H_5$ | $CH(CH_3)OC_2H_5$ | |
| 2.0722 | H | $SC_3H_7$-n | $CH(CH_3)OC_2H_5$ | |
| 2.0723 | H | $SO_2CH_3$ | $CH(CH_3)OC_2H_5$ | |
| 2.0724 | H | $SO_2C_2H_5$ | $CH(CH_3)OC_2H_5$ | |
| 2.0725 | H | $NO_2$ | $CH(CH_3)OC_2H_5$ | |
| 2.0726 | H | CN | $CH(CH_3)OC_2H_5$ | |
| 2.0727 | H | $CH_2OH$ | $CH(CH_3)OC_2H_5$ | |
| 2.0728 | H | $CH_2CH_2OH$ | $CH(CH_3)OC_2H_5$ | |
| 2.0729 | H | $CH_2CH_2CH_2OH$ | $CH(CH_3)OC_2H_5$ | |
| 2.0730 | H | $C_6H_5$ | $CH(CH_3)OC_2H_5$ | |
| 2.0731 | H | $CH_2Cl$ | $CH(CH_3)OC_2H_5$ | |
| 2.0732 | H | $CH_2CH_2Cl$ | $CH(CH_3)OC_2H_5$ | |
| 2.0733 | H | $CH_2CH_2CH_2Cl$ | $CH(CH_3)OC_2H_5$ | |

Tabelle 2.08

| No. | X | Y | Z | phys. Daten |
|---|---|---|---|---|
| 2.0801 | H | H | $C(CH_3)_2OCH_3$ | |
| 2.0802 | H | $CH_3$ | $C(CH_3)_2OCH_3$ | |
| 2.0803 | H | $C_2H_5$ | $C(CH_3)_2OCH_3$ | |
| 2.0804 | H | $C_3H_7$ | $C(CH_3)_2OCH_3$ | |
| 2.0805 | H | $OCH_3$ | $C(CH_3)_2OCH_3$ | |
| 2.0806 | H | $OC_2H_5$ | $C(CH_3)_2OCH_3$ | |
| 2.0807 | H | $OCH_2CH{=}CH_2$ | $C(CH_3)_2OCH_3$ | |
| 2.0808 | H | $OCH_2CH{=}CHCH_3$ | $C(CH_3)_2OCH_3$ | |
| 2.0809 | H | $OCH_2C(Cl){=}CH_2$ | $C(CH_3)_2OCH_3$ | |
| 2.0810 | H | $OCH_2CH{=}CHCl$ | $C(CH_3)_2OCH_3$ | |
| 2.0811 | H | $OCH_2C{\equiv}CH$ | $C(CH_3)_2OCH_3$ | |
| 2.0812 | H | $OCHF_2$ | $C(CH_3)_2OCH_3$ | |
| 2.0813 | H | $OCF_3$ | $C(CH_3)_2OCH_3$ | |
| 2.0814 | H | $OCF_2CHFCF_3$ | $C(CH_3)_2OCH_3$ | |
| 2.0815 | H | $OC_6H_5$ | $C(CH_3)_2OCH_3$ | |
| 2.0816 | H | $CF_3$ | $C(CH_3)_2OCH_3$ | |
| 2.0817 | H | Br | $C(CH_3)_2OCH_3$ | |
| 2.0818 | H | Cl | $C(CH_3)_2OCH_3$ | |
| 2.0819 | H | F | $C(CH_3)_2OCH_3$ | |
| 2.0820 | H | $SCH_3$ | $C(CH_3)_2OCH_3$ | |
| 2.0821 | H | $SC_2H_5$ | $C(CH_3)_2OCH_3$ | |
| 2.0822 | H | $SC_3H_7$-n | $C(CH_3)_2OCH_3$ | |
| 2.0823 | H | $SO_2CH_3$ | $C(CH_3)_2OCH_3$ | |
| 2.0824 | H | $SO_2C_2H_5$ | $C(CH_3)_2OCH_3$ | |
| 2.0825 | H | $NO_2$ | $C(CH_3)_2OCH_3$ | |
| 2.0826 | H | CN | $C(CH_3)_2OCH_3$ | |
| 2.0827 | H | $CH_2OH$ | $C(CH_3)_2OCH_3$ | |
| 2.0828 | H | $CH_2CH_2OH$ | $C(CH_3)_2OCH_3$ | |
| 2.0829 | H | $CH_2CH_2CH_2OH$ | $C(CH_3)_2OCH_3$ | |
| 2.0830 | H | $C_6H_5$ | $C(CH_3)_2OCH_3$ | |
| 2.0831 | H | $CH_2Cl$ | $C(CH_3)_2OCH_3$ | |
| 2.0832 | H | $CH_2CH_2Cl$ | $C(CH_3)_2OCH_3$ | |
| 2.0833 | H | $CH_2CH_2CH_2Cl$ | $C(CH_3)_2OCH_3$ | |

Tabelle 2.09

| No. | X | Y | Z | phys. Daten |
|---|---|---|---|---|
| 2.0901 | H | H | $CH_2OC_3H_7$ | |
| 2.0902 | H | $CH_3$ | $CH_2OC_3H_7$ | |
| 2.0903 | H | $C_2H_5$ | $CH_2OC_3H_7$ | |
| 2.0904 | H | $C_3H_7$ | $CH_2OC_3H_7$ | |
| 2.0905 | H | $OCH_3$ | $CH_2OC_3H_7$ | |
| 2.0906 | H | $OC_2H_5$ | $CH_2OC_3H_7$ | |
| 2.0907 | H | $OCH_2CH=CH_2$ | $CH_2OC_3H_7$ | |
| 2.0908 | H | $OCH_2CH=CHCH_3$ | $CH_2OC_3H_7$ | |
| 2.0909 | H | $OCH_2C(Cl)=CH_2$ | $CH_2OC_3H_7$ | |
| 2.0910 | H | $OCH_2CH=CHCl$ | $CH_2OC_3H_7$ | |
| 2.0911 | H | $OCH_2C\equiv CH$ | $CH_2OC_3H_7$ | |
| 2.0912 | H | $OCHF_2$ | $CH_2OC_3H_7$ | |
| 2.0913 | H | $OCF_3$ | $CH_2OC_3H_7$ | |
| 2.0914 | H | $OCF_2CHFCF_3$ | $CH_2OC_3H_7$ | |
| 2.0915 | H | $OC_6H_5$ | $CH_2OC_3H_7$ | |
| 2.0916 | H | $CF_3$ | $CH_2OC_3H_7$ | |
| 2.0917 | H | Br | $CH_2OC_3H_7$ | |
| 2.0918 | H | Cl | $CH_2OC_3H_7$ | |
| 2.0919 | H | F | $CH_2OC_3H_7$ | |
| 2.0920 | H | $SCH_3$ | $CH_2OC_3H_7$ | |
| 2.0921 | H | $SC_2H_5$ | $CH_2OC_3H_7$ | |
| 2.0922 | H | $SC_3H_7$-n | $CH_2OC_3H_7$ | |
| 2.0923 | H | $SO_2CH_3$ | $CH_2OC_3H_7$ | |
| 2.0924 | H | $SO_2C_2H_5$ | $CH_2OC_3H_7$ | |
| 2.0925 | H | $NO_2$ | $CH_2OC_3H_7$ | |
| 2.0926 | H | CN | $CH_2OC_3H_7$ | |
| 2.0927 | H | $CH_2OH$ | $CH_2OC_3H_7$ | |
| 2.0928 | H | $CH_2CH_2OH$ | $CH_2OC_3H_7$ | |
| 2.0929 | H | $CH_2CH_2CH_2OH$ | $CH_2OC_3H_7$ | |
| 2.0930 | H | $C_6H_5$ | $CH_2OC_3H_7$ | |
| 2.0931 | H | $CH_2Cl$ | $CH_2OC_3H_7$ | |
| 2.0932 | H | $CH_2CH_2Cl$ | $CH_2OC_3H_7$ | |
| 2.0933 | H | $CH_2CH_2CH_2Cl$ | $CH_2OC_3H_7$ | |

Tabelle 2.10

| No. | X | Y | Z | phys. Daten |
|---|---|---|---|---|
| 2.1001 | H | H | $C(CH_3)C_2H_4$(Cyclo) | Smp. 149-154°C |
| 2.1002 | H | $CH_3$ | $C(CH_3)C_2H_4$(Cyclo) | |
| 2.1003 | H | $C_2H_5$ | $C(CH_3)C_2H_4$(Cyclo) | |
| 2.1004 | H | $C_3H_7$ | $C(CH_3)C_2H_4$(Cyclo) | |
| 2.1005 | H | $OCH_3$ | $C(CH_3)C_2H_4$(Cyclo) | |
| 2.1006 | H | $OC_2H_5$ | $C(CH_3)C_2H_4$(Cyclo) | |
| 2.1007 | H | $OCH_2CH{=}CH_2$ | $C(CH_3)C_2H_4$(Cyclo) | |
| 2.1008 | H | $OCH_2CH{=}CHCH_3$ | $C(CH_3)C_2H_4$(Cyclo) | |
| 2.1009 | H | $OCH_2C(Cl){=}CH_2$ | $C(CH_3)C_2H_4$(Cyclo) | |
| 2.1010 | H | $OCH_2CH{=}CHCl$ | $C(CH_3)C_2H_4$(Cyclo) | |
| 2.1011 | H | $OCH_2C{\equiv}CH$ | $C(CH_3)C_2H_4$(Cyclo) | |
| 2.1012 | H | $OCHF_2$ | $C(CH_3)C_2H_4$(Cyclo) | |
| 2.1013 | H | $OCF_3$ | $C(CH_3)C_2H_4$(Cyclo) | |
| 2.1014 | H | $OCF_2CHFCF_3$ | $C(CH_3)C_2H_4$(Cyclo) | |
| 2.1015 | H | $OC_6H_5$ | $C(CH_3)C_2H_4$(Cyclo) | |
| 2.1016 | H | $CF_3$ | $C(CH_3)C_2H_4$(Cyclo) | |
| 2.1017 | H | Br | $C(CH_3)C_2H_4$(Cyclo) | |
| 2.1018 | H | Cl | $C(CH_3)C_2H_4$(Cyclo) | |
| 2.1019 | H | F | $C(CH_3)C_2H_4$(Cyclo) | |
| 2.1020 | H | $SCH_3$ | $C(CH_3)C_2H_4$(Cyclo) | |
| 2.1021 | H | $SC_2H_5$ | $C(CH_3)C_2H_4$(Cyclo) | |
| 2.1022 | H | $SC_3H_7$-n | $C(CH_3)C_2H_4$(Cyclo) | |
| 2.1023 | H | $SO_2CH_3$ | $C(CH_3)C_2H_4$(Cyclo) | |
| 2.1024 | H | $SO_2C_2H_5$ | $C(CH_3)C_2H_4$(Cyclo) | |
| 2.1025 | H | $NO_2$ | $C(CH_3)C_2H_4$(Cyclo) | |
| 2.1026 | H | CN | $C(CH_3)C_2H_4$(Cyclo) | |
| 2.1027 | H | $CH_2OH$ | $C(CH_3)C_2H_4$(Cyclo) | |
| 2.1028 | H | $CH_2CH_2OH$ | $C(CH_3)C_2H_4$(Cyclo) | |
| 2.1029 | H | $CH_2CH_2CH_2OH$ | $C(CH_3)C_2H_4$(Cyclo) | |
| 2.1030 | H | $C_6H_5$ | $C(CH_3)C_2H_4$(Cyclo) | |
| 2.1031 | H | $CH_2Cl$ | $C(CH_3)C_2H_4$(Cyclo) | |
| 2.1032 | H | $CH_2CH_2Cl$ | $C(CH_3)C_2H_4$(Cyclo) | |
| 2.1033 | H | $CH_2CH_2CH_2Cl$ | $C(CH_3)C_2H_4$(Cyclo) | |

Tabelle 2.11

| No. | X | Y | Z | phys. Daten |
|---|---|---|---|---|
| 2.1101 | H | H | $CH_2OC_6H_5$ | Smp. 157-162°C |
| 2.1102 | H | $CH_3$ | $CH_2OC_6H_5$ | |
| 2.1103 | H | $C_2H_5$ | $CH_2OC_6H_5$ | |
| 2.1104 | H | $C_3H_7$ | $CH_2OC_6H_5$ | |
| 2.1105 | H | $OCH_3$ | $CH_2OC_6H_5$ | |
| 2.1106 | H | $OC_2H_5$ | $CH_2OC_6H_5$ | |
| 2.1107 | H | $OCH_2CH=CH_2$ | $CH_2OC_6H_5$ | |
| 2.1108 | H | $OCH_2CH=CHCH_3$ | $CH_2OC_6H_5$ | |
| 2.1109 | H | $OCH_2C(Cl)=CH_2$ | $CH_2OC_6H_5$ | |
| 2.1110 | H | $OCH_2CH=CHCl$ | $CH_2OC_6H_5$ | |
| 2.1111 | H | $OCH_2C\equiv CH$ | $CH_2OC_6H_5$ | |
| 2.1112 | H | $OCHF_2$ | $CH_2OC_6H_5$ | |
| 2.1113 | H | $OCF_3$ | $CH_2OC_6H_5$ | |
| 2.1114 | H | $OCF_2CHFCF_3$ | $CH_2OC_6H_5$ | |
| 2.1115 | H | $OC_6H_5$ | $CH_2OC_6H_5$ | |
| 2.1116 | H | $CF_3$ | $CH_2OC_6H_5$ | |
| 2.1117 | H | Br | $CH_2OC_6H_5$ | |
| 2.1118 | H | Cl | $CH_2OC_6H_5$ | |
| 2.1119 | H | F | $CH_2OC_6H_5$ | |
| 2.1120 | H | $SCH_3$ | $CH_2OC_6H_5$ | |
| 2.1121 | H | $SC_2H_5$ | $CH_2OC_6H_5$ | |
| 2.1122 | H | $SC_3H_7$-n | $CH_2OC_6H_5$ | |
| 2.1123 | H | $SO_2CH_3$ | $CH_2OC_6H_5$ | |
| 2.1124 | H | $SO_2C_2H_5$ | $CH_2OC_6H_5$ | |
| 2.1125 | H | $NO_2$ | $CH_2OC_6H_5$ | |
| 2.1126 | H | CN | $CH_2OC_6H_5$ | |
| 2.1127 | H | $CH_2OH$ | $CH_2OC_6H_5$ | |
| 2.1128 | H | $CH_2CH_2OH$ | $CH_2OC_6H_5$ | |
| 2.1129 | H | $CH_2CH_2CH_2OH$ | $CH_2OC_6H_5$ | |
| 2.1130 | H | $C_6H_5$ | $CH_2OC_6H_5$ | |
| 2.1131 | H | $CH_2Cl$ | $CH_2OC_6H_5$ | |
| 2.1132 | H | $CH_2CH_2Cl$ | $CH_2OC_6H_5$ | |
| 2.1133 | H | $CH_2CH_2CH_2Cl$ | $CH_2OC_6H_5$ | |

Tabelle 2.12

| No. | X | Y | Z | phys. Daten |
|---|---|---|---|---|
| 2.1201 | H | H | Furan-2-yl | Smp. 197-199°C |
| 2.1202 | H | $CH_3$ | Furan-2-yl | |
| 2.1203 | H | $C_2H_5$ | Furan-2-yl | |
| 2.1204 | H | $C_3H_7$ | Furan-2-yl | |
| 2.1205 | H | $OCH_3$ | Furan-2-yl | |
| 2.1206 | H | $OC_2H_5$ | Furan-2-yl | |
| 2.1207 | H | $OCH_2CH=CH_2$ | Furan-2-yl | |
| 2.1208 | H | $OCH_2CH=CHCH_3$ | Furan-2-yl | |
| 2.1209 | H | $OCH_2C(Cl)=CH_2$ | Furan-2-yl | |
| 2.1210 | H | $OCH_2CH=CHCl$ | Furan-2-yl | |
| 2.1211 | H | $OCH_2C\equiv CH$ | Furan-2-yl | |
| 2.1212 | H | $OCHF_2$ | Furan-2-yl | |
| 2.1213 | H | $OCF_3$ | Furan-2-yl | |
| 2.1214 | H | $OCF_2CHFCF_3$ | Furan-2-yl | |
| 2.1215 | H | $OC_6H_5$ | Furan-2-yl | |
| 2.1216 | H | $CF_3$ | Furan-2-yl | |
| 2.1217 | H | Br | Furan-2-yl | |
| 2.1218 | H | Cl | Furan-2-yl | |
| 2.1219 | H | F | Furan-2-yl | |
| 2.1220 | H | $SCH_3$ | Furan-2-yl | |
| 2.1221 | H | $SC_2H_5$ | Furan-2-yl | |
| 2.1222 | H | $SC_3H_7$-n | Furan-2-yl | |
| 2.1223 | H | $SO_2CH_3$ | Furan-2-yl | |
| 2.1224 | H | $SO_2C_2H_5$ | Furan-2-yl | |
| 2.1225 | H | $NO_2$ | Furan-2-yl | |
| 2.1226 | H | CN | Furan-2-yl | |
| 2.1227 | H | $CH_2OH$ | Furan-2-yl | |
| 2.1228 | H | $CH_2CH_2OH$ | Furan-2-yl | |
| 2.1229 | H | $CH_2CH_2CH_2OH$ | Furan-2-yl | |
| 2.1230 | H | $C_6H_5$ | Furan-2-yl | |
| 2.1231 | H | $CH_2Cl$ | Furan-2-yl | |
| 2.1232 | H | $CH_2CH_2Cl$ | Furan-2-yl | |
| 2.1233 | H | $CH_2CH_2CH_2Cl$ | Furan-2-yl | |

Tabelle 2.13

| No. | X | Y | Z | phys. Daten |
|---|---|---|---|---|
| 2.1301 | H | H | Dioxan-2-yl | Smp. 203-206°C |
| 2.1302 | H | $CH_3$ | Dioxan-2-yl | |
| 2.1303 | H | $C_2H_5$ | Dioxan-2-yl | |
| 2.1304 | H | $C_3H_7$ | Dioxan-2-yl | |
| 2.1305 | H | $OCH_3$ | Dioxan-2-yl | |
| 2.1306 | H | $OC_2H_5$ | Dioxan-2-yl | |
| 2.1307 | H | $OCH_2CH=CH_2$ | Dioxan-2-yl | |
| 2.1308 | H | $OCH_2CH=CHCH_3$ | Dioxan-2-yl | |
| 2.1309 | H | $OCH_2C(Cl)=CH_2$ | Dioxan-2-yl | |
| 2.1310 | H | $OCH_2CH=CHCl$ | Dioxan-2-yl | |
| 2.1311 | H | $OCH_2C\equiv CH$ | Dioxan-2-yl | |
| 2.1312 | H | $OCHF_2$ | Dioxan-2-yl | |
| 2.1313 | H | $OCF_3$ | Dioxan-2-yl | |
| 2.1314 | H | $OCF_2CHFCF_3$ | Dioxan-2-yl | |
| 2.1315 | H | $OC_6H_5$ | Dioxan-2-yl | |
| 2.1316 | H | $CF_3$ | Dioxan-2-yl | |
| 2.1317 | H | Br | Dioxan-2-yl | |
| 2.1318 | H | Cl | Dioxan-2-yl | |
| 2.1319 | H | F | Dioxan-2-yl | |
| 2.1320 | H | $SCH_3$ | Dioxan-2-yl | |
| 2.1321 | H | $SC_2H_5$ | Dioxan-2-yl | |
| 2.1322 | H | $SC_3H_7$-n | Dioxan-2-yl | |
| 2.1323 | H | $SO_2CH_3$ | Dioxan-2-yl | |
| 2.1324 | H | $SO_2C_2H_5$ | Dioxan-2-yl | |
| 2.1325 | H | $NO_2$ | Dioxan-2-yl | |
| 2.1326 | H | CN | Dioxan-2-yl | |
| 2.1327 | H | $CH_2OH$ | Dioxan-2-yl | |
| 2.1328 | H | $CH_2CH_2OH$ | Dioxan-2-yl | |
| 2.1329 | H | $CH_2CH_2CH_2OH$ | Dioxan-2-yl | |
| 2.1330 | H | $C_6H_5$ | Dioxan-2-yl | |
| 2.1331 | H | $CH_2Cl$ | Dioxan-2-yl | |
| 2.1332 | H | $CH_2CH_2Cl$ | Dioxan-2-yl | |
| 2.1333 | H | $CH_2CH_2CH_2Cl$ | Dioxan-2-yl | |

Tabelle 2.14

| No. | X | Y | Z | phys. Daten |
|---|---|---|---|---|
| 2.1401 | H | H | $CH_2OC_2H_4OCH_3$ | Harz |
| 2.1402 | H | $CH_3$ | $CH_2OC_2H_4OCH_3$ | |
| 2.1403 | H | $C_2H_5$ | $CH_2OC_2H_4OCH_3$ | |
| 2.1404 | H | $C_3H_7$ | $CH_2OC_2H_4OCH_3$ | |
| 2.1405 | H | $OCH_3$ | $CH_2OC_2H_4OCH_3$ | |
| 2.1406 | H | $OC_2H_5$ | $CH_2OC_2H_4OCH_3$ | |
| 2.1407 | H | $OCH_2CH=CH_2$ | $CH_2OC_2H_4OCH_3$ | |
| 2.1408 | H | $OCH_2CH=CHCH_3$ | $CH_2OC_2H_4OCH_3$ | |
| 2.1409 | H | $OCH_2C(Cl)=CH_2$ | $CH_2OC_2H_4OCH_3$ | |
| 2.1410 | H | $OCH_2CH=CHCl$ | $CH_2OC_2H_4OCH_3$ | |
| 2.1411 | H | $OCH_2C{\equiv}CH$ | $CH_2OC_2H_4OCH_3$ | |
| 2.1412 | H | $OCHF_2$ | $CH_2OC_2H_4OCH_3$ | |
| 2.1413 | H | $OCF_3$ | $CH_2OC_2H_4OCH_3$ | |
| 2.1414 | H | $OCF_2CHFCF_3$ | $CH_2OC_2H_4OCH_3$ | |
| 2.1415 | H | $OC_6H_5$ | $CH_2OC_2H_4OCH_3$ | |
| 2.1416 | H | $CF_3$ | $CH_2OC_2H_4OCH_3$ | |
| 2.1417 | H | Br | $CH_2OC_2H_4OCH_3$ | |
| 2.1418 | H | Cl | $CH_2OC_2H_4OCH_3$ | |
| 2.1419 | H | F | $CH_2OC_2H_4OCH_3$ | |
| 2.1420 | H | $SCH_3$ | $CH_2OC_2H_4OCH_3$ | |
| 2.1421 | H | $SC_2H_5$ | $CH_2OC_2H_4OCH_3$ | |
| 2.1422 | H | $SC_3H_7$-n | $CH_2OC_2H_4OCH_3$ | |
| 2.1423 | H | $SO_2CH_3$ | $CH_2OC_2H_4OCH_3$ | |
| 2.1424 | H | $SO_2C_2H_5$ | $CH_2OC_2H_4OCH_3$ | |
| 2.1425 | H | $NO_2$ | $CH_2OC_2H_4OCH_3$ | |
| 2.1426 | H | CN | $CH_2OC_2H_4OCH_3$ | |
| 2.1427 | H | $CH_2OH$ | $CH_2OC_2H_4OCH_3$ | |
| 2.1428 | H | $CH_2CH_2OH$ | $CH_2OC_2H_4OCH_3$ | |
| 2.1429 | H | $CH_2CH_2CH_2OH$ | $CH_2OC_2H_4OCH_3$ | |
| 2.1430 | H | $C_6H_5$ | $CH_2OC_2H_4OCH_3$ | |
| 2.1431 | H | $CH_2Cl$ | $CH_2OC_2H_4OCH_3$ | |
| 2.1432 | H | $CH_2CH_2Cl$ | $CH_2OC_2H_4OCH_3$ | |
| 2.1433 | H | $CH_2CH_2CH_2Cl$ | $CH_2OC_2H_4OCH_3$ | |

Tabelle 2.15

| No. | X | Y | Z | phys. Daten |
|---|---|---|---|---|
| 2.1501 | H | H | $CH_2OC_2H_4OC_2H_4OCH_3$ | |
| 2.1502 | H | $CH_3$ | $CH_2OC_2H_4OC_2H_4OCH_3$ | |
| 2.1503 | H | $C_2H_5$ | $CH_2OC_2H_4OC_2H_4OCH_3$ | |
| 2.1504 | H | $C_3H_7$ | $CH_2OC_2H_4OC_2H_4OCH_3$ | |
| 2.1505 | H | $OCH_3$ | $CH_2OC_2H_4OC_2H_4OCH_3$ | |
| 2.1506 | H | $OC_2H_5$ | $CH_2OC_2H_4OC_2H_4OCH_3$ | |
| 2.1507 | H | $OCH_2CH=CH_2$ | $CH_2OC_2H_4OC_2H_4OCH_3$ | |
| 2.1508 | H | $OCH_2CH=CHCH_3$ | $CH_2OC_2H_4OC_2H_4OCH_3$ | |
| 2.1509 | H | $OCH_2C(Cl)=CH_2$ | $CH_2OC_2H_4OC_2H_4OCH_3$ | |
| 2.1510 | H | $OCH_2CH=CHCl$ | $CH_2OC_2H_4OC_2H_4OCH_3$ | |
| 2.1511 | H | $OCH_2C\equiv CH$ | $CH_2OC_2H_4OC_2H_4OCH_3$ | |
| 2.1512 | H | $OCHF_2$ | $CH_2OC_2H_4OC_2H_4OCH_3$ | |
| 2.1513 | H | $OCF_3$ | $CH_2OC_2H_4OC_2H_4OCH_3$ | |
| 2.1514 | H | $OCF_2CHFCF_3$ | $CH_2OC_2H_4OC_2H_4OCH_3$ | |
| 2.1515 | H | $OC_6H_5$ | $CH_2OC_2H_4OC_2H_4OCH_3$ | |
| 2.1516 | H | $CF_3$ | $CH_2OC_2H_4OC_2H_4OCH_3$ | |
| 2.1517 | H | Br | $CH_2OC_2H_4OC_2H_4OCH_3$ | |
| 2.1518 | H | Cl | $CH_2OC_2H_4OC_2H_4OCH_3$ | |
| 2.1519 | H | F | $CH_2OC_2H_4OC_2H_4OCH_3$ | |
| 2.1520 | H | $SCH_3$ | $CH_2OC_2H_4OC_2H_4OCH_3$ | |
| 2.1521 | H | $SC_2H_5$ | $CH_2OC_2H_4OC_2H_4OCH_3$ | |
| 2.1522 | H | $SC_3H_7$-n | $CH_2OC_2H_4OC_2H_4OCH_3$ | |
| 2.1523 | H | $SO_2CH_3$ | $CH_2OC_2H_4OC_2H_4OCH_3$ | |
| 2.1524 | H | $SO_2C_2H_5$ | $CH_2OC_2H_4OC_2H_4OCH_3$ | |
| 2.1525 | H | $NO_2$ | $CH_2OC_2H_4OC_2H_4OCH_3$ | |
| 2.1526 | H | CN | $CH_2OC_2H_4OC_2H_4OCH_3$ | |
| 2.1527 | H | $CH_2OH$ | $CH_2OC_2H_4OC_2H_4OCH_3$ | |
| 2.1528 | H | $CH_2CH_2OH$ | $CH_2OC_2H_4OC_2H_4OCH_3$ | |
| 2.1529 | H | $CH_2CH_2CH_2OH$ | $CH_2OC_2H_4OC_2H_4OCH_3$ | |
| 2.1530 | H | $C_6H_5$ | $CH_2OC_2H_4OC_2H_4OCH_3$ | |
| 2.1531 | H | $CH_2Cl$ | $CH_2OC_2H_4OC_2H_4OCH_3$ | |
| 2.1532 | H | $CH_2CH_2Cl$ | $CH_2OC_2H_4OC_2H_4OCH_3$ | |
| 2.1533 | H | $CH_2CH_2CH_2Cl$ | $CH_2OC_2H_4OC_2H_4OCH_3$ | |

Tabelle 2.16

| No. | X | Y | Z | phys. Daten |
|---|---|---|---|---|
| 2.1601 | H | H | $CH_2OCH_2CONH_2$ | Wachs |
| 2.1602 | H | $CH_3$ | $CH_2OCH_2CONH_2$ | |
| 2.1603 | H | $C_2H_5$ | $CH_2OCH_2CONH_2$ | |
| 2.1604 | H | $C_3H_7$ | $CH_2OCH_2CONH_2$ | |
| 2.1605 | H | $OCH_3$ | $CH_2OCH_2CONH_2$ | |
| 2.1606 | H | $OC_2H_5$ | $CH_2OCH_2CONH_2$ | |
| 2.1607 | H | $OCH_2CH=CH_2$ | $CH_2OCH_2CONH_2$ | |
| 2.1608 | H | $OCH_2CH=CHCH_3$ | $CH_2OCH_2CONH_2$ | |
| 2.1609 | H | $OCH_2C(Cl)=CH_2$ | $CH_2OCH_2CONH_2$ | |
| 2.1610 | H | $OCH_2CH=CHCl$ | $CH_2OCH_2CONH_2$ | |
| 2.1611 | H | $OCH_2C\equiv CH$ | $CH_2OCH_2CONH_2$ | |
| 2.1612 | H | $OCHF_2$ | $CH_2OCH_2CONH_2$ | |
| 2.1613 | H | $OCF_3$ | $CH_2OCH_2CONH_2$ | |
| 2.1614 | H | $OCF_2CHFCF_3$ | $CH_2OCH_2CONH_2$ | |
| 2.1615 | H | $OC_6H_5$ | $CH_2OCH_2CONH_2$ | |
| 2.1616 | H | $CF_3$ | $CH_2OCH_2CONH_2$ | |
| 2.1617 | H | Br | $CH_2OCH_2CONH_2$ | |
| 2.1618 | H | Cl | $CH_2OCH_2CONH_2$ | |
| 2.1619 | H | F | $CH_2OCH_2CONH_2$ | |
| 2.1620 | H | $SCH_3$ | $CH_2OCH_2CONH_2$ | |
| 2.1621 | H | $SC_2H_5$ | $CH_2OCH_2CONH_2$ | |
| 2.1622 | H | $SC_3H_7$-n | $CH_2OCH_2CONH_2$ | |
| 2.1623 | H | $SO_2CH_3$ | $CH_2OCH_2CONH_2$ | |
| 2.1624 | H | $SO_2C_2H_5$ | $CH_2OCH_2CONH_2$ | |
| 2.1625 | H | $NO_2$ | $CH_2OCH_2CONH_2$ | |
| 2.1626 | H | CN | $CH_2OCH_2CONH_2$ | |
| 2.1627 | H | $CH_2OH$ | $CH_2OCH_2CONH_2$ | |
| 2.1628 | H | $CH_2CH_2OH$ | $CH_2OCH_2CONH_2$ | |
| 2.1629 | H | $CH_2CH_2CH_2OH$ | $CH_2OCH_2CONH_2$ | |
| 2.1630 | H | $C_6H_5$ | $CH_2OCH_2CONH_2$ | |
| 2.1631 | H | $CH_2Cl$ | $CH_2OCH_2CONH_2$ | |
| 2.1632 | H | $CH_2CH_2Cl$ | $CH_2OCH_2CONH_2$ | |
| 2.1633 | H | $CH_2CH_2CH_2Cl$ | $CH_2OCH_2CONH_2$ | |

Tabelle 2.17

| No. | X | Y | Z | phys. Daten |
|---|---|---|---|---|
| 2.1701 | H | H | Thiophen-2-yl | Smp.247-248°C |
| 2.1702 | H | $CH_3$ | Thiophen-2-yl | |
| 2.1703 | H | $C_2H_5$ | Thiophen-2-yl | |
| 2.1704 | H | $C_3H_7$ | Thiophen-2-yl | |
| 2.1705 | H | $OCH_3$ | Thiophen-2-yl | |
| 2.1706 | H | $OC_2H_5$ | Thiophen-2-yl | |
| 2.1707 | H | $OCH_2CH{=}CH_2$ | Thiophen-2-yl | |
| 2.1708 | H | $OCH_2CH{=}CHCH_3$ | Thiophen-2-yl | |
| 2.1709 | H | $OCH_2C(Cl){=}CH_2$ | Thiophen-2-yl | |
| 2.1710 | H | $OCH_2CH{=}CHCl$ | Thiophen-2-yl | |
| 2.1711 | H | $OCH_2C{\equiv}CH$ | Thiophen-2-yl | |
| 2.1712 | H | $OCHF_2$ | Thiophen-2-yl | |
| 2.1713 | H | $OCF_3$ | Thiophen-2-yl | |
| 2.1714 | H | $OCF_2CHFCF_3$ | Thiophen-2-yl | |
| 2.1715 | H | $OC_6H_5$ | Thiophen-2-yl | |
| 2.1716 | H | $CF_3$ | Thiophen-2-yl | |
| 2.1717 | H | Br | Thiophen-2-yl | |
| 2.1718 | H | Cl | Thiophen-2-yl | |
| 2.1719 | H | F | Thiophen-2-yl | |
| 2.1720 | H | $SCH_3$ | Thiophen-2-yl | |
| 2.1721 | H | $SC_2H_5$ | Thiophen-2-yl | |
| 2.1722 | H | $SC_3H_7$-n | Thiophen-2-yl | |
| 2.1723 | H | $SO_2CH_3$ | Thiophen-2-yl | |
| 2.1724 | H | $SO_2C_2H_5$ | Thiophen-2-yl | |
| 2.1725 | H | $NO_2$ | Thiophen-2-yl | |
| 2.1726 | H | CN | Thiophen-2-yl | |
| 2.1727 | H | $CH_2OH$ | Thiophen-2-yl | |
| 2.1728 | H | $CH_2CH_2OH$ | Thiophen-2-yl | |
| 2.1729 | H | $CH_2CH_2CH_2OH$ | Thiophen-2-yl | |
| 2.1730 | H | $C_6H_5$ | Thiophen-2-yl | |
| 2.1731 | H | $CH_2Cl$ | Thiophen-2-yl | |
| 2.1732 | H | $CH_2CH_2Cl$ | Thiophen-2-yl | |
| 2.1733 | H | $CH_2CH_2CH_2Cl$ | Thiophen-2-yl | |

Tabelle 2.18

| No. | X | Y | Z | phys. Daten |
|---|---|---|---|---|
| 2.1801 | H | H | Thiophen-3-yl | Smp.247-248°C |
| 2.1802 | H | $CH_3$ | Thiophen-3-yl | |
| 2.1803 | H | $C_2H_5$ | Thiophen-3-yl | |
| 2.1804 | H | $C_3H_7$ | Thiophen-3-yl | |
| 2.1805 | H | $OCH_3$ | Thiophen-3-yl | |
| 2.1806 | H | $OC_2H_5$ | Thiophen-3-yl | |
| 2.1807 | H | $OCH_2CH=CH_2$ | Thiophen-3-yl | |
| 2.1808 | H | $OCH_2CH=CHCH_3$ | Thiophen-3-yl | |
| 2.1809 | H | $OCH_2C(Cl)=CH_2$ | Thiophen-3-yl | |
| 2.1810 | H | $OCH_2CH=CHCl$ | Thiophen-3-yl | |
| 2.1811 | H | $OCH_2C≡CH$ | Thiophen-3-yl | |
| 2.1812 | H | $OCHF_2$ | Thiophen-3-yl | |
| 2.1813 | H | $OCF_3$ | Thiophen-3-yl | |
| 2.1814 | H | $OCF_2CHFCF_3$ | Thiophen-3-yl | |
| 2.1815 | H | $OC_6H_5$ | Thiophen-3-yl | |
| 2.1816 | H | $CF_3$ | Thiophen-3-yl | |
| 2.1817 | H | Br | Thiophen-3-yl | |
| 2.1818 | H | Cl | Thiophen-3-yl | |
| 2.1819 | H | F | Thiophen-3-yl | |
| 2.1820 | H | $SCH_3$ | Thiophen-3-yl | |
| 2.1821 | H | $SC_2H_5$ | Thiophen-3-yl | |
| 2.1822 | H | $SC_3H_7$-n | Thiophen-3-yl | |
| 2.1823 | H | $SO_2CH_3$ | Thiophen-3-yl | |
| 2.1824 | H | $SO_2C_2H_5$ | Thiophen-3-yl | |
| 2.1825 | H | $NO_2$ | Thiophen-3-yl | |
| 2.1826 | H | CN | Thiophen-3-yl | |
| 2.1827 | H | $CH_2OH$ | Thiophen-3-yl | |
| 2.1828 | H | $CH_2CH_2OH$ | Thiophen-3-yl | |
| 2.1829 | H | $CH_2CH_2CH_2OH$ | Thiophen-3-yl | |
| 2.1830 | H | $C_6H_5$ | Thiophen-3-yl | |
| 2.1831 | H | $CH_2Cl$ | Thiophen-3-yl | |
| 2.1832 | H | $CH_2CH_2Cl$ | Thiophen-3-yl | |
| 2.1833 | H | $CH_2CH_2CH_2Cl$ | Thiophen-3-yl | |

Tabelle 2.19

| No. | X | Y | Z | phys. Daten |
|---|---|---|---|---|
| 2.1901 | H | H | Pyridin-3-yl | Smp.240-242°C |
| 2.1902 | H | $CH_3$ | Pyridin-3-yl | |
| 2.1903 | H | $C_2H_5$ | Pyridin-3-yl | |
| 2.1904 | H | $C_3H_7$ | Pyridin-3-yl | |
| 2.1905 | H | $OCH_3$ | Pyridin-3-yl | |
| 2.1906 | H | $OC_2H_5$ | Pyridin-3-yl | |
| 2.1907 | H | $OCH_2CH=CH_2$ | Pyridin-3-yl | |
| 2.1908 | H | $OCH_2CH=CHCH_3$ | Pyridin-3-yl | |
| 2.1909 | H | $OCH_2C(Cl)=CH_2$ | Pyridin-3-yl | |
| 2.1910 | H | $OCH_2CH=CHCl$ | Pyridin-3-yl | |
| 2.1911 | H | $OCH_2C{\equiv}CH$ | Pyridin-3-yl | |
| 2.1912 | H | $OCHF_2$ | Pyridin-3-yl | |
| 2.1913 | H | $OCF_3$ | Pyridin-3-yl | |
| 2.1914 | H | $OCF_2CHFCF_3$ | Pyridin-3-yl | |
| 2.1915 | H | $OC_6H_5$ | Pyridin-3-yl | |
| 2.1916 | H | $CF_3$ | Pyridin-3-yl | |
| 2.1917 | H | Br | Pyridin-3-yl | |
| 2.1918 | H | Cl | Pyridin-3-yl | |
| 2.1919 | H | F | Pyridin-3-yl | |
| 2.1920 | H | $SCH_3$ | Pyridin-3-yl | |
| 2.1921 | H | $SC_2H_5$ | Pyridin-3-yl | |
| 2.1922 | H | $SC_3H_7$-n | Pyridin-3-yl | |
| 2.1923 | H | $SO_2CH_3$ | Pyridin-3-yl | |
| 2.1924 | H | $SO_2C_2H_5$ | Pyridin-3-yl | |
| 2.1925 | H | $NO_2$ | Pyridin-3-yl | |
| 2.1926 | H | CN | Pyridin-3-yl | |
| 2.1927 | H | $CH_2OH$ | Pyridin-3-yl | |
| 2.1928 | H | $CH_2CH_2OH$ | Pyridin-3-yl | |
| 2.1929 | H | $CH_2CH_2CH_2OH$ | Pyridin-3-yl | |
| 2.1930 | H | $C_6H_5$ | Pyridin-3-yl | |
| 2.1931 | H | $CH_2Cl$ | Pyridin-3-yl | |
| 2.1932 | H | $CH_2CH_2Cl$ | Pyridin-3-yl | |
| 2.1933 | H | $CH_2CH_2CH_2Cl$ | Pyridin-3-yl | |

Tabelle 2.20

| No. | X | Y | Z | phys. Daten |
|---|---|---|---|---|
| 2.2001 | H | H | Pyridin-4-yl | |
| 2.2002 | H | $CH_3$ | Pyridin-4-yl | |
| 2.2003 | H | $C_2H_5$ | Pyridin-4-yl | |
| 2.2004 | H | $C_3H_7$ | Pyridin-4-yl | |
| 2.2005 | H | $OCH_3$ | Pyridin-4-yl | |
| 2.2006 | H | $OC_2H_5$ | Pyridin-4-yl | |
| 2.2007 | H | $OCH_2CH=CH_2$ | Pyridin-4-yl | |
| 2.2008 | H | $OCH_2CH=CHCH_3$ | Pyridin-4-yl | |
| 2.2009 | H | $OCH_2C(Cl)=CH_2$ | Pyridin-4-yl | |
| 2.2010 | H | $OCH_2CH=CHCl$ | Pyridin-4-yl | |
| 2.2011 | H | $OCH_2C{\equiv}CH$ | Pyridin-4-yl | |
| 2.2012 | H | $OCHF_2$ | Pyridin-4-yl | |
| 2.2013 | H | $OCF_3$ | Pyridin-4-yl | |
| 2.2014 | H | $OCF_2CHFCF_3$ | Pyridin-4-yl | |
| 2.2015 | H | $OC_6H_5$ | Pyridin-4-yl | |
| 2.2016 | H | $CF_3$ | Pyridin-4-yl | |
| 2.2017 | H | Br | Pyridin-4-yl | |
| 2.2018 | H | Cl | Pyridin-4-yl | |
| 2.2019 | H | F | Pyridin-4-yl | |
| 2.2020 | H | $SCH_3$ | Pyridin-4-yl | |
| 2.2021 | H | $SC_2H_5$ | Pyridin-4-yl | |
| 2.2022 | H | $SC_3H_7$-n | Pyridin-4-yl | |
| 2.2023 | H | $SO_2CH_3$ | Pyridin-4-yl | |
| 2.2024 | H | $SO_2C_2H_5$ | Pyridin-4-yl | |
| 2.2025 | H | $NO_2$ | Pyridin-4-yl | |
| 2.2026 | H | CN | Pyridin-4-yl | |
| 2.2027 | H | $CH_2OH$ | Pyridin-4-yl | |
| 2.2028 | H | $CH_2CH_2OH$ | Pyridin-4-yl | |
| 2.2029 | H | $CH_2CH_2CH_2OH$ | Pyridin-4-yl | |
| 2.2030 | H | $C_6H_5$ | Pyridin-4-yl | |
| 2.2031 | H | $CH_2Cl$ | Pyridin-4-yl | |
| 2.2032 | H | $CH_2CH_2Cl$ | Pyridin-4-yl | |
| 2.2033 | H | $CH_2CH_2CH_2Cl$ | Pyridin-4-yl | |

Tabelle 2.21

| No. | X | Y | Z | phys. Daten |
|---|---|---|---|---|
| 2.2101 | H | H | Pyridin-2-yl | |
| 2.2102 | H | $CH_3$ | Pyridin-2-yl | |
| 2.2103 | H | $C_2H_5$ | Pyridin-2-yl | |
| 2.2104 | H | $C_3H_7$ | Pyridin-2-yl | |
| 2.2105 | H | $OCH_3$ | Pyridin-2-yl | |
| 2.2106 | H | $OC_2H_5$ | Pyridin-2-yl | |
| 2.2107 | H | $OCH_2CH=CH_2$ | Pyridin-2-yl | |
| 2.2108 | H | $OCH_2CH=CHCH_3$ | Pyridin-2-yl | |
| 2.2109 | H | $OCH_2C(Cl)=CH_2$ | Pyridin-2-yl | |
| 2.2110 | H | $OCH_2CH=CHCl$ | Pyridin-2-yl | |
| 2.2111 | H | $OCH_2C{\equiv}CH$ | Pyridin-2-yl | |
| 2.2112 | H | $OCHF_2$ | Pyridin-2-yl | |
| 2.2113 | H | $OCF_3$ | Pyridin-2-yl | |
| 2.2114 | H | $OCF_2CHFCF_3$ | Pyridin-2-yl | |
| 2.2115 | H | $OC_6H_5$ | Pyridin-2-yl | |
| 2.2116 | H | $CF_3$ | Pyridin-2-yl | |
| 2.2117 | H | Br | Pyridin-2-yl | |
| 2.2118 | H | Cl | Pyridin-2-yl | |
| 2.2119 | H | F | Pyridin-2-yl | |
| 2.2120 | H | $SCH_3$ | Pyridin-2-yl | |
| 2.2121 | H | $SC_2H_5$ | Pyridin-2-yl | |
| 2.2122 | H | $SC_3H_7$-n | Pyridin-2-yl | |
| 2.2123 | H | $SO_2CH_3$ | Pyridin-2-yl | |
| 2.2124 | H | $SO_2C_2H_5$ | Pyridin-2-yl | |
| 2.2125 | H | $NO_2$ | Pyridin-2-yl | |
| 2.2126 | H | CN | Pyridin-2-yl | |
| 2.2127 | H | $CH_2OH$ | Pyridin-2-yl | |
| 2.2128 | H | $CH_2CH_2OH$ | Pyridin-2-yl | |
| 2.2129 | H | $CH_2CH_2CH_2OH$ | Pyridin-2-yl | |
| 2.2130 | H | $C_6H_5$ | Pyridin-2-yl | |
| 2.2131 | H | $CH_2Cl$ | Pyridin-2-yl | |
| 2.2132 | H | $CH_2CH_2Cl$ | Pyridin-2-yl | |
| 2.2133 | H | $CH_2CH_2CH_2Cl$ | Pyridin-2-yl | |

Tabelle 2.22

| No. | X | Y | Z | phys. Daten |
|---|---|---|---|---|
| 2.2201 | H | H | Furan-3-yl | |
| 2.2202 | H | $CH_3$ | Furan-3-yl | |
| 2.2203 | H | $C_2H_5$ | Furan-3-yl | |
| 2.2204 | H | $C_3H_7$ | Furan-3-yl | |
| 2.2205 | H | $OCH_3$ | Furan-3-yl | |
| 2.2206 | H | $OC_2H_5$ | Furan-3-yl | |
| 2.2207 | H | $OCH_2CH=CH_2$ | Furan-3-yl | |
| 2.2208 | H | $OCH_2CH=CHCH_3$ | Furan-3-yl | |
| 2.2209 | H | $OCH_2C(Cl)=CH_2$ | Furan-3-yl | |
| 2.2210 | H | $OCH_2CH=CHCl$ | Furan-3-yl | |
| 2.2211 | H | $OCH_2C \equiv CH$ | Furan-3-yl | |
| 2.2212 | H | $OCHF_2$ | Furan-3-yl | |
| 2.2213 | H | $OCF_3$ | Furan-3-yl | |
| 2.2214 | H | $OCF_2CHFCF_3$ | Furan-3-yl | |
| 2.2215 | H | $OC_6H_5$ | Furan-3-yl | |
| 2.2216 | H | $CF_3$ | Furan-3-yl | |
| 2.2217 | H | Br | Furan-3-yl | |
| 2.2218 | H | Cl | Furan-3-yl | |
| 2.2219 | H | F | Furan-3-yl | |
| 2.2220 | H | $SCH_3$ | Furan-3-yl | |
| 2.2221 | H | $SC_2H_5$ | Furan-3-yl | |
| 2.2222 | H | $SC_3H_7$-n | Furan-3-yl | |
| 2.2223 | H | $SO_2CH_3$ | Furan-3-yl | |
| 2.2224 | H | $SO_2C_2H_5$ | Furan-3-yl | |
| 2.2225 | H | $NO_2$ | Furan-3-yl | |
| 2.2226 | H | CN | Furan-3-yl | |
| 2.2227 | H | $CH_2OH$ | Furan-3-yl | |
| 2.2228 | H | $CH_2CH_2OH$ | Furan-3-yl | |
| 2.2229 | H | $CH_2CH_2CH_2OH$ | Furan-3-yl | |
| 2.2230 | H | $C_6H_5$ | Furan-3-yl | |
| 2.2231 | H | $CH_2Cl$ | Furan-3-yl | |
| 2.2232 | H | $CH_2CH_2Cl$ | Furan-3-yl | |
| 2.2233 | H | $CH_2CH_2CH_2Cl$ | Furan-3-yl | |

Tabelle 2.23

| No. | X | Y | Z | phys. Daten |
|---|---|---|---|---|
| 2.2301 | H | H | 4-Methylpyrimidin-6-yl | |
| 2.2302 | H | $CH_3$ | 4-Methylpyrimidin-6-yl | |
| 2.2303 | H | $C_2H_5$ | 4-Methylpyrimidin-6-yl | |
| 2.2304 | H | $C_3H_7$ | 4-Methylpyrimidin-6-yl | |
| 2.2305 | H | $OCH_3$ | 4-Methylpyrimidin-6-yl | |
| 2.2306 | H | $OC_2H_5$ | 4-Methylpyrimidin-6-yl | |
| 2.2307 | H | $OCH_2CH=CH_2$ | 4-Methylpyrimidin-6-yl | |
| 2.2308 | H | $OCH_2CH=CHCH_3$ | 4-Methylpyrimidin-6-yl | |
| 2.2309 | H | $OCH_2C(Cl)=CH_2$ | 4-Methylpyrimidin-6-yl | |
| 2.2310 | H | $OCH_2CH=CHCl$ | 4-Methylpyrimidin-6-yl | |
| 2.2311 | H | $OCH_2C\equiv CH$ | 4-Methylpyrimidin-6-yl | |
| 2.2312 | H | $OCHF_2$ | 4-Methylpyrimidin-6-yl | |
| 2.2313 | H | $OCF_3$ | 4-Methylpyrimidin-6-yl | |
| 2.2314 | H | $OCF_2CHFCF_3$ | 4-Methylpyrimidin-6-yl | |
| 2.2315 | H | $OC_6H_5$ | 4-Methylpyrimidin-6-yl | |
| 2.2316 | H | $CF_3$ | 4-Methylpyrimidin-6-yl | |
| 2.2317 | H | Br | 4-Methylpyrimidin-6-yl | |
| 2.2318 | H | Cl | 4-Methylpyrimidin-6-yl | |
| 2.2319 | H | F | 4-Methylpyrimidin-6-yl | |
| 2.2320 | H | $SCH_3$ | 4-Methylpyrimidin-6-yl | |
| 2.2321 | H | $SC_2H_5$ | 4-Methylpyrimidin-6-yl | |
| 2.2322 | H | $SC_3H_7$-n | 4-Methylpyrimidin-6-yl | |
| 2.2323 | H | $SO_2CH_3$ | 4-Methylpyrimidin-6-yl | |
| 2.2324 | H | $SO_2C_2H_5$ | 4-Methylpyrimidin-6-yl | |
| 2.2325 | H | $NO_2$ | 4-Methylpyrimidin-6-yl | |
| 2.2326 | H | CN | 4-Methylpyrimidin-6-yl | |
| 2.2327 | H | $CH_2OH$ | 4-Methylpyrimidin-6-yl | |
| 2.2328 | H | $CH_2CH_2OH$ | 4-Methylpyrimidin-6-yl | |
| 2.2329 | H | $CH_2CH_2CH_2OH$ | 4-Methylpyrimidin-6-yl | |
| 2.2330 | H | $C_6H_5$ | 4-Methylpyrimidin-6-yl | |
| 2.2331 | H | $CH_2Cl$ | 4-Methylpyrimidin-6-yl | |
| 2.2332 | H | $CH_2CH_2Cl$ | 4-Methylpyrimidin-6-yl | |
| 2.2333 | H | $CH_2CH_2CH_2Cl$ | 4-Methylpyrimidin-6-yl | |

Tabelle 2.24

| No. | X | Y | Z | phys. Daten |
|---|---|---|---|---|
| 2.2401 | H | H | 1,3,5-Triazin-2-yl | |
| 2.2402 | H | $CH_3$ | 1,3,5-Triazin-2-yl | |
| 2.2403 | H | $C_2H_5$ | 1,3,5-Triazin-2-yl | |
| 2.2404 | H | $C_3H_7$ | 1,3,5-Triazin-2-yl | |
| 2.2405 | H | $OCH_3$ | 1,3,5-Triazin-2-yl | |
| 2.2406 | H | $OC_2H_5$ | 1,3,5-Triazin-2-yl | |
| 2.2407 | H | $OCH_2CH{=}CH_2$ | 1,3,5-Triazin-2-yl | |
| 2.2408 | H | $OCH_2CH{=}CHCH_3$ | 1,3,5-Triazin-2-yl | |
| 2.2409 | H | $OCH_2C(Cl){=}CH_2$ | 1,3,5-Triazin-2-yl | |
| 2.2410 | H | $OCH_2CH{=}CHCl$ | 1,3,5-Triazin-2-yl | |
| 2.2411 | H | $OCH_2C{\equiv}CH$ | 1,3,5-Triazin-2-yl | |
| 2.2412 | H | $OCHF_2$ | 1,3,5-Triazin-2-yl | |
| 2.2413 | H | $OCF_3$ | 1,3,5-Triazin-2-yl | |
| 2.2414 | H | $OCF_2CHFCF_3$ | 1,3,5-Triazin-2-yl | |
| 2.2415 | H | $OC_6H_5$ | 1,3,5-Triazin-2-yl | |
| 2.2416 | H | $CF_3$ | 1,3,5-Triazin-2-yl | |
| 2.2417 | H | Br | 1,3,5-Triazin-2-yl | |
| 2.2418 | H | Cl | 1,3,5-Triazin-2-yl | |
| 2.2419 | H | F | 1,3,5-Triazin-2-yl | |
| 2.2420 | H | $SCH_3$ | 1,3,5-Triazin-2-yl | |
| 2.2421 | H | $SC_2H_5$ | 1,3,5-Triazin-2-yl | |
| 2.2422 | H | $SC_3H_7$-n | 1,3,5-Triazin-2-yl | |
| 2.2423 | H | $SO_2CH_3$ | 1,3,5-Triazin-2-yl | |
| 2.2424 | H | $SO_2C_2H_5$ | 1,3,5-Triazin-2-yl | |
| 2.2425 | H | $NO_2$ | 1,3,5-Triazin-2-yl | |
| 2.2426 | H | CN | 1,3,5-Triazin-2-yl | |
| 2.2427 | H | $CH_2OH$ | 1,3,5-Triazin-2-yl | |
| 2.2428 | H | $CH_2CH_2OH$ | 1,3,5-Triazin-2-yl | |
| 2.2429 | H | $CH_2CH_2CH_2OH$ | 1,3,5-Triazin-2-yl | |
| 2.2430 | H | $C_6H_5$ | 1,3,5-Triazin-2-yl | |
| 2.2431 | H | $CH_2Cl$ | 1,3,5-Triazin-2-yl | |
| 2.2432 | H | $CH_2CH_2Cl$ | 1,3,5-Triazin-2-yl | |
| 2.2433 | H | $CH_2CH_2CH_2Cl$ | 1,3,5-Triazin-2-yl | |

Tabelle 2.25

| No. | X | Y | Z | phys. Daten |
|---|---|---|---|---|
| 2.2501 | H | H | Oxazol-2-yl | |
| 2.2502 | H | $CH_3$ | Oxazol-2-yl | |
| 2.2503 | H | $C_2H_5$ | Oxazol-2-yl | |
| 2.2504 | H | $C_3H_7$ | Oxazol-2-yl | |
| 2.2505 | H | $OCH_3$ | Oxazol-2-yl | |
| 2.2506 | H | $OC_2H_5$ | Oxazol-2-yl | |
| 2.2507 | H | $OCH_2CH{=}CH_2$ | Oxazol-2-yl | |
| 2.2508 | H | $OCH_2CH{=}CHCH_3$ | Oxazol-2-yl | |
| 2.2509 | H | $OCH_2C(Cl){=}CH_2$ | Oxazol-2-yl | |
| 2.2510 | H | $OCH_2CH{=}CHCl$ | Oxazol-2-yl | |
| 2.2511 | H | $OCH_2C{\equiv}CH$ | Oxazol-2-yl | |
| 2.2512 | H | $OCHF_2$ | Oxazol-2-yl | |
| 2.2513 | H | $OCF_3$ | Oxazol-2-yl | |
| 2.2514 | H | $OCF_2CHFCF_3$ | Oxazol-2-yl | |
| 2.2515 | H | $OC_6H_5$ | Oxazol-2-yl | |
| 2.2516 | H | $CF_3$ | Oxazol-2-yl | |
| 2.2517 | H | Br | Oxazol-2-yl | |
| 2.2518 | H | Cl | Oxazol-2-yl | |
| 2.2519 | H | F | Oxazol-2-yl | |
| 2.2520 | H | $SCH_3$ | Oxazol-2-yl | |
| 2.2521 | H | $SC_2H_5$ | Oxazol-2-yl | |
| 2.2522 | H | $SC_3H_7$-n | Oxazol-2-yl | |
| 2.2523 | H | $SO_2CH_3$ | Oxazol-2-yl | |
| 2.2524 | H | $SO_2C_2H_5$ | Oxazol-2-yl | |
| 2.2525 | H | $NO_2$ | Oxazol-2-yl | |
| 2.2526 | H | CN | Oxazol-2-yl | |
| 2.2527 | H | $CH_2OH$ | Oxazol-2-yl | |
| 2.2528 | H | $CH_2CH_2OH$ | Oxazol-2-yl | |
| 2.2529 | H | $CH_2CH_2CH_2OH$ | Oxazol-2-yl | |
| 2.2530 | H | $C_6H_5$ | Oxazol-2-yl | |
| 2.2531 | H | $CH_2Cl$ | Oxazol-2-yl | |
| 2.2532 | H | $CH_2CH_2Cl$ | Oxazol-2-yl | |
| 2.2533 | H | $CH_2CH_2CH_2Cl$ | Oxazol-2-yl | |

Tabelle 2.26

| No. | X | Y | Z | phys. Daten |
|---|---|---|---|---|
| 2.2601 | H | H | Triazol-2-yl | |
| 2.2602 | H | $CH_3$ | Triazol-2-yl | |
| 2.2603 | H | $C_2H_5$ | Triazol-2-yl | |
| 2.2604 | H | $C_3H_7$ | Triazol-2-yl | |
| 2.2605 | H | $OCH_3$ | Triazol-2-yl | |
| 2.2606 | H | $OC_2H_5$ | Triazol-2-yl | |
| 2.2607 | H | $OCH_2CH=CH_2$ | Triazol-2-yl | |
| 2.2608 | H | $OCH_2CH=CHCH_3$ | Triazol-2-yl | |
| 2.2609 | H | $OCH_2C(Cl)=CH_2$ | Triazol-2-yl | |
| 2.2610 | H | $OCH_2CH=CHCl$ | Triazol-2-yl | |
| 2.2611 | H | $OCH_2C\equiv CH$ | Triazol-2-yl | |
| 2.2612 | H | $OCHF_2$ | Triazol-2-yl | |
| 2.2613 | H | $OCF_3$ | Triazol-2-yl | |
| 2.2614 | H | $OCF_2CHFCF_3$ | Triazol-2-yl | |
| 2.2615 | H | $OC_6H_5$ | Triazol-2-yl | |
| 2.2616 | H | $CF_3$ | Triazol-2-yl | |
| 2.2617 | H | Br | Triazol-2-yl | |
| 2.2618 | H | Cl | Triazol-2-yl | |
| 2.2619 | H | F | Triazol-2-yl | |
| 2.2620 | H | $SCH_3$ | Triazol-2-yl | |
| 2.2621 | H | $SC_2H_5$ | Triazol-2-yl | |
| 2.2622 | H | $SC_3H_7$-n | Triazol-2-yl | |
| 2.2623 | H | $SO_2CH_3$ | Triazol-2-yl | |
| 2.2624 | H | $SO_2C_2H_5$ | Triazol-2-yl | |
| 2.2625 | H | $NO_2$ | Triazol-2-yl | |
| 2.2626 | H | CN | Triazol-2-yl | |
| 2.2627 | H | $CH_2OH$ | Triazol-2-yl | |
| 2.2628 | H | $CH_2CH_2OH$ | Triazol-2-yl | |
| 2.2629 | H | $CH_2CH_2CH_2OH$ | Triazol-2-yl | |
| 2.2630 | H | $C_6H_5$ | Triazol-2-yl | |
| 2.2631 | H | $CH_2Cl$ | Triazol-2-yl | |
| 2.2632 | H | $CH_2CH_2Cl$ | Triazol-2-yl | |
| 2.2633 | H | $CH_2CH_2CH_2Cl$ | Triazol-2-yl | |

Beispiel 9 Herstellung von 8-Cyclopropyl-2,7-dimethyl-3,5-dioxo-2-isopropyl-imidazo-[2′,1′:1,2]5H-pyrrolo[3,4-6]pyridin

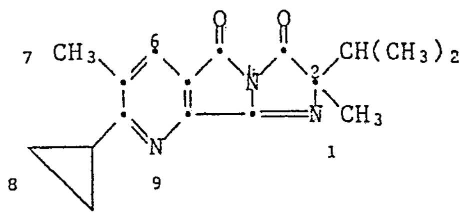

Eine Lösung von 13 g 6-Cyclopropyl-5-methyl-2-(5-isopropyl-5-methyl-4-oxo-imidazolin-2-yl)-pyridin-3-carbonsäure und 9,3-Dicyclohexylcarbodiimid in 200 ml Methylenchlorid wird während einer Stunde bei Zimmertemperatur gerührt. Das Reaktionsgemisch wird dann filtriert und das Filtrat eingeengt. Der Rückstand wird mit Aether berrührt und kristallisiert. Man erhält so 7,2 Titelverbindung vom Schmelzpunkt 185-187°C.

Beispiel 10 Herstellung von 7-Aethyl-3,5-dioxo-2-isopropyl-8-methoxymethyl-2-methyl-imidazo-[2,1:5,1]5H-pyrrolo[3,4-e]pyridin

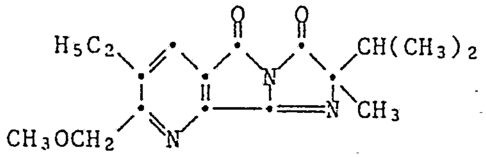

Zu einer Lösung von 7,9 g 5-Aethyl-6-methoxymethyl-2-(5-isopropyl-5-methyl-4-oxo-imidazolin-2-yl)pyridin-3-carbonsäure in 150 ml Methylenchlorid gibt man 4,9 g Dicyclohexylcarbodiimid und rührt eine Stunde bei Raumtemperatur. Das Reaktionsgemisch wird dann filtriert, das Filtrat eingeengt und der Rückstand mit Aether verrührt. Man erhält so 3,4 g kristallines Titelprodukt, welches bei 121-123°C schmilzt.

In analoger Weise zu den Beispielen 9 und 10 werden die tricyclischen Verbindungen der Tabellen 3.01 bis 3.16 hergestellt

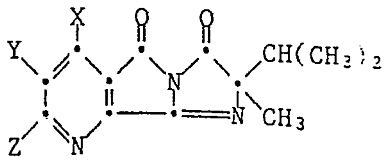

(II)

Tabelle 3.00

| No. | X | Y | Z | phys. Daten |
|---|---|---|---|---|
| 3.0001 | H | H | $C_3H_5$(Cyclo) | |
| 3.0002 | H | $CH_3$ | $C_3H_5$(Cyclo) | Smp. 187-192°C |
| 3.0003 | H | $C_2H_5$ | $C_3H_5$(Cyclo) | Smp. 170-180°C |
| 3.0004 | H | $C_3H_7$ | $C_3H_5$(Cyclo) | |
| 3.0005 | H | $OCH_3$ | $C_3H_5$(Cyclo) | |
| 3.0006 | H | $OC_2H_5$ | $C_3H_5$(Cyclo) | |
| 3.0007 | H | $OCH_2CH=CH_2$ | $C_3H_5$(Cyclo) | |
| 3.0008 | H | $OCH_2CH=CHCH_3$ | $C_3H_5$(Cyclo) | |
| 3.0009 | H | $OCH_2C(Cl)=CH_2$ | $C_3H_5$(Cyclo) | |
| 3.0010 | H | $OCH_2CH=CHCl$ | $C_3H_5$(Cyclo) | |
| 3.0011 | H | $OCH_2C\equiv CH$ | $C_3H_5$(Cyclo) | |
| 3.0012 | H | $OCHF_2$ | $C_3H_5$(Cyclo) | |
| 3.0013 | H | $OCF_3$ | $C_3H_5$(Cyclo) | |
| 3.0014 | H | $OCF_2CHFCF_3$ | $C_3H_5$(Cyclo) | |
| 3.0015 | H | $OC_6H_5$ | $C_3H_5$(Cyclo) | |
| 3.0016 | H | $CF_3$ | $C_3H_5$(Cyclo) | |
| 3.0017 | H | Br | $C_3H_5$(Cyclo) | |
| 3.0018 | H | Cl | $C_3H_5$(Cyclo) | |
| 3.0019 | H | F | $C_3H_5$(Cyclo) | |
| 3.0020 | H | $SCH_3$ | $C_3H_5$(Cyclo) | |
| 3.0021 | H | $SC_2H_5$ | $C_3H_5$(Cyclo) | |
| 3.0022 | H | $SC_3H_7$-n | $C_3H_5$(Cyclo) | |
| 3.0023 | H | $SO_2CH_3$ | $C_3H_5$(Cyclo) | |
| 3.0024 | H | $SO_2C_2H_5$ | $C_3H_5$(Cyclo) | |
| 3.0025 | H | $NO_2$ | $C_3H_5$(Cyclo) | |
| 3.0026 | H | CN | $C_3H_5$(Cyclo) | |
| 3.0027 | H | $CH_2OH$ | $C_3H_5$(Cyclo) | |
| 3.0028 | H | $CH_2CH_2OH$ | $C_3H_5$(Cyclo) | |
| 3.0029 | H | $CH_2CH_2CH_2OH$ | $C_3H_5$(Cyclo) | |
| 3.0030 | H | $C_6H_5$ | $C_3H_5$(Cyclo) | |
| 3.0031 | H | $CH_2Cl$ | $C_3H_5$(Cyclo) | |
| 3.0032 | H | $CH_2CH_2Cl$ | $C_3H_5$(Cyclo) | |
| 3.0033 | H | $CH_2CH_2CH_2Cl$ | $C_3H_5$(Cyclo) | |

Tabelle 3.01

| No. | X | Y | Z | phys. Daten |
|---|---|---|---|---|
| 3.0101 | H | H | $C_4H_7$(Cyclo) | |
| 3.0102 | H | $CH_3$ | $C_4H_7$(Cyclo) | |
| 3.0103 | H | $C_2H_5$ | $C_4H_7$(Cyclo) | |
| 3.0104 | H | $C_3H_7$ | $C_4H_7$(Cyclo) | |
| 3.0105 | H | $OCH_3$ | $C_4H_7$(Cyclo) | |
| 3.0106 | H | $OC_2H_5$ | $C_4H_7$(Cyclo) | |
| 3.0107 | H | $OCH_2CH{=}CH_2$ | $C_4H_7$(Cyclo) | |
| 3.0108 | H | $OCH_2CH{=}CHCH_3$ | $C_4H_7$(Cyclo) | |
| 3.0109 | H | $OCH_2C(Cl){=}CH_2$ | $C_4H_7$(Cyclo) | |
| 3.0110 | H | $OCH_2CH{=}CHCl$ | $C_4H_7$(Cyclo) | |
| 3.0111 | H | $OCH_2C{\equiv}CH$ | $C_4H_7$(Cyclo) | |
| 3.0112 | H | $OCHF_2$ | $C_4H_7$(Cyclo) | |
| 3.0113 | H | $OCF_3$ | $C_4H_7$(Cyclo) | |
| 3.0114 | H | $OCF_2CHFCF_3$ | $C_4H_7$(Cyclo) | |
| 3.0115 | H | $OC_6H_5$ | $C_4H_7$(Cyclo) | |
| 3.0116 | H | $CF_3$ | $C_4H_7$(Cyclo) | |
| 3.0117 | H | Br | $C_4H_7$(Cyclo) | |
| 3.0118 | H | Cl | $C_4H_7$(Cyclo) | |
| 3.0119 | H | F | $C_4H_7$(Cyclo) | |
| 3.0120 | H | $SCH_3$ | $C_4H_7$(Cyclo) | |
| 3.0121 | H | $SC_2H_5$ | $C_4H_7$(Cyclo) | |
| 3.0122 | H | $SC_3H_7$-n | $C_4H_7$(Cyclo) | |
| 3.0123 | H | $SO_2CH_3$ | $C_4H_7$(Cyclo) | |
| 3.0124 | H | $SO_2C_2H_5$ | $C_4H_7$(Cyclo) | |
| 3.0125 | H | $NO_2$ | $C_4H_7$(Cyclo) | |
| 3.0126 | H | CN | $C_4H_7$(Cyclo) | |
| 3.0127 | H | $CH_2OH$ | $C_4H_7$(Cyclo) | |
| 3.0128 | H | $CH_2CH_2OH$ | $C_4H_7$(Cyclo) | |
| 3.0129 | H | $CH_2CH_2CH_2OH$ | $C_4H_7$(Cyclo) | |
| 3.0130 | H | $C_6H_5$ | $C_4H_7$(Cyclo) | |
| 3.0131 | H | $CH_2Cl$ | $C_4H_7$(Cyclo) | |
| 3.0132 | H | $CH_2CH_2Cl$ | $C_4H_7$(Cyclo) | |
| 3.0133 | H | $CH_2CH_2CH_2Cl$ | $C_4H_7$(Cyclo) | |

Tabelle 3.02

| No. | X | Y | Z | phys. Daten |
|---|---|---|---|---|
| 3.0201 | H | H | $C_5H_9$(Cyclo) | |
| 3.0202 | H | $CH_3$ | $C_5H_9$(Cyclo) | |
| 3.0203 | H | $C_2H_5$ | $C_5H_9$(Cyclo) | |
| 3.0204 | H | $C_3H_7$ | $C_5H_9$(Cyclo) | |
| 3.0205 | H | $OCH_3$ | $C_5H_9$(Cyclo) | |
| 3.0206 | H | $OC_2H_5$ | $C_5H_9$(Cyclo) | |
| 3.0207 | H | $OCH_2CH{=}CH_2$ | $C_5H_9$(Cyclo) | |
| 3.0208 | H | $OCH_2CH{=}CHCH_3$ | $C_5H_9$(Cyclo) | |
| 3.0209 | H | $OCH_2C(Cl){=}CH_2$ | $C_5H_9$(Cyclo) | |
| 3.0210 | H | $OCH_2CH{=}CHCl$ | $C_5H_9$(Cyclo) | |
| 3.0211 | H | $OCH_2C{\equiv}CH$ | $C_5H_9$(Cyclo) | |
| 3.0212 | H | $OCHF_2$ | $C_5H_9$(Cyclo) | |
| 3.0213 | H | $OCF_3$ | $C_5H_9$(Cyclo) | |
| 3.0214 | H | $OCF_2CHFCF_3$ | $C_5H_9$(Cyclo) | |
| 3.0215 | H | $OC_6H_5$ | $C_5H_9$(Cyclo) | |
| 3.0216 | H | $CF_3$ | $C_5H_9$(Cyclo) | |
| 3.0217 | H | Br | $C_5H_9$(Cyclo) | |
| 3.0218 | H | Cl | $C_5H_9$(Cyclo) | |
| 3.0219 | H | F | $C_5H_9$(Cyclo) | |
| 3.0220 | H | $SCH_3$ | $C_5H_9$(Cyclo) | |
| 3.0221 | H | $SC_2H_5$ | $C_5H_9$(Cyclo) | |
| 3.0222 | H | $SC_3H_7$-n | $C_5H_9$(Cyclo) | |
| 3.0223 | H | $SO_2CH_3$ | $C_5H_9$(Cyclo) | |
| 3.0224 | H | $SO_2C_2H_5$ | $C_5H_9$(Cyclo) | |
| 3.0225 | H | $NO_2$ | $C_5H_9$(Cyclo) | |
| 3.0226 | H | CN | $C_5H_9$(Cyclo) | |
| 3.0227 | H | $CH_2OH$ | $C_5H_9$(Cyclo) | |
| 3.0228 | H | $CH_2CH_2OH$ | $C_5H_9$(Cyclo) | |
| 3.0229 | H | $CH_2CH_2CH_2OH$ | $C_5H_9$(Cyclo) | |
| 3.0230 | H | $C_6H_5$ | $C_5H_9$(Cyclo) | |
| 3.0231 | H | $CH_2Cl$ | $C_5H_9$(Cyclo) | |
| 3.0232 | H | $CH_2CH_2Cl$ | $C_5H_9$(Cyclo) | |
| 3.0233 | H | $CH_2CH_2CH_2Cl$ | $C_5H_9$(Cyclo) | |

Tabelle 3.03

| No. | X | Y | Z | phys. Daten |
|---|---|---|---|---|
| 3.0301 | H | H | $C_6H_{11}$(Cyclo) | |
| 3.0302 | H | $CH_3$ | $C_6H_{11}$(Cyclo) | |
| 3.0303 | H | $C_2H_5$ | $C_6H_{11}$(Cyclo) | |
| 3.0304 | H | $C_3H_7$ | $C_6H_{11}$(Cyclo) | |
| 3.0305 | H | $OCH_3$ | $C_6H_{11}$(Cyclo) | |
| 3.0306 | H | $OC_2H_5$ | $C_6H_{11}$(Cyclo) | |
| 3.0307 | H | $OCH_2CH{=}CH_2$ | $C_6H_{11}$(Cyclo) | |
| 3.0308 | H | $OCH_2CH{=}CHCH_3$ | $C_6H_{11}$(Cyclo) | |
| 3.0309 | H | $OCH_2C(Cl){=}CH_2$ | $C_6H_{11}$(Cyclo) | |
| 3.0310 | H | $OCH_2CH{=}CHCl$ | $C_6H_{11}$(Cyclo) | |
| 3.0311 | H | $OCH_2C{\equiv}CH$ | $C_6H_{11}$(Cyclo) | |
| 3.0312 | H | $OCHF_2$ | $C_6H_{11}$(Cyclo) | |
| 3.0313 | H | $OCF_3$ | $C_6H_{11}$(Cyclo) | |
| 3.0314 | H | $OCF_2CHFCF_3$ | $C_6H_{11}$(Cyclo) | |
| 3.0315 | H | $OC_6H_5$ | $C_6H_{11}$(Cyclo) | |
| 3.0316 | H | $CF_3$ | $C_6H_{11}$(Cyclo) | |
| 3.0317 | H | Br | $C_6H_{11}$(Cyclo) | |
| 3.0318 | H | Cl | $C_6H_{11}$(Cyclo) | |
| 3.0319 | H | F | $C_6H_{11}$(Cyclo) | |
| 3.0320 | H | $SCH_3$ | $C_6H_{11}$(Cyclo) | |
| 3.0321 | H | $SC_2H_5$ | $C_6H_{11}$(Cyclo) | |
| 3.0322 | H | $SC_3H_7$-n | $C_6H_{11}$(Cyclo) | |
| 3.0323 | H | $SO_2CH_3$ | $C_6H_{11}$(Cyclo) | |
| 3.0324 | H | $SO_2C_2H_5$ | $C_6H_{11}$(Cyclo) | |
| 3.0325 | H | $NO_2$ | $C_6H_{11}$(Cyclo) | |
| 3.0326 | H | CN | $C_6H_{11}$(Cyclo) | |
| 3.0327 | H | $CH_2OH$ | $C_6H_{11}$(Cyclo) | |
| 3.0328 | H | $CH_2CH_2OH$ | $C_6H_{11}$(Cyclo) | |
| 3.0329 | H | $CH_2CH_2CH_2OH$ | $C_6H_{11}$(Cyclo) | |
| 3.0330 | H | $C_6H_5$ | $C_6H_{11}$(Cyclo) | |
| 3.0331 | H | $CH_2Cl$ | $C_6H_{11}$(Cyclo) | |
| 3.0332 | H | $CH_2CH_2Cl$ | $C_6H_{11}$(Cyclo) | |
| 3.0333 | H | $CH_2CH_2CH_2Cl$ | $C_6H_{11}$(Cyclo) | |

Tabelle 3.04

| No. | X | Y | Z | phys. Daten |
|---|---|---|---|---|
| 3.0401 | H | H | $CH_2OCH_3$ | |
| 3.0402 | H | $CH_3$ | $CH_2OCH_3$ | |
| 3.0403 | H | $C_2H_5$ | $CH_2OCH_3$ | 121-123°C |
| 3.0404 | H | $C_3H_7$ | $CH_2OCH_3$ | |
| 3.0405 | H | $OCH_3$ | $CH_2OCH_3$ | |
| 3.0406 | H | $OC_2H_5$ | $CH_2OCH_3$ | |
| 3.0407 | H | $OCH_2CH=CH_2$ | $CH_2OCH_3$ | |
| 3.0408 | H | $OCH_2CH=CHCH_3$ | $CH_2OCH_3$ | |
| 3.0409 | H | $OCH_2C(Cl)=CH_2$ | $CH_2OCH_3$ | |
| 3.0410 | H | $OCH_2CH=CHCl$ | $CH_2OCH_3$ | |
| 3.0411 | H | $OCH_2C{\equiv}CH$ | $CH_2OCH_3$ | |
| 3.0412 | H | $OCHF_2$ | $CH_2OCH_3$ | |
| 3.0413 | H | $OCF_3$ | $CH_2OCH_3$ | |
| 3.0414 | H | $OCF_2CHFCF_3$ | $CH_2OCH_3$ | |
| 3.0415 | H | $OC_6H_5$ | $CH_2OCH_3$ | |
| 3.0416 | H | $CF_3$ | $CH_2OCH_3$ | |
| 3.0417 | H | Br | $CH_2OCH_3$ | |
| 3.0418 | H | Cl | $CH_2OCH_3$ | |
| 3.0419 | H | F | $CH_2OCH_3$ | |
| 3.0420 | H | $SCH_3$ | $CH_2OCH_3$ | |
| 3.0421 | H | $SC_2H_5$ | $CH_2OCH_3$ | |
| 3.0422 | H | $SC_3H_7$-n | $CH_2OCH_3$ | |
| 3.0423 | H | $SO_2CH_3$ | $CH_2OCH_3$ | |
| 3.0424 | H | $SO_2C_2H_5$ | $CH_2OCH_3$ | |
| 3.0425 | H | $NO_2$ | $CH_2OCH_3$ | |
| 3.0426 | H | CN | $CH_2OCH_3$ | |
| 3.0427 | H | $CH_2OH$ | $CH_2OCH_3$ | |
| 3.0428 | H | $CH_2CH_2OH$ | $CH_2OCH_3$ | |
| 3.0429 | H | $CH_2CH_2CH_2OH$ | $CH_2OCH_3$ | |
| 3.0430 | H | $C_6H_5$ | $CH_2OCH_3$ | |
| 3.0431 | H | $CH_2Cl$ | $CH_2OCH_3$ | |
| 3.0432 | H | $CH_2CH_2Cl$ | $CH_2OCH_3$ | |
| 3.0433 | H | $CH_2CH_2CH_2Cl$ | $CH_2OCH_3$ | |

Tabelle 3.05

| No. | X | Y | Z | phys. Daten |
|---|---|---|---|---|
| 3.0501 | H | H | $CH(CH_3)OCH_3$ | |
| 3.0502 | H | $CH_3$ | $CH(CH_3)OCH_3$ | |
| 3.0503 | H | $C_2H_5$ | $CH(CH_3)OCH_3$ | |
| 3.0504 | H | $C_3H_7$ | $CH(CH_3)OCH_3$ | |
| 3.0505 | H | $OCH_3$ | $CH(CH_3)OCH_3$ | |
| 3.0506 | H | $OC_2H_5$ | $CH(CH_3)OCH_3$ | |
| 3.0507 | H | $OCH_2CH=CH_2$ | $CH(CH_3)OCH_3$ | |
| 3.0508 | H | $OCH_2CH=CHCH_3$ | $CH(CH_3)OCH_3$ | |
| 3.0509 | H | $OCH_2C(Cl)=CH_2$ | $CH(CH_3)OCH_3$ | |
| 3.0510 | H | $OCH_2CH=CHCl$ | $CH(CH_3)OCH_3$ | |
| 3.0511 | H | $OCH_2C\equiv CH$ | $CH(CH_3)OCH_3$ | |
| 3.0512 | H | $OCHF_2$ | $CH(CH_3)OCH_3$ | |
| 3.0513 | H | $OCF_3$ | $CH(CH_3)OCH_3$ | |
| 3.0514 | H | $OCF_2CHFCF_3$ | $CH(CH_3)OCH_3$ | |
| 3.0515 | H | $OC_6H_5$ | $CH(CH_3)OCH_3$ | |
| 3.0516 | H | $CF_3$ | $CH(CH_3)OCH_3$ | |
| 3.0517 | H | Br | $CH(CH_3)OCH_3$ | |
| 3.0518 | H | Cl | $CH(CH_3)OCH_3$ | |
| 3.0519 | H | F | $CH(CH_3)OCH_3$ | |
| 3.0520 | H | $SCH_3$ | $CH(CH_3)OCH_3$ | |
| 3.0521 | H | $SC_2H_5$ | $CH(CH_3)OCH_3$ | |
| 3.0522 | H | $SC_3H_7$-n | $CH(CH_3)OCH_3$ | |
| 3.0523 | H | $SO_2CH_3$ | $CH(CH_3)OCH_3$ | |
| 3.0524 | H | $SO_2C_2H_5$ | $CH(CH_3)OCH_3$ | |
| 3.0525 | H | $NO_2$ | $CH(CH_3)OCH_3$ | |
| 3.0526 | H | CN | $CH(CH_3)OCH_3$ | |
| 3.0527 | H | $CH_2OH$ | $CH(CH_3)OCH_3$ | |
| 3.0528 | H | $CH_2CH_2OH$ | $CH(CH_3)OCH_3$ | |
| 3.0529 | H | $CH_2CH_2CH_2OH$ | $CH(CH_3)OCH_3$ | |
| 3.0530 | H | $C_6H_5$ | $CH(CH_3)OCH_3$ | |
| 3.0531 | H | $CH_2Cl$ | $CH(CH_3)OCH_3$ | |
| 3.0532 | H | $CH_2CH_2Cl$ | $CH(CH_3)OCH_3$ | |
| 3.0533 | H | $CH_2CH_2CH_2Cl$ | $CH(CH_3)OCH_3$ | |

Tabelle 3.06

| No. | X | Y | Z | phys. Daten |
|---|---|---|---|---|
| 3.0601 | H | H | $CH_2OC_2H_5$ | Harz |
| 3.0602 | H | $CH_3$ | $CH_2OC_2H_5$ | |
| 3.0603 | H | $C_2H_5$ | $CH_2OC_2H_5$ | |
| 3.0604 | H | $C_3H_7$ | $CH_2OC_2H_5$ | |
| 3.0605 | H | $OCH_3$ | $CH_2OC_2H_5$ | |
| 3.0606 | H | $OC_2H_5$ | $CH_2OC_2H_5$ | |
| 3.0607 | H | $OCH_2CH{=}CH_2$ | $CH_2OC_2H_5$ | |
| 3.0608 | H | $OCH_2CH{=}CHCH_3$ | $CH_2OC_2H_5$ | |
| 3.0609 | H | $OCH_2C(Cl){=}CH_2$ | $CH_2OC_2H_5$ | |
| 3.0610 | H | $OCH_2CH{=}CHCl$ | $CH_2OC_2H_5$ | |
| 3.0611 | H | $OCH_2C{\equiv}CH$ | $CH_2OC_2H_5$ | |
| 3.0612 | H | $OCHF_2$ | $CH_2OC_2H_5$ | |
| 3.0613 | H | $OCF_3$ | $CH_2OC_2H_5$ | |
| 3.0614 | H | $OCF_2CHFCF_3$ | $CH_2OC_2H_5$ | |
| 3.0615 | H | $OC_6H_5$ | $CH_2OC_2H_5$ | |
| 3.0616 | H | $CF_3$ | $CH_2OC_2H_5$ | |
| 3.0617 | H | Br | $CH_2OC_2H_5$ | |
| 3.0618 | H | Cl | $CH_2OC_2H_5$ | |
| 3.0619 | H | F | $CH_2OC_2H_5$ | |
| 3.0620 | H | $SCH_3$ | $CH_2OC_2H_5$ | |
| 3.0621 | H | $SC_2H_5$ | $CH_2OC_2H_5$ | |
| 3.0622 | H | $SC_3H_7$-n | $CH_2OC_2H_5$ | |
| 3.0623 | H | $SO_2CH_3$ | $CH_2OC_2H_5$ | |
| 3.0624 | H | $SO_2C_2H_5$ | $CH_2OC_2H_5$ | |
| 3.0625 | H | $NO_2$ | $CH_2OC_2H_5$ | |
| 3.0626 | H | CN | $CH_2OC_2H_5$ | |
| 3.0627 | H | $CH_2OH$ | $CH_2OC_2H_5$ | |
| 3.0628 | H | $CH_2CH_2OH$ | $CH_2OC_2H_5$ | |
| 3.0629 | H | $CH_2CH_2CH_2OH$ | $CH_2OC_2H_5$ | |
| 3.0630 | H | $C_6H_5$ | $CH_2OC_2H_5$ | |
| 3.0631 | H | $CH_2Cl$ | $CH_2OC_2H_5$ | |
| 3.0632 | H | $CH_2CH_2Cl$ | $CH_2OC_2H_5$ | |
| 3.0633 | H | $CH_2CH_2CH_2Cl$ | $CH_2OC_2H_5$ | |

Tabelle 3.07

| No. | X | Y | Z | phys. Daten |
|---|---|---|---|---|
| 3.0701 | H | H | $CH(CH_3)OC_2H_5$ | |
| 3.0702 | H | $CH_3$ | $CH(CH_3)OC_2H_5$ | |
| 3.0703 | H | $C_2H_5$ | $CH(CH_3)OC_2H_5$ | |
| 3.0704 | H | $C_3H_7$ | $CH(CH_3)OC_2H_5$ | |
| 3.0705 | H | $OCH_3$ | $CH(CH_3)OC_2H_5$ | |
| 3.0706 | H | $OC_2H_5$ | $CH(CH_3)OC_2H_5$ | |
| 3.0707 | H | $OCH_2CH=CH_2$ | $CH(CH_3)OC_2H_5$ | |
| 3.0708 | H | $OCH_2CH=CHCH_3$ | $CH(CH_3)OC_2H_5$ | |
| 3.0709 | H | $OCH_2C(Cl)=CH_2$ | $CH(CH_3)OC_2H_5$ | |
| 3.0710 | H | $OCH_2CH=CHCl$ | $CH(CH_3)OC_2H_5$ | |
| 3.0711 | H | $OCH_2C\equiv CH$ | $CH(CH_3)OC_2H_5$ | |
| 3.0712 | H | $OCHF_2$ | $CH(CH_3)OC_2H_5$ | |
| 3.0713 | H | $OCF_3$ | $CH(CH_3)OC_2H_5$ | |
| 3.0714 | H | $OCF_2CHFCF_3$ | $CH(CH_3)OC_2H_5$ | |
| 3.0715 | H | $OC_6H_5$ | $CH(CH_3)OC_2H_5$ | |
| 3.0716 | H | $CF_3$ | $CH(CH_3)OC_2H_5$ | |
| 3.0717 | H | Br | $CH(CH_3)OC_2H_5$ | |
| 3.0718 | H | Cl | $CH(CH_3)OC_2H_5$ | |
| 3.0719 | H | F | $CH(CH_3)OC_2H_5$ | |
| 3.0720 | H | $SCH_3$ | $CH(CH_3)OC_2H_5$ | |
| 3.0721 | H | $SC_2H_5$ | $CH(CH_3)OC_2H_5$ | |
| 3.0722 | H | $SC_3H_7$-n | $CH(CH_3)OC_2H_5$ | |
| 3.0723 | H | $SO_2CH_3$ | $CH(CH_3)OC_2H_5$ | |
| 3.0724 | H | $SO_2C_2H_5$ | $CH(CH_3)OC_2H_5$ | |
| 3.0725 | H | $NO_2$ | $CH(CH_3)OC_2H_5$ | |
| 3.0726 | H | CN | $CH(CH_3)OC_2H_5$ | |
| 3.0727 | H | $CH_2OH$ | $CH(CH_3)OC_2H_5$ | |
| 3.0728 | H | $CH_2CH_2OH$ | $CH(CH_3)OC_2H_5$ | |
| 3.0729 | H | $CH_2CH_2CH_2OH$ | $CH(CH_3)OC_2H_5$ | |
| 3.0730 | H | $C_6H_5$ | $CH(CH_3)OC_2H_5$ | |
| 3.0731 | H | $CH_2Cl$ | $CH(CH_3)OC_2H_5$ | |
| 3.0732 | H | $CH_2CH_2Cl$ | $CH(CH_3)OC_2H_5$ | |
| 3.0733 | H | $CH_2CH_2CH_2Cl$ | $CH(CH_3)OC_2H_5$ | |

Tabelle 3.08

| No. | X | Y | Z | phys. Daten |
|---|---|---|---|---|
| 3.0801 | H | H | $C(CH_3)_2OCH_3$ | |
| 3.0802 | H | $CH_3$ | $C(CH_3)_2OCH_3$ | |
| 3.0803 | H | $C_2H_5$ | $C(CH_3)_2OCH_3$ | |
| 3.0804 | H | $C_3H_7$ | $C(CH_3)_2OCH_3$ | |
| 3.0805 | H | $OCH_3$ | $C(CH_3)_2OCH_3$ | |
| 3.0806 | H | $OC_2H_5$ | $C(CH_3)_2OCH_3$ | |
| 3.0807 | H | $OCH_2CH=CH_2$ | $C(CH_3)_2OCH_3$ | |
| 3.0808 | H | $OCH_2CH=CHCH_3$ | $C(CH_3)_2OCH_3$ | |
| 3.0809 | H | $OCH_2C(Cl)=CH_2$ | $C(CH_3)_2OCH_3$ | |
| 3.0810 | H | $OCH_2CH=CHCl$ | $C(CH_3)_2OCH_3$ | |
| 3.0811 | H | $OCH_2C{\equiv}CH$ | $C(CH_3)_2OCH_3$ | |
| 3.0812 | H | $OCHF_2$ | $C(CH_3)_2OCH_3$ | |
| 3.0813 | H | $OCF_3$ | $C(CH_3)_2OCH_3$ | |
| 3.0814 | H | $OCF_2CHFCF_3$ | $C(CH_3)_2OCH_3$ | |
| 3.0815 | H | $OC_6H_5$ | $C(CH_3)_2OCH_3$ | |
| 3.0816 | H | $CF_3$ | $C(CH_3)_2OCH_3$ | |
| 3.0817 | H | Br | $C(CH_3)_2OCH_3$ | |
| 3.0818 | H | Cl | $C(CH_3)_2OCH_3$ | |
| 3.0819 | H | F | $C(CH_3)_2OCH_3$ | |
| 3.0820 | H | $SCH_3$ | $C(CH_3)_2OCH_3$ | |
| 3.0821 | H | $SC_2H_5$ | $C(CH_3)_2OCH_3$ | |
| 3.0822 | H | $SC_3H_7$-n | $C(CH_3)_2OCH_3$ | |
| 3.0823 | H | $SO_2CH_3$ | $C(CH_3)_2OCH_3$ | |
| 3.0824 | H | $SO_2C_2H_5$ | $C(CH_3)_2OCH_3$ | |
| 3.0825 | H | $NO_2$ | $C(CH_3)_2OCH_3$ | |
| 3.0826 | H | CN | $C(CH_3)_2OCH_3$ | |
| 3.0827 | H | $CH_2OH$ | $C(CH_3)_2OCH_3$ | |
| 3.0828 | H | $CH_2CH_2OH$ | $C(CH_3)_2OCH_3$ | |
| 3.0829 | H | $CH_2CH_2CH_2OH$ | $C(CH_3)_2OCH_3$ | |
| 3.0830 | H | $C_6H_5$ | $C(CH_3)_2OCH_3$ | |
| 3.0831 | H | $CH_2Cl$ | $C(CH_3)_2OCH_3$ | |
| 3.0832 | H | $CH_2CH_2Cl$ | $C(CH_3)_2OCH_3$ | |
| 3.0833 | H | $CH_2CH_2CH_2Cl$ | $C(CH_3)_2OCH_3$ | |

Tabelle 3.09

| No. | X | Y | Z | phys. Daten |
|---|---|---|---|---|
| 3.0901 | H | H | $CH_2OC_3H_7$ | |
| 3.0902 | H | $CH_3$ | $CH_2OC_3H_7$ | |
| 3.0903 | H | $C_2H_5$ | $CH_2OC_3H_7$ | |
| 3.0904 | H | $C_3H_7$ | $CH_2OC_3H_7$ | |
| 3.0905 | H | $OCH_3$ | $CH_2OC_3H_7$ | |
| 3.0906 | H | $OC_2H_5$ | $CH_2OC_3H_7$ | |
| 3.0907 | H | $OCH_2CH=CH_2$ | $CH_2OC_3H_7$ | |
| 3.0908 | H | $OCH_2CH=CHCH_3$ | $CH_2OC_3H_7$ | |
| 3.0909 | H | $OCH_2C(Cl)=CH_2$ | $CH_2OC_3H_7$ | |
| 3.0910 | H | $OCH_2CH=CHCl$ | $CH_2OC_3H_7$ | |
| 3.0911 | H | $OCH_2C{\equiv}CH$ | $CH_2OC_3H_7$ | |
| 3.0912 | H | $OCHF_2$ | $CH_2OC_3H_7$ | |
| 3.0913 | H | $OCF_3$ | $CH_2OC_3H_7$ | |
| 3.0914 | H | $OCF_2CHFCF_3$ | $CH_2OC_3H_7$ | |
| 3.0915 | H | $OC_6H_5$ | $CH_2OC_3H_7$ | |
| 3.0916 | H | $CF_3$ | $CH_2OC_3H_7$ | |
| 3.0917 | H | Br | $CH_2OC_3H_7$ | |
| 3.0918 | H | Cl | $CH_2OC_3H_7$ | |
| 3.0919 | H | F | $CH_2OC_3H_7$ | |
| 3.0920 | H | $SCH_3$ | $CH_2OC_3H_7$ | |
| 3.0921 | H | $SC_2H_5$ | $CH_2OC_3H_7$ | |
| 3.0922 | H | $SC_3H_7$-n | $CH_2OC_3H_7$ | |
| 3.0923 | H | $SO_2CH_3$ | $CH_2OC_3H_7$ | |
| 3.0924 | H | $SO_2C_2H_5$ | $CH_2OC_3H_7$ | |
| 3.0925 | H | $NO_2$ | $CH_2OC_3H_7$ | |
| 3.0926 | H | CN | $CH_2OC_3H_7$ | |
| 3.0927 | H | $CH_2OH$ | $CH_2OC_3H_7$ | |
| 3.0928 | H | $CH_2CH_2OH$ | $CH_2OC_3H_7$ | |
| 3.0929 | H | $CH_2CH_2CH_2OH$ | $CH_2OC_3H_7$ | |
| 3.0930 | H | $C_6H_5$ | $CH_2OC_3H_7$ | |
| 3.0931 | H | $CH_2Cl$ | $CH_2OC_3H_7$ | |
| 3.0932 | H | $CH_2CH_2Cl$ | $CH_2OC_3H_7$ | |
| 3.0933 | H | $CH_2CH_2CH_2Cl$ | $CH_2OC_3H_7$ | |

Tabelle 3.10

| No. | X | Y | Z | phys. Daten |
|---|---|---|---|---|
| 3.1001 | H | H | $C(CH_3)C_2H_4$(Cyclo) | |
| 3.1002 | H | $CH_3$ | $C(CH_3)C_2H_4$(Cyclo) | |
| 3.1003 | H | $C_2H_5$ | $C(CH_3)C_2H_4$(Cyclo) | |
| 3.1004 | H | $C_3H_7$ | $C(CH_3)C_2H_4$(Cyclo) | |
| 3.1005 | H | $OCH_3$ | $C(CH_3)C_2H_4$(Cyclo) | |
| 3.1006 | H | $OC_2H_5$ | $C(CH_3)C_2H_4$(Cyclo) | |
| 3.1007 | H | $OCH_2CH{=}CH_2$ | $C(CH_3)C_2H_4$(Cyclo) | |
| 3.1008 | H | $OCH_2CH{=}CHCH_3$ | $C(CH_3)C_2H_4$(Cyclo) | |
| 3.1009 | H | $OCH_2C(Cl){=}CH_2$ | $C(CH_3)C_2H_4$(Cyclo) | |
| 3.1010 | H | $OCH_2CH{=}CHCl$ | $C(CH_3)C_2H_4$(Cyclo) | |
| 3.1011 | H | $OCH_2C{\equiv}CH$ | $C(CH_3)C_2H_4$(Cyclo) | |
| 3.1012 | H | $OCHF_2$ | $C(CH_3)C_2H_4$(Cyclo) | |
| 3.1013 | H | $OCF_3$ | $C(CH_3)C_2H_4$(Cyclo) | |
| 3.1014 | H | $OCF_2CHFCF_3$ | $C(CH_3)C_2H_4$(Cyclo) | |
| 3.1015 | H | $OC_6H_5$ | $C(CH_3)C_2H_4$(Cyclo) | |
| 3.1016 | H | $CF_3$ | $C(CH_3)C_2H_4$(Cyclo) | |
| 3.1017 | H | Br | $C(CH_3)C_2H_4$(Cyclo) | |
| 3.1018 | H | Cl | $C(CH_3)C_2H_4$(Cyclo) | |
| 3.1019 | H | F | $C(CH_3)C_2H_4$(Cyclo) | |
| 3.1020 | H | $SCH_3$ | $C(CH_3)C_2H_4$(Cyclo) | |
| 3.1021 | H | $SC_2H_5$ | $C(CH_3)C_2H_4$(Cyclo) | |
| 3.1022 | H | $SC_3H_7$-n | $C(CH_3)C_2H_4$(Cyclo) | |
| 3.1023 | H | $SO_2CH_3$ | $C(CH_3)C_2H_4$(Cyclo) | |
| 3.1024 | H | $SO_2C_2H_5$ | $C(CH_3)C_2H_4$(Cyclo) | |
| 3.1025 | H | $NO_2$ | $C(CH_3)C_2H_4$(Cyclo) | |
| 3.1026 | H | CN | $C(CH_3)C_2H_4$(Cyclo) | |
| 3.1027 | H | $CH_2OH$ | $C(CH_3)C_2H_4$(Cyclo) | |
| 3.1028 | H | $CH_2CH_2OH$ | $C(CH_3)C_2H_4$(Cyclo) | |
| 3.1029 | H | $CH_2CH_2CH_2OH$ | $C(CH_3)C_2H_4$(Cyclo) | |
| 3.1030 | H | $C_6H_5$ | $C(CH_3)C_2H_4$(Cyclo) | |
| 3.1031 | H | $CH_2Cl$ | $C(CH_3)C_2H_4$(Cyclo) | |
| 3.1032 | H | $CH_2CH_2Cl$ | $C(CH_3)C_2H_4$(Cyclo) | |
| 3.1033 | H | $CH_2CH_2CH_2Cl$ | $C(CH_3)C_2H_4$(Cyclo) | |

Tabelle 3.11

| No. | X | Y | Z | phys. Daten |
|---|---|---|---|---|
| 3.1101 | H | H | $CH_2OC_6H_5$ | |
| 3.1102 | H | $CH_3$ | $CH_2OC_6H_5$ | |
| 3.1103 | H | $C_2H_5$ | $CH_2OC_6H_5$ | |
| 3.1104 | H | $C_3H_7$ | $CH_2OC_6H_5$ | |
| 3.1105 | H | $OCH_3$ | $CH_2OC_6H_5$ | |
| 3.1106 | H | $OC_2H_5$ | $CH_2OC_6H_5$ | |
| 3.1107 | H | $OCH_2CH=CH_2$ | $CH_2OC_6H_5$ | |
| 3.1108 | H | $OCH_2CH=CHCH_3$ | $CH_2OC_6H_5$ | |
| 3.1109 | H | $OCH_2C(Cl)=CH_2$ | $CH_2OC_6H_5$ | |
| 3.1110 | H | $OCH_2CH=CHCl$ | $CH_2OC_6H_5$ | |
| 3.1111 | H | $OCH_2C\equiv CH$ | $CH_2OC_6H_5$ | |
| 3.1112 | H | $OCHF_2$ | $CH_2OC_6H_5$ | |
| 3.1113 | H | $OCF_3$ | $CH_2OC_6H_5$ | |
| 3.1114 | H | $OCF_2CHFCF_3$ | $CH_2OC_6H_5$ | |
| 3.1115 | H | $OC_6H_5$ | $CH_2OC_6H_5$ | |
| 3.1116 | H | $CF_3$ | $CH_2OC_6H_5$ | |
| 3.1117 | H | Br | $CH_2OC_6H_5$ | |
| 3.1118 | H | Cl | $CH_2OC_6H_5$ | |
| 3.1119 | H | F | $CH_2OC_6H_5$ | |
| 3.1120 | H | $SCH_3$ | $CH_2OC_6H_5$ | |
| 3.1121 | H | $SC_2H_5$ | $CH_2OC_6H_5$ | |
| 3.1122 | H | $SC_3H_7$-n | $CH_2OC_6H_5$ | |
| 3.1123 | H | $SO_2CH_3$ | $CH_2OC_6H_5$ | |
| 3.1124 | H | $SO_2C_2H_5$ | $CH_2OC_6H_5$ | |
| 3.1125 | H | $NO_2$ | $CH_2OC_6H_5$ | |
| 3.1126 | H | CN | $CH_2OC_6H_5$ | |
| 3.1127 | H | $CH_2OH$ | $CH_2OC_6H_5$ | |
| 3.1128 | H | $CH_2CH_2OH$ | $CH_2OC_6H_5$ | |
| 3.1129 | H | $CH_2CH_2CH_2OH$ | $CH_2OC_6H_5$ | |
| 3.1130 | H | $C_6H_5$ | $CH_2OC_6H_5$ | |
| 3.1131 | H | $CH_2Cl$ | $CH_2OC_6H_5$ | |
| 3.1132 | H | $CH_2CH_2Cl$ | $CH_2OC_6H_5$ | |
| 3.1133 | H | $CH_2CH_2CH_2Cl$ | $CH_2OC_6H_5$ | |

Tabelle 3.12

| No. | X | Y | Z | phys. Daten |
|---|---|---|---|---|
| 3.1201 | H | H | Furan-2-yl | |
| 3.1202 | H | $CH_3$ | Furan-2-yl | |
| 3.1203 | H | $C_2H_5$ | Furan-2-yl | |
| 3.1204 | H | $C_3H_7$ | Furan-2-yl | |
| 3.1205 | H | $OCH_3$ | Furan-2-yl | |
| 3.1206 | H | $OC_2H_5$ | Furan-2-yl | |
| 3.1207 | H | $OCH_2CH=CH_2$ | Furan-2-yl | |
| 3.1208 | H | $OCH_2CH=CHCH_3$ | Furan-2-yl | |
| 3.1209 | H | $OCH_2C(Cl)=CH_2$ | Furan-2-yl | |
| 3.1210 | H | $OCH_2CH=CHCl$ | Furan-2-yl | |
| 3.1211 | H | $OCH_2C\equiv CH$ | Furan-2-yl | |
| 3.1212 | H | $OCHF_2$ | Furan-2-yl | |
| 3.1213 | H | $OCF_3$ | Furan-2-yl | |
| 3.1214 | H | $OCF_2CHFCF_3$ | Furan-2-yl | |
| 3.1215 | H | $OC_6H_5$ | Furan-2-yl | |
| 3.1216 | H | $CF_3$ | Furan-2-yl | |
| 3.1217 | H | Br | Furan-2-yl | |
| 3.1218 | H | Cl | Furan-2-yl | |
| 3.1219 | H | F | Furan-2-yl | |
| 3.1220 | H | $SCH_3$ | Furan-2-yl | |
| 3.1221 | H | $SC_2H_5$ | Furan-2-yl | |
| 3.1222 | H | $SC_3H_7$-n | Furan-2-yl | |
| 3.1223 | H | $SO_2CH_3$ | Furan-2-yl | |
| 3.1224 | H | $SO_2C_2H_5$ | Furan-2-yl | |
| 3.1225 | H | $NO_2$ | Furan-2-yl | |
| 3.1226 | H | CN | Furan-2-yl | |
| 3.1227 | H | $CH_2OH$ | Furan-2-yl | |
| 3.1228 | H | $CH_2CH_2OH$ | Furan-2-yl | |
| 3.1229 | H | $CH_2CH_2CH_2OH$ | Furan-2-yl | |
| 3.1230 | H | $C_6H_5$ | Furan-2-yl | |
| 3.1231 | H | $CH_2Cl$ | Furan-2-yl | |
| 3.1232 | H | $CH_2CH_2Cl$ | Furan-2-yl | |
| 3.1233 | H | $CH_2CH_2CH_2Cl$ | Furan-2-yl | |

Tabelle 3.13

| No. | X | Y | Z | phys. Daten |
|---|---|---|---|---|
| 3.1301 | H | H | Dioxan-2-yl | |
| 3.1302 | H | $CH_3$ | Dioxan-2-yl | |
| 3.1303 | H | $C_2H_5$ | Dioxan-2-yl | |
| 3.1304 | H | $C_3H_7$ | Dioxan-2-yl | |
| 3.1305 | H | $OCH_3$ | Dioxan-2-yl | |
| 3.1306 | H | $OC_2H_5$ | Dioxan-2-yl | |
| 3.1307 | H | $OCH_2CH=CH_2$ | Dioxan-2-yl | |
| 3.1308 | H | $OCH_2CH=CHCH_3$ | Dioxan-2-yl | |
| 3.1309 | H | $OCH_2C(Cl)=CH_2$ | Dioxan-2-yl | |
| 3.1310 | H | $OCH_2CH=CHCl$ | Dioxan-2-yl | |
| 3.1311 | H | $OCH_2C{\equiv}CH$ | Dioxan-2-yl | |
| 3.1312 | H | $OCHF_2$ | Dioxan-2-yl | |
| 3.1313 | H | $OCF_3$ | Dioxan-2-yl | |
| 3.1314 | H | $OCF_2CHFCF_3$ | Dioxan-2-yl | |
| 3.1315 | H | $OC_6H_5$ | Dioxan-2-yl | |
| 3.1316 | H | $CF_3$ | Dioxan-2-yl | |
| 3.1317 | H | Br | Dioxan-2-yl | |
| 3.1318 | H | Cl | Dioxan-2-yl | |
| 3.1319 | H | F | Dioxan-2-yl | |
| 3.1320 | H | $SCH_3$ | Dioxan-2-yl | |
| 3.1321 | H | $SC_2H_5$ | Dioxan-2-yl | |
| 3.1322 | H | $SC_3H_7$-n | Dioxan-2-yl | |
| 3.1323 | H | $SO_2CH_3$ | Dioxan-2-yl | |
| 3.1324 | H | $SO_2C_2H_5$ | Dioxan-2-yl | |
| 3.1325 | H | $NO_2$ | Dioxan-2-yl | |
| 3.1326 | H | CN | Dioxan-2-yl | |
| 3.1327 | H | $CH_2OH$ | Dioxan-2-yl | |
| 3.1328 | H | $CH_2CH_2OH$ | Dioxan-2-yl | |
| 3.1329 | H | $CH_2CH_2CH_2OH$ | Dioxan-2-yl | |
| 3.1330 | H | $C_6H_5$ | Dioxan-2-yl | |
| 3.1331 | H | $CH_2Cl$ | Dioxan-2-yl | |
| 3.1332 | H | $CH_2CH_2Cl$ | Dioxan-2-yl | |
| 3.1333 | H | $CH_2CH_2CH_2Cl$ | Dioxan-2-yl | |

Tabelle 3.14

| No. | X | Y | Z | phys. Daten |
|---|---|---|---|---|
| 3.1401 | H | H | $CH_2OC_2H_4OCH_3$ | |
| 3.1402 | H | $CH_3$ | $CH_2OC_2H_4OCH_3$ | |
| 3.1403 | H | $C_2H_5$ | $CH_2OC_2H_4OCH_3$ | |
| 3.1404 | H | $C_3H_7$ | $CH_2OC_2H_4OCH_3$ | |
| 3.1405 | H | $OCH_3$ | $CH_2OC_2H_4OCH_3$ | |
| 3.1406 | H | $OC_2H_5$ | $CH_2OC_2H_4OCH_3$ | |
| 3.1407 | H | $OCH_2CH=CH_2$ | $CH_2OC_2H_4OCH_3$ | |
| 3.1408 | H | $OCH_2CH=CHCH_3$ | $CH_2OC_2H_4OCH_3$ | |
| 3.1409 | H | $OCH_2C(Cl)=CH_2$ | $CH_2OC_2H_4OCH_3$ | |
| 3.1410 | H | $OCH_2CH=CHCl$ | $CH_2OC_2H_4OCH_3$ | |
| 3.1411 | H | $OCH_2C\equiv CH$ | $CH_2OC_2H_4OCH_3$ | |
| 3.1412 | H | $OCHF_2$ | $CH_2OC_2H_4OCH_3$ | |
| 3.1413 | H | $OCF_3$ | $CH_2OC_2H_4OCH_3$ | |
| 3.1414 | H | $OCF_2CHFCF_3$ | $CH_2OC_2H_4OCH_3$ | |
| 3.1415 | H | $OC_6H_5$ | $CH_2OC_2H_4OCH_3$ | |
| 3.1416 | H | $CF_3$ | $CH_2OC_2H_4OCH_3$ | |
| 3.1417 | H | Br | $CH_2OC_2H_4OCH_3$ | |
| 3.1418 | H | Cl | $CH_2OC_2H_4OCH_3$ | |
| 3.1419 | H | F | $CH_2OC_2H_4OCH_3$ | |
| 3.1420 | H | $SCH_3$ | $CH_2OC_2H_4OCH_3$ | |
| 3.1421 | H | $SC_2H_5$ | $CH_2OC_2H_4OCH_3$ | |
| 3.1422 | H | $SC_3H_7$-n | $CH_2OC_2H_4OCH_3$ | |
| 3.1423 | H | $SO_2CH_3$ | $CH_2OC_2H_4OCH_3$ | |
| 3.1424 | H | $SO_2C_2H_5$ | $CH_2OC_2H_4OCH_3$ | |
| 3.1425 | H | $NO_2$ | $CH_2OC_2H_4OCH_3$ | |
| 3.1426 | H | CN | $CH_2OC_2H_4OCH_3$ | |
| 3.1427 | H | $CH_2OH$ | $CH_2OC_2H_4OCH_3$ | |
| 3.1428 | H | $CH_2CH_2OH$ | $CH_2OC_2H_4OCH_3$ | |
| 3.1429 | H | $CH_2CH_2CH_2OH$ | $CH_2OC_2H_4OCH_3$ | |
| 3.1430 | H | $C_6H_5$ | $CH_2OC_2H_4OCH_3$ | |
| 3.1431 | H | $CH_2Cl$ | $CH_2OC_2H_4OCH_3$ | |
| 3.1432 | H | $CH_2CH_2Cl$ | $CH_2OC_2H_4OCH_3$ | |
| 3.1433 | H | $CH_2CH_2CH_2Cl$ | $CH_2OC_2H_4OCH_3$ | |

Tabelle 3.15

| No. | X | Y | Z | phys. Daten |
|---|---|---|---|---|
| 3.1501 | H | H | $CH_2OC_2H_4OC_2H_4OCH_3$ | |
| 3.1502 | H | $CH_3$ | $CH_2OC_2H_4OC_2H_4OCH_3$ | |
| 3.1503 | H | $C_2H_5$ | $CH_2OC_2H_4OC_2H_4OCH_3$ | |
| 3.1504 | H | $C_3H_7$ | $CH_2OC_2H_4OC_2H_4OCH_3$ | |
| 3.1505 | H | $OCH_3$ | $CH_2OC_2H_4OC_2H_4OCH_3$ | |
| 3.1506 | H | $OC_2H_5$ | $CH_2OC_2H_4OC_2H_4OCH_3$ | |
| 3.1507 | H | $OCH_2CH=CH_2$ | $CH_2OC_2H_4OC_2H_4OCH_3$ | |
| 3.1508 | H | $OCH_2CH=CHCH_3$ | $CH_2OC_2H_4OC_2H_4OCH_3$ | |
| 3.1509 | H | $OCH_2C(Cl)=CH_2$ | $CH_2OC_2H_4OC_2H_4OCH_3$ | |
| 3.1510 | H | $OCH_2CH=CHCl$ | $CH_2OC_2H_4OC_2H_4OCH_3$ | |
| 3.1511 | H | $OCH_2C\equiv CH$ | $CH_2OC_2H_4OC_2H_4OCH_3$ | |
| 3.1512 | H | $OCHF_2$ | $CH_2OC_2H_4OC_2H_4OCH_3$ | |
| 3.1513 | H | $OCF_3$ | $CH_2OC_2H_4OC_2H_4OCH_3$ | |
| 3.1514 | H | $OCF_2CHFCF_3$ | $CH_2OC_2H_4OC_2H_4OCH_3$ | |
| 3.1515 | H | $OC_6H_5$ | $CH_2OC_2H_4OC_2H_4OCH_3$ | |
| 3.1516 | H | $CF_3$ | $CH_2OC_2H_4OC_2H_4OCH_3$ | |
| 3.1517 | H | Br | $CH_2OC_2H_4OC_2H_4OCH_3$ | |
| 3.1518 | H | Cl | $CH_2OC_2H_4OC_2H_4OCH_3$ | |
| 3.1519 | H | F | $CH_2OC_2H_4OC_2H_4OCH_3$ | |
| 3.1520 | H | $SCH_3$ | $CH_2OC_2H_4OC_2H_4OCH_3$ | |
| 3.1521 | H | $SC_2H_5$ | $CH_2OC_2H_4OC_2H_4OCH_3$ | |
| 3.1522 | H | $SC_3H_7$-n | $CH_2OC_2H_4OC_2H_4OCH_3$ | |
| 3.1523 | H | $SO_2CH_3$ | $CH_2OC_2H_4OC_2H_4OCH_3$ | |
| 3.1524 | H | $SO_2C_2H_5$ | $CH_2OC_2H_4OC_2H_4OCH_3$ | |
| 3.1525 | H | $NO_2$ | $CH_2OC_2H_4OC_2H_4OCH_3$ | |
| 3.1526 | H | CN | $CH_2OC_2H_4OC_2H_4OCH_3$ | |
| 3.1527 | H | $CH_2OH$ | $CH_2OC_2H_4OC_2H_4OCH_3$ | |
| 3.1528 | H | $CH_2CH_2OH$ | $CH_2OC_2H_4OC_2H_4OCH_3$ | |
| 3.1529 | H | $CH_2CH_2CH_2OH$ | $CH_2OC_2H_4OC_2H_4OCH_3$ | |
| 3.1530 | H | $C_6H_5$ | $CH_2OC_2H_4OC_2H_4OCH_3$ | |
| 3.1531 | H | $CH_2Cl$ | $CH_2OC_2H_4OC_2H_4OCH_3$ | |
| 3.1532 | H | $CH_2CH_2Cl$ | $CH_2OC_2H_4OC_2H_4OCH_3$ | |
| 3.1533 | H | $CH_2CH_2CH_2Cl$ | $CH_2OC_2H_4OC_2H_4OCH_3$ | |

Tabelle 3.16

| No. | X | Y | Z | phys. Daten |
|---|---|---|---|---|
| 3.1601 | H | H | $CH_2OCH_2CONH_2$ | |
| 3.1602 | H | $CH_3$ | $CH_2OCH_2CONH_2$ | |
| 3.1603 | H | $C_2H_5$ | $CH_2OCH_2CONH_2$ | |
| 3.1604 | H | $C_3H_7$ | $CH_2OCH_2CONH_2$ | |
| 3.1605 | H | $OCH_3$ | $CH_2OCH_2CONH_2$ | |
| 3.1606 | H | $OC_2H_5$ | $CH_2OCH_2CONH_2$ | |
| 3.1607 | H | $OCH_2CH{=}CH_2$ | $CH_2OCH_2CONH_2$ | |
| 3.1608 | H | $OCH_2CH{=}CHCH_3$ | $CH_2OCH_2CONH_2$ | |
| 3.1609 | H | $OCH_2C(Cl){=}CH_2$ | $CH_2OCH_2CONH_2$ | |
| 3.1610 | H | $OCH_2CH{=}CHCl$ | $CH_2OCH_2CONH_2$ | |
| 3.1611 | H | $OCH_2C{\equiv}CH$ | $CH_2OCH_2CONH_2$ | |
| 3.1612 | H | $OCHF_2$ | $CH_2OCH_2CONH_2$ | |
| 3.1613 | H | $OCF_3$ | $CH_2OCH_2CONH_2$ | |
| 3.1614 | H | $OCF_2CHFCF_3$ | $CH_2OCH_2CONH_2$ | |
| 3.1615 | H | $OC_6H_5$ | $CH_2OCH_2CONH_2$ | |
| 3.1616 | H | $CF_3$ | $CH_2OCH_2CONH_2$ | |
| 3.1617 | H | Br | $CH_2OCH_2CONH_2$ | |
| 3.1618 | H | Cl | $CH_2OCH_2CONH_2$ | |
| 3.1619 | H | F | $CH_2OCH_2CONH_2$ | |
| 3.1620 | H | $SCH_3$ | $CH_2OCH_2CONH_2$ | |
| 3.1621 | H | $SC_2H_5$ | $CH_2OCH_2CONH_2$ | |
| 3.1622 | H | $SC_3H_7$-n | $CH_2OCH_2CONH_2$ | |
| 3.1623 | H | $SO_2CH_3$ | $CH_2OCH_2CONH_2$ | |
| 3.1624 | H | $SO_2C_2H_5$ | $CH_2OCH_2CONH_2$ | |
| 3.1625 | H | $NO_2$ | $CH_2OCH_2CONH_2$ | |
| 3.1626 | H | CN | $CH_2OCH_2CONH_2$ | |
| 3.1627 | H | $CH_2OH$ | $CH_2OCH_2CONH_2$ | |
| 3.1628 | H | $CH_2CH_2OH$ | $CH_2OCH_2CONH_2$ | |
| 3.1629 | H | $CH_2CH_2CH_2OH$ | $CH_2OCH_2CONH_2$ | |
| 3.1630 | H | $C_6H_5$ | $CH_2OCH_2CONH_2$ | |
| 3.1631 | H | $CH_2Cl$ | $CH_2OCH_2CONH_2$ | |
| 3.1632 | H | $CH_2CH_2Cl$ | $CH_2OCH_2CONH_2$ | |
| 3.1633 | H | $CH_2CH_2CH_2Cl$ | $CH_2OCH_2CONH_2$ | |

Beispiel 11 Herstellung von 5-Aethyl-2-(5-isopropyl-5-methyl-4-oxoimidazolin-2-yl)-6-methoxymethyl-pyridin-3-carbonsäure-methylester.

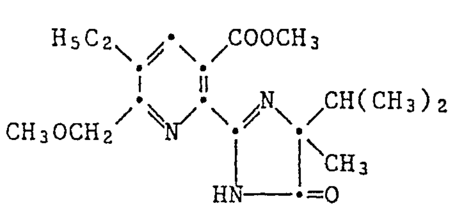

Zu einem Gemisch von 0.37 g Methanol und 60 g Triäthylamin in 30 ml Methylenchlorid gibt man unter Rühren bei Raumtemperatur 3,4 g 7-Aethyl-8-methoxymethyl-2-isopropyl-2-methyl-2H-imidazo[1,2:1′,2′]-pyr-

rolo[3,4-b]pyridin-3,5-dion. Nachdem eine homogene Lösung entstanden ist. Lässt man 2 Tage bei Zimmertemperatur stehen. Dann wird eingedampft und der Rückstand über eine Silikayelsäule mittels Aether chromatographiert. Nach verdampfen des Aethers verbleiben 2,8 g kristalline Titelsubstanz, welche einen Schmelzpunkt von 87-90°C hat.

Analog zum Beispiel 11 werden die Ester der Tabellen 4.00 bis 4.016 der Formel I hergestellt

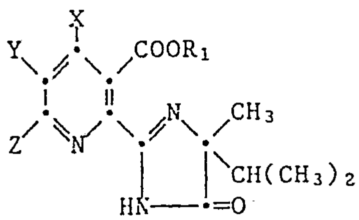

(I)

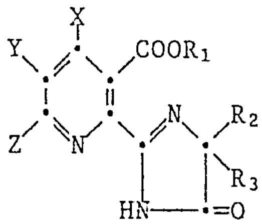

(I)

X = H
$R_2$ = $CH_3$
$R_3$ = $C_3H_7$-iso

Tabelle 4.00

| No. | X | Y | $R_1$ | Z | phys. Daten |
|---|---|---|---|---|---|
| 4.0001 | H | H | $CH_3$ | $C_3H_5$(Cyclo) | |
| 4.0002 | H | $CH_3$ | $CH_3$ | $C_3H_5$(Cyclo) | Smp. 184-187°C |
| 4.0003 | H | $C_2H_5$ | $CH_3$ | $C_3H_5$(Cyclo) | Smp. 167-168°C |
| 4.0004 | H | $C_3H_7$ | $CH_3$ | $C_3H_5$(Cyclo) | |
| 4.0005 | H | $OCH_3$ | $CH_3$ | $C_3H_5$(Cyclo) | |
| 4.0006 | H | $OC_2H_5$ | $CH_3$ | $C_3H_5$(Cyclo) | |
| 4.0007 | H | $OCH_2CH=CH_2$ | $CH_3$ | $C_3H_5$(Cyclo) | |
| 4.0008 | H | $OCH_2CH=CHCH_3$ | $CH_3$ | $C_3H_5$(Cyclo) | |
| 4.0009 | H | $OCH_2C(Cl)=CH_2$ | $CH_3$ | $C_3H_5$(Cyclo) | |
| 4.0010 | H | $OCH_2CH=CHCl$ | $CH_3$ | $C_3H_5$(Cyclo) | |
| 4.0011 | H | $OCH_2C\equiv CH$ | $CH_3$ | $C_3H_5$(Cyclo) | |
| 4.0012 | H | $OCHF_2$ | $CH_3$ | $C_3H_5$(Cyclo) | |
| 4.0013 | H | $OCF_3$ | $CH_3$ | $C_3H_5$(Cyclo) | |
| 4.0014 | H | $OCF_2CHFCF_3$ | $CH_3$ | $C_3H_5$(Cyclo) | |
| 4.0015 | H | $OC_6H_5$ | $CH_3$ | $C_3H_5$(Cyclo) | |
| 4.0016 | H | $CF_3$ | $CH_3$ | $C_3H_5$(Cyclo) | |
| 4.0017 | H | Br | $CH_3$ | $C_3H_5$(Cyclo) | |
| 4.0018 | H | Cl | $CH_3$ | $C_3H_5$(Cyclo) | |
| 4.0019 | H | F | $CH_3$ | $C_3H_5$(Cyclo) | |
| 4.0020 | H | $SCH_3$ | $CH_3$ | $C_3H_5$(Cyclo) | |
| 4.0021 | H | $SC_2H_5$ | $CH_3$ | $C_3H_5$(Cyclo) | |
| 4.0022 | H | $SC_3H_7$-n | $CH_3$ | $C_3H_5$(Cyclo) | |
| 4.0023 | H | $SO_2CH_3$ | $CH_3$ | $C_3H_5$(Cyxlo) | |
| 4.0024 | H | $SO_2C_2H_5$ | $CH_3$ | $C_3H_5$(Cyclo) | |
| 4.0025 | H | $NO_2$ | $CH_3$ | $C_3H_5$(Cyclo) | |
| 4.0026 | H | CN | $CH_3$ | $C_3H_5$(Cyclo) | |
| 4.0027 | H | $CH_2OH$ | $CH_3$ | $C_3H_5$(Cyclo) | |
| 4.0028 | H | $CH_2CH_2OH$ | $CH_3$ | $C_3H_5$(Cyclo) | |
| 4.0029 | H | $CH_2CH_2CH_2OH$ | $CH_3$ | $C_3H_5$(Cyclo) | |
| 4.0030 | H | $C_6H_5$ | $CH_3$ | $C_3H_5$(Cyclo) | |
| 4.0031 | H | $CH_2Cl$ | $CH_3$ | $C_3H_5$(Cyclo) | |
| 4.0032 | H | $CH_2CH_2Cl$ | $CH_3$ | $C_3H_5$(Cyclo) | |
| 4.0033 | H | $CH_2CH_2CH_2Cl$ | $CH_3$ | $C_3H_5$(Cyclo) | |

Tabelle 4.01

| No. | X | Y | $R_1$ | Z | phys. Daten |
|---|---|---|---|---|---|
| 4.0101 | H | H | $C_2H_5$ | $C_4H_7$(Cyclo) | |
| 4.0102 | H | $CH_3$ | $C_2H_5$ | $C_4H_7$(Cyclo) | |
| 4.0103 | H | $C_2H_5$ | $C_2H_5$ | $C_4H_7$(Cyclo) | |
| 4.0104 | H | $C_3H_7$ | $C_2H_5$ | $C_4H_7$(Cyclo) | |
| 4.0105 | H | $OCH_3$ | $C_2H_5$ | $C_4H_7$(Cyclo) | |
| 4.0106 | H | $OC_2H_5$ | $C_2H_5$ | $C_4H_7$(Cyclo) | |
| 4.0107 | H | $OCH_2CH=CH_2$ | $C_2H_5$ | $C_4H_7$(Cyclo) | |
| 4.0108 | H | $OCH_2CH=CHCH_3$ | $C_2H_5$ | $C_4H_7$(Cyclo) | |
| 4.0109 | H | $OCH_2C(Cl)=CH_2$ | $C_2H_5$ | $C_4H_7$(Cyclo) | |
| 4.0110 | H | $OCH_2CH=CHCl$ | $C_2H_5$ | $C_4H_7$(Cyclo) | |
| 4.0111 | H | $OCH_2C\equiv CH$ | $C_2H_5$ | $C_4H_7$(Cyclo) | |
| 4.0112 | H | $OCHF_2$ | $C_2H_5$ | $C_4H_7$(Cyclo) | |
| 4.0113 | H | $OCF_3$ | $C_2H_5$ | $C_4H_7$(Cyclo) | |
| 4.0114 | H | $OCF_2CHFCF_3$ | $C_2H_5$ | $C_4H_7$(Cyclo) | |
| 4.0115 | H | $OC_6H_5$ | $C_2H_5$ | $C_4H_7$(Cyclo) | |
| 4.0116 | H | $CF_3$ | $C_2H_5$ | $C_4H_7$(Cyclo) | |
| 4.0117 | H | Br | $C_2H_5$ | $C_4H_7$(Cyclo) | |
| 4.0118 | H | Cl | $C_2H_5$ | $C_4H_7$(Cyclo) | |
| 4.0119 | H | F | $C_2H_5$ | $C_4H_7$(Cyclo) | |
| 4.0120 | H | $SCH_3$ | $C_2H_5$ | $C_4H_7$(Cyclo) | |
| 4.0121 | H | $SC_2H_5$ | $C_2H_5$ | $C_4H_7$(Cyclo) | |
| 4.0122 | H | $SC_3H_7$-n | $C_2H_5$ | $C_4H_7$(Cyclo) | |
| 4.0123 | H | $SO_2CH_3$ | $C_2H_5$ | $C_4H_7$(Cyxlo) | |
| 4.0124 | H | $SO_2C_2H_5$ | $C_2H_5$ | $C_4H_7$(Cyclo) | |
| 4.0125 | H | $NO_2$ | $C_2H_5$ | $C_4H_7$(Cyclo) | |
| 4.0126 | H | CN | $C_2H_5$ | $C_4H_7$(Cyclo) | |
| 4.0127 | H | $CH_2OH$ | $C_2H_5$ | $C_4H_7$(Cyclo) | |
| 4.0128 | H | $CH_2CH_2OH$ | $C_2H_5$ | $C_4H_7$(Cyclo) | |
| 4.0129 | H | $CH_2CH_2CH_2OH$ | $C_2H_5$ | $C_4H_7$(Cyclo) | |
| 4.0130 | H | $C_6H_5$ | $C_2H_5$ | $C_4H_7$(Cyclo) | |
| 4.0131 | H | $CH_2Cl$ | $C_2H_5$ | $C_4H_7$(Cyclo) | |
| 4.0132 | H | $CH_2CH_2Cl$ | $C_2H_5$ | $C_4H_7$(Cyclo) | |
| 4.0133 | H | $CH_2CH_2CH_2Cl$ | $C_2H_5$ | $C_4H_7$(Cyclo) | |

Tabelle 4.02

| No. | X | Y | $R_1$ | Z | phys. Daten |
|---|---|---|---|---|---|
| 4.0201 | H | H | $CH_3$ | $C_5H_9$(Cyclo) | |
| 4.0202 | H | $CH_3$ | $CH_3$ | $C_5H_9$(Cyclo) | |
| 4.0203 | H | $C_2H_5$ | $CH_3$ | $C_5H_9$(Cyclo) | |
| 4.0204 | H | $C_3H_7$ | $CH_3$ | $C_5H_9$(Cyclo) | |
| 4.0205 | H | $OCH_3$ | $CH_3$ | $C_5H_9$(Cyclo) | |
| 4.0206 | H | $OC_2H_5$ | $CH_3$ | $C_5H_9$(Cyclo) | |
| 4.0207 | H | $OCH_2CH{=}CH_2$ | $CH_3$ | $C_5H_9$(Cyclo) | |
| 4.0208 | H | $OCH_2CH{=}CHCH_3$ | $CH_3$ | $C_5H_9$(Cyclo) | |
| 4.0209 | H | $OCH_2C(Cl){=}CH_2$ | $CH_3$ | $C_5H_9$(Cyclo) | |
| 4.0210 | H | $OCH_2CH{=}CHCl$ | $CH_3$ | $C_5H_9$(Cyclo) | |
| 4.0211 | H | $OCH_2C{\equiv}CH$ | $CH_3$ | $C_5H_9$(Cyclo) | |
| 4.0212 | H | $OCHF_2$ | $CH_3$ | $C_5H_9$(Cyclo) | |
| 4.0213 | H | $OCF_3$ | $CH_3$ | $C_5H_9$(Cyclo) | |
| 4.0214 | H | $OCF_2CHFCF_3$ | $CH_3$ | $C_5H_9$(Cyclo) | |
| 4.0215 | H | $OC_6H_5$ | $CH_3$ | $C_5H_9$(Cyclo) | |
| 4.0216 | H | $CF_3$ | $CH_3$ | $C_5H_9$(Cyclo) | |
| 4.0217 | H | Br | $CH_3$ | $C_5H_9$(Cyclo) | |
| 4.0218 | H | Cl | $CH_3$ | $C_5H_9$(Cyclo) | |
| 4.0219 | H | F | $CH_3$ | $C_5H_9$(Cyclo) | |
| 4.0220 | H | $SCH_3$ | $CH_3$ | $C_5H_9$(Cyclo) | |
| 4.0221 | H | $SC_2H_5$ | $CH_3$ | $C_5H_9$(Cyclo) | |
| 4.0222 | H | $SC_3H_7$-n | $CH_3$ | $C_5H_9$(Cyclo) | |
| 4.0223 | H | $SO_2CH_3$ | $CH_3$ | $C_5H_9$(Cyxlo) | |
| 4.0224 | H | $SO_2C_2H_5$ | $CH_3$ | $C_5H_9$(Cyclo) | |
| 4.0225 | H | $NO_2$ | $CH_3$ | $C_5H_9$(Cyclo) | |
| 4.0226 | H | CN | $CH_3$ | $C_5H_9$(Cyclo) | |
| 4.0227 | H | $CH_2OH$ | $CH_3$ | $C_5H_9$(Cyclo) | |
| 4.0228 | H | $CH_2CH_2OH$ | $CH_3$ | $C_5H_9$(Cyclo) | |
| 4.0229 | H | $CH_2CH_2CH_2OH$ | $CH_3$ | $C_5H_9$(Cyclo) | |
| 4.0230 | H | $C_6H_5$ | $CH_3$ | $C_5H_9$(Cyclo) | |
| 4.0231 | H | $CH_2Cl$ | $CH_3$ | $C_5H_9$(Cyclo) | |
| 4.0232 | H | $CH_2CH_2Cl$ | $CH_3$ | $C_5H_9$(Cyclo) | |
| 4.0233 | H | $CH_2CH_2CH_2Cl$ | $CH_3$ | $C_5H_9$(Cyclo) | |

Tabelle 4.03

| No. | X | Y | $R_1$ | Z | phys. Daten |
|---|---|---|---|---|---|
| 4.0301 | H | H | $CH_3$ | $C_6H_{11}$(Cyclo) | |
| 4.0302 | H | $CH_3$ | $CH_3$ | $C_6H_{11}$(Cyclo) | |
| 4.0303 | H | $C_2H_5$ | $CH_3$ | $C_6H_{11}$(Cyclo) | |
| 4.0304 | H | $C_3H_7$ | $CH_3$ | $C_6H_{11}$(Cyclo) | |
| 4.0305 | H | $OCH_3$ | $CH_3$ | $C_6H_{11}$(Cyclo) | |
| 4.0306 | H | $OC_2H_5$ | $CH_3$ | $C_6H_{11}$(Cyclo) | |
| 4.0307 | H | $OCH_2CH=CH_2$ | $CH_3$ | $C_6H_{11}$(Cyclo) | |
| 4.0308 | H | $OCH_2CH=CHCH_3$ | $CH_3$ | $C_6H_{11}$(Cyclo) | |
| 4.0309 | H | $OCH_2C(Cl)=CH_2$ | $CH_3$ | $C_6H_{11}$(Cyclo) | |
| 4.0310 | H | $OCH_2CH=CHCl$ | $CH_3$ | $C_6H_{11}$(Cyclo) | |
| 4.0311 | H | $OCH_2C\equiv CH$ | $CH_3$ | $C_6H_{11}$(Cyclo) | |
| 4.0312 | H | $OCHF_2$ | $CH_3$ | $C_6H_{11}$(Cyclo) | |
| 4.0313 | H | $OCF_3$ | $CH_3$ | $C_6H_{11}$(Cyclo) | |
| 4.0314 | H | $OCF_2CHFCF_3$ | $CH_3$ | $C_6H_{11}$(Cyclo) | |
| 4.0315 | H | $OC_6H_5$ | $CH_3$ | $C_6H_{11}$(Cyclo) | |
| 4.0316 | H | $CF_3$ | $CH_3$ | $C_6H_{11}$(Cyclo) | |
| 4.0317 | H | Br | $CH_3$ | $C_6H_{11}$(Cyclo) | |
| 4.0318 | H | Cl | $CH_3$ | $C_6H_{11}$(Cyclo) | |
| 4.0319 | H | F | $CH_3$ | $C_6H_{11}$(Cyclo) | |
| 4.0320 | H | $SCH_3$ | $CH_3$ | $C_6H_{11}$(Cyclo) | |
| 4.0321 | H | $SC_2H_5$ | $CH_3$ | $C_6H_{11}$(Cyclo) | |
| 4.0322 | H | $SC_3H_7$-n | $CH_3$ | $C_6H_{11}$(Cyclo) | |
| 4.0323 | H | $SO_2CH_3$ | $CH_3$ | $C_6H_{11}$(Cyxlo) | |
| 4.0324 | H | $SO_2C_2H_5$ | $CH_3$ | $C_6H_{11}$(Cyclo) | |
| 4.0325 | H | $NO_2$ | $CH_3$ | $C_6H_{11}$(Cyclo) | |
| 4.0326 | H | CN | $CH_3$ | $C_6H_{11}$(Cyclo) | |
| 4.0327 | H | $CH_2OH$ | $CH_3$ | $C_6H_{11}$(Cyclo) | |
| 4.0328 | H | $CH_2CH_2OH$ | $CH_3$ | $C_6H_{11}$(Cyclo) | |
| 4.0329 | H | $CH_2CH_2CH_2OH$ | $CH_3$ | $C_6H_{11}$(Cyclo) | |
| 4.0330 | H | $C_6H_5$ | $CH_3$ | $C_6H_{11}$(Cyclo) | |
| 4.0331 | H | $CH_2Cl$ | $CH_3$ | $C_6H_{11}$(Cyclo) | |
| 4.0332 | H | $CH_2CH_2Cl$ | $CH_3$ | $C_6H_{11}$(Cyclo) | |
| 4.0333 | H | $CH_2CH_2CH_2Cl$ | $CH_3$ | $C_6H_{11}$(Cyclo) | |

Tabelle 4.03

| No. | X | Y | $R_1$ | Z | phys. Daten |
|---|---|---|---|---|---|
| 4.0401 | H | H | $CH_3$ | $CH_2OCH_3$ | |
| 4.0402 | H | $CH_3$ | $CH_3$ | $CH_2OCH_3$ | |
| 4.0403 | H | $C_2H_5$ | $CH_3$ | $CH_2OCH_3$ | Smp. 87-90°C |
| 4.0404 | H | $C_3H_7$ | $CH_3$ | $CH_2OCH_3$ | |
| 4.0405 | H | $OCH_3$ | $CH_3$ | $CH_2OCH_3$ | |
| 4.0406 | H | $OC_2H_5$ | $CH_3$ | $CH_2OCH_3$ | |
| 4.0407 | H | $OCH_2CH=CH_2$ | $CH_3$ | $CH_2OCH_3$ | |
| 4.0408 | H | $OCH_2CH=CHCH_3$ | $CH_3$ | $CH_2OCH_3$ | |
| 4.0409 | H | $OCH_2C(Cl)=CH_2$ | $CH_3$ | $CH_2OCH_3$ | |
| 4.0410 | H | $OCH_2CH=CHCl$ | $CH_3$ | $CH_2OCH_3$ | |
| 4.0411 | H | $OCH_2C\equiv CH$ | $CH_3$ | $CH_2OCH_3$ | |
| 4.0412 | H | $OCHF_2$ | $CH_3$ | $CH_2OCH_3$ | |
| 4.0413 | H | $OCF_3$ | $CH_3$ | $CH_2OCH_3$ | |
| 4.0414 | H | $OCF_2CHFCF_3$ | $CH_3$ | $CH_2OCH_3$ | |
| 4.0415 | H | $OC_6H_5$ | $CH_3$ | $CH_2OCH_3$ | |
| 4.0416 | H | $CF_3$ | $CH_3$ | $CH_2OCH_3$ | |
| 4.0417 | H | Br | $CH_3$ | $CH_2OCH_3$ | |
| 4.0418 | H | Cl | $CH_3$ | $CH_2OCH_3$ | |
| 4.0419 | H | F | $CH_3$ | $CH_2OCH_3$ | |
| 4.0420 | H | $SCH_3$ | $CH_3$ | $CH_2OCH_3$ | |
| 4.0421 | H | $SC_2H_5$ | $CH_3$ | $CH_2OCH_3$ | |
| 4.0422 | H | $SC_3H_7$-n | $CH_3$ | $CH_2OCH_3$ | |
| 4.0423 | H | $SO_2CH_3$ | $CH_3$ | $CH_2OCH_3$ | |
| 4.0424 | H | $SO_2C_2H_5$ | $CH_3$ | $CH_2OCH_3$ | |
| 4.0425 | H | $NO_2$ | $CH_3$ | $CH_2OCH_3$ | |
| 4.0426 | H | CN | $CH_3$ | $CH_2OCH_3$ | |
| 4.0427 | H | $CH_2OH$ | $CH_3$ | $CH_2OCH_3$ | |
| 4.0428 | H | $CH_2CH_2OH$ | $CH_3$ | $CH_2OCH_3$ | |
| 4.0429 | H | $CH_2CH_2CH_2OH$ | $CH_3$ | $CH_2OCH_3$ | |
| 4.0430 | H | $C_6H_5$ | $CH_3$ | $CH_2OCH_3$ | |
| 4.0431 | H | $CH_2Cl$ | $CH_3$ | $CH_2OCH_3$ | |
| 4.0432 | H | $CH_2CH_2Cl$ | $CH_3$ | $CH_2OCH_3$ | |
| 4.0433 | H | $CH_2CH_2CH_2Cl$ | $CH_3$ | $CH_2OCH_3$ | |

Tabelle 4.04

| No. | X | Y | $R_1$ | Z | phys. Daten |
|---|---|---|---|---|---|
| 4.0501 | H | H | $CH_3$ | $CH(CH_3)OCH_3$ | |
| 4.0502 | H | $CH_3$ | $CH_3$ | $CH(CH_3)OCH_3$ | |
| 4.0503 | H | $C_2H_5$ | $CH_3$ | $CH(CH_3)OCH_3$ | |
| 4.0504 | H | $C_3H_7$ | $CH_3$ | $CH(CH_3)OCH_3$ | |
| 4.0505 | H | $OCH_3$ | $CH_3$ | $CH(CH_3)OCH_3$ | |
| 4.0506 | H | $OC_2H_5$ | $CH_3$ | $CH(CH_3)OCH_3$ | |
| 4.0507 | H | $OCH_2CH=CH_2$ | $CH_3$ | $CH(CH_3)OCH_3$ | |
| 4.0508 | H | $OCH_2CH=CHCH_3$ | $CH_3$ | $CH(CH_3)OCH_3$ | |
| 4.0509 | H | $OCH_2C(Cl)=CH_2$ | $CH_3$ | $CH(CH_3)OCH_3$ | |
| 4.0510 | H | $OCH_2CH=CHCl$ | $CH_3$ | $CH(CH_3)OCH_3$ | |
| 4.0511 | H | $OCH_2C\equiv CH$ | $CH_3$ | $CH(CH_3)OCH_3$ | |
| 4.0512 | H | $OCHF_2$ | $CH_3$ | $CH(CH_3)OCH_3$ | |
| 4.0513 | H | $OCF_3$ | $CH_3$ | $CH(CH_3)OCH_3$ | |
| 4.0514 | H | $OCF_2CHFCF_3$ | $CH_3$ | $CH(CH_3)OCH_3$ | |
| 4.0515 | H | $OC_6H_5$ | $CH_3$ | $CH(CH_3)OCH_3$ | |
| 4.0516 | H | $CF_3$ | $CH_3$ | $CH(CH_3)OCH_3$ | |
| 4.0517 | H | Br | $CH_3$ | $CH(CH_3)OCH_3$ | |
| 4.0518 | H | Cl | $CH_3$ | $CH(CH_3)OCH_3$ | |
| 4.0519 | H | F | $CH_3$ | $CH(CH_3)OCH_3$ | |
| 4.0520 | H | $SCH_3$ | $CH_3$ | $CH(CH_3)OCH_3$ | |
| 4.0521 | H | $SC_2H_5$ | $CH_3$ | $CH(CH_3)OCH_3$ | |
| 4.0522 | H | $SC_3H_7$-n | $CH_3$ | $CH(CH_3)OCH_3$ | |
| 4.0523 | H | $SO_2CH_3$ | $CH_3$ | $CH(CH_3)OCH_3$ | |
| 4.0524 | H | $SO_2C_2H_5$ | $CH_3$ | $CH(CH_3)OCH_3$ | |
| 4.0525 | H | $NO_2$ | $CH_3$ | $CH(CH_3)OCH_3$ | |
| 4.0526 | H | CN | $CH_3$ | $CH(CH_3)OCH_3$ | |
| 4.0527 | H | $CH_2OH$ | $CH_3$ | $CH(CH_3)OCH_3$ | |
| 4.0528 | H | $CH_2CH_2OH$ | $CH_3$ | $CH(CH_3)OCH_3$ | |
| 4.0529 | H | $CH_2CH_2CH_2OH$ | $CH_3$ | $CH(CH_3)OCH_3$ | |
| 4.0530 | H | $C_6H_5$ | $CH_3$ | $CH(CH_3)OCH_3$ | |
| 4.0531 | H | $CH_2Cl$ | $CH_3$ | $CH(CH_3)OCH_3$ | |
| 4.0532 | H | $CH_2CH_2Cl$ | $CH_3$ | $CH(CH_3)OCH_3$ | |
| 4.0533 | H | $CH_2CH_2CH_2Cl$ | $CH_3$ | $CH(CH_3)OCH_3$ | |

Tabelle 4.05

| No. | X | Y | $R_1$ | Z | phys. Daten |
|---|---|---|---|---|---|
| 4.0601 | H | H | $CH_3$ | $CH_2OC_2H_5$ | |
| 4.0602 | H | $CH_3$ | $CH_3$ | $CH_2OC_2H_5$ | |
| 4.0603 | H | $C_2H_5$ | $CH_3$ | $CH_2OC_2H_5$ | |
| 4.0604 | H | $C_3H_7$ | $CH_3$ | $CH_2OC_2H_5$ | |
| 4.0605 | H | $OCH_3$ | $CH_3$ | $CH_2OC_2H_5$ | |
| 4.0606 | H | $OC_2H_5$ | $CH_3$ | $CH_2OC_2H_5$ | |
| 4.0607 | H | $OCH_2CH=CH_2$ | $CH_3$ | $CH_2OC_2H_5$ | |
| 4.0608 | H | $OCH_2CH=CHCH_3$ | $CH_3$ | $CH_2OC_2H_5$ | |
| 4.0609 | H | $OCH_2C(Cl)=CH_2$ | $CH_3$ | $CH_2OC_2H_5$ | |
| 4.0610 | H | $OCH_2CH=CHCl$ | $CH_3$ | $CH_2OC_2H_5$ | |
| 4.0611 | H | $OCH_2C\equiv CH$ | $CH_3$ | $CH_2OC_2H_5$ | |
| 4.0612 | H | $OCHF_2$ | $CH_3$ | $CH_2OC_2H_5$ | |
| 4.0613 | H | $OCF_3$ | $CH_3$ | $CH_2OC_2H_5$ | |
| 4.0614 | H | $OCF_2CHFCF_3$ | $CH_3$ | $CH_2OC_2H_5$ | |
| 4.0615 | H | $OC_6H_5$ | $CH_3$ | $CH_2OC_2H_5$ | |
| 4.0616 | H | $CF_3$ | $CH_3$ | $CH_2OC_2H_5$ | |
| 4.0617 | H | Br | $CH_3$ | $CH_2OC_2H_5$ | |
| 4.0618 | H | Cl | $CH_3$ | $CH_2OC_2H_5$ | |
| 4.0619 | H | F | $CH_3$ | $CH_2OC_2H_5$ | |
| 4.0620 | H | $SCH_3$ | $CH_3$ | $CH_2OC_2H_5$ | |
| 4.0621 | H | $SC_2H_5$ | $CH_3$ | $CH_2OC_2H_5$ | |
| 4.0622 | H | $SC_3H_7$-n | $CH_3$ | $CH_2OC_2H_5$ | |
| 4.0623 | H | $SO_2CH_3$ | $CH_3$ | $CH_2OC_2H_5$ | |
| 4.0624 | H | $SO_2C_2H_5$ | $CH_3$ | $CH_2OC_2H_5$ | |
| 4.0625 | H | $NO_2$ | $CH_3$ | $CH_2OC_2H_5$ | |
| 4.0626 | H | CN | $CH_3$ | $CH_2OC_2H_5$ | |
| 4.0627 | H | $CH_2OH$ | $CH_3$ | $CH_2OC_2H_5$ | |
| 4.0628 | H | $CH_2CH_2OH$ | $CH_3$ | $CH_2OC_2H_5$ | |
| 4.0629 | H | $CH_2CH_2CH_2OH$ | $CH_3$ | $CH_2OC_2H_5$ | |
| 4.0630 | H | $C_6H_5$ | $CH_3$ | $CH_2OC_2H_5$ | |
| 4.0631 | H | $CH_2Cl$ | $CH_3$ | $CH_2OC_2H_5$ | |
| 4.0632 | H | $CH_2CH_2Cl$ | $CH_3$ | $CH_2OC_2H_5$ | |
| 4.0633 | H | $CH_2CH_2CH_2Cl$ | $CH_3$ | $CH_2OC_2H_5$ | |

Tabelle 4.06

| No. | X | Y | $R_1$ | Z | phys. Daten |
|---|---|---|---|---|---|
| 4.0701 | H | H | $CH_3$ | $CH(CH_3)OC_2H_5$ | |
| 4.0702 | H | $CH_3$ | $CH_3$ | $CH(CH_3)OC_2H_5$ | |
| 4.0703 | H | $C_2H_5$ | $CH_3$ | $CH(CH_3)OC_2H_5$ | |
| 4.0704 | H | $C_3H_7$ | $CH_3$ | $CH(CH_3)OC_2H_5$ | |
| 4.0705 | H | $OCH_3$ | $CH_3$ | $CH(CH_3)OC_2H_5$ | |
| 4.0706 | H | $OC_2H_5$ | $CH_3$ | $CH(CH_3)OC_2H_5$ | |
| 4.0707 | H | $OCH_2CH=CH_2$ | $CH_3$ | $CH(CH_3)OC_2H_5$ | |
| 4.0708 | H | $OCH_2CH=CHCH_3$ | $CH_3$ | $CH(CH_3)OC_2H_5$ | |
| 4.0709 | H | $OCH_2C(Cl)=CH_2$ | $CH_3$ | $CH(CH_3)OC_2H_5$ | |
| 4.0710 | H | $OCH_2CH=CHCl$ | $CH_3$ | $CH(CH_3)OC_2H_5$ | |
| 4.0711 | H | $OCH_2C{\equiv}CH$ | $CH_3$ | $CH(CH_3)OC_2H_5$ | |
| 4.0712 | H | $OCHF_2$ | $CH_3$ | $CH(CH_3)OC_2H_5$ | |
| 4.0713 | H | $OCF_3$ | $CH_3$ | $CH(CH_3)OC_2H_5$ | |
| 4.0714 | H | $OCF_2CHFCF_3$ | $CH_3$ | $CH(CH_3)OC_2H_5$ | |
| 4.0715 | H | $OC_6H_5$ | $CH_3$ | $CH(CH_3)OC_2H_5$ | |
| 4.0716 | H | $CF_3$ | $CH_3$ | $CH(CH_3)OC_2H_5$ | |
| 4.0717 | H | Br | $CH_3$ | $CH(CH_3)OC_2H_5$ | |
| 4.0718 | H | Cl | $CH_3$ | $CH(CH_3)OC_2H_5$ | |
| 4.0719 | H | F | $CH_3$ | $CH(CH_3)OC_2H_5$ | |
| 4.0720 | H | $SCH_3$ | $CH_3$ | $CH(CH_3)OC_2H_5$ | |
| 4.0721 | H | $SC_2H_5$ | $CH_3$ | $CH(CH_3)OC_2H_5$ | |
| 4.0722 | H | $SC_3H_7$-n | $CH_3$ | $CH(CH_3)OC_2H_5$ | |
| 4.0723 | H | $SO_2CH_3$ | $CH_3$ | $CH(CH_3)OC_2H_5$ | |
| 4.0724 | H | $SO_2C_2H_5$ | $CH_3$ | $CH(CH_3)OC_2H_5$ | |
| 4.0725 | H | $NO_2$ | $CH_3$ | $CH(CH_3)OC_2H_5$ | |
| 4.0726 | H | CN | $CH_3$ | $CH(CH_3)OC_2H_5$ | |
| 4.0727 | H | $CH_2OH$ | $CH_3$ | $CH(CH_3)OC_2H_5$ | |
| 4.0728 | H | $CH_2CH_2OH$ | $CH_3$ | $CH(CH_3)OC_2H_5$ | |
| 4.0729 | H | $CH_2CH_2CH_2OH$ | $CH_3$ | $CH(CH_3)OC_2H_5$ | |
| 4.0730 | H | $C_6H_5$ | $CH_3$ | $CH(CH_3)OC_2H_5$ | |
| 4.0731 | H | $CH_2Cl$ | $CH_3$ | $CH(CH_3)OC_2H_5$ | |
| 4.0732 | H | $CH_2CH_2Cl$ | $CH_3$ | $CH(CH_3)OC_2H_5$ | |
| 4.0733 | H | $CH_2CH_2CH_2Cl$ | $CH_3$ | $CH(CH_3)OC_2H_5$ | |

Tabelle 4.08

| No. | X | Y | $R_1$ | Z | phys. Daten |
|---|---|---|---|---|---|
| 4.0801 | H | H | $CH_3$ | $C(CH_3)_2OCH_3$ | |
| 4.0802 | H | $CH_3$ | $CH_3$ | $C(CH_3)_2OCH_3$ | |
| 4.0803 | H | $C_2H_5$ | $CH_3$ | $C(CH_3)_2OCH_3$ | |
| 4.0804 | H | $C_3H_7$ | $CH_3$ | $C(CH_3)_2OCH_3$ | |
| 4.0805 | H | $OCH_3$ | $CH_3$ | $C(CH_3)_2OCH_3$ | |
| 4.0806 | H | $OC_2H_5$ | $CH_3$ | $C(CH_3)_2OCH_3$ | |
| 4.0807 | H | $OCH_2CH=CH_2$ | $CH_3$ | $C(CH_3)_2OCH_3$ | |
| 4.0808 | H | $OCH_2CH=CHCH_3$ | $CH_3$ | $C(CH_3)_2OCH_3$ | |
| 4.0809 | H | $OCH_2C(Cl)=CH_2$ | $CH_3$ | $C(CH_3)_2OCH_3$ | |
| 4.0810 | H | $OCH_2CH=CHCl$ | $CH_3$ | $C(CH_3)_2OCH_3$ | |
| 4.0811 | H | $OCH_2C\equiv CH$ | $CH_3$ | $C(CH_3)_2OCH_3$ | |
| 4.0812 | H | $OCHF_2$ | $CH_3$ | $C(CH_3)_2OCH_3$ | |
| 4.0813 | H | $OCF_3$ | $CH_3$ | $C(CH_3)_2OCH_3$ | |
| 4.0814 | H | $OCF_2CHFCF_3$ | $CH_3$ | $C(CH_3)_2OCH_3$ | |
| 4.0815 | H | $OC_6H_5$ | $CH_3$ | $C(CH_3)_2OCH_3$ | |
| 4.0816 | H | $CF_3$ | $CH_3$ | $C(CH_3)_2OCH_3$ | |
| 4.0817 | H | Br | $CH_3$ | $C(CH_3)_2OCH_3$ | |
| 4.0818 | H | Cl | $CH_3$ | $C(CH_3)_2OCH_3$ | |
| 4.0819 | H | F | $CH_3$ | $C(CH_3)_2OCH_3$ | |
| 4.0820 | H | $SCH_3$ | $CH_3$ | $C(CH_3)_2OCH_3$ | |
| 4.0821 | H | $SC_2H_5$ | $CH_3$ | $C(CH_3)_2OCH_3$ | |
| 4.0822 | H | $SC_3H_7$-n | $CH_3$ | $C(CH_3)_2OCH_3$ | |
| 4.0823 | H | $SO_2CH_3$ | $CH_3$ | $C(CH_3)_2OCH_3$ | |
| 4.0824 | H | $SO_2C_2H_5$ | $CH_3$ | $C(CH_3)_2OCH_3$ | |
| 4.0825 | H | $NO_2$ | $CH_3$ | $C(CH_3)_2OCH_3$ | |
| 4.0826 | H | CN | $CH_3$ | $C(CH_3)_2OCH_3$ | |
| 4.0827 | H | $CH_2OH$ | $CH_3$ | $C(CH_3)_2OCH_3$ | |
| 4.0828 | H | $CH_2CH_2OH$ | $CH_3$ | $C(CH_3)_2OCH_3$ | |
| 4.0829 | H | $CH_2CH_2CH_2OH$ | $CH_3$ | $C(CH_3)_2OCH_3$ | |
| 4.0830 | H | $C_6H_5$ | $CH_3$ | $C(CH_3)_2OCH_3$ | |
| 4.0831 | H | $CH_2Cl$ | $CH_3$ | $C(CH_3)_2OCH_3$ | |
| 4.0832 | H | $CH_2CH_2Cl$ | $CH_3$ | $C(CH_3)_2OCH_3$ | |
| 4.0833 | H | $CH_2CH_2CH_2Cl$ | $CH_3$ | $C(CH_3)_2OCH_3$ | |

Tabelle 4.09

| No. | X | Y | $R_1$ | Z | phys. Daten |
|---|---|---|---|---|---|
| 4.0901 | H | H | $CH_3$ | $CH_2OC_3H_7$ | |
| 4.0902 | H | $CH_3$ | $CH_3$ | $CH_2OC_3H_7$ | |
| 4.0903 | H | $C_2H_5$ | $CH_3$ | $CH_2OC_3H_7$ | |
| 4.0904 | H | $C_3H_7$ | $CH_3$ | $CH_2OC_3H_7$ | |
| 4.0905 | H | $OCH_3$ | $CH_3$ | $CH_2OC_3H_7$ | |
| 4.0906 | H | $OC_2H_5$ | $CH_3$ | $CH_2OC_3H_7$ | |
| 4.0907 | H | $OCH_2CH=CH_2$ | $CH_3$ | $CH_2OC_3H_7$ | |
| 4.0908 | H | $OCH_2CH=CHCH_3$ | $CH_3$ | $CH_2OC_3H_7$ | |
| 4.0909 | H | $OCH_2C(Cl)=CH_2$ | $CH_3$ | $CH_2OC_3H_7$ | |
| 4.0910 | H | $OCH_2CH=CHCl$ | $CH_3$ | $CH_2OC_3H_7$ | |
| 4.0911 | H | $OCH_2C\equiv CH$ | $CH_3$ | $CH_2OC_3H_7$ | |
| 4.0912 | H | $OCHF_2$ | $CH_3$ | $CH_2OC_3H_7$ | |
| 4.0913 | H | $OCF_3$ | $CH_3$ | $CH_2OC_3H_7$ | |
| 4.0914 | H | $OCF_2CHFCF_3$ | $CH_3$ | $CH_2OC_3H_7$ | |
| 4.0915 | H | $OC_6H_5$ | $CH_3$ | $CH_2OC_3H_7$ | |
| 4.0916 | H | $CF_3$ | $CH_3$ | $CH_2OC_3H_7$ | |
| 4.0917 | H | Br | $CH_3$ | $CH_2OC_3H_7$ | |
| 4.0918 | H | Cl | $CH_3$ | $CH_2OC_3H_7$ | |
| 4.0919 | H | F | $CH_3$ | $CH_2OC_3H_7$ | |
| 4.0920 | H | $SCH_3$ | $CH_3$ | $CH_2OC_3H_7$ | |
| 4.0921 | H | $SC_2H_5$ | $CH_3$ | $CH_2OC_3H_7$ | |
| 4.0922 | H | $SC_3H_7$-n | $CH_3$ | $CH_2OC_3H_7$ | |
| 4.0923 | H | $SO_2CH_3$ | $CH_3$ | $CH_2OC_3H_7$ | |
| 4.0924 | H | $SO_2C_2H_5$ | $CH_3$ | $CH_2OC_3H_7$ | |
| 4.0925 | H | $NO_2$ | $CH_3$ | $CH_2OC_3H_7$ | |
| 4.0926 | H | CN | $CH_3$ | $CH_2OC_3H_7$ | |
| 4.0927 | H | $CH_2OH$ | $CH_3$ | $CH_2OC_3H_7$ | |
| 4.0928 | H | $CH_2CH_2OH$ | $CH_3$ | $CH_2OC_3H_7$ | |
| 4.0929 | H | $CH_2CH_2CH_2OH$ | $CH_3$ | $CH_2OC_3H_7$ | |
| 4.0930 | H | $C_6H_5$ | $CH_3$ | $CH_2OC_3H_7$ | |
| 4.0931 | H | $CH_2Cl$ | $CH_3$ | $CH_2OC_3H_7$ | |
| 4.0932 | H | $CH_2CH_2Cl$ | $CH_3$ | $CH_2OC_3H_7$ | |
| 4.0933 | H | $CH_2CH_2CH_2Cl$ | $CH_3$ | $CH_2OC_3H_7$ | |

Tabelle 4.10

| No. | X | Y | $R_1$ | Z | phys. Daten |
|---|---|---|---|---|---|
| 4.1001 | H | H | $CH_3$ | $C(CH_3)C_2H_4$(Cyclo) | Smp. 160-161°C |
| 4.1002 | H | $CH_3$ | $CH_3$ | $C(CH_3)C_2H_4$(Cyclo) | |
| 4.1003 | H | $C_2H_5$ | $CH_3$ | $C(CH_3)C_2H_4$(Cyclo) | |
| 4.1004 | H | $C_3H_7$ | $CH_3$ | $C(CH_3)C_2H_4$(Cyclo) | |
| 4.1005 | H | $OCH_3$ | $CH_3$ | $C(CH_3)C_2H_4$(Cyclo) | |
| 4.1006 | H | $OC_2H_5$ | $CH_3$ | $C(CH_3)C_2H_4$(Cyclo) | |
| 4.1007 | H | $OCH_2CH=CH_2$ | $CH_3$ | $C(CH_3)C_2H_4$(Cyclo) | |
| 4.1008 | H | $OCH_2CH=CHCH_3$ | $CH_3$ | $C(CH_3)C_2H_4$(Cyclo) | |
| 4.1009 | H | $OCH_2C(Cl)=CH_2$ | $CH_3$ | $C(CH_3)C_2H_4$(Cyclo) | |
| 4.1010 | H | $OCH_2CH=CHCl$ | $CH_3$ | $C(CH_3)C_2H_4$(Cyclo) | |
| 4.1011 | H | $OCH_2C\equiv CH$ | $CH_3$ | $C(CH_3)C_2H_4$(Cyclo) | |
| 4.1012 | H | $OCHF_2$ | $CH_3$ | $C(CH_3)C_2H_4$(Cyclo) | |
| 4.1013 | H | $OCF_3$ | $CH_3$ | $C(CH_3)C_2H_4$(Cyclo) | |
| 4.1014 | H | $OCF_2CHFCF_3$ | $CH_3$ | $C(CH_3)C_2H_4$(Cyclo) | |
| 4.1015 | H | $OC_6H_5$ | $CH_3$ | $C(CH_3)C_2H_4$(Cyclo) | |
| 4.1016 | H | $CF_3$ | $CH_3$ | $C(CH_3)C_2H_4$(Cyclo) | |
| 4.1017 | H | Br | $CH_3$ | $C(CH_3)C_2H_4$(Cyclo) | |
| 4.1018 | H | Cl | $CH_3$ | $C(CH_3)C_2H_4$(Cyclo) | |
| 4.1019 | H | F | $CH_3$ | $C(CH_3)C_2H_4$(Cyclo) | |
| 4.1020 | H | $SCH_3$ | $CH_3$ | $C(CH_3)C_2H_4$(Cyclo) | |
| 4.1021 | H | $SC_2H_5$ | $CH_3$ | $C(CH_3)C_2H_4$(Cyclo) | |
| 4.1022 | H | $SC_3H_7$-n | $CH_3$ | $C(CH_3)C_2H_4$(Cyclo) | |
| 4.1023 | H | $SO_2CH_3$ | $CH_3$ | $C(CH_3)C_2H_4$(Cyclo) | |
| 4.1024 | H | $SO_2C_2H_5$ | $CH_3$ | $C(CH_3)C_2H_4$(Cyclo) | |
| 4.1025 | H | $NO_2$ | $CH_3$ | $C(CH_3)C_2H_4$(Cyclo) | |
| 4.1026 | H | CN | $CH_3$ | $C(CH_3)C_2H_4$(Cyclo) | |
| 4.1027 | H | $CH_2OH$ | $CH_3$ | $C(CH_3)C_2H_4$(Cyclo) | |
| 4.1028 | H | $CH_2CH_2OH$ | $CH_3$ | $C(CH_3)C_2H_4$(Cyclo) | |
| 4.1029 | H | $CH_2CH_2CH_2OH$ | $CH_3$ | $C(CH_3)C_2H_4$(Cyclo) | |
| 4.1030 | H | $C_6H_5$ | $CH_3$ | $C(CH_3)C_2H_4$(Cyclo) | |
| 4.1031 | H | $CH_2Cl$ | $CH_3$ | $C(CH_3)C_2H_4$(Cyclo) | |
| 4.1032 | H | $CH_2CH_2Cl$ | $CH_3$ | $C(CH_3)C_2H_4$(Cyclo) | |
| 4.1033 | H | $CH_2CH_2CH_2Cl$ | $CH_3$ | $C(CH_3)C_2H_4$(Cyclo) | |

Tabelle 4.11

| No. | X | Y | $R_1$ | Z | phys. Daten |
|---|---|---|---|---|---|
| 4.1101 | H | H | $CH_3$ | $CH_2OC_6H_5$ | |
| 4.1102 | H | $CH_3$ | $CH_3$ | $CH_2OC_6H_5$ | |
| 4.1103 | H | $C_2H_5$ | $CH_3$ | $CH_2OC_6H_5$ | |
| 4.1104 | H | $C_3H_7$ | $CH_3$ | $CH_2OC_6H_5$ | |
| 4.1105 | H | $OCH_3$ | $CH_3$ | $CH_2OC_6H_5$ | |
| 4.1106 | H | $OC_2H_5$ | $CH_3$ | $CH_2OC_6H_5$ | |
| 4.1107 | H | $OCH_2CH=CH_2$ | $CH_3$ | $CH_2OC_6H_5$ | |
| 4.1108 | H | $OCH_2CH=CHCH_3$ | $CH_3$ | $CH_2OC_6H_5$ | |
| 4.1109 | H | $OCH_2C(Cl)=CH_2$ | $CH_3$ | $CH_2OC_6H_5$ | |
| 4.1110 | H | $OCH_2CH=CHCl$ | $CH_3$ | $CH_2OC_6H_5$ | |
| 4.1111 | H | $OCH_2C\equiv CH$ | $CH_3$ | $CH_2OC_6H_5$ | |
| 4.1112 | H | $OCHF_2$ | $CH_3$ | $CH_2OC_6H_5$ | |
| 4.1113 | H | $OCF_3$ | $CH_3$ | $CH_2OC_6H_5$ | |
| 4.1114 | H | $OCF_2CHFCF_3$ | $CH_3$ | $CH_2OC_6H_5$ | |
| 4.1115 | H | $OC_6H_5$ | $CH_3$ | $CH_2OC_6H_5$ | |
| 4.1116 | H | $CF_3$ | $CH_3$ | $CH_2OC_6H_5$ | |
| 4.1117 | H | Br | $CH_3$ | $CH_2OC_6H_5$ | |
| 4.1118 | H | Cl | $CH_3$ | $CH_2OC_6H_5$ | |
| 4.1119 | H | F | $CH_3$ | $CH_2OC_6H_5$ | |
| 4.1120 | H | $SCH_3$ | $CH_3$ | $CH_2OC_6H_5$ | |
| 4.1121 | H | $SC_2H_5$ | $CH_3$ | $CH_2OC_6H_5$ | |
| 4.1122 | H | $SC_3H_7$-n | $CH_3$ | $CH_2OC_6H_5$ | |
| 4.1123 | H | $SO_2CH_3$ | $CH_3$ | $CH_2OC_6H_5$ | |
| 4.1124 | H | $SO_2C_2H_5$ | $CH_3$ | $CH_2OC_6H_5$ | |
| 4.1125 | H | $NO_2$ | $CH_3$ | $CH_2OC_6H_5$ | |
| 4.1126 | H | CN | $CH_3$ | $CH_2OC_6H_5$ | |
| 4.1127 | H | $CH_2OH$ | $CH_3$ | $CH_2OC_6H_5$ | |
| 4.1128 | H | $CH_2CH_2OH$ | $CH_3$ | $CH_2OC_6H_5$ | |
| 4.1129 | H | $CH_2CH_2CH_2OH$ | $CH_3$ | $CH_2OC_6H_5$ | |
| 4.1130 | H | $C_6H_5$ | $CH_3$ | $CH_2OC_6H_5$ | |
| 4.1131 | H | $CH_2Cl$ | $CH_3$ | $CH_2OC_6H_5$ | |
| 4.1132 | H | $CH_2CH_2Cl$ | $CH_3$ | $CH_2OC_6H_5$ | |
| 4.1133 | H | $CH_2CH_2CH_2Cl$ | $CH_3$ | $CH_2OC_6H_5$ | |

Tabelle 4.12

| No. | X | Y | $R_1$ | Z | phys. Daten |
|---|---|---|---|---|---|
| 4.1201 | H | H | $CH_3$ | Furan-2-yl | |
| 4.1202 | H | $CH_3$ | $CH_3$ | Furan-2-yl | |
| 4.1203 | H | $C_2H_5$ | $CH_3$ | Furan-2-yl | |
| 4.1204 | H | $C_3H_7$ | $CH_3$ | Furan-2-yl | |
| 4.1205 | H | $OCH_3$ | $CH_3$ | Furan-2-yl | |
| 4.1206 | H | $OC_2H_5$ | $CH_3$ | Furan-2-yl | |
| 4.1207 | H | $OCH_2CH=CH_2$ | $CH_3$ | Furan-2-yl | |
| 4.1208 | H | $OCH_2CH=CHCH_3$ | $CH_3$ | Furan-2-yl | |
| 4.1209 | H | $OCH_2C(Cl)=CH_2$ | $CH_3$ | Furan-2-yl | |
| 4.1210 | H | $OCH_2CH=CHCl$ | $CH_3$ | Furan-2-yl | |
| 4.1211 | H | $OCH_2C\equiv CH$ | $CH_3$ | Furan-2-yl | |
| 4.1212 | H | $OCHF_2$ | $CH_3$ | Furan-2-yl | |
| 4.1213 | H | $OCF_3$ | $CH_3$ | Furan-2-yl | |
| 4.1214 | H | $OCF_2CHFCF_3$ | $CH_3$ | Furan-2-yl | |
| 4.1215 | H | $OC_6H_5$ | $CH_3$ | Furan-2-yl | |
| 4.1216 | H | $CF_3$ | $CH_3$ | Furan-2-yl | |
| 4.1217 | H | Br | $CH_3$ | Furan-2-yl | |
| 4.1218 | H | Cl | $CH_3$ | Furan-2-yl | |
| 4.1219 | H | F | $CH_3$ | Furan-2-yl | |
| 4.1220 | H | $SCH_3$ | $CH_3$ | Furan-2-yl | |
| 4.1221 | H | $SC_2H_5$ | $CH_3$ | Furan-2-yl | |
| 4.1222 | H | $SC_3H_7$-n | $CH_3$ | Furan-2-yl | |
| 4.1223 | H | $SO_2CH_3$ | $CH_3$ | Furan-2-yl | |
| 4.1224 | H | $SO_2C_2H_5$ | $CH_3$ | Furan-2-yl | |
| 4.1225 | H | $NO_2$ | $CH_3$ | Furan-2-yl | |
| 4.1226 | H | CN | $CH_3$ | Furan-2-yl | |
| 4.1227 | H | $CH_2OH$ | $CH_3$ | Furan-2-yl | |
| 4.1228 | H | $CH_2CH_2OH$ | $CH_3$ | Furan-2-yl | |
| 4.1229 | H | $CH_2CH_2CH_2OH$ | $CH_3$ | Furan-2-yl | |
| 4.1230 | H | $C_6H_5$ | $CH_3$ | Furan-2-yl | |
| 4.1231 | H | $CH_2Cl$ | $CH_3$ | Furan-2-yl | |
| 4.1232 | H | $CH_2CH_2Cl$ | $CH_3$ | Furan-2-yl | |
| 4.1233 | H | $CH_2CH_2CH_2Cl$ | $CH_3$ | Furan-2-yl | |

Tabelle 4.13

| No. | X | Y | $R_1$ | Z | phys. Daten |
|---|---|---|---|---|---|
| 4.1301 | H | H | $CH_3$ | Dioxan-2-yl | |
| 4.1302 | H | $CH_3$ | $CH_3$ | Dioxan-2-yl | |
| 4.1303 | H | $C_2H_5$ | $CH_3$ | Dioxan-2-yl | |
| 4.1304 | H | $C_3H_7$ | $CH_3$ | Dioxan-2-yl | |
| 4.1305 | H | $OCH_3$ | $CH_3$ | Dioxan-2-yl | |
| 4.1306 | H | $OC_2H_5$ | $CH_3$ | Dioxan-2-yl | |
| 4.1307 | H | $OCH_2CH=CH_2$ | $CH_3$ | Dioxan-2-yl | |
| 4.1308 | H | $OCH_2CH=CHCH_3$ | $CH_3$ | Dioxan-2-yl | |
| 4.1309 | H | $OCH_2C(Cl)=CH_2$ | $CH_3$ | Dioxan-2-yl | |
| 4.1310 | H | $OCH_2CH=CHCl$ | $CH_3$ | Dioxan-2-yl | |
| 4.1311 | H | $OCH_2C{\equiv}CH$ | $CH_3$ | Dioxan-2-yl | |
| 4.1312 | H | $OCHF_2$ | $CH_3$ | Dioxan-2-yl | |
| 4.1313 | H | $OCF_3$ | $CH_3$ | Dioxan-2-yl | |
| 4.1314 | H | $OCF_2CHFCF_3$ | $CH_3$ | Dioxan-2-yl | |
| 4.1315 | H | $OC_6H_5$ | $CH_3$ | Dioxan-2-yl | |
| 4.1316 | H | $CF_3$ | $CH_3$ | Dioxan-2-yl | |
| 4.1317 | H | Br | $CH_3$ | Dioxan-2-yl | |
| 4.1318 | H | Cl | $CH_3$ | Dioxan-2-yl | |
| 4.1319 | H | F | $CH_3$ | Dioxan-2-yl | |
| 4.1320 | H | $SCH_3$ | $CH_3$ | Dioxan-2-yl | |
| 4.1321 | H | $SC_2H_5$ | $CH_3$ | Dioxan-2-yl | |
| 4.1322 | H | $SC_3H_7$-n | $CH_3$ | Dioxan-2-yl | |
| 4.1323 | H | $SO_2CH_3$ | $CH_3$ | Dioxan-2-yl | |
| 4.1324 | H | $SO_2C_2H_5$ | $CH_3$ | Dioxan-2-yl | |
| 4.1325 | H | $NO_2$ | $CH_3$ | Dioxan-2-yl | |
| 4.1326 | H | CN | $CH_3$ | Dioxan-2-yl | |
| 4.1327 | H | $CH_2OH$ | $CH_3$ | Dioxan-2-yl | |
| 4.1328 | H | $CH_2CH_2OH$ | $CH_3$ | Dioxan-2-yl | |
| 4.1329 | H | $CH_2CH_2CH_2OH$ | $CH_3$ | Dioxan-2-yl | |
| 4.1330 | H | $C_6H_5$ | $CH_3$ | Dioxan-2-yl | |
| 4.1331 | H | $CH_2Cl$ | $CH_3$ | Dioxan-2-yl | |
| 4.1332 | H | $CH_2CH_2Cl$ | $CH_3$ | Dioxan-2-yl | |
| 4.1333 | H | $CH_2CH_2CH_2Cl$ | $CH_3$ | Dioxan-2-yl | |

Tabelle 4.14

| No. | X | Y | $R_1$ | Z | phys. Daten |
|---|---|---|---|---|---|
| 4.1401 | H | H | $CH_3$ | $CH_2OC_2H_4OCH_3$ | |
| 4.1402 | H | $CH_3$ | $CH_3$ | $CH_2OC_2H_4OCH_3$ | |
| 4.1403 | H | $C_2H_5$ | $CH_3$ | $CH_2OC_2H_4OCH_3$ | |
| 4.1404 | H | $C_3H_7$ | $CH_3$ | $CH_2OC_2H_4OCH_3$ | |
| 4.1405 | H | $OCH_3$ | $CH_3$ | $CH_2OC_2H_4OCH_3$ | |
| 4.1406 | H | $OC_2H_5$ | $CH_3$ | $CH_2OC_2H_4OCH_3$ | |
| 4.1407 | H | $OCH_2CH{=}CH_2$ | $CH_3$ | $CH_2OC_2H_4OCH_3$ | |
| 4.1408 | H | $OCH_2CH{=}CHCH_3$ | $CH_3$ | $CH_2OC_2H_4OCH_3$ | |
| 4.1409 | H | $OCH_2C(Cl){=}CH_2$ | $CH_3$ | $CH_2OC_2H_4OCH_3$ | |
| 4.1410 | H | $OCH_2CH{=}CHCl$ | $CH_3$ | $CH_2OC_2H_4OCH_3$ | |
| 4.1411 | H | $OCH_2C{\equiv}CH$ | $CH_3$ | $CH_2OC_2H_4OCH_3$ | |
| 4.1412 | H | $OCHF_2$ | $CH_3$ | $CH_2OC_2H_4OCH_3$ | |
| 4.1413 | H | $OCF_3$ | $CH_3$ | $CH_2OC_2H_4OCH_3$ | |
| 4.1414 | H | $OCF_2CHFCF_3$ | $CH_3$ | $CH_2OC_2H_4OCH_3$ | |
| 4.1415 | H | $OC_6H_5$ | $CH_3$ | $CH_2OC_2H_4OCH_3$ | |
| 4.1416 | H | $CF_3$ | $CH_3$ | $CH_2OC_2H_4OCH_3$ | |
| 4.1417 | H | Br | $CH_3$ | $CH_2OC_2H_4OCH_3$ | |
| 4.1418 | H | Cl | $CH_3$ | $CH_2OC_2H_4OCH_3$ | |
| 4.1419 | H | F | $CH_3$ | $CH_2OC_2H_4OCH_3$ | |
| 4.1420 | H | $SCH_3$ | $CH_3$ | $CH_2OC_2H_4OCH_3$ | |
| 4.1421 | H | $SC_2H_5$ | $CH_3$ | $CH_2OC_2H_4OCH_3$ | |
| 4.1422 | H | $SC_3H_7$-n | $CH_3$ | $CH_2OC_2H_4OCH_3$ | |
| 4.1423 | H | $SO_2CH_3$ | $CH_3$ | $CH_2OC_2H_4OCH_3$ | |
| 4.1424 | H | $SO_2C_2H_5$ | $CH_3$ | $CH_2OC_2H_4OCH_3$ | |
| 4.1425 | H | $NO_2$ | $CH_3$ | $CH_2OC_2H_4OCH_3$ | |
| 4.1426 | H | CN | $CH_3$ | $CH_2OC_2H_4OCH_3$ | |
| 4.1427 | H | $CH_2OH$ | $CH_3$ | $CH_2OC_2H_4OCH_3$ | |
| 4.1428 | H | $CH_2CH_2OH$ | $CH_3$ | $CH_2OC_2H_4OCH_3$ | |
| 4.1429 | H | $CH_2CH_2CH_2OH$ | $CH_3$ | $CH_2OC_2H_4OCH_3$ | |
| 4.1430 | H | $C_6H_5$ | $CH_3$ | $CH_2OC_2H_4OCH_3$ | |
| 4.1431 | H | $CH_2Cl$ | $CH_3$ | $CH_2OC_2H_4OCH_3$ | |
| 4.1432 | H | $CH_2CH_2Cl$ | $CH_3$ | $CH_2OC_2H_4OCH_3$ | |
| 4.1433 | H | $CH_2CH_2CH_2Cl$ | $CH_3$ | $CH_2OC_2H_4OCH_3$ | |

Tabelle 4.15

| No. | X | Y | $R_1$ | Z | phys. Daten |
|---|---|---|---|---|---|
| 4.1501 | H | H | $CH_3$ | $CH_2OC_2H_4OC_2H_4OCH_3$ | |
| 4.1502 | H | $CH_3$ | $CH_3$ | $CH_2OC_2H_4OC_2H_4OCH_3$ | |
| 4.1503 | H | $C_2H_5$ | $CH_3$ | $CH_2OC_2H_4OC_2H_4OCH_3$ | |
| 4.1504 | H | $C_3H_7$ | $CH_3$ | $CH_2OC_2H_4OC_2H_4OCH_3$ | |
| 4.1505 | H | $OCH_3$ | $CH_3$ | $CH_2OC_2H_4OC_2H_4OCH_3$ | |
| 4.1506 | H | $OC_2H_5$ | $CH_3$ | $CH_2OC_2H_4OC_2H_4OCH_3$ | |
| 4.1507 | H | $OCH_2CH=CH_2$ | $CH_3$ | $CH_2OC_2H_4OC_2H_4OCH_3$ | |
| 4.1508 | H | $OCH_2CH=CHCH_3$ | $CH_3$ | $CH_2OC_2H_4OC_2H_4OCH_3$ | |
| 4.1509 | H | $OCH_2C(Cl)=CH_2$ | $CH_3$ | $CH_2OC_2H_4OC_2H_4OCH_3$ | |
| 4.1510 | H | $OCH_2CH=CHCl$ | $CH_3$ | $CH_2OC_2H_4OC_2H_4OCH_3$ | |
| 4.1511 | H | $OCH_2C{\equiv}CH$ | $CH_3$ | $CH_2OC_2H_4OC_2H_4OCH_3$ | |
| 4.1512 | H | $OCHF_2$ | $CH_3$ | $CH_2OC_2H_4OC_2H_4OCH_3$ | |
| 4.1513 | H | $OCF_3$ | $CH_3$ | $CH_2OC_2H_4OC_2H_4OCH_3$ | |
| 4.1514 | H | $OCF_2CHFCF_3$ | $CH_3$ | $CH_2OC_2H_4OC_2H_4OCH_3$ | |
| 4.1515 | H | $OC_6H_5$ | $CH_3$ | $CH_2OC_2H_4OC_2H_4OCH_3$ | |
| 4.1516 | H | $CF_3$ | $CH_3$ | $CH_2OC_2H_4OC_2H_4OCH_3$ | |
| 4.1517 | H | Br | $CH_3$ | $CH_2OC_2H_4OC_2H_4OCH_3$ | |
| 4.1518 | H | Cl | $CH_3$ | $CH_2OC_2H_4OC_2H_4OCH_3$ | |
| 4.1519 | H | F | $CH_3$ | $CH_2OC_2H_4OC_2H_4OCH_3$ | |
| 4.1520 | H | $SCH_3$ | $CH_3$ | $CH_2OC_2H_4OC_2H_4OCH_3$ | |
| 4.1521 | H | $SC_2H_5$ | $CH_3$ | $CH_2OC_2H_4OC_2H_4OCH_3$ | |
| 4.1522 | H | $SC_3H_7$-n | $CH_3$ | $CH_2OC_2H_4OC_2H_4OCH_3$ | |
| 4.1523 | H | $SO_2CH_3$ | $CH_3$ | $CH_2OC_2H_4OC_2H_4OCH_3$ | |
| 4.1524 | H | $SO_2C_2H_5$ | $CH_3$ | $CH_2OC_2H_4OC_2H_4OCH_3$ | |
| 4.1525 | H | $NO_2$ | $CH_3$ | $CH_2OC_2H_4OC_2H_4OCH_3$ | |
| 4.1526 | H | CN | $CH_3$ | $CH_2OC_2H_4OC_2H_4OCH_3$ | |
| 4.1527 | H | $CH_2OH$ | $CH_3$ | $CH_2OC_2H_4OC_2H_4OCH_3$ | |
| 4.1528 | H | $CH_2CH_2OH$ | $CH_3$ | $CH_2OC_2H_4OC_2H_4OCH_3$ | |
| 4.1529 | H | $CH_2CH_2CH_2OH$ | $CH_3$ | $CH_2OC_2H_4OC_2H_4OCH_3$ | |
| 4.1530 | H | $C_6H_5$ | $CH_3$ | $CH_2OC_2H_4OC_2H_4OCH_3$ | |
| 4.1531 | H | $CH_2Cl$ | $CH_3$ | $CH_2OC_2H_4OC_2H_4OCH_3$ | |
| 4.1532 | H | $CH_2CH_2Cl$ | $CH_3$ | $CH_2OC_2H_4OC_2H_4OCH_3$ | |
| 4.1533 | H | $CH_2CH_2CH_2Cl$ | $CH_3$ | $CH_2OC_2H_4OC_2H_4OCH_3$ | |

Tabelle 4.16

| No. | X | Y | $R_1$ | Z | phys. Daten |
|---|---|---|---|---|---|
| 4.1601 | H | H | $CH_3$ | $CH_2OCH_2CONH_2$ | |
| 4.1602 | H | $CH_3$ | $CH_3$ | $CH_2OCH_2CONH_2$ | |
| 4.1603 | H | $C_2H_5$ | $CH_3$ | $CH_2OCH_2CONH_2$ | |
| 4.1604 | H | $C_3H_7$ | $CH_3$ | $CH_2OCH_2CONH_2$ | |
| 4.1605 | H | $OCH_3$ | $CH_3$ | $CH_2OCH_2CONH_2$ | |
| 4.1606 | H | $OC_2H_5$ | $CH_3$ | $CH_2OCH_2CONH_2$ | |
| 4.1607 | H | $OCH_2CH=CH_2$ | $CH_3$ | $CH_2OCH_2CONH_2$ | |
| 4.1608 | H | $OCH_2CH=CHCH_3$ | $CH_3$ | $CH_2OCH_2CONH_2$ | |
| 4.1609 | H | $OCH_2C(Cl)=CH_2$ | $CH_3$ | $CH_2OCH_2CONH_2$ | |
| 4.1610 | H | $OCH_2CH=CHCl$ | $CH_3$ | $CH_2OCH_2CONH_2$ | |
| 4.1611 | H | $OCH_2C\equiv CH$ | $CH_3$ | $CH_2OCH_2CONH_2$ | |
| 4.1612 | H | $OCHF_2$ | $CH_3$ | $CH_2OCH_2CONH_2$ | |
| 4.1613 | H | $OCF_3$ | $CH_3$ | $CH_2OCH_2CONH_2$ | |
| 4.1614 | H | $OCF_2CHFCF_3$ | $CH_3$ | $CH_2OCH_2CONH_2$ | |
| 4.1615 | H | $OC_6H_5$ | $CH_3$ | $CH_2OCH_2CONH_2$ | |
| 4.1616 | H | $CF_3$ | $CH_3$ | $CH_2OCH_2CONH_2$ | |
| 4.1617 | H | Br | $CH_3$ | $CH_2OCH_2CONH_2$ | |
| 4.1618 | H | Cl | $CH_3$ | $CH_2OCH_2CONH_2$ | |
| 4.1619 | H | F | $CH_3$ | $CH_2OCH_2CONH_2$ | |
| 4.1620 | H | $SCH_3$ | $CH_3$ | $CH_2OCH_2CONH_2$ | |
| 4.1621 | H | $SC_2H_5$ | $CH_3$ | $CH_2OCH_2CONH_2$ | |
| 4.1622 | H | $SC_3H_7$-n | $CH_3$ | $CH_2OCH_2CONH_2$ | |
| 4.1623 | H | $SO_2CH_3$ | $CH_3$ | $CH_2OCH_2CONH_2$ | |
| 4.1624 | H | $SO_2C_2H_5$ | $CH_3$ | $CH_2OCH_2CONH_2$ | |
| 4.1625 | H | $NO_2$ | $CH_3$ | $CH_2OCH_2CONH_2$ | |
| 4.1626 | H | CN | $CH_3$ | $CH_2OCH_2CONH_2$ | |
| 4.1627 | H | $CH_2OH$ | $CH_3$ | $CH_2OCH_2CONH_2$ | |
| 4.1628 | H | $CH_2CH_2OH$ | $CH_3$ | $CH_2OCH_2CONH_2$ | |
| 4.1629 | H | $CH_2CH_2CH_2OH$ | $CH_3$ | $CH_2OCH_2CONH_2$ | |
| 4.1630 | H | $C_6H_5$ | $CH_3$ | $CH_2OCH_2CONH_2$ | |
| 4.1631 | H | $CH_2Cl$ | $CH_3$ | $CH_2OCH_2CONH_2$ | |
| 4.1632 | H | $CH_2CH_2Cl$ | $CH_3$ | $CH_2OCH_2CONH_2$ | |
| 4.1633 | H | $CH_2CH_2CH_2Cl$ | $CH_3$ | $CH_2OCH_2CONH_2$ | |

Beispiel 12 Herstellung von 6-Aethoxymethyl-2,3-pyridin dicarbonsäure

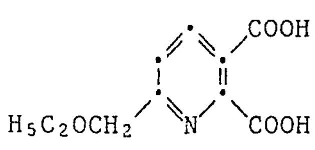

Zu einer Lösung von 0.5 g Tetrabutylammoniumbromid in 140 ml wässriger 15%iger Natriumhydroxydlösung gibt man unter Rühren 37,4 g 6-Aethoxymethyl-2,3-pyridincarbonsäure-diäthylester. Es wird eine leicht exotherme Reaktion beobachtet. Das Reaktionsgemisch wird 3 Stunden bei Raumtemperatur nachgerührt und dann weitgehend eingedampft wobei das Dinatriumsalz der 6-Aethoxymethyl-2,3-pyridindicarbonsäure ausfällt. Der Rückstand wird 50 ml Wasser versetzt und mit Aether extrahiert. Im Aetherextrakt verbleibt 0,5 g Edukt. Die wässrige Phase wird mit konzentrierter Salzsäure auf ph 2 ausgesäuert und dann 5 mal mit je 100 ml

Aethylacetat extrahiert. Die organischen Phasen werden gesammelt, über Magnesiumsulfat getrocknet und eingedampft. Dabei erhält man 26 g der obigen Säure als honiggelbe harzartige Substanz.

Beispiel 13 Herstellung von 6-Phenoxymethyl-pyridin-2,3-dicarbonsäure

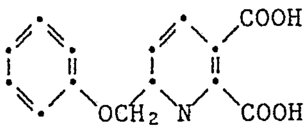

Zu einer Lösung von 0,5 g Tetrabutylammonium-bromid in 140 ml 15%iger wässriger Natriumhydroxydlösung gibt man unter Rühren 42 g 6-Phenoxymethyl-pyridin-2,3-dicarbonsäure-diäthylester. Die entstandene Suspension wird auf 80° erwärmt und eine Stunde gerührt. Dann verdünnt man das Reaktionsgemisch mit 300 ml Wasser und säuert die Lösung mittels konzentrierter Salzsäsure auf pH 2 an. Dabei fällt ein Niederschlag aus, der abgenutscht wird. Das Nutschgut wird mit Methylenchlorid/Tetrahydrafuran verrührt und filtriert. Das Filtrat wird über Magnesiumsulfat getrocknet, eingeengt und mit dem Nut scheninhalt vereinigt. Nachdem das ganze nochmals über Phosphorpentoxid getrocknet wurde erhält man 34 g der obigen Säure, welche bei 270° unter Zersetzen schmilzt.

In analoger Weise zu den Beispielen 12 und 13 werden die in den Tabellen 6.00 bis 6.02 aufgeführten 2,3-Pyridindicarbonsäuren der Formel VI hergestellt:

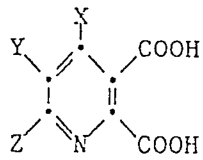

(VI)

Tabelle 6.00

| No. | X | Y | Z | phys. Daten |
|---|---|---|---|---|
| 6.0001 | H | H | $C_3H_5$(Cyclo) | |
| 6.0002 | H | $CH_3$ | $C_3H_5$(Cyclo) | |
| 6.0003 | H | $C_2H_5$ | $C_3H_5$(Cyclo) | |
| 6.0004 | H | $C_3H_7$ | $C_3H_5$(Cyclo) | |
| 6.0005 | H | $OCH_3$ | $C_3H_5$(Cyclo) | |
| 6.0006 | H | $OC_2H_5$ | $C_3H_5$(Cyclo) | |
| 6.0007 | H | $OCH_2CH=CH_2$ | $C_3H_5$(Cyclo) | |
| 6.0008 | H | $OCH_2CH=CHCH_3$ | $C_3H_5$(Cyclo) | |
| 6.0009 | H | $OCH_2C(Cl)=CH_2$ | $C_3H_5$(Cyclo) | |
| 6.0010 | H | $OCH_2CH=CHCl$ | $C_3H_5$(Cyclo) | |
| 6.0011 | H | $OCH_2C \equiv CH$ | $C_3H_5$(Cyclo) | |
| 6.0012 | H | $OCHF_2$ | $C_3H_5$(Cyclo) | |
| 6.0013 | H | $OCF_3$ | $C_3H_5$(Cyclo) | |
| 6.0014 | H | $OCF_2CHFCF_3$ | $C_3H_5$(Cyclo) | |
| 6.0015 | H | $OC_6H_5$ | $C_3H_5$(Cyclo) | |
| 6.0016 | H | $CF_3$ | $C_3H_5$(Cyclo) | |
| 6.0017 | H | Br | $C_3H_5$(Cyclo) | |
| 6.0018 | H | Cl | $C_3H_5$(Cyclo) | |
| 6.0019 | H | F | $C_3H_5$(Cyclo) | |
| 6.0020 | H | $SCH_3$ | $C_3H_5$(Cyclo) | |
| 6.0021 | H | $SC_2H_5$ | $C_3H_5$(Cyclo) | |
| 6.0022 | H | $SC_3H_7$-n | $C_3H_5$(Cyclo) | |
| 6.0023 | H | $SO_2CH_3$ | $C_3H_5$(Cyclo) | |
| 6.0024 | H | $SO_2C_2H_5$ | $C_3H_5$(Cyclo) | |
| 6.0025 | H | $NO_2$ | $C_3H_5$(Cyclo) | |
| 6.0026 | H | CN | $C_3H_5$(Cyclo) | |
| 6.0027 | H | $CH_2OH$ | $C_3H_5$(Cyclo) | |
| 6.0028 | H | $CH_2CH_2OH$ | $C_3H_5$(Cyclo) | |
| 6.0029 | H | $CH_2CH_2CH_2OH$ | $C_3H_5$(Cyclo) | |
| 6.0030 | H | $C_6H_5$ | $C_3H_5$(Cyclo) | |
| 6.0031 | H | $CH_2Cl$ | $C_3H_5$(Cyclo) | |
| 6.0032 | H | $CH_2CH_2Cl$ | $C_3H_5$(Cyclo) | |
| 6.0033 | H | $CH_2CH_2CH_2Cl$ | $C_3H_5$(Cyclo) | |

Tabelle 6.01

| No. | X | Y | Z | phys. Daten |
| --- | --- | --- | --- | --- |
| 6.0101 | H | H | $CH_2OC_2H_5$ | |
| 6.0102 | H | $CH_3$ | $CH_2OC_2H_5$ | |
| 6.0103 | H | $C_2H_5$ | $CH_2OC_2H_5$ | |
| 6.0104 | H | $C_3H_7$ | $CH_2OC_2H_5$ | |
| 6.0105 | H | $OCH_3$ | $CH_2OC6H_5$ | |
| 6.0213 | H | $OCF_3$ | $CH_2OC_6H_5$ | |
| 6.0214 | H | $OCF_2CHFCF_3$ | $CH_2OC_6H_5$ | |
| 6.0215 | H | $OC_6H_5$ | $CH_2OC_6H_5$ | |
| 6.0216 | H | $CF_3$ | $CH_2OC_6H_5$ | |
| 6.0217 | H | Br | $CH_2OC_6H_5$ | |
| 6.0218 | H | Cl | $CH_2OC_6H_5$ | |
| 6.0219 | H | F | $CH_2OC_6H_5$ | |
| 6.0220 | H | $SCH_3$ | $CH_2OC_6H_5$ | |
| 6.0221 | H | $SC_2H_5$ | $CH_2OC_6H_5$ | |
| 6.0222 | H | $SC_3H_7$-n | $CH_2OC_6H_5$ | |
| 6.0223 | H | $SO_2CH_3$ | $CH_2OC_6H_5$ | |
| 6.0224 | H | $SO_2C_2H_5$ | $CH_2OC_6H_5$ | |
| 6.0225 | H | $NO_2$ | $CH_2OC_6H_5$ | |
| 6.0226 | H | CN | $CH_2OC_6H_5$ | |
| 6.0227 | H | $CH_2OH$ | $CH_2OC_6H_5$ | |
| 6.0228 | H | $CH_2CH_2OH$ | $CH_2OC_6H_5$ | |
| 6.0229 | H | $CH_2CH_2CH_2OH$ | $CH_2OC_6H_5$ | |
| 6.0230 | H | $C_6H_5$ | $CH_2OC_6H_5$ | |
| 6.0231 | H | $CH_2Cl$ | $CH_2OC_6H_5$ | |
| 6.0232 | H | $CH_2CH_2Cl$ | $CH_2OC_6H_5$ | |
| 6.0233 | H | $CH_2CH_2CH_2Cl$ | $CH_2OC_6H_5$ | |

Beispiel 14 Herstellung von 6-Aethoxymethyl-2,3-pyridin-dicarbonsäureanhydrid

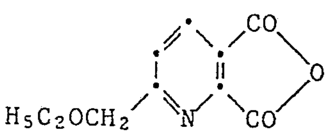

Zu einem Gemisch von 100 ml Dimethoxyäthan, 28 ml Acetanhydrid und 15 ml Pyridin gibt man unter Rühren 21 g 6-Aethoxymethyl-2,3-dicarbonsäure und rührt während 6 Stunden bei Raumtemperatur weiter bevor man das Ganze am Hochvakuum eindampft. Es verbleiben 20 g Anhydrid als ölige Flüssigkeit mit Brechpunkt $n_D^{22}$ 1,5480.

Beispiel 15 Herstellung von 6-Phenoxymethyl-2,3-pyridinicarbonsäureanhydrid

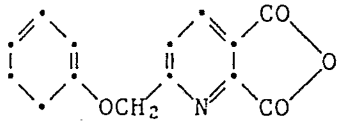

Zum Gemisch von 100 ml Dimethoxyäthan, 28 ml Acetanhydrid und 15 ml Pyridin gibt man unter Rühren 30 g 6-Phenoxymethyl-2,3-pyridindicarbonsäure und rührt eine Stunde bei Raumtemperatur weiter. Dann wird das Ganze am Hochvakuum eingedampft. Das obige Anhydrid verbleibt als helles Oel.

In analoger Weise zu den Beispielen 14 und 15 werden die in den Tabellen 7.00-7.02 aufgeführten 2,3-Pyridindicarbonsäureanhydrid der Formel VII hergestellt.

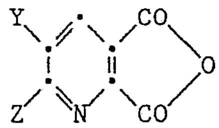

(VII)

Tabelle 7.00

| No. | X | Y | Z | phys. Daten |
|---|---|---|---|---|
| 7.0001 | H | H | $C_3H_5$(Cyclo) | |
| 7.0002 | H | $CH_3$ | $C_3H_5$(Cyclo) | |
| 7.0003 | H | $C_2H_5$ | $C_3H_5$(Cyclo) | |
| 7.0004 | H | $C_3H_7$ | $C_3H_5$(Cyclo) | |
| 7.0005 | H | $OCH_3$ | $C_3H_5$(Cyclo) | |
| 7.0006 | H | $OC_2H_5$ | $C_3H_5$(Cyclo) | |
| 7.0007 | H | $OCH_2CH{=}CH_2$ | $C_3H_5$(Cyclo) | |
| 7.0008 | H | $OCH_2CH{=}CHCH_3$ | $C_3H_5$(Cyclo) | |
| 7.0009 | H | $OCH_2C(Cl){=}CH_2$ | $C_3H_5$(Cyclo) | |
| 7.0010 | H | $OCH_2CH{=}CHCl$ | $C_3H_5$(Cyclo) | |
| 7.0011 | H | $OCH_2C{\equiv}CH$ | $C_3H_5$(Cyclo) | |
| 7.0012 | H | $OCHF_2$ | $C_3H_5$(Cyclo) | |
| 7.0013 | H | $OCF_3$ | $C_3H_5$(Cyclo) | |
| 7.0014 | H | $OCF_2CHFCF_3$ | $C_3H_5$(Cyclo) | |
| 7.0015 | H | $OC_6H_5$ | $C_3H_5$(Cyclo) | |
| 7.0016 | H | $CF_3$ | $C_3H_5$(Cyclo) | |
| 7.0017 | H | Br | $C_3H_5$(Cyclo) | |
| 7.0018 | H | Cl | $C_3H_5$(Cyclo) | |
| 7.0019 | H | F | $C_3H_5$(Cyclo) | |
| 7.0020 | H | $SCH_3$ | $C_3H_5$(Cyclo) | |
| 7.0021 | H | $SC_2H_5$ | $C_3H_5$(Cyclo) | |
| 7.0022 | H | $SC_3H_7$-n | $C_3H_5$(Cyclo) | |
| 7.0023 | H | $SO_2CH_3$ | $C_3H_5$(Cyclo) | |
| 7.0024 | H | $SO_2C_2H_5$ | $C_3H_5$(Cyclo) | |
| 7.0025 | H | $NO_2$ | $C_3H_5$(Cyclo) | |
| 7.0026 | H | CN | $C_3H_5$(Cyclo) | |
| 7.0027 | H | $CH_2OH$ | $C_3H_5$(Cyclo) | |
| 7.0028 | H | $CH_2CH_2OH$ | $C_3H_5$(Cyclo) | |
| 7.0029 | H | $CH_2CH_2CH_2OH$ | $C_3H_5$(Cyclo) | |
| 7.0030 | H | $C_6H_5$ | $C_3H_5$(Cyclo) | |
| 7.0031 | H | $CH_2Cl$ | $C_3H_5$(Cyclo) | |
| 7.0032 | H | $CH_2CH_2Cl$ | $C_3H_5$(Cyclo) | |
| 7.0033 | H | $CH_2CH_2CH_2Cl$ | $C_3H_5$(Cyclo) | |

Tabelle 7.01

| No. | X | Y | Z | phys. Daten |
|---|---|---|---|---|
| 7.0101 | H | H | $CH_2OC_2H_5$ | |
| 7.0102 | H | $CH_3$ | $CH_2OC_2H_5$ | |
| 7.0103 | H | $C_2H_5$ | $CH_2OC_2H_5$ | |
| 7.0104 | H | $C_3H_7$ | $CH_2OC_2H_5$ | |
| 7.0105 | H | $OCH_3$ | $CH_2OC_2H_5$ | |
| 7.0106 | H | $OC_2H_5$ | $CH_2OC_2H_5$ | |
| 7.0107 | H | $OCH_2CH=CH_2$ | $CH_2OC_2H_5$ | |
| 7.0108 | H | $OCH_2CH=CHCH_3$ | $CH_2OC_2H_5$ | |
| 7.0109 | H | $OCH_2C(Cl)=CH_2$ | $CH_2OC_2H_5$ | |
| 7.0110 | H | $OCH_2CH=CHCl$ | $CH_2OC_2H_5$ | |
| 7.0111 | H | $OCH_2C{\equiv}CH$ | $CH_2OC_2H_5$ | |
| 7.0112 | H | $OCHF_2$ | $CH_2OC_2H_5$ | |
| 7.0113 | H | $OCF_3$ | $CH_2OC_2H_5$ | |
| 7.0114 | H | $OCF_2CHFCF_3$ | $CH_2OC_2H_5$ | |
| 7.0115 | H | $OC_6H_5$ | $CH_2OC_2H_5$ | |
| 7.0116 | H | $CF_3$ | $CH_2OC_2H_5$ | |
| 7.0117 | H | Br | $CH_2OC_2H_5$ | |
| 7.0118 | H | Cl | $CH_2OC_2H_5$ | |
| 7.0119 | H | F | $CH_2OC_2H_5$ | |
| 7.0120 | H | $SCH_3$ | $CH_2OC_2H_5$ | |
| 7.0121 | H | $SC_2H_5$ | $CH_2OC_2H_5$ | |
| 7.0122 | H | $SC_3H_7$-n | $CH_2OC_2H_5$ | |
| 7.0123 | H | $SO_2CH_3$ | $CH_2OC_2H_5$ | |
| 7.0124 | H | $SO_2C_2H_5$ | $CH_2OC_2H_5$ | |
| 7.0125 | H | $NO_2$ | $CH_2OC_2H_5$ | |
| 7.0126 | H | CN | $CH_2OC_2H_5$ | |
| 7.0127 | H | $CH_2OH$ | $CH_2OC_2H_5$ | |
| 7.0128 | H | $CH_2CH_2OH$ | $CH_2OC_2H_5$ | |
| 7.0129 | H | $CH_2CH_2CH_2OH$ | $CH_2OC_2H_5$ | |
| 7.0130 | H | $C_6H_5$ | $CH_2OC_2H_5$ | |
| 7.0131 | H | $CH_2Cl$ | $CH_2OC_2H_5$ | |
| 7.0132 | H | $CH_2CH_2Cl$ | $CH_2OC_2H_5$ | |
| 7.0133 | H | $CH_2CH_2CH_2Cl$ | $CH_2OC_2H_5$ | |

Tabelle 7.02

| No. | X | Y | Z | phys. Daten |
|---|---|---|---|---|
| 7.0201 | H | H | $CH_2OC_6H_5$ | |
| 7.0202 | H | $CH_3$ | $CH_2OC_6H_5$ | |
| 7.0203 | H | $C_2H_5$ | $CH_2OC_6H_5$ | |
| 7.0204 | H | $C_3H_7$ | $CH_2OC_6H_5$ | |
| 7.0205 | H | $OCH_3$ | $CH_2OC_6H_5$ | |
| 7.0206 | H | $OC_2H_5$ | $CH_2OC_6H_5$ | |
| 7.0207 | H | $OCH_2CH=CH_2$ | $CH_2OC_6H_5$ | |
| 7.0208 | H | $OCH_2CH=CHCH_3$ | $CH_2OC_6H_5$ | |
| 7.0209 | H | $OCH_2C(Cl)=CH_2$ | $CH_2OC_6H_5$ | |
| 7.0210 | H | $OCH_2CH=CHCl$ | $CH_2OC_6H_5$ | |
| 7.0211 | H | $OCH_2C\equiv CH$ | $CH_2OC_6H_5$ | |
| 7.0212 | H | $OCHF_2$ | $CH_2OC_6H_5$ | |
| 7.0213 | H | $OCF_3$ | $CH_2OC_6H_5$ | |
| 7.0214 | H | $OCF_2CHFCF_3$ | $CH_2OC_6H_5$ | |
| 7.0215 | H | $OC_6H_5$ | $CH_2OC_6H_5$ | |
| 7.0216 | H | $CF_3$ | $CH_2OC_6H_5$ | |
| 7.0217 | H | Br | $CH_2OC_6H_5$ | |
| 7.0218 | H | Cl | $CH_2OC_6H_5$ | |
| 7.0219 | H | F | $CH_2OC_6H_5$ | |
| 7.0220 | H | $SCH_3$ | $CH_2OC_6H_5$ | |
| 7.0221 | H | $SC_2H_5$ | $CH_2OC_6H_5$ | |
| 7.0222 | H | $SC_3H_7$-n | $CH_2OC_6H_5$ | |
| 7.0223 | H | $SO_2CH_3$ | $CH_2OC_6H_5$ | |
| 7.0224 | H | $SO_2C_2H_5$ | $CH_2OC_6H_5$ | |
| 7.0225 | H | $NO_2$ | $CH_2OC_6H_5$ | |
| 7.0226 | H | CN | $CH_2OC_6H_5$ | |
| 7.0227 | H | $CH_2OH$ | $CH_2OC_6H_5$ | |
| 7.0228 | H | $CH_2CH_2OH$ | $CH_2OC_6H_5$ | |
| 7.0229 | H | $CH_2CH_2CH_2OH$ | $CH_2OC_6H_5$ | |
| 7.0230 | H | $C_6H_5$ | $CH_2OC_6H_5$ | |
| 7.0231 | H | $CH_2Cl$ | $CH_2OC_6H_5$ | |
| 7.0232 | H | $CH_2CH_2Cl$ | $CH_2OC_6H_5$ | |
| 7.0233 | H | $CH_2CH_2CH_2Cl$ | $CH_2OC_6H_5$ | |

Beispiel 16 Herstellung von 2-(N-2-methyl-valylamid)-carbamoyl-6-phenoxymethyl-nicotinsäure

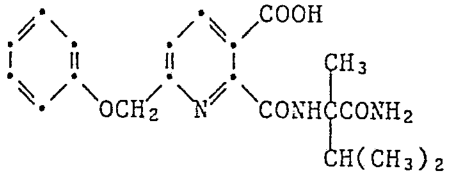

Zu einer Lösung von 2.55 g 6-Phenoxy-methyl-2,3-pyridindicarbonsäure in 30 ml Tetrahydrofuran gibt man unter Rühren 13 g 2-Methyl-valinamid und rührt während 14 Stunden bei Raumtemperatur weiter, bevor das Reaktionsgemisch eingedampft wird. Es verbleibt ein öliger Rückstand, welcher durch Kristallisation aus

Aether/Petroläther gereinigt wird. Man erhält 3,4 g weisse Kristalle vom Smp. 108-110°.

In analoger Weise zum Beispiel 16 werden die in den Tabelle 8.00-8.02 aufgeführten 2-Carbamoyl-nicotinsäurederivate der Formel VIII hergestellt.

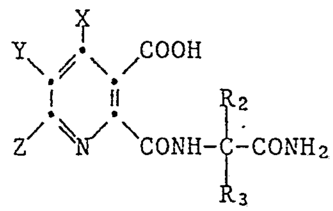

(VII)

Tabelle 8.00

| No. | X | Y | $R_2$ | $R_3$ | Z | phys. Daten |
|---|---|---|---|---|---|---|
| 8.0001 | H | H | $CH_3$ | $C_3H_7$(iso) | $C_3H_5$(Cyclo) | |
| 8.0002 | H | $CH_3$ | $CH_3$ | $C_3H_7$(iso) | $C_3H_5$(Cyclo) | |
| 8.0003 | H | $C_2H_5$ | $CH_3$ | $C_3H_7$(iso) | $C_3H_5$(Cyclo) | |
| 8.0004 | H | $C_3H_7$ | $CH_3$ | $C_3H_7$(iso) | $C_3H_5$(Cyclo) | |
| 8.0005 | H | $OCH_3$ | $CH_3$ | $C_3H_7$(iso) | $C_3H_5$(Cyclo) | |
| 8.0006 | H | $OC_2H_5$ | $CH_3$ | $C_3H_7$(iso) | $C_3H_5$(Cyclo) | |
| 8.0007 | H | $OCH_2CH{=}CH_2$ | $CH_3$ | $C_3H_7$(iso) | $C_3H_5$(Cyclo) | |
| 8.0008 | H | $OCH_2CH{=}CHCH_3$ | $CH_3$ | $C_3H_7$(iso) | $C_3H_5$(Cyclo) | |
| 8.0009 | H | $OCH_2C(Cl){=}CH_2$ | $CH_3$ | $C_3H_7$(iso) | $C_3H_5$(Cyclo) | |
| 8.0010 | H | $OCH_2CH{=}CHCl$ | $CH_3$ | $C_3H_7$(iso) | $C_3H_5$(Cyclo) | |
| 8.0011 | H | $OCH_2C{\equiv}CH$ | $CH_3$ | $C_3H_7$(iso) | $C_3H_5$(Cyclo) | |
| 8.0012 | H | $OCHF_2$ | $CH_3$ | $C_3H_7$(iso) | $C_3H_5$(Cyclo) | |
| 8.0013 | H | $OCF_3$ | $CH_3$ | $C_3H_7$(iso) | $C_3H_5$(Cyclo) | |
| 8.0014 | H | $OCF_2CHFCF_3$ | $CH_3$ | $C_3H_7$(iso) | $C_3H_5$(Cyclo) | |
| 8.0015 | H | $OC_6H_5$ | $CH_3$ | $C_3H_7$(iso) | $C_3H_5$(Cyclo) | |
| 8.0016 | H | $CF_3$ | $CH_3$ | $C_3H_7$(iso) | $C_3H_5$(Cyclo) | |
| 8.0017 | H | Br | $CH_3$ | $C_3H_7$(iso) | $C_3H_5$(Cyclo) | |
| 8.0018 | H | Cl | $CH_3$ | $C_3H_7$(iso) | $C_3H_5$(Cyclo) | |
| 8.0019 | H | F | $CH_3$ | $C_3H_7$(iso) | $C_3H_5$(Cyclo) | |
| 8.0020 | H | $SCH_3$ | $CH_3$ | $C_3H_7$(iso) | $C_3H_5$(Cyclo) | |
| 8.0021 | H | $SCH_3$ | $CH_3$ | $C_3H_7$(iso) | $C_3H_5$(Cyclo) | |
| 8.0022 | H | $SC_3H_7$-n | $CH_3$ | $C_3H_7$(iso) | $C_3H_5$(Cyclo) | |
| 8.0023 | H | $SO_2CH_3$ | $CH_3$ | $C_3H_7$(iso) | $C_3H_5$(Cyclo) | |
| 8.0024 | H | $SO_2CH_3$ | $CH_3$ | $C_3H_7$(iso) | $C_3H_5$(Cyclo) | |
| 8.0025 | H | $NO_2$ | $CH_3$ | $C_3H_7$(iso) | $C_3H_5$(Cyclo) | |
| 8.0026 | H | CN | $CH_3$ | $C_3H_7$(iso) | $C_3H_5$(Cyclo) | |
| 8.0027 | H | $CH_2OH$ | $CH_3$ | $C_3H_7$(iso) | $C_3H_5$(Cyclo) | |
| 8.0028 | H | $CH_2CH_2OH$ | $CH_3$ | $C_3H_7$(iso) | $C_3H_5$(Cyclo) | |
| 8.0029 | H | $CH_2CH_2CH_2OH$ | $CH_3$ | $C_3H_7$(iso) | $C_3H_5$(Cyclo) | |
| 8.0030 | H | $C_6H_5$ | $CH_3$ | $C_3H_7$(iso) | $C_3H_5$(Cyclo) | |
| 8.0031 | H | $CH_2Cl$ | $CH_3$ | $C_3H_7$(iso) | $C_3H_5$(Cyclo) | |
| 8.0032 | H | $CH_2CH_2Cl$ | $CH_3$ | $C_3H_7$(iso) | $C_3H_5$(Cyclo) | |
| 8.0033 | H | $CH_2CH_2CH_2Cl$ | $CH_3$ | $C_3H_7$(iso) | $C_3H_5$(Cyclo) | |

Tabelle 8.01

| No. | X | Y | $R_2$ | $R_3$ | Z | phys. Daten |
|---|---|---|---|---|---|---|
| 8.0101 | H | H | $CH_3$ | $C_3H_7$(iso) | $CH_2OC_2H_5$ | |
| 8.0102 | H | $CH_3$ | $CH_3$ | $C_3H_7$(iso) | $CH_2OC_2H_5$ | |
| 8.0103 | H | $C_2H_5$ | $CH_3$ | $C_3H_7$(iso) | $CH_2OC_2H_5$ | |
| 8.0104 | H | $C_3H_7$ | $CH_3$ | $C_3H_7$(iso) | $CH_2OC_2H_5$ | |
| 8.0105 | H | $OCH_3$ | $CH_3$ | $C_3H_7$(iso) | $CH_2OC_2H_5$ | |
| 8.0106 | H | $OC_2H_5$ | $CH_3$ | $C_3H_7$(iso) | $CH_2OC_2H_5$ | |
| 8.0107 | H | $OCH_2CH{=}CH_2$ | $CH_3$ | $C_3H_7$(iso) | $CH_2OC_2H_5$ | |
| 8.0108 | H | $OCH_2CH{=}CHCH_3$ | $CH_3$ | $C_3H_7$(iso) | $CH_2OC_2H_5$ | |
| 8.0109 | H | $OCH_2C(Cl){=}CH_2$ | $CH_3$ | $C_3H_7$(iso) | $CH_2OC_2H_5$ | |
| 8.0110 | H | $OCH_2CH{=}CHCl$ | $CH_3$ | $C_3H_7$(iso) | $CH_2OC_2H_5$ | |
| 8.0111 | H | $OCH_2C{\equiv}CH$ | $CH_3$ | $C_3H_7$(iso) | $CH_2OC_2H_5$ | |
| 8.0112 | H | $OCHF_2$ | $CH_3$ | $C_3H_7$(iso) | $CH_2OC_2H_5$ | |
| 8.0113 | H | $OCF_3$ | $CH_3$ | $C_3H_7$(iso) | $CH_2OC_2H_5$ | |
| 8.0114 | H | $OCF_2CHFCF_3$ | $CH_3$ | $C_3H_7$(iso) | $CH_2OC_2H_5$ | |
| 8.0115 | H | $OC_6H_5$ | $CH_3$ | $C_3H_7$(iso) | $CH_2OC_2H_5$ | |
| 8.0116 | H | $CF_3$ | $CH_3$ | $C_3H_7$(iso) | $CH_2OC_2H_5$ | |
| 8.0117 | H | Br | $CH_3$ | $C_3H_7$(iso) | $CH_2OC_2H_5$ | |
| 8.0118 | H | Cl | $CH_3$ | $C_3H_7$(iso) | $CH_2OC_2H_5$ | |
| 8.0119 | H | F | $CH_3$ | $C_3H_7$(iso) | $CH_2OC_2H_5$ | |
| 8.0120 | H | $SCH_3$ | $CH_3$ | $C_3H_7$(iso) | $CH_2OC_2H_5$ | |
| 8.0121 | H | $SCH_3$ | $CH_3$ | $C_3H_7$(iso) | $CH_2OC_2H_5$ | |
| 8.0122 | H | $SC_3H_7$-n | $CH_3$ | $C_3H_7$(iso) | $CH_2OC_2H_5$ | |
| 8.0123 | H | $SO_2CH_3$ | $CH_3$ | $C_3H_7$(iso) | $CH_2OC_2H_5$ | |
| 8.0124 | H | $SO_2CH_3$ | $CH_3$ | $C_3H_7$(iso) | $CH_2OC_2H_5$ | |
| 8.0125 | H | $NO_2$ | $CH_3$ | $C_3H_7$(iso) | $CH_2OC_2H_5$ | |
| 8.0126 | H | CN | $CH_3$ | $C_3H_7$(iso) | $CH_2OC_2H_5$ | |
| 8.0127 | H | $CH_2OH$ | $CH_3$ | $C_3H_7$(iso) | $CH_2OC_2H_5$ | |
| 8.0128 | H | $CH_2CH_2OH$ | $CH_3$ | $C_3H_7$(iso) | $CH_2OC_2H_5$ | |
| 8.0129 | H | $CH_2CH_2CH_2OH$ | $CH_3$ | $C_3H_7$(iso) | $CH_2OC_2H_5$ | |
| 8.0130 | H | $C_6H_5$ | $CH_3$ | $C_3H_7$(iso) | $CH_2OC_2H_5$ | |
| 8.0131 | H | $CH_2Cl$ | $CH_3$ | $C_3H_7$(iso) | $CH_2OC_2H_5$ | |
| 8.0132 | H | $CH_2CH_2Cl$ | $CH_3$ | $C_3H_7$(iso) | $CH_2OC_2H_5$ | |
| 8.0133 | H | $CH_2CH_2CH_2Cl$ | $CH_3$ | $C_3H_7$(iso) | $CH_2OC_2H_5$ | |

Tabelle 8.02

| No. | X | Y | $R_2$ | $R_3$ | Z | phys. Daten |
|---|---|---|---|---|---|---|
| 8.0201 | H | H | $CH_3$ | $C_3H_7$(iso) | $CH_2OC_6H_5$ | Smp. 108-110° |
| 8.0202 | H | $CH_3$ | $CH_3$ | $C_3H_7$(iso) | $CH_2OC_6H_5$ | |
| 8.0203 | H | $C_2H_5$ | $CH_3$ | $C_3H_7$(iso) | $CH_2OC_6H_5$ | |
| 8.0204 | H | $C_3H_7$ | $CH_3$ | $C_3H_7$(iso) | $CH_2OC_6H_5$ | |
| 8.0205 | H | $OCH_3$ | $CH_3$ | $C_3H_7$(iso) | $CH_2OC_6H_5$ | |
| 8.0206 | H | $OC_2H_5$ | $CH_3$ | $C_3H_7$(iso) | $CH_2OC_6H_5$ | |
| 8.0207 | H | $OCH_2CH{=}CH_2$ | $CH_3$ | $C_3H_7$(iso) | $CH_2OC_6H_5$ | |
| 8.0208 | H | $OCH_2CH{=}CHCH_3$ | $CH_3$ | $C_3H_7$(iso) | $CH_2OC_6H_5$ | |
| 8.0209 | H | $OCH_2C(Cl){=}CH_2$ | $CH_3$ | $C_3H_7$(iso) | $CH_2OC_6H_5$ | |
| 8.0210 | H | $OCH_2CH{=}CHCl$ | $CH_3$ | $C_3H_7$(iso) | $CH_2OC_6H_5$ | |
| 8.0211 | H | $OCH_2C{\equiv}CH$ | $CH_3$ | $C_3H_7$(iso) | $CH_2OC_6H_5$ | |
| 8.0212 | H | $OCHF_2$ | $CH_3$ | $C_3H_7$(iso) | $CH_2OC_6H_5$ | |
| 8.0213 | H | $OCF_3$ | $CH_3$ | $C_3H_7$(iso) | $CH_2OC_6H_5$ | |
| 8.0214 | H | $OCF_2CHFCF_3$ | $CH_3$ | $C_3H_7$(iso) | $CH_2OC_6H_5$ | |
| 8.0215 | H | $OC_6H_5$ | $CH_3$ | $C_3H_7$(iso) | $CH_2OC_6H_5$ | |
| 8.0216 | H | $CF_3$ | $CH_3$ | $C_3H_7$(iso) | $CH_2OC_6H_5$ | |
| 8.0217 | H | Br | $CH_3$ | $C_3H_7$(iso) | $CH_2OC_6H_5$ | |
| 8.0218 | H | Cl | $CH_3$ | $C_3H_7$(iso) | $CH_2OC_6H_5$ | |
| 8.0219 | H | F | $CH_3$ | $C_3H_7$(iso) | $CH_2OC_6H_5$ | |
| 8.0220 | H | $SCH_3$ | $CH_3$ | $C_3H_7$(iso) | $CH_2OC_6H_5$ | |
| 8.0221 | H | $SCH_3$ | $CH_3$ | $C_3H_7$(iso) | $CH_2OC_6H_5$ | |
| 8.0222 | H | $SC_3H_7$-n | $CH_3$ | $C_3H_7$(iso) | $CH_2OC_6H_5$ | |
| 8.0223 | H | $SO_2CH_3$ | $CH_3$ | $C_3H_7$(iso) | $CH_2OC_6H_5$ | |
| 8.0224 | H | $SO_2CH_3$ | $CH_3$ | $C_3H_7$(iso) | $CH_2OC_6H_5$ | |
| 8.0225 | H | $NO_2$ | $CH_3$ | $C_3H_7$(iso) | $CH_2OC_6H_5$ | |
| 8.0226 | H | CN | $CH_3$ | $C_3H_7$(iso) | $CH_2OC_6H_5$ | |
| 8.0227 | H | $CH_2OH$ | $CH_3$ | $C_3H_7$(iso) | $CH_2OC_6H_5$ | |
| 8.0228 | H | $CH_2CH_2OH$ | $CH_3$ | $C_3H_7$(iso) | $CH_2OC_6H_5$ | |
| 8.0229 | H | $CH_2CH_2CH_2OH$ | $CH_3$ | $C_3H_7$(iso) | $CH_2OC_6H_5$ | |
| 8.0230 | H | $C_6H_5$ | $CH_3$ | $C_3H_7$(iso) | $CH_2OC_6H_5$ | |
| 8.0231 | H | $CH_2Cl$ | $CH_3$ | $C_3H_7$(iso) | $CH_2OC_6H_5$ | |
| 8.0232 | H | $CH_2CH_2Cl$ | $CH_3$ | $C_3H_7$(iso) | $CH_2OC_6H_5$ | |
| 8.0233 | H | $CH_2CH_2CH_2Cl$ | $CH_3$ | $C_3H_7$(iso) | $CH_2OC_6H_5$ | |

Beispiel 17 Herstellung von 2-(N-valylnitril)carbamoyl-6-phenoxymethyl-nicotinsäure

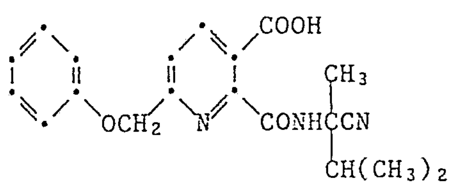

Zu einer Lösung von 2,55 g 6-Phenoxymethyl-2,3-pyridindicarbonsäureanhydrid in 3,0 ml Tetrahydrofuran gibt man unter Rühren 1,2 g 2-Methylvalylnitril (2-Amino-2-isopropyl-2-methyl-acetonitril) und rührt eine Stunde bei Raumtemperatur weiter, bevor man das Reaktionsgemisch am Vakuum eindampft. Der Rückstand wird in wenig Aether verrieben und kristallisiert. Man erhält so 3,2 der obigen Nicotinsäure. Schmelzpunkt 82-87°C.

In Analogie zum Beispiel 18 werden die in den Tabellen 9.00-9.02 aufgeführten Nicotinsäurederivat der Formel IX hergestellt.

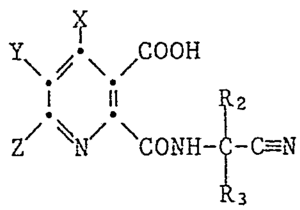

(IX)

Tabelle 9.00

| No. | X | Y | $R_2$ | $R_3$ | Z | phys. Daten |
|---|---|---|---|---|---|---|
| 9.0001 | H | H | $CH_3$ | $C_3H_7$(iso) | $C_3H_5$(Cyclo) | |
| 9.0002 | H | $CH_3$ | $CH_3$ | $C_3H_7$(iso) | $C_3H_5$(Cyclo) | |
| 9.0003 | H | $C_2H_5$ | $CH_3$ | $C_3H_7$(iso) | $C_3H_5$(Cyclo) | |
| 9.0004 | H | $C_3H_7$ | $CH_3$ | $C_3H_7$(iso) | $C_3H_5$(Cyclo) | |
| 9.0005 | H | $OCH_3$ | $CH_3$ | $C_3H_7$(iso) | $C_3H_5$(Cyclo) | |
| 9.0006 | H | $OC_2H_5$ | $CH_3$ | $C_3H_7$(iso) | $C_3H_5$(Cyclo) | |
| 9.0007 | H | $OCH_2CH=CH_2$ | $CH_3$ | $C_3H_7$(iso) | $C_3H_5$(Cyclo) | |
| 9.0008 | H | $OCH_2CH=CHCH_3$ | $CH_3$ | $C_3H_7$(iso) | $C_3H_5$(Cyclo) | |
| 9.0009 | H | $OCH_2C(Cl)=CH_2$ | $CH_3$ | $C_3H_7$(iso) | $C_3H_5$(Cyclo) | |
| 9.0010 | H | $OCH_2CH=CHCl$ | $CH_3$ | $C_3H_7$(iso) | $C_3H_5$(Cyclo) | |
| 9.0011 | H | $OCH_2C{\equiv}CH$ | $CH_3$ | $C_3H_7$(iso) | $C_3H_5$(Cyclo) | |
| 9.0012 | H | $OCHF_2$ | $CH_3$ | $C_3H_7$(iso) | $C_3H_5$(Cyclo) | |
| 9.0013 | H | $OCF_3$ | $CH_3$ | $C_3H_7$(iso) | $C_3H_5$(Cyclo) | |
| 9.0014 | H | $OCF_2CHFCF_3$ | $CH_3$ | $C_3H_7$(iso) | $C_3H_5$(Cyclo) | |
| 9.0015 | H | $OC_6H_5$ | $CH_3$ | $C_3H_7$(iso) | $C_3H_5$(Cyclo) | |
| 9.0016 | H | $CF_3$ | $CH_3$ | $C_3H_7$(iso) | $C_3H_5$(Cyclo) | |
| 9.0017 | H | Br | $CH_3$ | $C_3H_7$(iso) | $C_3H_5$(Cyclo) | |
| 9.0018 | H | Cl | $CH_3$ | $C_3H_7$(iso) | $C_3H_5$(Cyclo) | |
| 9.0019 | H | F | $CH_3$ | $C_3H_7$(iso) | $C_3H_5$(Cyclo) | |
| 9.0020 | H | $SCH_3$ | $CH_3$ | $C_3H_7$(iso) | $C_3H_5$(Cyclo) | |
| 9.0021 | H | $SCH_3$ | $CH_3$ | $C_3H_7$(iso) | $C_3H_5$(Cyclo) | |
| 9.0022 | H | $SC_3H_7$-n | $CH_3$ | $C_3H_7$(iso) | $C_3H_5$(Cyclo) | |
| 9.0023 | H | $SO_2CH_3$ | $CH_3$ | $C_3H_7$(iso) | $C_3H_5$(Cyclo) | |
| 9.0024 | H | $SO_2CH_3$ | $CH_3$ | $C_3H_7$(iso) | $C_3H_5$(Cyclo) | |
| 9.0025 | H | $NO_2$ | $CH_3$ | $C_3H_7$(iso) | $C_3H_5$(Cyclo) | |
| 9.0026 | H | CN | $CH_3$ | $C_3H_7$(iso) | $C_3H_5$(Cyclo) | |
| 9.0027 | H | $CH_2OH$ | $CH_3$ | $C_3H_7$(iso) | $C_3H_5$(Cyclo) | |
| 9.0028 | H | $CH_2CH_2OH$ | $CH_3$ | $C_3H_7$(iso) | $C_3H_5$(Cyclo) | |
| 9.0029 | H | $CH_2CH_2CH_2OH$ | $CH_3$ | $C_3H_7$(iso) | $C_3H_5$(Cyclo) | |
| 9.0030 | H | $C_6H_5$ | $CH_3$ | $C_3H_7$(iso) | $C_3H_5$(Cyclo) | |
| 9.0031 | H | $CH_2Cl$ | $CH_3$ | $C_3H_7$(iso) | $C_3H_5$(Cyclo) | |
| 9.0032 | H | $CH_2CH_2Cl$ | $CH_3$ | $C_3H_7$(iso) | $C_3H_5$(Cyclo) | |
| 9.0033 | H | $CH_2CH_2CH_2Cl$ | $CH_3$ | $C_3H_7$(iso) | $C_3H_5$(Cyclo) | |

Tabelle 9.01

| No. | X | Y | $R_2$ | $R_3$ | Z | phys. Daten |
|---|---|---|---|---|---|---|
| 9.0101 | H | H | $CH_3$ | $C_3H_7$(iso) | $CH_2OC_2H_5$ | |
| 9.0102 | H | $CH_3$ | $CH_3$ | $C_3H_7$(iso) | $CH_2OC_2H_5$ | |
| 9.0103 | H | $C_2H_5$ | $CH_3$ | $C_3H_7$(iso) | $CH_2OC_2H_5$ | |
| 9.0104 | H | $C_3H_7$ | $CH_3$ | $C_3H_7$(iso) | $CH_2OC_2H_5$ | |
| 9.0105 | H | $OCH_3$ | $CH_3$ | $C_3H_7$(iso) | $CH_2OC_2H_5$ | |
| 9.0106 | H | $OC_2H_5$ | $CH_3$ | $C_3H_7$(iso) | $CH_2OC_2H_5$ | |
| 9.0107 | H | $OCH_2CH=CH_2$ | $CH_3$ | $C_3H_7$(iso) | $CH_2OC_2H_5$ | |
| 9.0108 | H | $OCH_2CH=CHCH_3$ | $CH_3$ | $C_3H_7$(iso) | $CH_2OC_2H_5$ | |
| 9.0109 | H | $OCH_2C(Cl)=CH_2$ | $CH_3$ | $C_3H_7$(iso) | $CH_2OC_2H_5$ | |
| 9.0110 | H | $OCH_2CH=CHCl$ | $CH_3$ | $C_3H_7$(iso) | $CH_2OC_2H_5$ | |
| 9.0111 | H | $OCH_2C{\equiv}CH$ | $CH_3$ | $C_3H_7$(iso) | $CH_2OC_2H_5$ | |
| 9.0112 | H | $OCHF_2$ | $CH_3$ | $C_3H_7$(iso) | $CH_2OC_2H_5$ | |
| 9.0113 | H | $OCF_3$ | $CH_3$ | $C_3H_7$(iso) | $CH_2OC_2H_5$ | |
| 9.0114 | H | $OCF_2CHFCF_3$ | $CH_3$ | $C_3H_7$(iso) | $CH_2OC_2H_5$ | |
| 9.0115 | H | $OC_6H_5$ | $CH_3$ | $C_3H_7$(iso) | $CH_2OC_2H_5$ | |
| 9.0116 | H | $CF_3$ | $CH_3$ | $C_3H_7$(iso) | $CH_2OC_2H_5$ | |
| 9.0117 | H | Br | $CH_3$ | $C_3H_7$(iso) | $CH_2OC_2H_5$ | |
| 9.0118 | H | Cl | $CH_3$ | $C_3H_7$(iso) | $CH_2OC_2H_5$ | |
| 9.0119 | H | F | $CH_3$ | $C_3H_7$(iso) | $CH_2OC_2H_5$ | |
| 9.0120 | H | $SCH_3$ | $CH_3$ | $C_3H_7$(iso) | $CH_2OC_2H_5$ | |
| 9.0121 | H | $SCH_3$ | $CH_3$ | $C_3H_7$(iso) | $CH_2OC_2H_5$ | |
| 9.0122 | H | $SC_3H_7$-n | $CH_3$ | $C_3H_7$(iso) | $CH_2OC_2H_5$ | |
| 9.0123 | H | $SO_2CH_3$ | $CH_3$ | $C_3H_7$(iso) | $CH_2OC_2H_5$ | |
| 9.0124 | H | $SO_2CH_3$ | $CH_3$ | $C_3H_7$(iso) | $CH_2OC_2H_5$ | |
| 9.0125 | H | $NO_2$ | $CH_3$ | $C_3H_7$(iso) | $CH_2OC_2H_5$ | |
| 9.0126 | H | CN | $CH_3$ | $C_3H_7$(iso) | $CH_2OC_2H_5$ | |
| 9.0127 | H | $CH_2OH$ | $CH_3$ | $C_3H_7$(iso) | $CH_2OC_2H_5$ | |
| 9.0128 | H | $CH_2CH_2OH$ | $CH_3$ | $C_3H_7$(iso) | $CH_2OC_2H_5$ | |
| 9.0129 | H | $CH_2CH_2CH_2OH$ | $CH_3$ | $C_3H_7$(iso) | $CH_2OC_2H_5$ | |
| 9.0130 | H | $C_6H_5$ | $CH_3$ | $C_3H_7$(iso) | $CH_2OC_2H_5$ | |
| 9.0131 | H | $CH_2Cl$ | $CH_3$ | $C_3H_7$(iso) | $CH_2OC_2H_5$ | |
| 9.0132 | H | $CH_2CH_2Cl$ | $CH_3$ | $C_3H_7$(iso) | $CH_2OC_2H_5$ | |
| 9.0133 | H | $CH_2CH_2CH_2Cl$ | $CH_3$ | $C_3H_7$(iso) | $CH_2OC_2H_5$ | |

Tabelle 9.02

| No. | X | Y | $R_2$ | $R_3$ | Z | phys. Daten |
|---|---|---|---|---|---|---|
| 9.0201 | H | H | $CH_3$ | $C_3H_7$(iso) | $CH_2OC_6H_5$ | Smp. 82-87° |
| 9.0202 | H | $CH_3$ | $CH_3$ | $C_3H_7$(iso) | $CH_2OC_6H_5$ | |
| 9.0203 | H | $C_2H_5$ | $CH_3$ | $C_3H_7$(iso) | $CH_2OC_6H_5$ | |
| 9.0204 | H | $C_3H_7$ | $CH_3$ | $C_3H_7$(iso) | $CH_2OC_6H_5$ | |
| 9.0205 | H | $OCH_3$ | $CH_3$ | $C_3H_7$(iso) | $CH_2OC_6H_5$ | |
| 9.0206 | H | $OC_2H_5$ | $CH_3$ | $C_3H_7$(iso) | $CH_2OC_6H_5$ | |
| 9.0207 | H | $OCH_2CH=CH_2$ | $CH_3$ | $C_3H_7$(iso) | $CH_2OC_6H_5$ | |
| 9.0208 | H | $OCH_2CH=CHCH_3$ | $CH_3$ | $C_3H_7$(iso) | $CH_2OC_6H_5$ | |
| 9.0209 | H | $OCH_2C(Cl)=CH_2$ | $CH_3$ | $C_3H_7$(iso) | $CH_2OC_6H_5$ | |
| 9.0210 | H | $OCH_2CH=CHCl$ | $CH_3$ | $C_3H_7$(iso) | $CH_2OC_6H_5$ | |
| 9.0211 | H | $OCH_2C\equiv CH$ | $CH_3$ | $C_3H_7$(iso) | $CH_2OC_6H_5$ | |
| 9.0212 | H | $OCHF_2$ | $CH_3$ | $C_3H_7$(iso) | $CH_2OC_6H_5$ | |
| 9.0213 | H | $OCF_3$ | $CH_3$ | $C_3H_7$(iso) | $CH_2OC_6H_5$ | |
| 9.0214 | H | $OCF_2CHFCF_3$ | $CH_3$ | $C_3H_7$(iso) | $CH_2OC_6H_5$ | |
| 9.0215 | H | $OC_6H_5$ | $CH_3$ | $C_3H_7$(iso) | $CH_2OC_6H_5$ | |
| 9.0216 | H | $CF_3$ | $CH_3$ | $C_3H_7$(iso) | $CH_2OC_6H_5$ | |
| 9.0217 | H | Br | $CH_3$ | $C_3H_7$(iso) | $CH_2OC_6H_5$ | |
| 9.0218 | H | Cl | $CH_3$ | $C_3H_7$(iso) | $CH_2OC_6H_5$ | |
| 9.0219 | H | F | $CH_3$ | $C_3H_7$(iso) | $CH_2OC_6H_5$ | |
| 9.0220 | H | $SCH_3$ | $CH_3$ | $C_3H_7$(iso) | $CH_2OC_6H_5$ | |
| 9.0221 | H | $SCH_3$ | $CH_3$ | $C_3H_7$(iso) | $CH_2OC_6H_5$ | |
| 9.0222 | H | $SC_3H_7$-n | $CH_3$ | $C_3H_7$(iso) | $CH_2OC_6H_5$ | |
| 9.0223 | H | $SO_2CH_3$ | $CH_3$ | $C_3H_7$(iso) | $CH_2OC_6H_5$ | |
| 9.0224 | H | $SO_2CH_3$ | $CH_3$ | $C_3H_7$(iso) | $CH_2OC_6H_5$ | |
| 9.0225 | H | $NO_2$ | $CH_3$ | $C_3H_7$(iso) | $CH_2OC_6H_5$ | |
| 9.0226 | H | CN | $CH_3$ | $C_3H_7$(iso) | $CH_2OC_6H_5$ | |
| 9.0227 | H | $CH_2OH$ | $CH_3$ | $C_3H_7$(iso) | $CH_2OC_6H_5$ | |
| 9.0228 | H | $CH_2CH_2OH$ | $CH_3$ | $C_3H_7$(iso) | $CH_2OC_6H_5$ | |
| 9.0229 | H | $CH_2CH_2CH_2OH$ | $CH_3$ | $C_3H_7$(iso) | $CH_2OC_6H_5$ | |
| 9.0230 | H | $C_6H_5$ | $CH_3$ | $C_3H_7$(iso) | $CH_2OC_6H_5$ | |
| 9.0231 | H | $CH_2Cl$ | $CH_3$ | $C_3H_7$(iso) | $CH_2OC_6H_5$ | |
| 9.0232 | H | $CH_2CH_2Cl$ | $CH_3$ | $C_3H_7$(iso) | $CH_2OC_6H_5$ | |
| 9.0233 | H | $CH_2CH_2CH_2Cl$ | $CH_3$ | $C_3H_7$(iso) | $CH_2OC_6H_5$ | |

Beispiel 18 Herstellung von N-(2-Isopropyl-2-methyl-glycylnitril)-6-phenoxymethyl-2,3-pyridindincarbonsäureimid

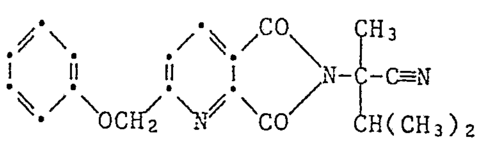

Zu einer Lösung von einem Teil 2-(N-valylnitril)carbamoyl-6-phenoxymethylnicotinsäure (Beispiel 17) in 4 Teilen Essigsäureanhydrid gibt man 0,02 Teile Natriumacetat und rührt das Gemisch unter Rückfluss während 6 Stunden bie 110°. Dann wird alles eingedampft, der Rückstand in etwas Toluol aufgenommen und nochmals eingedampft.

Der Rückstand wird in Aether/Hexan verrieben, die entstandenen kristalline Masse abgenutscht, dann in

Methylenchlorid gelöst und an Kieselgel filtriert. Nach Verdampfen des Methylenchlorides verbleiben 0.85 Teile des obigen Imides.

In analoger Weise zum Beispiel 18 werden die in den Tabellen 10.00-10.02 aufgeführten 2,3-Pyridindicarbonsäureimide der Formel X hergestellt.

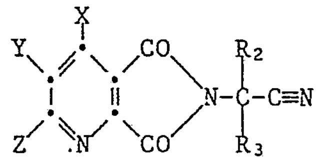

(X)

Tabelle 10.00

| No. | X | Y | $R_2$ | $R_3$ | Z | phys. Daten |
|---|---|---|---|---|---|---|
| 10.0001 | H | H | $CH_3$ | $C_3H_7$(iso) | $C_3H_5$(Cyclo) | |
| 10.0002 | H | $CH_3$ | $CH_3$ | $C_3H_7$(iso) | $C_3H_5$(Cyclo) | |
| 10.0003 | H | $C_2H_5$ | $CH_3$ | $C_3H_7$(iso) | $C_3H_5$(Cyclo) | |
| 10.0004 | H | $C_3H_7$ | $CH_3$ | $C_3H_7$(iso) | $C_3H_5$(Cyclo) | |
| 10.0005 | H | $OCH_3$ | $CH_3$ | $C_3H_7$(iso) | $C_3H_5$(Cyclo) | |
| 10.0006 | H | $OC_2H_5$ | $CH_3$ | $C_3H_7$(iso) | $C_3H_5$(Cyclo) | |
| 10.0007 | H | $OCH_2CH=CH_2$ | $CH_3$ | $C_3H_7$(iso) | $C_3H_5$(Cyclo) | |
| 10.0008 | H | $OCH_2CH=CHCH_3$ | $CH_3$ | $C_3H_7$(iso) | $C_3H_5$(Cyclo) | |
| 10.0009 | H | $OCH_2C(Cl)=CH_2$ | $CH_3$ | $C_3H_7$(iso) | $C_3H_5$(Cyclo) | |
| 10.0010 | H | $OCH_2CH=CHCl$ | $CH_3$ | $C_3H_7$(iso) | $C_3H_5$(Cyclo) | |
| 10.0011 | H | $OCH_2C{\equiv}CH$ | $CH_3$ | $C_3H_7$(iso) | $C_3H_5$(Cyclo) | |
| 10.0012 | H | $OCHF_2$ | $CH_3$ | $C_3H_7$(iso) | $C_3H_5$(Cyclo) | |
| 10.0013 | H | $OCF_3$ | $CH_3$ | $C_3H_7$(iso) | $C_3H_5$(Cyclo) | |
| 10.0014 | H | $OCF_2CHFCF_3$ | $CH_3$ | $C_3H_7$(iso) | $C_3H_5$(Cyclo) | |
| 10.0015 | H | $OC_6H_5$ | $CH_3$ | $C_3H_7$(iso) | $C_3H_5$(Cyclo) | |
| 10.0016 | H | $CF_3$ | $CH_3$ | $C_3H_7$(iso) | $C_3H_5$(Cyclo) | |
| 10.0017 | H | Br | $CH_3$ | $C_3H_7$(iso) | $C_3H_5$(Cyclo) | |
| 10.0018 | H | Cl | $CH_3$ | $C_3H_7$(iso) | $C_3H_5$(Cyclo) | |
| 10.0019 | H | F | $CH_3$ | $C_3H_7$(iso) | $C_3H_5$(Cyclo) | |
| 10.0020 | H | $SCH_3$ | $CH_3$ | $C_3H_7$(iso) | $C_3H_5$(Cyclo) | |
| 10.0021 | H | $SCH_3$ | $CH_3$ | $C_3H_7$(iso) | $C_3H_5$(Cyclo) | |
| 10.0022 | H | $SC_3H_7$-n | $CH_3$ | $C_3H_7$(iso) | $C_3H_5$(Cyclo) | |
| 10.0023 | H | $SO_2CH_3$ | $CH_3$ | $C_3H_7$(iso) | $C_3H_5$(Cyclo) | |
| 10.0024 | H | $SO_2CH_3$ | $CH_3$ | $C_3H_7$(iso) | $C_3H_5$(Cyclo) | |
| 10.0025 | H | $NO_2$ | $CH_3$ | $C_3H_7$(iso) | $C_3H_5$(Cyclo) | |
| 10.0026 | H | CN | $CH_3$ | $C_3H_7$(iso) | $C_3H_5$(Cyclo) | |
| 10.0027 | H | $CH_2OH$ | $CH_3$ | $C_3H_7$(iso) | $C_3H_5$(Cyclo) | |
| 10.0028 | H | $CH_2CH_2OH$ | $CH_3$ | $C_3H_7$(iso) | $C_3H_5$(Cyclo) | |
| 10.0029 | H | $CH_2CH_2CH_2OH$ | $CH_3$ | $C_3H_7$(iso) | $C_3H_5$(Cyclo) | |
| 10.0030 | H | $C_6H_5$ | $CH_3$ | $C_3H_7$(iso) | $C_3H_5$(Cyclo) | |
| 10.0031 | H | $CH_2Cl$ | $CH_3$ | $C_3H_7$(iso) | $C_3H_5$(Cyclo) | |
| 10.0032 | H | $CH_2CH_2Cl$ | $CH_3$ | $C_3H_7$(iso) | $C_3H_5$(Cyclo) | |
| 10.0033 | H | $CH_2CH_2CH_2Cl$ | $CH_3$ | $C_3H_7$(iso) | $C_3H_5$(Cyclo) | |

Tabelle 10.01

| No. | X | Y | $R_2$ | $R_3$ | Z | phys. Daten |
|---|---|---|---|---|---|---|
| 10.0101 | H | H | $CH_3$ | $C_3H_7$(iso) | $CH_2OC_2H_5$ | |
| 10.0102 | H | $CH_3$ | $CH_3$ | $C_3H_7$(iso) | $CH_2OC_2H_5$ | |
| 10.0103 | H | $C_2H_5$ | $CH_3$ | $C_3H_7$(iso) | $CH_2OC_2H_5$ | |
| 10.0104 | H | $C_3H_7$ | $CH_3$ | $C_3H_7$(iso) | $CH_2OC_2H_5$ | |
| 10.0105 | H | $OCH_3$ | $CH_3$ | $C_3H_7$(iso) | $CH_2OC_2H_5$ | |
| 10.0106 | H | $OC_2H_5$ | $CH_3$ | $C_3H_7$(iso) | $CH_2OC_2H_5$ | |
| 10.0107 | H | $OCH_2CH{=}CH_2$ | $CH_3$ | $C_3H_7$(iso) | $CH_2OC_2H_5$ | |
| 10.0108 | H | $OCH_2CH{=}CHCH_3$ | $CH_3$ | $C_3H_7$(iso) | $CH_2OC_2H_5$ | |
| 10.0109 | H | $OCH_2C(Cl){=}CH_2$ | $CH_3$ | $C_3H_7$(iso) | $CH_2OC_2H_5$ | |
| 10.0110 | H | $OCH_2CH{=}CHCl$ | $CH_3$ | $C_3H_7$(iso) | $CH_2OC_2H_5$ | |
| 10.0111 | H | $OCH_2C{\equiv}CH$ | $CH_3$ | $C_3H_7$(iso) | $CH_2OC_2H_5$ | |
| 10.0112 | H | $OCHF_2$ | $CH_3$ | $C_3H_7$(iso) | $CH_2OC_2H_5$ | |
| 10.0113 | H | $OCF_3$ | $CH_3$ | $C_3H_7$(iso) | $CH_2OC_2H_5$ | |
| 10.0114 | H | $OCF_2CHFCF_3$ | $CH_3$ | $C_3H_7$(iso) | $CH_2OC_2H_5$ | |
| 10.0115 | H | $OC_6H_5$ | $CH_3$ | $C_3H_7$(iso) | $CH_2OC_2H_5$ | |
| 10.0116 | H | $CF_3$ | $CH_3$ | $C_3H_7$(iso) | $CH_2OC_2H_5$ | |
| 10.0117 | H | Br | $CH_3$ | $C_3H_7$(iso) | $CH_2OC_2H_5$ | |
| 10.0118 | H | Cl | $CH_3$ | $C_3H_7$(iso) | $CH_2OC_2H_5$ | |
| 10.0119 | H | F | $CH_3$ | $C_3H_7$(iso) | $CH_2OC_2H_5$ | |
| 10.0120 | H | $SCH_3$ | $CH_3$ | $C_3H_7$(iso) | $CH_2OC_2H_5$ | |
| 10.0121 | H | $SCH_3$ | $CH_3$ | $C_3H_7$(iso) | $CH_2OC_2H_5$ | |
| 10.0122 | H | $SC_3H_7$-n | $CH_3$ | $C_3H_7$(iso) | $CH_2OC_2H_5$ | |
| 10.0123 | H | $SO_2CH_3$ | $CH_3$ | $C_3H_7$(iso) | $CH_2OC_2H_5$ | |
| 10.0124 | H | $SO_2CH_3$ | $CH_3$ | $C_3H_7$(iso) | $CH_2OC_2H_5$ | |
| 10.0125 | H | $NO_2$ | $CH_3$ | $C_3H_7$(iso) | $CH_2OC_2H_5$ | |
| 10.0126 | H | CN | $CH_3$ | $C_3H_7$(iso) | $CH_2OC_2H_5$ | |
| 10.0127 | H | $CH_2OH$ | $CH_3$ | $C_3H_7$(iso) | $CH_2OC_2H_5$ | |
| 10.0128 | H | $CH_2CH_2OH$ | $CH_3$ | $C_3H_7$(iso) | $CH_2OC_2H_5$ | |
| 10.0129 | H | $CH_2CH_2CH_2OH$ | $CH_3$ | $C_3H_7$(iso) | $CH_2OC_2H_5$ | |
| 10.0130 | H | $C_6H_5$ | $CH_3$ | $C_3H_7$(iso) | $CH_2OC_2H_5$ | |
| 10.0131 | H | $CH_2Cl$ | $CH_3$ | $C_3H_7$(iso) | $CH_2OC_2H_5$ | |
| 10.0132 | H | $CH_2CH_2Cl$ | $CH_3$ | $C_3H_7$(iso) | $CH_2OC_2H_5$ | |
| 10.0133 | H | $CH_2CH_2CH_2Cl$ | $CH_3$ | $C_3H_7$(iso) | $CH_2OC_2H_5$ | |

Tabelle 10.02

| No. | X | Y | $R_2$ | $R_3$ | Z | phys. Daten |
|---|---|---|---|---|---|---|
| 10.0201 | H | H | $CH_3$ | $C_3H_7$(iso) | $CH_2OC_6H_5$ | |
| 10.0202 | H | $CH_3$ | $CH_3$ | $C_3H_7$(iso) | $CH_2OC_6H_5$ | |
| 10.0203 | H | $C_2H_5$ | $CH_3$ | $C_3H_7$(iso) | $CH_2OC_6H_5$ | |
| 10.0204 | H | $C_3H_7$ | $CH_3$ | $C_3H_7$(iso) | $CH_2OC_6H_5$ | |
| 10.0205 | H | $OCH_3$ | $CH_3$ | $C_3H_7$(iso) | $CH_2OC_6H_5$ | |
| 10.0206 | H | $OC_2H_5$ | $CH_3$ | $C_3H_7$(iso) | $CH_2OC_6H_5$ | |
| 10.0207 | H | $OCH_2CH{=}CH_2$ | $CH_3$ | $C_3H_7$(iso) | $CH_2OC_6H_5$ | |
| 10.0208 | H | $OCH_2CH{=}CHCH_3$ | $CH_3$ | $C_3H_7$(iso) | $CH_2OC_6H_5$ | |
| 10.0209 | H | $OCH_2C(Cl){=}CH_2$ | $CH_3$ | $C_3H_7$(iso) | $CH_2OC_6H_5$ | |
| 10.0210 | H | $OCH_2CH{=}CHCl$ | $CH_3$ | $C_3H_7$(iso) | $CH_2OC_6H_5$ | |
| 10.0211 | H | $OCH_2C{\equiv}CH$ | $CH_3$ | $C_3H_7$(iso) | $CH_2OC_6H_5$ | |
| 10.0212 | H | $OCHF_2$ | $CH_3$ | $C_3H_7$(iso) | $CH_2OC_6H_5$ | |
| 10.0213 | H | $OCF_3$ | $CH_3$ | $C_3H_7$(iso) | $CH_2OC_6H_5$ | |
| 10.0214 | H | $OCF_2CHFCF_3$ | $CH_3$ | $C_3H_7$(iso) | $CH_2OC_6H_5$ | |
| 10.0215 | H | $OC_6H_5$ | $CH_3$ | $C_3H_7$(iso) | $CH_2OC_6H_5$ | |
| 10.0216 | H | $CF_3$ | $CH_3$ | $C_3H_7$(iso) | $CH_2OC_6H_5$ | |
| 10.0217 | H | Br | $CH_3$ | $C_3H_7$(iso) | $CH_2OC_6H_5$ | |
| 10.0218 | H | Cl | $CH_3$ | $C_3H_7$(iso) | $CH_2OC_6H_5$ | |
| 10.0219 | H | F | $CH_3$ | $C_3H_7$(iso) | $CH_2OC_6H_5$ | |
| 10.0220 | H | $SCH_3$ | $CH_3$ | $C_3H_7$(iso) | $CH_2OC_6H_5$ | |
| 10.0221 | H | $SCH_3$ | $CH_3$ | $C_3H_7$(iso) | $CH_2OC_6H_5$ | |
| 10.0222 | H | $SC_3H_7$-n | $CH_3$ | $C_3H_7$(iso) | $CH_2OC_6H_5$ | |
| 10.0223 | H | $SO_2CH_3$ | $CH_3$ | $C_3H_7$(iso) | $CH_2OC_6H_5$ | |
| 10.0224 | H | $SO_2CH_3$ | $CH_3$ | $C_3H_7$(iso) | $CH_2OC_6H_5$ | |
| 10.0225 | H | $NO_2$ | $CH_3$ | $C_3H_7$(iso) | $CH_2OC_6H_5$ | |
| 10.0226 | H | CN | $CH_3$ | $C_3H_7$(iso) | $CH_2OC_6H_5$ | |
| 10.0227 | H | $CH_2OH$ | $CH_3$ | $C_3H_7$(iso) | $CH_2OC_6H_5$ | |
| 10.0228 | H | $CH_2CH_2OH$ | $CH_3$ | $C_3H_7$(iso) | $CH_2OC_6H_5$ | |
| 10.0229 | H | $CH_2CH_2CH_2OH$ | $CH_3$ | $C_3H_7$(iso) | $CH_2OC_6H_5$ | |
| 10.0230 | H | $C_6H_5$ | $CH_3$ | $C_3H_7$(iso) | $CH_2OC_6H_5$ | |
| 10.0231 | H | $CH_2Cl$ | $CH_3$ | $C_3H_7$(iso) | $CH_2OC_6H_5$ | |
| 10.0232 | H | $CH_2CH_2Cl$ | $CH_3$ | $C_3H_7$(iso) | $CH_2OC_6H_5$ | |
| 10.0233 | H | $CH_2CH_2CH_2Cl$ | $CH_3$ | $C_3H_7$(iso) | $CH_2OC_6H_5$ | |

Beispiel 19 Herstellung von N-(2-Methylvalylamid)-6-phenoxymethyl-2,3-pyridincarbonsäureimid

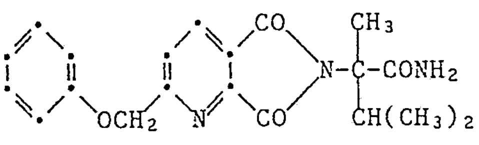

Man gibt unter Rühren einen Teil 2-Phenoxymethyl 5,7-dihydro-α-isopropyl-α-methyl-5,7-dioxo-OH-pyrolo-[3,4-6]pyridin-6-acetonitril [N-(2-Isopropyl-2-methyl-glycylnitril)-6-phenoxymethyl-2,3-pyridindicarbonsäureimid vom Beispiel 18] zu einem Teil konzentrierter Schwefelsäure wobei man durch Kühlen die Reaktionstemperatur auf weniger als 70° hält. Nachdem alles zugegeben ist rührt man eine Stunde bei 65° weiter. Dann wird das Reaktionsgemisch gekühlt und auf 12 Teile Eis gegossen. Zum entstandenen Gemisch werden unter Rühren 1,5 Teile Natriumacetat gegeben und noch 2 Stunden wird abgenutscht. Das Nutschgut

wird mit Natriumacetat-Lösung und Wasser gewaschen und über Phosphorpentoxid getrocknet. Man erhält so 0,82 Teile des obigen Imides.

In analoger Weise zum Beispiel 19 werden die in Tabellen 11.00-11.02 aufgeführten 2,3-Pyridinicarbonsäsureimide der Formel XI hergestellt.

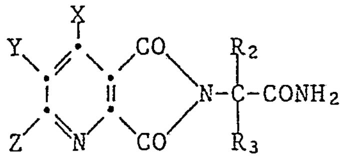

(XI)

Tabelle 11.00

| No. | X | Y | $R_2$ | $R_3$ | Z | phys. Daten |
|---|---|---|---|---|---|---|
| 11.0001 | H | H | $CH_3$ | $C_3H_7$(iso) | $C_3H_5$(Cyclo) | |
| 11.0002 | H | $CH_3$ | $CH_3$ | $C_3H_7$(iso) | $C_3H_5$(Cyclo) | |
| 11.0003 | H | $C_2H_5$ | $CH_3$ | $C_3H_7$(iso) | $C_3H_5$(Cyclo) | |
| 11.0004 | H | $C_3H_7$ | $CH_3$ | $C_3H_7$(iso) | $C_3H_5$(Cyclo) | |
| 11.0005 | H | $OCH_3$ | $CH_3$ | $C_3H_7$(iso) | $C_3H_5$(Cyclo) | |
| 11.0006 | H | $OC_2H_5$ | $CH_3$ | $C_3H_7$(iso) | $C_3H_5$(Cyclo) | |
| 11.0007 | H | $OCH_2CH{=}CH_2$ | $CH_3$ | $C_3H_7$(iso) | $C_3H_5$(Cyclo) | |
| 11.0008 | H | $OCH_2CH{=}CHCH_3$ | $CH_3$ | $C_3H_7$(iso) | $C_3H_5$(Cyclo) | |
| 11.0009 | H | $OCH_2C(Cl){=}CH_2$ | $CH_3$ | $C_3H_7$(iso) | $C_3H_5$(Cyclo) | |
| 11.0010 | H | $OCH_2CH{=}CHCl$ | $CH_3$ | $C_3H_7$(iso) | $C_3H_5$(Cyclo) | |
| 11.0011 | H | $OCH_2C{\equiv}CH$ | $CH_3$ | $C_3H_7$(iso) | $C_3H_5$(Cyclo) | |
| 11.0012 | H | $OCHF_2$ | $CH_3$ | $C_3H_7$(iso) | $C_3H_5$(Cyclo) | |
| 11.0013 | H | $OCF_3$ | $CH_3$ | $C_3H_7$(iso) | $C_3H_5$(Cyclo) | |
| 11.0014 | H | $OCF_2CHFCF_3$ | $CH_3$ | $C_3H_7$(iso) | $C_3H_5$(Cyclo) | |
| 11.0015 | H | $OC_6H_5$ | $CH_3$ | $C_3H_7$(iso) | $C_3H_5$(Cyclo) | |
| 11.0016 | H | $CF_3$ | $CH_3$ | $C_3H_7$(iso) | $C_3H_5$(Cyclo) | |
| 11.0017 | H | Br | $CH_3$ | $C_3H_7$(iso) | $C_3H_5$(Cyclo) | |
| 11.0018 | H | Cl | $CH_3$ | $C_3H_7$(iso) | $C_3H_5$(Cyclo) | |
| 11.0019 | H | F | $CH_3$ | $C_3H_7$(iso) | $C_3H_5$(Cyclo) | |
| 11.0020 | H | $SCH_3$ | $CH_3$ | $C_3H_7$(iso) | $C_3H_5$(Cyclo) | |
| 11.0021 | H | $SCH_3$ | $CH_3$ | $C_3H_7$(iso) | $C_3H_5$(Cyclo) | |
| 11.0022 | H | $SC_3H_7$-n | $CH_3$ | $C_3H_7$(iso) | $C_3H_5$(Cyclo) | |
| 11.0023 | H | $SO_2CH_3$ | $CH_3$ | $C_3H_7$(iso) | $C_3H_5$(Cyclo) | |
| 11.0024 | H | $SO_2CH_3$ | $CH_3$ | $C_3H_7$(iso) | $C_3H_5$(Cyclo) | |
| 11.0025 | H | $NO_2$ | $CH_3$ | $C_3H_7$(iso) | $C_3H_5$(Cyclo) | |
| 11.0026 | H | CN | $CH_3$ | $C_3H_7$(iso) | $C_3H_5$(Cyclo) | |
| 11.0027 | H | $CH_2OH$ | $CH_3$ | $C_3H_7$(iso) | $C_3H_5$(Cyclo) | |
| 11.0028 | H | $CH_2CH_2OH$ | $CH_3$ | $C_3H_7$(iso) | $C_3H_5$(Cyclo) | |
| 11.0029 | H | $CH_2CH_2CH_2OH$ | $CH_3$ | $C_3H_7$(iso) | $C_3H_5$(Cyclo) | |
| 11.0030 | H | $C_6H_5$ | $CH_3$ | $C_3H_7$(iso) | $C_3H_5$(Cyclo) | |
| 11.0031 | H | $CH_2Cl$ | $CH_3$ | $C_3H_7$(iso) | $C_3H_5$(Cyclo) | |
| 11.0032 | H | $CH_2CH_2Cl$ | $CH_3$ | $C_3H_7$(iso) | $C_3H_5$(Cyclo) | |
| 11.0033 | H | $CH_2CH_2CH_2Cl$ | $CH_3$ | $C_3H_7$(iso) | $C_3H_5$(Cyclo) | |

Tabelle 11.01

| No. | X | Y | $R_2$ | $R_3$ | Z | phys. Daten |
|---|---|---|---|---|---|---|
| 11.0101 | H | H | $CH_3$ | $C_3H_7$(iso) | $CH_2OC_2H_5$ | |
| 11.0102 | H | $CH_3$ | $CH_3$ | $C_3H_7$(iso) | $CH_2OC_2H_5$ | |
| 11.0103 | H | $C_2H_5$ | $CH_3$ | $C_3H_7$(iso) | $CH_2OC_2H_5$ | |
| 11.0104 | H | $C_3H_7$ | $CH_3$ | $C_3H_7$(iso) | $CH_2OC_2H_5$ | |
| 11.0105 | H | $OCH_3$ | $CH_3$ | $C_3H_7$(iso) | $CH_2OC_2H_5$ | |
| 11.0106 | H | $OC_2H_5$ | $CH_3$ | $C_3H_7$(iso) | $CH_2OC_2H_5$ | |
| 11.0107 | H | $OCH_2CH=CH_2$ | $CH_3$ | $C_3H_7$(iso) | $CH_2OC_2H_5$ | |
| 11.0108 | H | $OCH_2CH=CHCH_3$ | $CH_3$ | $C_3H_7$(iso) | $CH_2OC_2H_5$ | |
| 11.0109 | H | $OCH_2C(Cl)=CH_2$ | $CH_3$ | $C_3H_7$(iso) | $CH_2OC_2H_5$ | |
| 11.0110 | H | $OCH_2CH=CHCl$ | $CH_3$ | $C_3H_7$(iso) | $CH_2OC_2H_5$ | |
| 11.0111 | H | $OCH_2C{\equiv}CH$ | $CH_3$ | $C_3H_7$(iso) | $CH_2OC_2H_5$ | |
| 11.0112 | H | $OCHF_2$ | $CH_3$ | $C_3H_7$(iso) | $CH_2OC_2H_5$ | |
| 11.0113 | H | $OCF_3$ | $CH_3$ | $C_3H_7$(iso) | $CH_2OC_2H_5$ | |
| 11.0114 | H | $OCF_2CHFCF_3$ | $CH_3$ | $C_3H_7$(iso) | $CH_2OC_2H_5$ | |
| 11.0115 | H | $OC_6H_5$ | $CH_3$ | $C_3H_7$(iso) | $CH_2OC_2H_5$ | |
| 11.0116 | H | $CF_3$ | $CH_3$ | $C_3H_7$(iso) | $CH_2OC_2H_5$ | |
| 11.0117 | H | Br | $CH_3$ | $C_3H_7$(iso) | $CH_2OC_2H_5$ | |
| 11.0118 | H | Cl | $CH_3$ | $C_3H_7$(iso) | $CH_2OC_2H_5$ | |
| 11.0119 | H | F | $CH_3$ | $C_3H_7$(iso) | $CH_2OC_2H_5$ | |
| 11.0120 | H | $SCH_3$ | $CH_3$ | $C_3H_7$(iso) | $CH_2OC_2H_5$ | |
| 11.0121 | H | $SCH_3$ | $CH_3$ | $C_3H_7$(iso) | $CH_2OC_2H_5$ | |
| 11.0122 | H | $SC_3H_7$-n | $CH_3$ | $C_3H_7$(iso) | $CH_2OC_2H_5$ | |
| 11.0123 | H | $SO_2CH_3$ | $CH_3$ | $C_3H_7$(iso) | $CH_2OC_2H_5$ | |
| 11.0124 | H | $SO_2CH_3$ | $CH_3$ | $C_3H_7$(iso) | $CH_2OC_2H_5$ | |
| 11.0125 | H | $NO_2$ | $CH_3$ | $C_3H_7$(iso) | $CH_2OC_2H_5$ | |
| 11.0126 | H | CN | $CH_3$ | $C_3H_7$(iso) | $CH_2OC_2H_5$ | |
| 11.0127 | H | $CH_2OH$ | $CH_3$ | $C_3H_7$(iso) | $CH_2OC_2H_5$ | |
| 11.0128 | H | $CH_2CH_2OH$ | $CH_3$ | $C_3H_7$(iso) | $CH_2OC_2H_5$ | |
| 11.0129 | H | $CH_2CH_2CH_2OH$ | $CH_3$ | $C_3H_7$(iso) | $CH_2OC_2H_5$ | |
| 11.0130 | H | $C_6H_5$ | $CH_3$ | $C_3H_7$(iso) | $CH_2OC_2H_5$ | |
| 11.0131 | H | $CH_2Cl$ | $CH_3$ | $C_3H_7$(iso) | $CH_2OC_2H_5$ | |
| 11.0132 | H | $CH_2CH_2Cl$ | $CH_3$ | $C_3H_7$(iso) | $CH_2OC_2H_5$ | |
| 11.0133 | H | $CH_2CH_2CH_2Cl$ | $CH_3$ | $C_3H_7$(iso) | $CH_2OC_2H_5$ | |

Tabelle 11.02

| No. | X | Y | $R_2$ | $R_3$ | Z | phys. Daten |
|---|---|---|---|---|---|---|
| 11.0201 | H | H | $CH_3$ | $C_3H_7$(iso) | $CH_2OC_6H_5$ | |
| 11.0202 | H | $CH_3$ | $CH_3$ | $C_3H_7$(iso) | $CH_2OC_6H_5$ | |
| 11.0203 | H | $C_2H_5$ | $CH_3$ | $C_3H_7$(iso) | $CH_2OC_6H_5$ | |
| 11.0204 | H | $C_3H_7$ | $CH_3$ | $C_3H_7$(iso) | $CH_2OC_6H_5$ | |
| 11.0205 | H | $OCH_3$ | $CH_3$ | $C_3H_7$(iso) | $CH_2OC_6H_5$ | |
| 11.0206 | H | $OC_2H_5$ | $CH_3$ | $C_3H_7$(iso) | $CH_2OC_6H_5$ | |
| 11.0207 | H | $OCH_2CH{=}CH_2$ | $CH_3$ | $C_3H_7$(iso) | $CH_2OC_6H_5$ | |
| 11.0208 | H | $OCH_2CH{=}CHCH_3$ | $CH_3$ | $C_3H_7$(iso) | $CH_2OC_6H_5$ | |
| 11.0209 | H | $OCH_2C(Cl){=}CH_2$ | $CH_3$ | $C_3H_7$(iso) | $CH_2OC_6H_5$ | |
| 11.0210 | H | $OCH_2CH{=}CHCl$ | $CH_3$ | $C_3H_7$(iso) | $CH_2OC_6H_5$ | |
| 11.0211 | H | $OCH_2C{\equiv}CH$ | $CH_3$ | $C_3H_7$(iso) | $CH_2OC_6H_5$ | |
| 11.0212 | H | $OCHF_2$ | $CH_3$ | $C_3H_7$(iso) | $CH_2OC_6H_5$ | |
| 11.0213 | H | $OCF_3$ | $CH_3$ | $C_3H_7$(iso) | $CH_2OC_6H_5$ | |
| 11.0214 | H | $OCF_2CHFCF_3$ | $CH_3$ | $C_3H_7$(iso) | $CH_2OC_6H_5$ | |
| 11.0215 | H | $OC_6H_5$ | $CH_3$ | $C_3H_7$(iso) | $CH_2OC_6H_5$ | |
| 11.0216 | H | $CF_3$ | $CH_3$ | $C_3H_7$(iso) | $CH_2OC_6H_5$ | |
| 11.0217 | H | Br | $CH_3$ | $C_3H_7$(iso) | $CH_2OC_6H_5$ | |
| 11.0218 | H | Cl | $CH_3$ | $C_3H_7$(iso) | $CH_2OC_6H_5$ | |
| 11.0219 | H | F | $CH_3$ | $C_3H_7$(iso) | $CH_2OC_6H_5$ | |
| 11.0220 | H | $SCH_3$ | $CH_3$ | $C_3H_7$(iso) | $CH_2OC_6H_5$ | |
| 11.0221 | H | $SCH_3$ | $CH_3$ | $C_3H_7$(iso) | $CH_2OC_6H_5$ | |
| 11.0222 | H | $SC_3H_7$-n | $CH_3$ | $C_3H_7$(iso) | $CH_2OC_6H_5$ | |
| 11.0223 | H | $SO_2CH_3$ | $CH_3$ | $C_3H_7$(iso) | $CH_2OC_6H_5$ | |
| 11.0224 | H | $SO_2CH_3$ | $CH_3$ | $C_3H_7$(iso) | $CH_2OC_6H_5$ | |
| 11.0225 | H | $NO_2$ | $CH_3$ | $C_3H_7$(iso) | $CH_2OC_6H_5$ | |
| 11.0226 | H | CN | $CH_3$ | $C_3H_7$(iso) | $CH_2OC_6H_5$ | |
| 11.0227 | H | $CH_2OH$ | $CH_3$ | $C_3H_7$(iso) | $CH_2OC_6H_5$ | |
| 11.0228 | H | $CH_2CH_2OH$ | $CH_3$ | $C_3H_7$(iso) | $CH_2OC_6H_5$ | |
| 11.0229 | H | $CH_2CH_2CH_2OH$ | $CH_3$ | $C_3H_7$(iso) | $CH_2OC_6H_5$ | |
| 11.0230 | H | $C_6H_5$ | $CH_3$ | $C_3H_7$(iso) | $CH_2OC_6H_5$ | |
| 11.0231 | H | $CH_2Cl$ | $CH_3$ | $C_3H_7$(iso) | $CH_2OC_6H_5$ | |
| 11.0232 | H | $CH_2CH_2Cl$ | $CH_3$ | $C_3H_7$(iso) | $CH_2OC_6H_5$ | |
| 11.0233 | H | $CH_2CH_2CH_2Cl$ | $CH_3$ | $C_3H_7$(iso) | $CH_2OC_6H_5$ | |

Beispiel 20 Herstellung von
3-Isopropyl-3-methyl-8-phenoxymethyl-1H-Imidazo[1',2:1,2]pyrolo[3,4-6]pyridin-2,5(3H, 96H)-dion

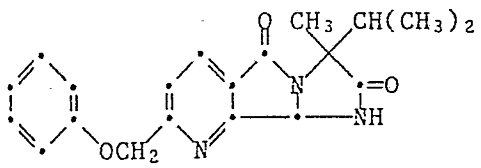

1 Teil N-(2-Methylvalylamid)-6-phenoxymethyl-2-3-pyridindicarbonsäureimid (Beispiel 19) werden in 9 Teilen Toluol gelöst und während 15 Stunden am Wasserabscheiden gekocht. Dann wird gekühlt und der trockenen Toluollösung 0,2 Teile einer 50%igen Natriumhydrid-Mineralöl-Suspension zugegeben. Die Suspension wird 24 Stunden am Rückfluss gekocht und noch heiss filtriert. Die Lösung wird dann eingedampft und der

kristalline Niederschlag mit Hexan vom Mineralöl befreit und anschliessend am Vakuum getrocknet. Man erhält so 0.78 Teile der obigen tricyclischen Verbindung.

Auf analoge Weise zum Beispiel 20 werden die in den Tabellen 12.00-12.02 aufgeführten tricyclischen Verbindungen der Formel XII hergestellt.

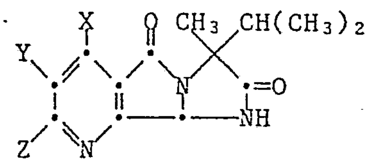

(XII)

Tabelle 12.00

| No. | X | Y | $R_2$ | $R_3$ | Z | phys. Daten |
|---|---|---|---|---|---|---|
| 12.0001 | H | H | $CH_3$ | $C_3H_7$(iso) | $C_3H_5$(Cyclo) | |
| 12.0002 | H | $CH_3$ | $CH_3$ | $C_3H_7$(iso) | $C_3H_5$(Cyclo) | |
| 12.0003 | H | $C_2H_5$ | $CH_3$ | $C_3H_7$(iso) | $C_3H_5$(Cyclo) | |
| 12.0004 | H | $C_3H_7$ | $CH_3$ | $C_3H_7$(iso) | $C_3H_5$(Cyclo) | |
| 12.0005 | H | $OCH_3$ | $CH_3$ | $C_3H_7$(iso) | $C_3H_5$(Cyclo) | |
| 12.0006 | H | $OC_2H_5$ | $CH_3$ | $C_3H_7$(iso) | $C_3H_5$(Cyclo) | |
| 12.0007 | H | $OCH_2CH=CH_2$ | $CH_3$ | $C_3H_7$(iso) | $C_3H_5$(Cyclo) | |
| 12.0008 | H | $OCH_2CH=CHCH_3$ | $CH_3$ | $C_3H_7$(iso) | $C_3H_5$(Cyclo) | |
| 12.0009 | H | $OCH_2C(Cl)=CH_2$ | $CH_3$ | $C_3H_7$(iso) | $C_3H_5$(Cyclo) | |
| 12.0010 | H | $OCH_2CH=CHCl$ | $CH_3$ | $C_3H_7$(iso) | $C_3H_5$(Cyclo) | |
| 12.0011 | H | $OCH_2C{\equiv}CH$ | $CH_3$ | $C_3H_7$(iso) | $C_3H_5$(Cyclo) | |
| 12.0012 | H | $OCHF_2$ | $CH_3$ | $C_3H_7$(iso) | $C_3H_5$(Cyclo) | |
| 12.0013 | H | $OCF_3$ | $CH_3$ | $C_3H_7$(iso) | $C_3H_5$(Cyclo) | |
| 12.0014 | H | $OCF_2CHFCF_3$ | $CH_3$ | $C_3H_7$(iso) | $C_3H_5$(Cyclo) | |
| 12.0015 | H | $OC_6H_5$ | $CH_3$ | $C_3H_7$(iso) | $C_3H_5$(Cyclo) | |
| 12.0016 | H | $CF_3$ | $CH_3$ | $C_3H_7$(iso) | $C_3H_5$(Cyclo) | |
| 12.0017 | H | Br | $CH_3$ | $C_3H_7$(iso) | $C_3H_5$(Cyclo) | |
| 12.0018 | H | Cl | $CH_3$ | $C_3H_7$(iso) | $C_3H_5$(Cyclo) | |
| 12.0019 | H | F | $CH_3$ | $C_3H_7$(iso) | $C_3H_5$(Cyclo) | |
| 12.0020 | H | $SCH_3$ | $CH_3$ | $C_3H_7$(iso) | $C_3H_5$(Cyclo) | |
| 12.0021 | H | $SCH_3$ | $CH_3$ | $C_3H_7$(iso) | $C_3H_5$(Cyclo) | |
| 12.0022 | H | $SC_3H_7$-n | $CH_3$ | $C_3H_7$(iso) | $C_3H_5$(Cyclo) | |
| 12.0023 | H | $SO_2CH_3$ | $CH_3$ | $C_3H_7$(iso) | $C_3H_5$(Cyclo) | |
| 12.0024 | H | $SO_2CH_3$ | $CH_3$ | $C_3H_7$(iso) | $C_3H_5$(Cyclo) | |
| 12.0025 | H | $NO_2$ | $CH_3$ | $C_3H_7$(iso) | $C_3H_5$(Cyclo) | |
| 12.0026 | H | CN | $CH_3$ | $C_3H_7$(iso) | $C_3H_5$(Cyclo) | |
| 12.0027 | H | $CH_2OH$ | $CH_3$ | $C_3H_7$(iso) | $C_3H_5$(Cyclo) | |
| 12.0028 | H | $CH_2CH_2OH$ | $CH_3$ | $C_3H_7$(iso) | $C_3H_5$(Cyclo) | |
| 12.0029 | H | $CH_2CH_2CH_2OH$ | $CH_3$ | $C_3H_7$(iso) | $C_3H_5$(Cyclo) | |
| 12.0030 | H | $C_6H_5$ | $CH_3$ | $C_3H_7$(iso) | $C_3H_5$(Cyclo) | |
| 12.0031 | H | $CH_2Cl$ | $CH_3$ | $C_3H_7$(iso) | $C_3H_5$(Cyclo) | |
| 12.0032 | H | $CH_2CH_2Cl$ | $CH_3$ | $C_3H_7$(iso) | $C_3H_5$(Cyclo) | |
| 12.0033 | H | $CH_2CH_2CH_2Cl$ | $CH_3$ | $C_3H_7$(iso) | $C_3H_5$(Cyclo) | |

Tabelle 12.01

| No. | X | Y | $R_2$ | $R_3$ | Z | phys. Daten |
|---|---|---|---|---|---|---|
| 12.0101 | H | H | $CH_3$ | $C_3H_7$(iso) | $CH_2OC_2H_5$ | |
| 12.0102 | H | $CH_3$ | $CH_3$ | $C_3H_7$(iso) | $CH_2OC_2H_5$ | |
| 12.0103 | H | $C_2H_5$ | $CH_3$ | $C_3H_7$(iso) | $CH_2OC_2H_5$ | |
| 12.0104 | H | $C_3H_7$ | $CH_3$ | $C_3H_7$(iso) | $CH_2OC_2H_5$ | |
| 12.0105 | H | $OCH_3$ | $CH_3$ | $C_3H_7$(iso) | $CH_2OC_2H_5$ | |
| 12.0106 | H | $OC_2H_5$ | $CH_3$ | $C_3H_7$(iso) | $CH_2OC_2H_5$ | |
| 12.0107 | H | $OCH_2CH{=}CH_2$ | $CH_3$ | $C_3H_7$(iso) | $CH_2OC_2H_5$ | |
| 12.0108 | H | $OCH_2CH{=}CHCH_3$ | $CH_3$ | $C_3H_7$(iso) | $CH_2OC_2H_5$ | |
| 12.0109 | H | $OCH_2C(Cl){=}CH_2$ | $CH_3$ | $C_3H_7$(iso) | $CH_2OC_2H_5$ | |
| 12.0110 | H | $OCH_2CH{=}CHCl$ | $CH_3$ | $C_3H_7$(iso) | $CH_2OC_2H_5$ | |
| 12.0111 | H | $OCH_2C{\equiv}CH$ | $CH_3$ | $C_3H_7$(iso) | $CH_2OC_2H_5$ | |
| 12.0112 | H | $OCHF_2$ | $CH_3$ | $C_3H_7$(iso) | $CH_2OC_2H_5$ | |
| 12.0113 | H | $OCF_3$ | $CH_3$ | $C_3H_7$(iso) | $CH_2OC_2H_5$ | |
| 12.0114 | H | $OCF_2CHFCF_3$ | $CH_3$ | $C_3H_7$(iso) | $CH_2OC_2H_5$ | |
| 12.0115 | H | $OC_6H_5$ | $CH_3$ | $C_3H_7$(iso) | $CH_2OC_2H_5$ | |
| 12.0116 | H | $CF_3$ | $CH_3$ | $C_3H_7$(iso) | $CH_2OC_2H_5$ | |
| 12.0117 | H | Br | $CH_3$ | $C_3H_7$(iso) | $CH_2OC_2H_5$ | |
| 12.0118 | H | Cl | $CH_3$ | $C_3H_7$(iso) | $CH_2OC_2H_5$ | |
| 12.0119 | H | F | $CH_3$ | $C_3H_7$(iso) | $CH_2OC_2H_5$ | |
| 12.0120 | H | $SCH_3$ | $CH_3$ | $C_3H_7$(iso) | $CH_2OC_2H_5$ | |
| 12.0121 | H | $SCH_3$ | $CH_3$ | $C_3H_7$(iso) | $CH_2OC_2H_5$ | |
| 12.0122 | H | $SC_3H_7$-n | $CH_3$ | $C_3H_7$(iso) | $CH_2OC_2H_5$ | |
| 12.0123 | H | $SO_2CH_3$ | $CH_3$ | $C_3H_7$(iso) | $CH_2OC_2H_5$ | |
| 12.0124 | H | $SO_2CH_3$ | $CH_3$ | $C_3H_7$(iso) | $CH_2OC_2H_5$ | |
| 12.0125 | H | $NO_2$ | $CH_3$ | $C_3H_7$(iso) | $CH_2OC_2H_5$ | |
| 12.0126 | H | CN | $CH_3$ | $C_3H_7$(iso) | $CH_2OC_2H_5$ | |
| 12.0127 | H | $CH_2OH$ | $CH_3$ | $C_3H_7$(iso) | $CH_2OC_2H_5$ | |
| 12.0128 | H | $CH_2CH_2OH$ | $CH_3$ | $C_3H_7$(iso) | $CH_2OC_2H_5$ | |
| 12.0129 | H | $CH_2CH_2CH_2OH$ | $CH_3$ | $C_3H_7$(iso) | $CH_2OC_2H_5$ | |
| 12.0130 | H | $C_6H_5$ | $CH_3$ | $C_3H_7$(iso) | $CH_2OC_2H_5$ | |
| 12.0131 | H | $CH_2Cl$ | $CH_3$ | $C_3H_7$(iso) | $CH_2OC_2H_5$ | |
| 12.0132 | H | $CH_2CH_2Cl$ | $CH_3$ | $C_3H_7$(iso) | $CH_2OC_2H_5$ | |
| 12.0133 | H | $CH_2CH_2CH_2Cl$ | $CH_3$ | $C_3H_7$(iso) | $CH_2OC_2H_5$ | |

Tabelle 12.02

| No. | X | Y | $R_2$ | $R_3$ | Z | phys. Daten |
|---|---|---|---|---|---|---|
| 12.0201 | H | H | $CH_3$ | $C_3H_7$(iso) | $CH_2OC_6H_5$ | |
| 12.0202 | H | $CH_3$ | $CH_3$ | $C_3H_7$(iso) | $CH_2OC_6H_5$ | |
| 12.0203 | H | $C_2H_5$ | $CH_3$ | $C_3H_7$(iso) | $CH_2OC_6H_5$ | |
| 12.0204 | H | $C_3H_7$ | $CH_3$ | $C_3H_7$(iso) | $CH_2OC_6H_5$ | |
| 12.0205 | H | $OCH_3$ | $CH_3$ | $C_3H_7$(iso) | $CH_2OC_6H_5$ | |
| 12.0206 | H | $OC_2H_5$ | $CH_3$ | $C_3H_7$(iso) | $CH_2OC_6H_5$ | |
| 12.0207 | H | $OCH_2CH{=}CH_2$ | $CH_3$ | $C_3H_7$(iso) | $CH_2OC_6H_5$ | |
| 12.0208 | H | $OCH_2CH{=}CHCH_3$ | $CH_3$ | $C_3H_7$(iso) | $CH_2OC_6H_5$ | |
| 12.0209 | H | $OCH_2C(Cl){=}CH_2$ | $CH_3$ | $C_3H_7$(iso) | $CH_2OC_6H_5$ | |
| 12.0210 | H | $OCH_2CH{=}CHCl$ | $CH_3$ | $C_3H_7$(iso) | $CH_2OC_6H_5$ | |
| 12.0211 | H | $OCH_2C{\equiv}CH$ | $CH_3$ | $C_3H_7$(iso) | $CH_2OC_6H_5$ | |
| 12.0212 | H | $OCHF_2$ | $CH_3$ | $C_3H_7$(iso) | $CH_2OC_6H_5$ | |
| 12.0213 | H | $OCF_3$ | $CH_3$ | $C_3H_7$(iso) | $CH_2OC_6H_5$ | |
| 12.0214 | H | $OCF_2CHFCF_3$ | $CH_3$ | $C_3H_7$(iso) | $CH_2OC_6H_5$ | |
| 12.0215 | H | $OC_6H_5$ | $CH_3$ | $C_3H_7$(iso) | $CH_2OC_6H_5$ | |
| 12.0216 | H | $CF_3$ | $CH_3$ | $C_3H_7$(iso) | $CH_2OC_6H_5$ | |
| 12.0217 | H | Br | $CH_3$ | $C_3H_7$(iso) | $CH_2OC_6H_5$ | |
| 12.0218 | H | Cl | $CH_3$ | $C_3H_7$(iso) | $CH_2OC_6H_5$ | |
| 12.0219 | H | F | $CH_3$ | $C_3H_7$(iso) | $CH_2OC_6H_5$ | |
| 12.0220 | H | $SCH_3$ | $CH_3$ | $C_3H_7$(iso) | $CH_2OC_6H_5$ | |
| 12.0221 | H | $SCH_3$ | $CH_3$ | $C_3H_7$(iso) | $CH_2OC_6H_5$ | |
| 12.0222 | H | $SC_3H_7$-n | $CH_3$ | $C_3H_7$(iso) | $CH_2OC_6H_5$ | |
| 12.0223 | H | $SO_2CH_3$ | $CH_3$ | $C_3H_7$(iso) | $CH_2OC_6H_5$ | |
| 12.0224 | H | $SO_2CH_3$ | $CH_3$ | $C_3H_7$(iso) | $CH_2OC_6H_5$ | |
| 12.0225 | H | $NO_2$ | $CH_3$ | $C_3H_7$(iso) | $CH_2OC_6H_5$ | |
| 12.0226 | H | CN | $CH_3$ | $C_3H_7$(iso) | $CH_2OC_6H_5$ | |
| 12.0227 | H | $CH_2OH$ | $CH_3$ | $C_3H_7$(iso) | $CH_2OC_6H_5$ | |
| 12.0228 | H | $CH_2CH_2OH$ | $CH_3$ | $C_3H_7$(iso) | $CH_2OC_6H_5$ | |
| 12.0229 | H | $CH_2CH_2CH_2OH$ | $CH_3$ | $C_3H_7$(iso) | $CH_2OC_6H_5$ | |
| 12.0230 | H | $C_6H_5$ | $CH_3$ | $C_3H_7$(iso) | $CH_2OC_6H_5$ | |
| 12.0231 | H | $CH_2Cl$ | $CH_3$ | $C_3H_7$(iso) | $CH_2OC_6H_5$ | |
| 12.0232 | H | $CH_2CH_2Cl$ | $CH_3$ | $C_3H_7$(iso) | $CH_2OC_6H_5$ | |
| 12.0233 | H | $CH_2CH_2CH_2Cl$ | $CH_3$ | $C_3H_7$(iso) | $CH_2OC_6H_5$ | |

Formulierungsbeispiele:

Beispiel 21 Formulierungsbeispiele für Wirkstoffe der Formel I (% = Gewichtsprozent)

a) Spritzpulver

| | a) | b) | c) |
|---|---|---|---|
| Wirkstoff gemäss Tabellen 1-4 | 20 % | 60 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyethylenglykol-ether (7-8 Mol EO) | - | 2 % | 2 % |
| Hochdisperse Kieselsäure | 5 % | 27 % | 27 % |
| Kaolin | 67 % | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

b) Emulsions-Konzentrat

| | a) | b) |
|---|---|---|
| Wirkstoff gemäss Tabellen 1-4 | 10 % | 1 % |
| Octylphenolpolyethylenglykol-ether (4-5 Mol EO) | 3 % | 3 % |
| Ca-Dodecylbenzolsulfonat | 3 % | 3 % |
| Ricinusölpolyglykolether (36 Mol EO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

c) Stäubemittel

| | a) | b) |
|---|---|---|
| Wirkstoff gemäss Tabellen 1-4 | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

d) Extruder Granulat

| | a) | b) |
|---|---|---|
| Wirkstoff gemäss Tabellen 1-4 | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff gemäss Tabellen 1-4 | 3 % |
| Polyethylenglykol (MG 200) | 3 % |
| Kaolin | 94 % |

EMI ID

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff gemäss Tabellen 1-4 | 40 % | 5 % |
| Ethylenglykol | 10 % | 10 % |
| Nonylphenolpolyethylenglykolether (15 Mol EO) | 6 % | 1 % |
| Na-Ligninsulfonat | 10 % | 5 % |
| Carboxymethylcellulose | 1 % | 1 % |
| 37 %ige wässrige Formaldehyd-Lösung | 0,2 % | 0,2 % |
| Silikonöl in Form einer 75 %igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

## Biologische Beispiele:

### Beispiel 22 Pre-emergente Herbizid-Wirkung

Im Gewächshaus wird unmittelbar nach der Einsaat der Versuchspflanzen in Saatschalen die Erdoberfläche mit einer wässrigen Dispersion der Wirkstoffe, erhalten aus einem 25 %igen Emulsionskonzentrat behandelt. Es werden Konzentrationen von 4 kg Wirksubstanz/Hektar angewendet. Die Saatschalen werden im Gewächshaus bei 22-25° C und 50-70 % relativer Luftfeuchtigkeit gehalten und der Versuch nach 3 Wochen ausgewertet und die Resultate nach folgender Notenskala ausgewertet.

1 Pflanze hat nicht gekeimt oder ist eingegangen
2-3 sehr starke Schäden
4 starke Schäden
5 mittlere Schäden, die Pflanzen sind verkümmert
6 Schäden, welche die Pflanze regenerieren kann
7-8 leichte Schäden
9 normales Wachstum, wie das unbehandelter Pflanzen

In diesem Versuch zeigten die Verbindungen der Tabellen 1-4 starke Herbizidwirkung.

### Beispiel 23 Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wässrigen Wirkstoffdispersion in einer Dosierung von 4 kg Wirksubstanz pro Hektar auf die Pflanzen gespritzt und diese bei 24°-26° C und 45-60 % relativer Luftfeuchtigkeit gehalten. 15 Tage

nach der Behandlung wird der Versuch nach der obigen Notenskala ausgewertet. Auch in diesem Versuch zeigen die Verbindungen der Tabellen 1-4 starke bis sehr starke Herbizidwirkung.

Beispiel Herbizidwirkung vor dem Auflaufen der Pflanzen

Kunststofftöpfe werden mit expandiertem Vermiculit (Dichte: 0,135 g/cm$^3$, Wasserabsorptionsvermögen: 0,565 1/1) gefüllt. Nach dem Sättigen des nicht adsorptiven Vermiculits mit einer wässrigen Wirkstoffemulsion werden Samen der folgenden Pflanzen auf die Oberfläche gesät: Nasturtium offcinalis, Agrostis tenuis, Stellaria media und Digitaria sanguinalis. Die Versuchsgefässe werden anschliessend in einer Klimakammer bei 20° C, einer Beleuchtung von ca. 20 kLux und einer relativen Luftfeuchtigkeit von 70 % gehalten. Während der Keimphase von 4 bis 6 Tagen werden die Töpfe zur Erhöhung der örtlichen Luftfeuchtigkeit mit lichtdurchlässigem Material abgedeckt und mit deinoisiertem Wasser begossen. Nach dem 5. Tag wird dem Giesswasser 0,5 % eines handelsüblichen Flüssigdüngers (®Greenzit) zugesetzt. 12 Tage nach der Aussaat wird der Versuch ausgewertet und die Wirkung auf die Versuchspflanzen nach dem in Beispiel 3 gegebenen Massstab bewertet.

Die Resultate sind in der Tabelle 13 zusammengefasst:

Tabelle 13

| Verb. No. | Aufwand Menge ppm | Nasturtium officinalis | Agrostis tenuis | Stellaria media | Digitaria sang. |
|---|---|---|---|---|---|
| 1.0003 | 100 | 2 | 2 | 2 | 2 |
| 1.0402 | 100 | 2 | 1 | 2 | 2 |
| 2.0001 | 100 | 2 | 2 | 2 | 2 |
| 2.0002 | 100 | 2 | 2 | 2 | 2 |
| 2.0003 | 100 | 2 | 2 | 2 | 2 |
| 2.0017 | 100 | 1 | 1 | 1 | 1 |
| 2.0034 | 100 | 2 | 3 | 4 | 4 |
| 2.0301 | 100 | 2 | 2 | 2 | 2 |
| 2.0401 | 100 | 2 | 2 | 2 | 2 |
| 2.0402 | 100 | 2 | 2 | 2 | 2 |
| 2.0403 | 100 | 2 | 2 | 2 | 2 |
| 2.0434 | 100 | 8 | 8 | 8 | 8 |
| 2.0601 | 100 | 2 | 2 | 2 | 2 |
| 2.0602 | 100 | 2 | 2 | 2 | 2 |
| 2.0603 | 100 | 2 | 2 | 2 | 2 |
| 2.1001 | 100 | 2 | 2 | 2 | 2 |
| 2.1201 | 100 | 4 | 4 | 3 | 4 |
| 2.1301 | 100 | 2 | 2 | 2 | 2 |
| 2.1401 | 100 | 3 | 3 | 3 | 3 |
| 2.1601 | 100 | 6 | 6 | 6 | 7 |
| 3.0002 | 100 | 1 | 1 | 2 | 2 |
| 3.0403 | 100 | 2 | 2 | 2 | 2 |
| 4.0002 | 100 | 3 | 3 | 3 | 3 |
| 4.0003 | 100 | 2 | 2 | 2 | 2 |
| 4.0403 | 100 | 2 | 2 | 2 | 2 |
| 4.1001 | 100 | 2 | 2 | 2 | 2 |

Beispiel 25 Herbizidwirkung für Wasserreis (paddy)

Die Wasserunkräuter Echinochloa crus galli und Monocharia vag. werden in Plastikbechern (60 cm$^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat mit einer wässrigen Emulsion der Prüfsubstanzen durch eine Spritzung auf die

Gefässe. Die Dosis entspricht einer Wirkstoffmenge von 4 kg AS pro Hektar (Spritzbrühmenge = 550 l/ha. Die Pflanzenbecher werden dann im Gewächshaus unter optimalen Wachstumsbedingungen für die Reisunkräuter aufgestellt, d.h. bei 25°-30°C und hoher Luftfeuchtigkeit. Die Auswertung der Versuche findet 3 Wochen nach Applikation statt.

Die Bonitur erfolgt nach im Beispiel 22 gegebenen linearen Notenskala.

Die geprüften Verbindungen der Tabellen 1-4 zeigen gute Wirkung gegen diese Unkräuter unter weitgehender Schonung der Reispflänzchen.

Beispiel 26 Wuchshemmung bei tropischen Bodenbedecker-Leguminosen (cover crops).

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 6000 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Tabelle 1 in Aufwandmengen von 50-3000 g/ha behandelten Pflanzen eine deutliche Redukrion des Neuzuwachses (weniger als 20 % des Neuzuwachses bei unbehandelten Kontrollpflanzen), ohne dass dabei die Versuchspflanzen geschädigt wurden.

Beispiel 27 Wuchsregulierung an Sojabohnen

In Kunststoffbehältern mit einem Erde-Torf-Sandgemisch im Verhältnis 6:3:1 werden Sojabohnen der Sorte "Hark" angesät und in eine Klimakammer gegeben. Durch optimale Tempeaturwahl, Beleuchtung, Düngerzugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleilch zu unbehandelten Kontrollpflanzen bewirken erfindungsgemässe Wirkstoffe der Tabelle 1 eine merklicher Erhöhung der Anzahl und des Gewichts der Schoten im Haupttrieb.

Beispiel 28 Wuchshemmung bei Getreide

In Kunststofftöpfen mit sterilisierter Erde werden in Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Tabelle 2 besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleilch zu unbehandelten Kontrollen eine Verringerung des Neuzuwachses (60-90 % der Kontrolle), sowie teilweise eine Zunahme der Stengeldurchmesser auf.

Beispiel 29 Wuchshemmung bei Gräsern

In Kunststoffschalen mit Erde-Torf-Sand-Gemisch (6:3:1) werden die Gräser Lolium perenne, Poa pratensis, Festuca ovina, Dactylis glomerate und Cynodon dactylon im Gewächshaus angesät und nach Bedarf bewässert. Die Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Tabellen 3-8 besprüht. Die Wirkstoffmenge beträgt umgerechnet bis zu 500 g Aktivsubstanz pro Hektar. 21 Tage nach Applikation wird das Wachstum der Gräser beurteilt.

Die geprüften Verbindungen der Tabelle 1 bewirken eine Reduzierung des Neuzuwachses von um 10-30 % im Vergleich zur unbehandelten Kontrolle.

Beispiel 30 Desiccations- und Defoliationswirkung

In einem Gewächshaus wurden Baumwollpflanzen der Sorte Deltapine in Tontöpfen angezogen. Nach abgeschlossener Kapselbildung wurden sie mit wässrigen Zubereitungen der Wirkstoffe in einer Aufwandmenge, die 1,2, 0,6 und 0,3 kg/ha im Feld entspräche, gespritzt. Unbehandelte Pflanzen wurden als Kontrolle belassen. Die Auswertung des Versuches erfolgte 3, 7 und 14 Tage nach Applikation der Wirksubstanz durch Bestimmen des Grades an Defoliation (% abgefallene Blätter) und an Dessikation (% Austrocknung der an der Pflanze verbleibenden Blätter).

In diesem Versuch beliessen die geprüften Verbindungen der Tabelle 1 bei Aufwandmengen von 0,6 und 1,2 kg/ha nach 7 Tagen bloss noch wenige vertrocknete Blätter auf den Pflanzen (> 80 % Blattfall und Austrocknung).

## Patentansprüche

1. Verfahren zur Herstellung von in der 6-Stellung substituierten 2-(Imidazolin-2-yl)nicotinsäurederivaten der Formel I

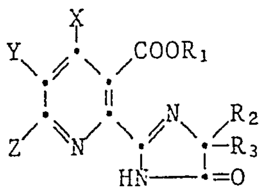

(I)

worin
$R_1$ Wasserstoff, das Kationäquivalent eines Alkalimetall-, Erdalkalimetall-, Magnesium-, Kupfer-, Eisen-, Zink-, Kobalt-, Blei-, Silber-, Nickel- oder quaternären Ammonium- oder Alkylammonium-Salzes, $C_1$-$C_6$-Alkyl, unsubstituiert oder substituiert durch Halogen, Hydroxyl, $C_1$-$C_3$-Alkoxy, $C_3$-$C_6$-Cycloalkyl, Benzyloxy, Furyl, Phenyl, Halophenyl, Niederalkylphenyl, Niederalkoxyphenyl, Nitrophenyl, Carboxyl, $C_1$-$C_4$-Alkoxycarbonyl oder Cyan; $C_3$-$C_6$-Cycloalkyl unsubstituiert oder 1-2fach substituiert durch $C_1$-$C_3$-Alkyl; $C_3$-$C_6$-Alkenyl, unsubstituiert oder substituiert durch Halogen, $C_1$-$C_3$-Alkoxy, Phenyl oder $C_1$-$C_4$-Alkoxycarbonyl; $C_3$-$C_6$-Alkinyl, unsubstituiert oder 1-2fach substituiert durch $C_1$-$C_3$-Alkyl;
$R_2$ $C_1$-$C_4$-Alkyl,
$R_3$ $C_1$-$C_4$-Alkyl oder $C_3$-$C_6$-Cycloalkyl oder
$R_2$ und $R_3$ zusammen mit dem Kohlenstoffatom, an das sie gebunden sind, auch einen $C_3$-$C_6$-Cycloalkylrest, der durch Methylreste substituiert sein kann,
X Wasserstoff oder Methyl,
Y Wasserstoff Halogen, $C_1$-$C_6$-Alkyl, $C_1$-$C_6$-Halogenalkyl, $C_1$-$C_6$-Hydroxyalkyl, $C_1$-$C_6$-Alkoxy, $C_1$-$C_6$-Alkylthio, Phenoxy, Nitro, Cyan, $C_1$-$C_4$-Alkylamino, Di-$C_1$-$C_4$-alkylamino, $C_1$-$C_4$-Alkylsulfonyl, Phenyl, Halophenyl, Niederalkylphenyl, Niederalkoxyphenyl, $C_3$-$C_8$-Alkenyloxy, $C_3$-$C_8$-Haloalkenyloxy, $C_3$-$C_8$-Alkinyloxy, $C_3$-$C_8$-Haloalkinyloxy und
Z einen Rest $-CQ_1Q_2Q_3$ oder $-CQ_1Q_4Q_5$ bedeuten worin
$Q_1$ Wasserstoff oder $C_1$-$C_4$-Alkyl,
$Q_2$ Wasserstoff oder $C_1$-$C_4$-Alkyl,
$Q_3$ $C_1$-$C_6$-Alkoxy welches unsubstituiert oder durch $C_1$-$C_4$-Alkyl, $C_1$-$C_4$-Alkoxy, $C_4$-$C_9$-Alkoxyalkoxy, Cyan oder Carbamoyl substituiert ist, Phenoxy welches unsubstituiert oder durch Halogen, $C_1$-$C_4$-Alkyl, $C_1$-$C_4$-Alkoxy, $C_1$-$C_4$-Haloalkyl oder Nitro substituiert ist; $C_3$-$C_6$-Alkenyloxy oder $C_3$-$C_6$-Alkinyloxy,
$Q_4$ und $Q_5$ zusammen mit dem Kohlenstoffatom an das sie gebunden sind $C_3$-$C_6$-Cycloalkyl oder einen durch ein oder zwei sich nicht in vicinaler Stellung befindliche Sauerstoffatome enthaltenden 5-6-gliedrigen Ring welche durch $C_1$-$C_4$-Alkyl substituiert sein können, bedeuten oder
Z bedeutet einen über Kohlenstoff gebundenen 5-6-gliedrigen, gesättigten oder ungesättigten heterocyclischen Rest, der unsubstituiert oder durch Niederalkyl substituiert ist, dadurch gekennzeichnet, dass man einen in der 6-Stellung unsubstituierten Pyridin-2,3-dicarbonsäure-diester der Formel V

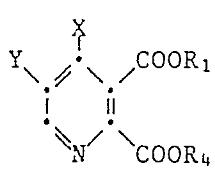

(V)

worin $R_4$, $R_1$, X und Y die oben gegebenen Bedeutung haben, und $R_4$ einen $C_1$-$C_8$-Alkylphenyl- oder Phenyl-$C_1$-$C_4$-alkylrest bedeutet, in wässriger Lösung, in Gegenwart einer katalytischen Menge von Silber II-ionen und einem Peroxysulfatsalz, mit einer Carbonsäure der Formel XVII

Z—COOH (XVII)

worin Z die oben gegebene Bedeutung hat, umsetzt zum in der 6-Stellung substituierten Pyridin-2,3-dicarbonsäure-diester der Formel IV

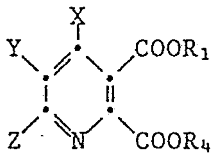

(IV)

worin $R_1$, $R_4$, X, Y und Z die oben gegebene Bedeutung haben und diesen Ester in einem inerten organischen Lösungsmittel, in Gegenwart einer starken Base mit dem 2-Aminoalkancarbonsäureamid der Formel XV

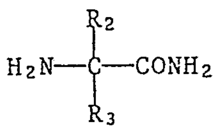

(XV)

in welchem $R_2$ und $R_3$ die im Anspruch 1 gegebene Bedeutung haben, umsetzt, das gebildete Salz der 2-(Imidazol-2-yl)-nicotinsäure der Formel III aus wässriger saurer Lösung aufnimmt, die freie 2-(Imidazolin-2-yl)nicotinsäure der Formel III

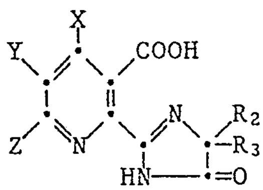

(III)

worin $R_2$, $R_3$, Y, Y und Z die oben gegebene Bedeutung haben, isoliert und dann mit einem wasserentziehenden Mittel odr Reagens zu einer tricyclischen 2H-Imidazo[1,2:1′,2′]pyrrolo[3,4-b]pyridin-3,5-dion Verbindung der Formel II umlagert,

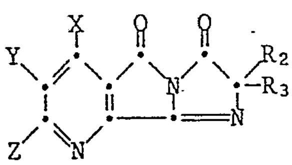

(II)

worin $R_2$, $R_3$, X, Y und Z die oben gegebene Bedeutung haben, und diese Verbindung dann bei erhöhterter Temperatur mit einem Hydroxid oder Alkohol der Formel XVI

$HOR_1$ (XVI)

worin $R_1$ die oben gegebene Bedeutung hat, zum 2-(Imidazolin-2-yl)nicotinsäurederivat der Formel I, aufspaltet.

2. 2-(Imidazolin-2-yl)nicotinsäurederivate der Formel V als Zwischenprodukte

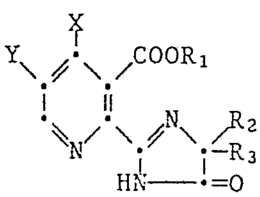

(V)

worin

$R_1$ Wasserstoff, das Kationäquivalent eines Alkalimetall-, Erdalkalimetall-, Magnesium-, Kupfer-, Eisen-,

Zink-, Kobalt-, Blei-, Silber-, Nickel- oder quaternären Ammonium- oder Alkylammonium-Salzes, $C_1$-$C_6$-Alkyl, unsubstituiert oder substituiert durch Halogen, Hydroxyl, $C_1$-$C_3$-Alkoxy, $C_3$-$C_6$-Cycloalkyl, Benzyloxy, Furyl, Phenyl, Halophenyl, Niederalkylphenyl, Niederalkoxyphenyl, Nitrophenyl, Carboxy, $C_1$-$C_4$-Alkoxycarbonyl oder Cyan; $C_3$-$C_6$-Cycloalkyl unsubstituiert oder 1-2fach substituiert durch $C_1$-$C_3$-Alkyl; $C_3$-$C_6$-Alkenyl, unsubstituiert oder substituiert durch Halogen, $C_1$-$C_3$-Alkoxy, Phenyl oder $C_1$-$C_4$-Alkoxycarbonyl; $C_3$-$C_6$-Alkinyl, unsubstituiert oder 1-2fach substituiert durch $C_1$-$C_3$-Alkyl;
$R_2$ $C_1$-$C_4$-Alkyl,
$R_3$ $C_1$-$C_4$-Alkyl oder $C_3$-$C_6$-Cycloalkyl oder
$R_2$ und $R_3$ zusammen mit dem Kohlenstoffatom, an das sie gebunden sind, auch einen $C_3$-$C_6$-Cycloalkylrest, der durch Methylreste substituiert sein kann,
X Wasserstoff oder Methyl,
Y Wasserstoff Halogen, $C_1$-$C_6$-Alkyl, $C_1$-$C_6$-Halogenalkyl, $C_1$-$C_6$-Hydroxyalkyl, $C_1$-$C_6$-Alkoxy, $C_1$-$C_6$-Alkylthio, Phenoxy, Nitro, Cyan, $C_1$-$C_4$-Alkylamino, Di-$C_1$-$C_4$-alkylamino, $C_1$-$C_4$-Alkylsulfonyl, Phenyl, Halophenyl, Niederalkylphenyl, Niederalkoxyphenyl, $C_3$-$C_8$-Alkenyloxy, $C_3$-$C_8$-Haloalkenyloxy, $C_3$-$C_8$-Alkinyloxy, $C_3$-$C_8$-Haloalkinyloxy und
Z einen Rest $-CQ_1Q_2Q_3$ oder $-CQ_1Q_4Q_5$ bedeuten worin
$Q_1$ Wasserstoff oder $C_1$-$C_4$-Alkyl,
$Q_2$ Wasserstoff oder $C_1$-$C_4$-Alkyl,
$Q_3$ $C_1$-$C_6$-Alkoxy welches durch $C_1$-$C_4$-Alkoxy, $C_4$-$C_9$-Alkoxyalkoxy, Cyan oder Carbamoyl substituiert ist, Phenoxy welches unsubstituiert oder durch Halogen, $C_1$-$C_4$-Alkyl, $C_1$-$C_4$-Alkoxy, $C_1$-$C_4$-Haloalkyl oder Nitro substituiert ist; $C_3$-$C_6$-Alkenyloxy oder $C_3$-$C_6$-Alkinyloxy,
$Q_4$ und $Q_5$ zusammen mit dem Kohlenstoffatom an das sie gebunden sind einen durch ein oder zwei sich nicht in vicinaler Stellung befindliche Sauerstoffatome enthaltenden 5-6-gliedrigen gesättigten Ring, welcher durch $C_1$-$C_4$-Alkyl substituiert sein kann, bedeuten.

3. Verfahren zur Herstellung der in der 6-Stellung substituierten 2-(Imidazolin-2-yl)nicotinsäurederivate der Formel I gemäss Anspruch 1, dadurch gekennzeichnet, dass man einen in der 6-Stellung substituierten Pyridin-2,3-dicarbonsäurediester der Formel IV

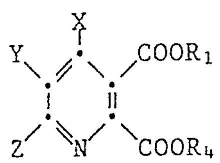

(IV)

worin $R_1$, $R_4$, X, Y und Z die im Anspruch 1 gegebene Bedeutung haben, mit einer starken Base in wässrigem Milieu verseift und die entstandene in 6-Stellung substituierte Pyridin-2,3-dicarbonsäure der Formel V

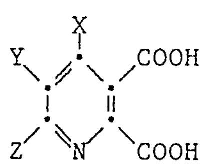

(V)

worin X, Y und Z die im Anspruch 1 gegebene Bedeutung haben, in einem wasserentziehenden Milieu zum in der 6-Stellung substituierten Pyridin-2,3-dicarbonsäureanhydrid der Formel VII

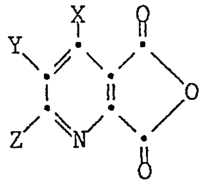

(VII)

worin X, Y und Z die im Anspruch 1 gegebene Bedeutung haben, und dieses Anhydrid dann mit einem Glycylamid der Formel XVIII

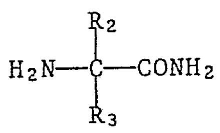

(XVIII)

worin $R_2$ und $R_3$ die im Anspruch 1 gegebene Bedeutung haben, kondensiert, die daraus enstandene, in der 6-Stellung substituierte 2-(N-Carbamoylmethylcarbamoyl)-nicotinsäuren der Formel VIII

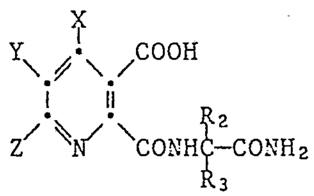

(VIII)

worin $R_2$, $R_3$, X, Y und Z die im Anspruch 1 gegebene Bedeutung haben, in einem inerten trockenen Lösungsmittel zum Ringschluss bringt, wobei die in der 6-Stellung substituierte 2-(Imidazolin-2-yl)-nicotinsäure der Formel III entsteht

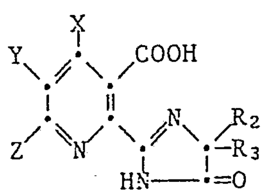

(III)

worin $R_2$, $R_3$, X, Y und Z die im Anspruch 1 gegebene Bedeutung haben isoliert und dann mit einem wasserentziehenden Mittel oder Reagens zu einer tricyclischen 2H-Imidazo[1,2:1′,2′]pyrrolo[3,4-b]pyridin-3,5-dion Verbindung der Formel II

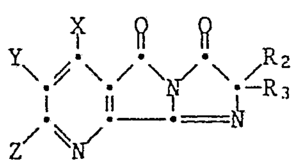

(II)

worin $R_2$, $R_3$, X, Y und Z die im Anspruch 1 gegebene Bedeutung haben, und diese Verbindung dann bei erhöhter Temperatur mit einem Hydroxid oder Alkohol der Formel XVI

$HOR_1$ (XVI)

worin $R_1$ die im Anspruch 1 gegebene Bedeutung hat, zum 2-(Imidazolin-2-yl)nicotinsäurederivat der Formel I aufspaltet.

4. In der 6-Stellung substituierte Pyridincarbonsäurederivate der Formel IV als Zwischenprodukte

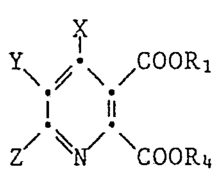

(IV)

worin

$R_1$ und $R_4$ unabhängig $C_1$-$C_6$-Alkyl, unsubstituiert oder substituiert durch Halogen, Hydroxyl, $C_1$-$C_3$-Alkoxy, $C_3$-$C_6$-Cycloalkyl, Benzyloxy, Furyl, Phenyl, Halophenyl, Niederalkylphenyl, Niederalkoxyphenyl, Nitrophenyl, Carboxyl, $C_1$-$C_4$-Alkoxycarbonyl oder Cyan; $C_3$-$C_6$-Cycloalkyl unsubstituiert oder 1-2fach substituiert durch $C_1$-$C_3$-Alkyl; $C_3$-$C_6$-Alkenyl, unsubstituiert oder substituiert durch Halogen,

$C_1$-$C_3$-Alkoxy, Phenyl oder $C_1$-$C_4$-Alkoxycarbonyl; $C_3$-$C_6$-Alkinyl, unsubstituiert oder 1-2fach substituiert durch $C_1$-$C_3$-Alkyl;
X Wasserstoff oder Methyl,
Y Wasserstoff Halogen, $C_1$-$C_6$-Alkyl, $C_1$-$C_6$-Halogenalkyl, $C_1$-$C_6$-Hydroxyalkyl, $C_1$-$C_6$-Alkoxy, $C_1$-$C_6$-Alkylthio, Phenoxy, Nitro, Cyan, $C_1$-$C_4$-Alkylamino, Di-$C_1$-$C_4$-alkylamino, $C_1$-$C_4$-Alkylsulfonyl, Phenyl, Halophenyl, Niederalkylphenyl, Niederalkoxyphenyl, $C_3$-$C_8$-Alkenyloxy, $C_3$-$C_8$-Haloalkenyloxy, $C_3$-$C_8$-Alkinyloxy, $C_3$-$C_8$-Haloalkinyloxy und
Z einen Rest $-CQ_1Q_2Q_3$ oder $-CQ_1Q_4Q_5$ bedeuten worin
$Q_1$ Wasserstoff oder $C_1$-$C_4$-Alkyl,
$Q_2$ Wasserstoff oder $C_1$-$C_4$-Alkyl,
$Q_3$ $C_1$-$C_6$-Alkoxy welches unsubstituiert oder durch $C_1$-$C_4$-Alkyl, $C_1$-$C_4$-Alkoxy, $C_4$-$C_9$-Alkoxyalkoxy, Cyan oder Carbamoyl substituiert ist, Phenoxy welches unsubstituiert oder durch Halogen, $C_1$-$C_3$-Alkyl, $C_1$-$N_4$-Alkoxy, $C_1$-$C_4$-Haloalkyl oder Nitro substituiert ist; $C_3$-$C_6$-Alkenyloxy oder $C_3$-$C_6$-Alkinyloxy,
$Q_4$ und $Q_5$ zusammen mit dem Kohlenstoffatom an das sie gebunden sind $C_3$-$C_6$-Cycloalkyl oder einen durch ein oder zwei sich nicht in vicinaler Stellung befindliche Sauerstoffatome enthaltenden 5-6-gliedrigen Ring welche durch $C_1$-$C_4$-Alkyl substituiert sein können, bedeuten oder
Z bedeutet einen über Kohlenstoff gebundenen 5-6-gliedrigen, gesättigten oder ungesättigten heterocyclischen Rest, der unsubstituiert oder durch Niederalkyl substituiert ist.

5. Verfahren zur Herstellung der in der 6-Stellung substituierten 2-(Imidazolin-2-yl)nicotinsäurederivate der Formel I, Anspruch 1, dadurch gekennzeichnet, dass man ein in der 6-Stellung substituiertes Pyridin-2,3-dicarbonsäureanhydrid der Formel VII

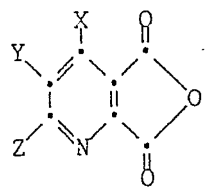

(VII)

worin X, Y und Z die im Anspruch 1 gegebene Bedeutung haben, in einem inerten organischen Lösungsmittel mit einem Glycylnitril der Formel XIX

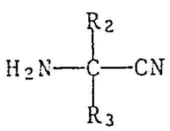

(XIX)

worin $R_2$ und $R_3$ die im Anspruch 1 gegebene Bedeutung haben, umsetzt, die dabei erhaltene in der 6-Stellung substituierte 2-(Cyanomethylcarbamoyl)-nicotinsäure der Formel IX

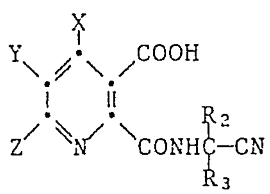

(IX)

worin $R_2$, $R_3$, X, Y und Z die im Anspruch 1 gegebene Bedeutung haben, dann in wasserentziehendem Milieu oder einem wasserentziehenden Mittel behandelt, so dass ein N-(Cyanomethylcarbamoyl)-pyridin-2,3-dicarbonsäureimid der Formel X entsteht,

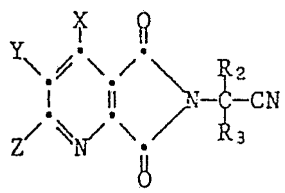

(X)

worin $R_2$, $R_3$, X, Y und Z die im Anspruch 1 gegebene Bedeutung haben, diese Imid in wässrig-saurem Medium, bei erhöhter Temperatur zum N-(Carbamoylmethyl)-pyridin-2,3-dicarbonsäureimid der Formel XI

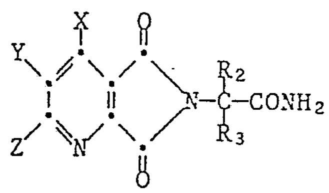

(XI)

worin $R_2$, $R_3$, X, Y und Z die im Anspruch 1 gegebene Bedeutung haben, welche Verbindung dann durch Erwärmen in basischem Milieu zur tricyclischen 3H-Imidazo[1,2:1′,2′]pyrrolo[3,4-b]pyridin-2,5-dion Verbindung der Formel XII umgewandelt wird,

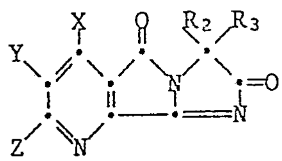

(XII)

worin $R_2$, $R_3$, X, Y und die im Anspruch 1 gegebene Bedeutung haben, und dieses Produkt dann bei erhöhter Temperatur mit einem Hydroxid oder Alkohol der Formel XVI

$HOR_1$ (XVI)

worin $R_1$ die im Anspruch 1 gegebene Bedeutung hat, zum 2-(Imidazolin-2-yl)nicotinsäurederivat der Formel I aufspaltet.

6. Verfahren zur Herstellung der in der 6-Stellung substituierten 2-(Imidazolin-2-yl)-nicotinsäurederivate der Formel I gemäss Anspruch 2, dadurch gekennzeichnet, dass man ein Methylketon der Formel XXIV

$Z-COCH_3$ (XXIV)

worin Z die im Anspruch 2 gegebene Bedeutung hat mit einem sekundären Amin der Formel XX

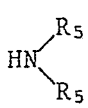 umsetzt (XX)

worin $R_5$ $C_1$-$C_4$-Alkyl, Phenyl-$C_1$-$C_4$-alkyl oder beide $R_5$ zusammen mit dem Stickstoffatom an das sie gebunden sind, einen 5-6 gliedrigen, gesättigten oder ungesättigten Ring bilden, der durch Sauerstoff unterbrochen sein kann, das entstandene Methylenamin der Formel XXI

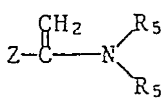

(XXI)

worin $R_5$ die oben gegebene Bedeutung hat, dann mit einem Akoxymethylenoxalessigester der Formel XXII

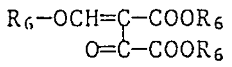

(XXII)

worin die $R_6$ unabhängig voneinander $C_1$-$C_6$-Alkyl- oder Phenyl-$C_1$-$C_6$-alkylreste bedeuten, kondensiert wobei ein 6-Amino-3-alkoxycarbonyl-hexan-3,5-dien-carbonsäureester der Formel XXIII entsteht,

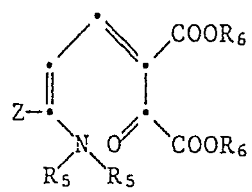

(XXIII)

worin $R_5$ und $R_6$ die obige und Z die im Anspruch 2 gegebene Bedeutung haben, diesem Ester dann in organischer Lösung mit Ammoniak ringschliesst zum in der 6-Stellung substituierten Pyridin-2,3-dicarbonsäurediester der Formel IVa

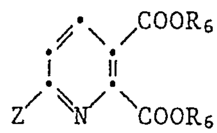

(IVa)

worin Z die im Anspruch 2 gegebene Bedeutung hat und $R_6$ einem $C_1$-$C_6$-Alkyl- oder Phenyl-$C_1$-$C_6$-alkylrest bedeutet steht, welcher dann gemäss dem Verfahren des Anspruchs 2 in Gegenwart einer starken Base mit dem 2-Aminoalkancarbonsäureamid der Formel XVIII

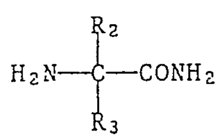

(XVIII)

in welchem $R_2$ und $R_3$ die im Anspruch 2 gegebene Bedeutung haben, umsetzt, das gebildete Salz der 2-(Imidazol-2-yl)nicotinsäure der Formel III wässriger saurer Lösung aufnimmt, die freie 2-(Imidazolin-2-yl)nicotinsäure der Formel III

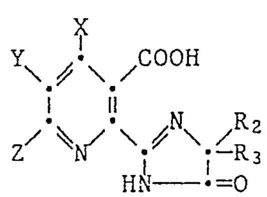

(III)

worin $R_2$, $R_3$, X, Y und Z die im Anspruch 2 gegebene Bedeutung haben, isoliert und dann mit einem wasserentziehenden Mittel oder Reagens zu einer tricyclischen 2H-Imidazo[1,2:1′,2′]pyrrolo[3,4-b]pyridin-3,5-dion Verbindung der Formel II umlagert,

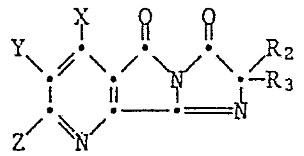

(II)

worin $R_2$, $R_3$, X, Y und Z die im Anspruch 1 gegebene Bedeutung haben, und diese Verbindung dann bei erhöhterter Temperatur mit einem Hydroxid oder Alkohol der Formel XVI

$HOR_1$ (XVI)

worin $R_1$ die im Anspruch 2 gegebene Bedeutung hat, zum 2-(Imidazolin-2-yl)nicotinsäurederivat der Formel I, Anspruch 1 aufspaltet.

7. Ein herbizides und das Pflanzenwachstum regulierendes Mittel, dadurch gekennzeichnet, dass es neben inerten Trägermaterialien und/oder anderen Zuschlagsstoffen als Wirkstoff eine herbizid oder den Pflanzenwuchs regulierend wirksame Menge eines 2-(Imidazolin-2-yl)nicotinsäurederivates gemäss Anspruch 1 enthält.

8. Die Verwendung des 2-(Imidazolin-2-yl)-nicotinsäurederivates gemäss Anspruch 1 oder eines ein solches Derivat enthaltenden Mittels zur Regulierung des Pfalnzenwuches oder zur Bekämpfung unerwünschten Pflanzenwachstums.

9. Verfahren zur selektiven pre- oder postemergenten Bekämpfung von Unkräutern in Nutzpflanzenkulturen, dadurch gekennzeichnet, dass man die Nutzpflanzenkulturen oder deren Anbaufläche mit einer wirksamen Menge eines 2-(Imidazolin-2-yl)-nicotinsäurederivates der Formel I, Anspruch 1 oder eines eine solche Verbindung enthaltendes Mittel behandelt.

10. Verfahren zur Unterdrückung des Pflanzenwachstums über das 2-Blattstadium hinaus, dadurch gekennzeichnet, dass man die Pflanzen während ihres Wachstums mit einer wirksamen Menge eines 2-(Imidazolin-2-yl)-nicotinsäurederivates der Formel I Anspruch 1 oder eines einen solchen Wirkstoff enthaltenden Mittels behandelt.

11. Neue in der 8-Stellung substituierte 2H-Imidazo[1,2:1′,2′]pyrrolo[3,4-b]pyridin-3,5-dion-Verbindungen der Formel II

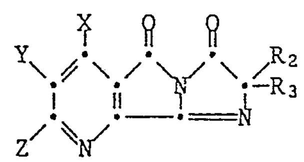

(II)

worin

$R_2$ $C_1$-$C_4$-Alkyl,

$R_3$ $C_1$-$C_4$-Alkyl oder $C_3$-$C_6$-Cycloalkyl oder

$R_2$ und $R_3$ zusammen mit dem Kohlenstoffatom, an das sie gebunden sind, auch einen $C_3$-$C_6$-Cycloalkylrest, der durch Methylreste substituiert sein kann,

X Wasserstoff oder Methyl,

Y Wasserstoff Halogen, $C_1$-$C_6$-Alkyl, $C_1$-$C_6$-Halogenalkyl, $C_1$-$C_6$-Hydroxyalkyl, $C_1$-$C_6$-Alkoxy, $C_1$-$C_6$-Alkylthio, Phenoxy, Nitro, Cyan, $C_1$-$C_4$-Alkylamino, Di-$C_1$-$C_4$-alkylamino, $C_1$-$C_4$-Alkylsulfonyl, Phenyl, Halophenyl, Niederalkylphenyl, Niederalkoxyphenyl, $C_3$-$C_8$-Alkenyloxy $C_3$-$C_8$-Haloalkenyloxy, $C_3$-$C_8$-Alkinyloxy, $C_3$-$C_8$-Haloalkinyloxy und

Z einen Rest $-CQ_1Q_2Q_3$ oder $-CQ_1Q_4Q_5$ bedeuten worin

$Q_1$ Wasserstoff oder $C_1$-$C_4$-Alkyl,

$Q_2$ Wasserstoff oder $C_1$-$C_4$-Alkyl,

$Q_3$ $C_1$-$C_6$-Alkoxy welches durch $C_1$-$C_4$-Alkoxy, $C_4$-$C_9$-Alkoxyalkoxy, Cyan oder Carbamoyl substituiert ist, Phenoxy welches unsubstituiert oder durch Halogen, $C_1$-$C_4$-Alkyl, $C_1$-Alkoxy, $C_1$-$C_4$-Haloalkyl oder Nitro substituiert ist; $C_3$-$C_6$-Alkenyloxy oder $C_3$-$C_6$-Alkinyloxy,

$Q_4$ und $Q_5$ zusammen mit dem Kohlenstoffatom, an das sie gebunden sind oder einen durch ein oder zwei sich nicht in vicinaler Stellung befindliche Sauerstoffatome enthaltenden 5-6 gliedrigen aliphatischen Ring, welcher durch $C_1$-$C_4$-Alkyl substituiert sein kann bedeuten.

12. Verbindungen gemäss Anspruch 11 der Formel II, worin $R_1$ Wasserstoff oder $C_1$-$C_6$-Alkyl bedeutet, $R_2$, $R_3$ und X die im Anspruch 1 gegebene Bedeutung haben, Y Wasserstoff, $C_1$-$C_6$-Alkyl oder Halogen, Z einen heterocyclischen Rest oder in der Bedeutung von $-CQ_1$-$Q_2Q_3$ einen $C_1$-$C_6$-Alkoxy-$C_1$-$C_4$-alkyl-, Phenoxy-$C_1$-$C_4$-alkyl oder Carbamoyl-$C_1$-$C_4$-alkoxy-$C_1$-$C_4$-alkylrest oder in der Bedeutung von $-CQ_1Q_4Q_5$ einen $C_3$-$C_6$-Cycloalkylrest, der durch $C_1$-$C_4$-Alkyl substituiert sein kann.

13. Verfahren zur Herstellung neuer, in der 8-Stellung substituierter 2H-Imidazo[1,2:1′,2′]pyrrolo[3,4-b]pyridin-3,4-dion-Verbindungen der Formel II gemäss Anspruch 11, dadurch gekennzeichnet, dass man einen in der 6-Stellung unsubstituierten Pyridin-2,3-dicarbonsäurediester der Formel V

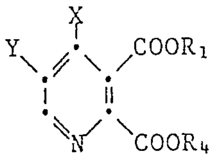

(V)

worin $R_1$, X und Y die im Anspruch 2 gegebene Bedeutung haben, und $R_4$ einen $C_1$-$C_8$-Alkylphenyl- oder Phenyl-$C_1$-$C_4$-alkylrest bedeutet, in wässriger Lösung, in Gegenwart einer katalytischen Menge von Silber II-ionen und einem Peroxysulfatsalz, mit einer Carbonsäure der Formel XVII

Z—COOH (XVII)

worin Z die im Anspruch 2 gegebene Bedeutung hat, umsetzt zum in der 6-Stellung substituierten Pyridin-2,3-dicarbonsäure-diester der Formel IV

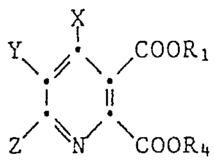

(IV)

worin $R_1$, $R_4$, X, Y und Z die im Anspruch 2 gegebene Bedeutung haben und diesen Ester in einem inerten organischen Lösungsmittel, in Gegenwart einer starken Base mit dem 2-Aminoalkancarbonsäureamid der Formel XVIII

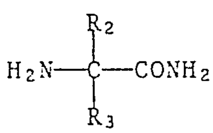

(XVIII)

in welchem $R_2$ und $R_3$ die im Anspruch 1 gegebene Bedeutung haben, umsetzt, das gebildete Salz der 2-(Imidazol-2-yl)nicotinsäure der Formel III wässriger saurer Lösung aufnimmt, die freie 2-(Imidazolin-2-yl)nicotinsäure der Formel III

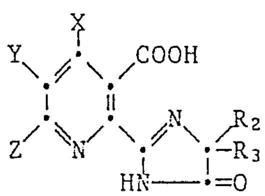

(III)

worin $R_2$, $R_3$, X, Y und Z die unter der Formel I gegebene Bedeutung haben, isoliert und dann mit einem wasserentziehenden Mittel oder Reagens zu einer tricyclischen 2H-Imidazo[1,2:1′,2′]pyrrolo[3,4-b]pyridin-3,5-dion Verbindung der Formel II umlagert.

14. Ein herbizides und das Pflanzenwachstum regulierendes Mittel, dadurch gekennzeichnet, dass es neben inerten Trägermaterialien und/oder anderen Zuschlagsstoffen als Wirkstoff eine herbizid oder den Pflanzenwuchs regulierend wirksame Menge einer 2H-Imidazo[1,2:1′,2′]pyrrolo[3,4-b]pyridin-3,5-dion-Verbindung gemäss Anspruch 11 enthält.

15. Die Verwendung der 2H-Imidazo[1,2:1′,2′]pyrrolo[3,4-b]pyridin-3,5-dion-Verbindung gemäss Anspruch 11 oder eines ein solches Derivat enthaltenden Mittels zur Regulierung des Pflanzenwuchses oder zur Bekämpfung unerwünschten Pflanzenwachstums.

16. Verfahren zur selektiven pre- oder postemergenten Bekämpfung von Unkräutern in Nutzpflanzenkulturen, dadurch gekennzeichnet, dass man die Nutzpflanzenkulturen oder deren Anbaufläche mit einer wirksamen Menge einer 2H-Imidazo[1,2:1′,2′]pyrrolo[3,4-b]pyridin-3,5-dion-Verbindung der Formel II,

Anspruch 11 oder eines eine solche Verbindung enthaltendes Mittel behandelt.

17. Verfahren zur Unterdrückung des Pflanzenwachstums über das 2-Blattstadium hinaus, dadurch gekennzeichnet, dass man die Pflanzen während ihres Wachstums mit einer wirksamen Menge einer 2H-Imidazo[1,2:1′,2′]pyrrolo[3,4-b]pyridin-3,4-dion-Verbindung der Formel II Anspruch 11 oder eines einen solchen Wirkstoff enthaltenden Mittels behandelt.

18. Neue in der 6-Stellung substituierte 2-(Imidazolin-2-yl)-nicotinsäuren der Formel III

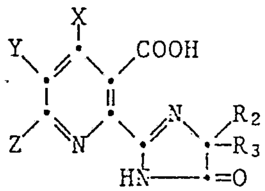

(III) .

$R_2$ $C_1$-$C_4$-Alkyl,
$R_3$ $C_1$-$C_4$-Alkyl oder $C_3$-$C_6$-Cycloalkyl oder
$R_2$ und $R_3$ zusammen mit dem Kohlenstoffatom, an das sie gebunden sind, auch einen $C_3$-$C_6$-Cycloalkylrest, der durch Methylreste substituiert sein kann,
X Wasserstoff oder Methyl,
Y Wasserstoff Halogen, $C_1$-$C_6$-Alkyl, $C_1$-$C_6$-Halogenalkyl, $C_1$-$C_6$-Hydroxyalkyl, $C_1$-$C_6$-Alkoxy, $C_1$-$C_6$-Alkylthio, Phenoxy, Nitro, Cyan, $C_1$-$C_4$-Alkylamino, Di-$C_1$-$C_4$-alkylamino, $C_1$-$C_4$-Alkylsulfonyl, Phenyl, Halophenyl, Niederalkylphenyl, Niederalkoxyphenyl, $C_3$-$C_8$-Alkenyloxy, $C_3$-$C_8$-Haloalkenyloxy, $C_3$-$C_8$-Alkinyloxy, $C_3$-$C_8$-Haloalkinyloxy und
Z einen Rest $-CQ_1Q_2Q_3$ oder $-CQ_1Q_4Q_5$ bedeuten worin
$Q_1$ Wasserstoff oder $C_1$-$C_4$-Alkyl,
$Q_2$ Wasserstoff oder $C_1$-$C_4$-Alkyl,
$Q_3$ $C_1$-$C_6$-Alkoxy welches durch $C_1$-$C_4$-Alkoxy, $C_4$-$C_9$-Alkoxyalkoxy, Cyan oder Carbamoyl substituiert ist, Phenoxy welches unsubstituiert oder durch Halogen, $C_1$-$C_4$-Alkyl, $C_1$-$C_4$-Alkoxy, $C_1$-$C_4$-Haloalkyl oder Nitro substituiert ist; $C_3$-$C_6$-Alkenyloxy oder $C_3$-$C_6$-Alkinyloxy,
$Q_4$ und $Q_5$ zusammen mit dem Kohlenstoffatom an das sie gebunden sind einen durch ein oder zwei sich nicht in vicinaler Stellung befindliche Sauerstoffatome enthaltenden 5-6-gliedrigen gesättigten Ring, welche durch $C_1$-$C_4$-Alkyl substituiert sein können.

19. Verbindungen gemäss Anspruch 18 der Formel III, worin $R_2$, $R_3$ und X die im Anspruch 1 gegebene Bedeutung haben, Y Wasserstoff, $C_6$-Alkyl oder Halogen, Z in der Bedeutung von $-CQ_1$-$Q_2Q_3$, Phenoxy-$C_1$-$C_4$-alkyl oder ein Carbamoyl-$C_1$-$C_4$-alkoxy-$C_1$-$C_4$-alkylrest sein kann.

20. Verbindungen gemäss Anspruch 18 der Formel III, worin $R_2$ Methyl, $R_3$ Isopropyl, Wasserstoff oder Methyl, Y $C_1$-$C_6$-Alkyl, Chlor oder Brom und Z einen Phenoxy-$C_1$-$C_4$-alkylrest bedeuten.

21. Verfahren zur Herstellung von neuen, in der 6-Stellung substituierten 2-(Imidazolin-2-yl)-nicotinsäuren der Formel III gemäss Anspruch 18, dadurch gekennzeichnet, dass man einen in der 6-Stellung unsubstituierten Pyridin-2,3-dicarbonsäurediester der Formel V

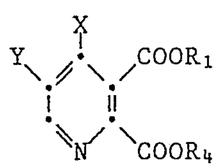

(V) ,

worin $R_1$, X und Y die im Anspruch 2 gegebene Bedeutung haben, und $R_4$ einen $C_1$-$C_8$-Alkylphenyl- oder Phenyl-$C_1$-$C_4$-alkylrest bedeutet, in wässriger Lösung, in Gegenwart einer katalytischen Menge von Silber II-ionen und einem Peroxysulfatsalz, mit einer Carbonsäure der Formel XVII

$Z-COOH$ (XVII)

worin Z die im Anspruch 2 gegebene Bedeutung hat, umsetzt zum in der 6-Stellung substituierten Pyridin-2,3-dicarbonsäure-diester der Formel IV

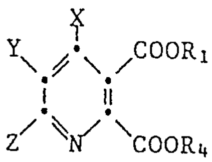

(IV)

worin $R_1$, $R_4$, X, Y und Z die im Anspruch 2 gegebene Bedeutung haben und diesen Ester in einem inerten organischen Lösungsmittel, in Gegenwart einer starken Base mit dem 2-Aminoalkancarbonsäureamid der Formel XVIII

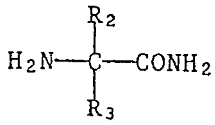

(XVIII)

in welchem $R_2$ und $R_3$ die im Anspruch 1 gegebene Bedeutung haben, umsetzt, das gebildete Salz der 2-(Imidazol-2-yl)nicotinsäure der Formel III aus wässriger saurer Lösung aufnimmt, die freie 2-(Imidazolin-2-yl)nicotinsäure der Formel III

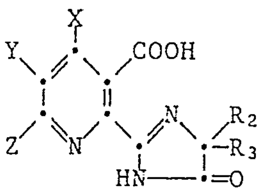

(III)

worin $R_2$, $R_3$, X, Y und Z die unter der Formel I gegebene Bedeutung haben, isoliert

22. Verfahren zur Herstellung von neuen in der 6-Stellung substituierten 2-(Imidazolin-2-yl)-nicotinsäuren der Formel III gemäss Anspruch 18, dadurch gekennzeichnet, dass man einen in der 6-Stellung substituierten Pyridin-2,3-dicarbonsäurediester der Formel IV

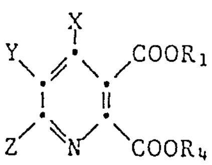

(IV)

worin $R_1$, $R_4$, X, Y und Z die im Anspruch 1 gegebene Bedeutung haben, mit einer starken Base in wässrigem Milieu verseift und die entstandene in 6-Stellung substituierte Pyridin-2,3-dicarbonsäure der Formel V

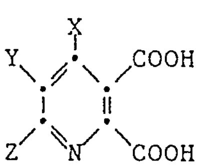

(V)

worin X, Y und Z die im Anspruch 1 gegebene Bedeutung haben, in einem wasserentziehenden Milieu zum in der 6-Stellung substituierten Pyridin-2,3-dicarbonsäureanhydrid der Formel VII

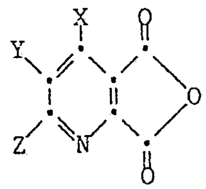

(VII)

worin X, Y und Z die im Anspruch 1 gegebene Bedeutung haben, und dieses Anhydrid dann mit einem Glycylamid der Formel XVIII

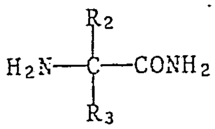

(XVIII)

worin $R_2$ und $R_3$ die im Anspruch 1 gegebene Bedeutung haben, kondensiert, die daraus enstandene, in der 6-Stellung substituierte 2-(N-Carbamoylmethylcarbamoyl)-nicotinsäuren der Formel VIII

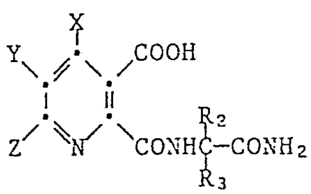

(VIII)

worin $R_2$, $R_3$, X, Y und Z die im Anspruch 1 gegebene Bedeutung haben, in einem inerten trockenen Lösungsmittel zum Ringschluss bringt, wobei die in der 6-Stellung substituierte 2-(Imidazolin-2-yl)-nicotinsäure der Formel III entsteht.

23. Ein herbizides und das Pflanzenwachstum regulierendes Mittel, dadurch gekennzeichnet, dass es neben inerten Trägermaterialien und/oder anderen Zuschlagsstoffen als Wirkstoff eine herbizid oder den Pflanzenwuchs regulierend wirksame Menge einer 2-(Imidazolin-2-yl)nicotinsäure gemäss Anspruch 18 enthält.

24. Die Verwendung der 2-(Imidazolin-2-yl)-nicotinsäure gemäss Anspruch 18 oder eines ein solches Derivat enthaltenden Mittels zur Regulierung des Pflanzenwuchses oder zur Bekämpfung unerwünschten Pflanzenwachstums.

25. Verfahren zur selektiven pre- oder postemergenten Bekämpfung von Unkräutern in Nutzpflanzenkulturen, dadurch gekennzeichnet, dass man die Nutzpflanzenkulturen oder deren Anbaufläche mit einer wirksamen Menge einer 2-(Imidazolin-2-yl)-nicotinsäure der Formel III, Anspruch 18 oder eines eine solche Verbindung enthaltendes Mittel behandelt.

26. Verfahren zur Unterdrückung des Pflanzenwachstums über das 2-Blattstadium hinaus, dadurch gekennzeichnet, dass man die Pflanzen während ihres Wachstums mit einer wirksamen Menge einer 2-(Imidazolin-2-yl)-nicotinsäure der Formel III Anspruch 20 oder eines einen solchen Wirkstoff enthaltenden Mittels behandelt.

27. Neue in der 8-Stellung substituierte 3H-Imidazo[1′,2′:1,2]pyrrolo[3,4-b]pyridin-2,5-dion-Verbindungen der Formel XII

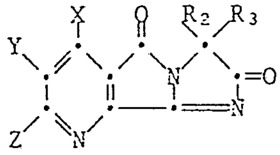

(XII)

worin
$R_2$ $C_1$-$C_4$-Alkyl,
$R_3$ $C_1$-$C_4$-Alkyl oder $C_3$-$C_6$-Cycloalkyl oder

$R_2$ und $R_3$ zusammen mit dem Kohlenstoffatom, an das sie gebunden sind, auch einen $C_3$-$C_6$-Cycloalkylrest, der durch Methylreste substituiert sein kann,
X Wasserstoff oder Methyl,
Y Wasserstoff Halogen, $C_1$-$C_6$-Alkyl, $C_1$-$C_6$-Halogenalkyl, $C_1$-$C_6$-Hydroxyalkyl, $C_1$-$C_6$-Alkoxy, $C_1$-$C_6$-Alkylthio, Phenoxy, Nitro, Cyan, $C_1$-$C_4$-Alkylamino, Di-$C_1$-$C_4$-alkylamino, $C_1$-$C_4$-Alkylsulfonyl, Phenyl, Halophenyl, Niederalkylphenyl, Niederalkoxyphenyl, $C_3$-$C_8$-Alkenyloxy, $C_3$-$C_8$-Haloalkenyloxy, $C_3$-$C_8$-Alkinyloxy, $C_3$-$C_8$-Haloalkinyloxy und
Z einen Rest -$CQ_1Q_2Q_3$ oder -$CQ_1Q_4Q_5$ bedeuten worin
$Q_1$ Wasserstoff oder $C_1$-$C_4$-Alkyl,
$Q_2$ Wasserstoff oder $C_1$-$C_4$-Alkyl,
$Q_3$ $C_1$-$C_6$-Alkoxy, welches durch $C_1$-$C_4$-Alkoxy, $C_4$-$C_9$-Alkoxyalkoxy, Cyan oder Carbamoyl substituiert ist, Phenoxy welches unsubstituiert oder durch Halogen, $C_1$-$C_4$-Alkyl, $C_1$-$C_4$-Alkoxy, $C_1$-$C_4$-Haloalkyl oder Nitro substituiert ist; $C_3$-$C_6$-Alkenyloxy oder $C_3$-$C_6$-Alkinyloxy,
$Q_4$ und $Q_5$ zusammen mit dem Kohlenstoffatom, an das sie gebunden sind einen durch ein oder zwei sich nicht in vicinaler Stellung befindliche Sauerstoffatome enthaltenden 5-6 gliedrigen gesättigten Ring, welcher durch $C_1$-$C_4$-Alkyl substituiert sein kann, bedeuten.

28. Verbindungen gemäss Anspruch 1 der Formel XII, worin $R_1$ Wasserstoff oder $C_1$-$C_6$-Alkyl bedeutet, $R_2$, $R_3$ und X die im Anspruch 1 gegebene Bedeutung haben, Y Wasserstoff, $C_1$-$C_6$-Alkyl oder Halogen, Z einen heterocyclischen Rest oder in der Bedeutung von -$CQ_1Q_2Q_3$ einen Phenoxy-$C_1$-$C_4$-alkyl oder Carbamoyl-$C_1$-$C_4$-alkoxy-$C_1$-$C_4$-alkylrest.

29. Verfahren zur Herstellung der 3H-Imidazo[1′,2′:1,2]pyrrolo[3,4-b]pyridin-2,5-dion-Verbindungen der Formel XII gemäss Anspruch 27, dadurch gekennzeichnet, dass man ein in der 6-Stellung substituiertes Pyridin-2,3-dicarbonsäureanhydrid der Formel VIII

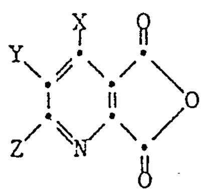

(VII)

worin X, Y und Z die im Anspruch 1 gegebene Bedeutung haben, in einem inerten organischen Lösungsmittel mit einem Glycylnitril der Formel XIX

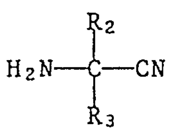

(XIX)

worin $R_2$ und $R_3$ die im Anspruch 1 gegebene Bedeutung haben, umsetzt, die dabei erhaltene in der 6-Stellung substituierte 2-(Cyanomethylcarbamoyl)-nicotinsäure der Formel IX

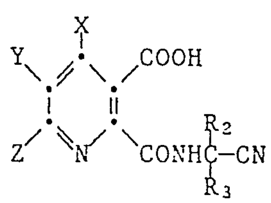

(IX)

worin $R_2$, $R_3$, X, Y und Z die im Anspruch 1 gegebene Bedeutung haben, dann in wasserentziehendem Milieu oder einem wasserentziehenden Mittel behandelt, so dass ein N-(Cyanomethylcarbamoyl)-pyridin-2,3-dicarbonsäureimid der Formel X entsteht,

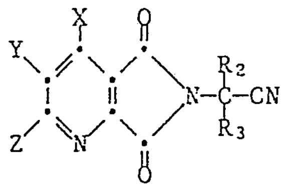

(X)

worin $R_2$, $R_3$, X, Y und Z die im Anspruch 1 gegebene Bedeutung haben, diese Imid in wässrig-saurem Medium, bei erhöhter Temperatur zum N-(Carbamoylmethyl)-pyridin-2,3-dicarbosäureimid der Formel XI

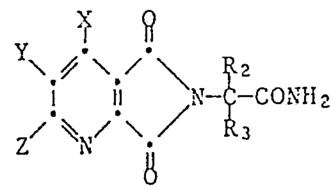

(XI)

worin $R_2$, $R_3$, X, Y und Z die im Anspruch 1 gegebene Bedeutung haben, welche Verbindung dann durch Erwärmen in basischem Milieu zur tricyclischen 3H-Imidazo[1,2:1′,2′]pyrrolo[3,4-b]pyridin-2,5-dion Verbindung der Formel XII umgewandelt wird.

30. Ein herbizides und das Pflanzenwachstum regulierendes Mittel, dadurch gekennzeichnet, dass es neben inerten Trägermaterialien und/oder anderen Zuschlagsstoffen als Wirkstoff eine herbizid oder den Pflanzenwuchs regulierend wirksame Menge einer 3H-Imidazo[1′,2′:1,2]pyrrolo[3,4-b]pyridin-2,5-dion-Verbindung gemäss Anspruch 27 enthält.

31. Die Verwendung der 3H-Imidazo[1′,2′:1,2]pyrrolo[3,4-b]pyridin-2,5-dion-Verbindung gemäss Anspruch 27 oder eines ein solches Derivat enthaltenden Mittels zur Regulierung des Pflanzenwuchses oder zur Bekämpfung unerwünschten Pflanzenwachstums.

32. Verfahren zur selektiven pre- oder postemergenten Bekämpfung von Unkräutern in Nutzpflanzenkulturen, dadurch gekennzeichnet, dass man die Nutzpflanzenkulturen oder deren Anbaufläche mit einer wirksamen Menge einer 3H-Imidazo[1′,2′:1,2]pyrrolo[3,4-b]pyridin-2,5-dion-Verbindung der Formel XII, Anspruch 27 oder eines eine solche Verbindung enthaltendes Mittel behandelt.

33. Verfahren zur Unterdrückung des Pflanzenwachstums über das 2-Blattstadium hinaus, dadurch gekennzeichnet, dass man die Pflanzen während ihres Wachstums mit einer wirksamen Menge einer 3H-Imidazo[1′,2′:1,2]pyrrolo[3,4-b]pyridin-2,5-dion-Verbindung der Formel XII Anspruch 27 oder eines einen solchen Wirkstoff enthaltenden Mittels behandelt.

34. Verfahren zur Herstellung neuer in der 6-Stellung substituierten Pyridin-2,3-dicarbonsäurederivate der Formel IV gemäss Anspruch 3, dadurch gekennzeichnet, dass man einen in der 6-Stellung unsubstituierten Pyridin-2,3-discarbonsäure-diester der Formel V

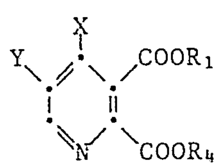

(V)

worin $R_1$, X und Y die im Anspruch 3 gegebene Bedeutung haben, und $R_4$ einen $C_1$-$C_8$-Alkylphenyl- oder Phenyl-$C_1$-$C_4$-alkylrest bedeutet, in wässriger Lösung, in Gegenwart einer katalytischen Menge von Silber II-ionen und einem Peroxysulfatsalz, mit einer Carbonsäure der Formel XVII

Z—COOH (XVII)

worin Z die im Anspruch 3 gegebene Bedeutung hat, umsetzt zum in der 6-Stellung substituierten Pyridin-2,3-dicarbonsäure-diester der Formel IV umsetzt.

35. Neue in der 6-Stellung substituierte Pyridin-2,3-dicarbonsäuren der Formel VI als Zwischenprodukte

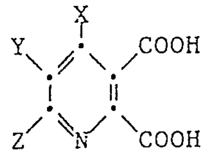

(VI)

worin X, Y und Z die im Anspruch 1 gegebene Bedeutung haben.

36. Die Herstellung der in der 6-Stellung substituierten Pyridin-2,3-dicarbonsäuren der Formel VI, Anspruch 1, dadurch gekennzeichnet, dass man einen gemäss Anspruch 13 oder 6 erhaltenen in der 6-Stellung substituierten Pyridin-2,3-dicarbonsäurediester der Formel IV gemäss Anspruch 22 mit einer starken Base in wässrigem Milieu verseift und die Säure nach Ansäuern des Reaktionsmittels isoliert.

37. Neue in der 6-Stellung substituierte Pyridin-2,3-dicarbonsäureanhydride der Formel VII als Zwischenprodukte

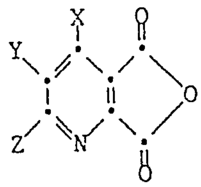

(VII)

worin X, Y und Z die im Anspruch 1 gegebene Bedeutung haben.

38. Verfahren zur Herstellung der in der 6-Stellung substituierten Pyridin-2,3-dicarbonsäureanhydride der Formel VII, Anspruch 37, dadurch gekennzeichnet, dass man eine Pyridin-2,3-dicarbonsäure der Formel VI, Anspruch 35 in einem waserentziehenden Medium zum Anhydrid umsetzt.

39. Neue in der 6-Stellung substituierte 2-(N-Carbamoylmethylcarbamoyl)-nicotinsäuren der Formel VIII als Zwischenprodukte

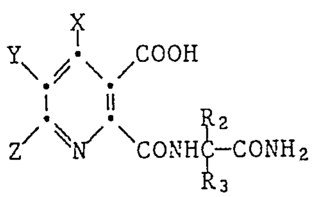

(VIII)

worin $R_2$, $R_3$, X, Y und Z die im Anspruch 2 gegebene Bedeutung haben.

40. Verfahren zur Herstellung der in der 6-Stellung substituierten 2-(N-Carbamoylmethylcarbamoyl)-nicotinsäuren der Formel VIII, Anspruch 39, dadurch gekennzeichnet, dass man ein in der 6-Stellung substituiertes Pyridin-2,3-dicarbonsäureanhydrid der Formel VII

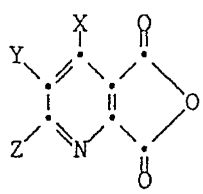

(VII)

worin X, Y und Z die im Anspruch 1 gegebene Bedeutung haben, mit einem Glycylamid der Formel XVIII

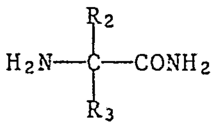

(XVIII)

worin $R_2$ und $R_3$ die in Anspruch 1 gegebene Bedeutung haben, kondensiert und die entstandene Säure aus dem Reaktionsmilieu isoliert.

41. Neue in der 6-Stellung substituierte N-(Cyanomethylcarbamoyl)-nicotinsäuren der Formel IX als Zwischenprodukte

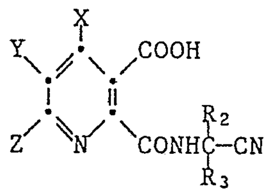

(IX)

worin $R_2$, $R_3$, X, Y und Z die im Anspruch 1 gegebene Bedeutung haben.

42. Verfahren zur Herstellung der in der 6-Stellung substituierten 2-(Cyanomethyl)-nicotinsäuren der Formel IX, Anspruch 41, dadurch gekennzeichnet, dass man ein in der 6-Stellung substituiertes Pyridin-2,3-dicarbonsäureanhydrid der Formel VII gemäss Anspruch 37

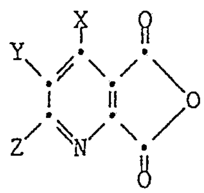

(VII)

worin X, Y und Z die im Anspruch 1 gegebene Bedeutungen haben, mit einem inerten organischen Lösungsmittel mit einem Glycylnitril der Formel XIX

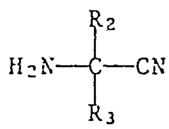

(XIX)

worin $R_2$ und $R_3$ die im Anspruch 1 gegebene Bedeutung haben, umsetzt und die erhaltene Säure aus dem Reaktionsmilieu isoliert.

43. Neue in der 6-Stellung substituierte N-(Cyanomethylcarbamoyl)-pyridin-dicarbonsäureimide der Formel X als Zwischenprodukte

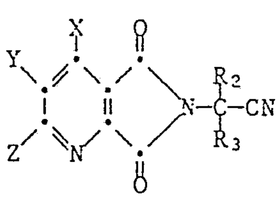

(X)

worin $R_2$, $R_3$, X, Y und Z die im Anspruch 1 gegebene Bedeutung haben.

44. Verfahren zur Herstellung von in der 6-Stellung substituierten N-(Cyanomethylcarbamoyl)-pyridin-2,3-dicarbonsäureimiden der Formel X, Anspruch 29, dadurch gekennzeichnet, dass man eine in der

6-Stellung substituierte N-(Cyanomethylcarbamoyl)-nicotinsäure der Formel IX gemäss Anspruch 41

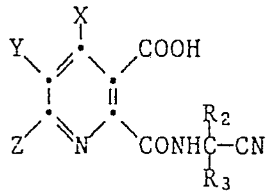

(IX)

worin $R_2$, $R_3$, X, Y und Z die im Anspruch 1 gegebene Bedeutung haben, in wasserentziehendem Milieu oder mit einem wasserentziehenden Mittel behandelt.

45. Neue in der 6-Stellung substituierte N-(Carbamoylmethylcarbamoyl)-pyridin-2,3-dicarbonsäureimide der Formel XI als Zwischenprodukte

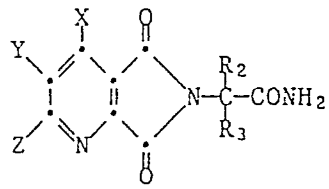

(XI)

worin $R_2$, $R_3$, X, Y und Z die im Anspruch 1 gegebene Bedeutung haben.

46. Verfahren zur Herstellung der in der 6-Stellung substituierten N-(Carbamoylmethylcarbamoyl)-pyridin-2,3-dicarbonsäureimide der Formel XI gemäss Anspruch 45, dadurch gekennzeichnet, dass man ein in der 6-Stellung substituiertes N-(Cyanomethylcarbamoyl)-pyridin-2,3-dicarbonsäureimid der Formel X gemäss Anspruch 43 in wässrigsaurem Medium bei erhöhten Temperaturen verseift.